(19) **Europäisches Patentamt / European Patent Office / Office européen des brevets**

(11) **EP 3 151 005 A1**

(12) **EUROPEAN PATENT APPLICATION**

(43) Date of publication:
05.04.2017 Bulletin 2017/14

(51) Int Cl.:
*G01N 33/53* (2006.01)    *G01N 33/68* (2006.01)

(21) Application number: 16189562.8

(22) Date of filing: 27.08.2010

(84) Designated Contracting States:
AL AT BE BG CH CY CZ DE DK EE ES FI FR GB
GR HR HU IE IS IT LI LT LU LV MC MK MT NL NO
PL PT RO SE SI SK SM TR

(30) Priority: 28.08.2009 US 238134 P
28.08.2009 US 238120 P
28.08.2009 US 238129 P
28.08.2009 US 238118 P
28.08.2009 US 238123 P
28.08.2009 US 238127 P
28.08.2009 US 238121 P
28.08.2009 US 238128 P
28.08.2009 US 238125 P
28.08.2009 US 238115 P
18.09.2009 US 244002 P
18.09.2009 US 243993 P
18.09.2009 US 243991 P
18.09.2009 US 243997 P

(62) Document number(s) of the earlier application(s) in
accordance with Art. 76 EPC:
15150059.2 / 2 894 473
10812639.2 / 2 470 905

(71) Applicant: **Astute Medical, Inc.**
San Diego, CA 92121 (US)

(72) Inventors:
• **ANDERBERG, Joseph**
Encinitas, CA 92024 (US)
• **GRAY, Jeff**
Solana Beach, CA 92075 (US)
• **MCPHERSON, Paul**
Encinitas, CA 92024 (US)
• **NAKAMURA, Kevin**
Cardiff by the Sea, CA 92007 (US)
• **KAMPF, James Patrick**
San Diego, CA 92130 (US)

(74) Representative: **Schiweck, Weinzierl & Koch**
**Patentanwälte Partnerschaft mbB**
**Landsberger Straße 98**
**80339 München (DE)**

Remarks:
This application was filed on 20-09-2016 as a
divisional application to the application mentioned
under INID code 62.

(54) **METHODS FOR DIAGNOSIS OF RENAL INJURY AND RENAL FAILURE**

(57) The present invention relates to methods and compositions for monitoring, diagnosis, prognosis, and determination of treatment regimens in subjects suffering from or suspected of having a renal injury. In particular, the invention relates to using assays that detect Carcinoembryonic antigen-related cell adhesion molecule 5, and optionally further one or more biomarkers of Cellular tumor antigen p53, Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, and as diagnostic and prognostic biomarker assays in renal injuries.

SEQUENCE LISTING

<110> ASTUTE MEDICAL, INC.

<120> METHODS AND COMPOSITIONS FOR DIAGNOSIS AND PROGNOSIS OF RENAL INJURY AND RENAL FAILURE

<130> BLG14431PCTEPD2

<140> PCT/US2010/046910
<141> 2010-08-27

<150> 61/243,991
<151> 2009-09-18

<150> 61/243,997
<151> 2009-09-18

<150> 61/244,002
<151> 2009-09-18

<150> 61/243,993
<151> 2009-09-18

<150> 61/238,115
<151> 2009-08-28

<150> 61/238,118
<151> 2009-08-28

<150> 61/238,120
<151> 2009-08-28

<150> 61/238,121
<151> 2009-08-28

<150> 61/238,123
<151> 2009-08-28

<150> 61/238,125
<151> 2009-08-28

<150> 61/238,127
<151> 2009-08-28

<150> 61/238,129
<151> 2009-08-28

<150> 61/238,134
<151> 2009-08-28

<150> 61/238,128
<151> 2009-08-28

<160> 16

<170> PatentIn version 3.5

<210> 1
<211> 440
<212> PRT
<213> Homo sapiens

<400> 1

Met Glu Gln Arg Gly Gln Asn Ala Pro Ala Ala Ser Gly Ala Arg Lys
1               5               10              15

Arg His Gly Pro Gly Pro Arg Glu Ala Arg Gly Ala Arg Pro Gly Pro
            20              25              30

Arg Val Pro Lys Thr Leu Val Leu Val Val Ala Ala Val Leu Leu Leu
        35              40              45

Val Ser Ala Glu Ser Ala Leu Ile Thr Gln Gln Asp Leu Ala Pro Gln
    50              55              60

Gln Arg Ala Ala Pro Gln Gln Lys Arg Ser Ser Pro Ser Glu Gly Leu
65              70              75              80

Cys Pro Pro Gly His His Ile Ser Glu Asp Gly Arg Asp Cys Ile Ser
            85              90              95

Cys Lys Tyr Gly Gln Asp Tyr Ser Thr His Trp Asn Asp Leu Leu Phe
            100             105             110

Cys Leu Arg Cys Thr Arg Cys Asp Ser Gly Glu Val Glu Leu Ser Pro
        115             120             125

Cys Thr Thr Thr Arg Asn Thr Val Cys Gln Cys Glu Glu Gly Thr Phe
    130             135             140

Arg Glu Glu Asp Ser Pro Glu Met Cys Arg Lys Cys Arg Thr Gly Cys
145             150             155             160

Pro Arg Gly Met Val Lys Val Gly Asp Cys Thr Pro Trp Ser Asp Ile
            165             170             175

Glu Cys Val His Lys Glu Ser Gly Thr Lys His Ser Gly Glu Ala Pro
            180             185             190

Ala Val Glu Glu Thr Val Thr Ser Ser Pro Gly Thr Pro Ala Ser Pro
    195             200             205

Cys Ser Leu Ser Gly Ile Ile Ile Gly Val Thr Val Ala Ala Val Val
    210             215             220

Leu Ile Val Ala Val Phe Val Cys Lys Ser Leu Leu Trp Lys Lys Val
225             230             235             240

Leu Pro Tyr Leu Lys Gly Ile Cys Ser Gly Gly Gly Gly Asp Pro Glu
            245             250             255

```
Arg Val Asp Arg Ser Ser Gln Arg Pro Gly Ala Glu Asp Asn Val Leu
            260             265             270

Asn Glu Ile Val Ser Ile Leu Gln Pro Thr Gln Val Pro Glu Gln Glu
            275             280             285

Met Glu Val Gln Glu Pro Ala Glu Pro Thr Gly Val Asn Met Leu Ser
            290             295             300

Pro Gly Glu Ser Glu His Leu Leu Glu Pro Ala Glu Ala Glu Arg Ser
305             310             315             320

Gln Arg Arg Arg Leu Leu Val Pro Ala Asn Glu Gly Asp Pro Thr Glu
                325             330             335

Thr Leu Arg Gln Cys Phe Asp Asp Phe Ala Asp Leu Val Pro Phe Asp
            340             345             350

Ser Trp Glu Pro Leu Met Arg Lys Leu Gly Leu Met Asp Asn Glu Ile
            355             360             365

Lys Val Ala Lys Ala Glu Ala Ala Gly His Arg Asp Thr Leu Tyr Thr
    370             375             380

Met Leu Ile Lys Trp Val Asn Lys Thr Gly Arg Asp Ala Ser Val His
385             390             395             400

Thr Leu Leu Asp Ala Leu Glu Thr Leu Gly Glu Arg Leu Ala Lys Gln
                405             410             415

Lys Ile Glu Asp His Leu Leu Ser Ser Gly Lys Phe Met Tyr Leu Glu
            420             425             430

Gly Asn Ala Asp Ser Ala Met Ser
            435             440


<210> 2
<211> 829
<212> PRT
<213> Homo sapiens

<400> 2
Met Val Pro Ala Trp Leu Trp Leu Leu Cys Val Ser Val Pro Gln Ala
1               5               10              15

Leu Pro Lys Ala Gln Pro Ala Glu Leu Ser Val Glu Val Pro Glu Asn
            20              25              30
```

Tyr Gly Gly Asn Phe Pro Leu Tyr Leu Thr Lys Leu Pro Leu Pro Arg
        35              40              45

Glu Gly Ala Glu Gly Gln Ile Val Leu Ser Gly Asp Ser Gly Lys Ala
        50              55              60

Thr Glu Gly Pro Phe Ala Met Asp Pro Asp Ser Gly Phe Leu Leu Val
65              70              75              80

Thr Arg Ala Leu Asp Arg Glu Glu Gln Ala Glu Tyr Gln Leu Gln Val
                85              90              95

Thr Leu Glu Met Gln Asp Gly His Val Leu Trp Gly Pro Gln Pro Val
            100             105             110

Leu Val His Val Lys Asp Glu Asn Asp Gln Val Pro His Phe Ser Gln
        115             120             125

Ala Ile Tyr Arg Ala Arg Leu Ser Arg Gly Thr Arg Pro Gly Ile Pro
    130             135             140

Phe Leu Phe Leu Glu Ala Ser Asp Arg Asp Glu Pro Gly Thr Ala Asn
145             150             155             160

Ser Asp Leu Arg Phe His Ile Leu Ser Gln Ala Pro Ala Gln Pro Ser
                165             170             175

Pro Asp Met Phe Gln Leu Glu Pro Arg Leu Gly Ala Leu Ala Leu Ser
            180             185             190

Pro Lys Gly Ser Thr Ser Leu Asp His Ala Leu Glu Arg Thr Tyr Gln
        195             200             205

Leu Leu Val Gln Val Lys Asp Met Gly Asp Gln Ala Ser Gly His Gln
    210             215             220

Ala Thr Ala Thr Val Glu Val Ser Ile Ile Glu Ser Thr Trp Val Ser
225             230             235             240

Leu Glu Pro Ile His Leu Ala Glu Asn Leu Lys Val Leu Tyr Pro His
            245             250             255

His Met Ala Gln Val His Trp Ser Gly Gly Asp Val His Tyr His Leu
        260             265             270

Glu Ser His Pro Pro Gly Pro Phe Glu Val Asn Ala Glu Gly Asn Leu
    275             280             285

162

Tyr Val Thr Arg Glu Leu Asp Arg Glu Ala Gln Ala Glu Tyr Leu Leu
290                 295                 300

Gln Val Arg Ala Gln Asn Ser His Gly Glu Asp Tyr Ala Ala Pro Leu
305                 310                 315                 320

Glu Leu His Val Leu Val Met Asp Glu Asn Asp Asn Val Pro Ile Cys
325                 330                 335

Pro Pro Arg Asp Pro Thr Val Ser Ile Pro Glu Leu Ser Pro Pro Gly
340                 345                 350

Thr Glu Val Thr Arg Leu Ser Ala Glu Asp Ala Asp Ala Pro Gly Ser
355                 360                 365

Pro Asn Ser His Val Val Tyr Gln Leu Leu Ser Pro Glu Pro Glu Asp
370                 375                 380

Gly Val Glu Gly Arg Ala Phe Gln Val Asp Pro Thr Ser Gly Ser Val
385                 390                 395                 400

Thr Leu Gly Val Leu Pro Leu Arg Ala Gly Gln Asn Ile Leu Leu Leu
405                 410                 415

Val Leu Ala Met Asp Leu Ala Gly Ala Glu Gly Gly Phe Ser Ser Thr
420                 425                 430

Cys Glu Val Glu Val Ala Val Thr Asp Ile Asn Asp His Ala Pro Glu
435                 440                 445

Phe Ile Thr Ser Gln Ile Gly Pro Ile Ser Leu Pro Glu Asp Val Glu
450                 455                 460

Pro Gly Thr Leu Val Ala Met Leu Thr Ala Ile Asp Ala Asp Leu Glu
465                 470                 475                 480

Pro Ala Phe Arg Leu Met Asp Phe Ala Ile Glu Arg Gly Asp Thr Glu
485                 490                 495

Gly Thr Phe Gly Leu Asp Trp Glu Pro Asp Ser Gly His Val Arg Leu
500                 505                 510

Arg Leu Cys Lys Asn Leu Ser Tyr Glu Ala Ala Pro Ser His Glu Val
515                 520                 525

Val Val Val Val Gln Ser Val Ala Lys Leu Val Gly Pro Gly Pro Gly

```
              530                    535                      540

      Pro Gly Ala Thr Ala Thr Val Thr Val Leu Val Glu Arg Val Met Pro
      545                 550                 555                 560

      Pro Pro Lys Leu Asp Gln Glu Ser Tyr Glu Ala Ser Val Pro Ile Ser
                      565                 570                 575

      Ala Pro Ala Gly Ser Phe Leu Leu Thr Ile Gln Pro Ser Asp Pro Ile
                      580                 585                 590

      Ser Arg Thr Leu Arg Phe Ser Leu Val Asn Asp Ser Glu Gly Trp Leu
                  595                 600                 605

      Cys Ile Glu Lys Phe Ser Gly Glu Val His Thr Ala Gln Ser Leu Gln
          610                 615                 620

      Gly Ala Gln Pro Gly Asp Thr Tyr Thr Val Leu Val Glu Ala Gln Asp
      625                 630                 635                 640

      Thr Asp Glu Pro Arg Leu Ser Ala Ser Ala Pro Leu Val Ile His Phe
                      645                 650                 655

      Leu Lys Ala Pro Pro Ala Pro Ala Leu Thr Leu Ala Pro Val Pro Ser
                  660                 665                 670

      Gln Tyr Leu Cys Thr Pro Arg Gln Asp His Gly Leu Ile Val Ser Gly
                  675                 680                 685

      Pro Ser Lys Asp Pro Asp Leu Ala Ser Gly His Gly Pro Tyr Ser Phe
          690                 695                 700

      Thr Leu Gly Pro Asn Pro Thr Val Gln Arg Asp Trp Arg Leu Gln Thr
      705                 710                 715                 720

      Leu Asn Gly Ser His Ala Tyr Leu Thr Leu Ala Leu His Trp Val Glu
                      725                 730                 735

      Pro Arg Glu His Ile Ile Pro Val Val Val Ser His Asn Ala Gln Met
                  740                 745                 750

      Trp Gln Leu Leu Val Arg Val Ile Val Cys Arg Cys Asn Val Glu Gly
                  755                 760                 765

      Gln Cys Met Arg Lys Val Gly Arg Met Lys Gly Met Pro Thr Lys Leu
          770                 775                 780
```

Ser Ala Val Gly Ile Leu Val Gly Thr Leu Val Ala Ile Gly Ile Phe
785             790             795             800

Leu Ile Leu Ile Phe Thr His Trp Thr Met Ser Arg Lys Lys Asp Pro
                805             810             815

Asp Gln Pro Ala Asp Ser Val Pro Leu Lys Ala Thr Val
                820             825

<210> 3
<211> 416
<212> PRT
<213> Homo sapiens

<400> 3
Met Asp Glu Ala Asp Arg Arg Leu Leu Arg Arg Cys Arg Leu Arg Leu
1               5               10              15

Val Glu Glu Leu Gln Val Asp Gln Leu Trp Asp Ala Leu Leu Ser Arg
                20              25              30

Glu Leu Phe Arg Pro His Met Ile Glu Asp Ile Gln Arg Ala Gly Ser
        35              40              45

Gly Ser Arg Arg Asp Gln Ala Arg Gln Leu Ile Ile Asp Leu Glu Thr
    50              55              60

Arg Gly Ser Gln Ala Leu Pro Leu Phe Ile Ser Cys Leu Glu Asp Thr
65              70              75              80

Gly Gln Asp Met Leu Ala Ser Phe Leu Arg Thr Asn Arg Gln Ala Ala
                85              90              95

Lys Leu Ser Lys Pro Thr Leu Glu Asn Leu Thr Pro Val Val Leu Arg
                100             105             110

Pro Glu Ile Arg Lys Pro Glu Val Leu Arg Pro Glu Thr Pro Arg Pro
        115             120             125

Val Asp Ile Gly Ser Gly Gly Phe Gly Asp Val Gly Ala Leu Glu Ser
    130             135             140

Leu Arg Gly Asn Ala Asp Leu Ala Tyr Ile Leu Ser Met Glu Pro Cys
145             150             155             160

Gly His Cys Leu Ile Ile Asn Asn Val Asn Phe Cys Arg Glu Ser Gly
                165             170             175

Leu Arg Thr Arg Thr Gly Ser Asn Ile Asp Cys Glu Lys Leu Arg Arg

165

```
                180                    185                        190

        Arg Phe Ser Ser Leu His Phe Met Val Glu Val Lys Gly Asp Leu Thr
                195                    200                    205

        Ala Lys Lys Met Val Leu Ala Leu Leu Glu Leu Ala Gln Gln Asp His
                210                    215                    220

        Gly Ala Leu Asp Cys Cys Val Val Val Ile Leu Ser His Gly Cys Gln
        225                    230                    235                    240

        Ala Ser His Leu Gln Phe Pro Gly Ala Val Tyr Gly Thr Asp Gly Cys
                        245                    250                    255

        Pro Val Ser Val Glu Lys Ile Val Asn Ile Phe Asn Gly Thr Ser Cys
                260                    265                    270

        Pro Ser Leu Gly Gly Lys Pro Lys Leu Phe Phe Ile Gln Ala Cys Gly
                275                    280                    285

        Gly Glu Gln Lys Asp His Gly Phe Glu Val Ala Ser Thr Ser Pro Glu
                290                    295                    300

        Asp Glu Ser Pro Gly Ser Asn Pro Glu Pro Asp Ala Thr Pro Phe Gln
        305                    310                    315                    320

        Glu Gly Leu Arg Thr Phe Asp Gln Leu Asp Ala Ile Ser Ser Leu Pro
                        325                    330                    335

        Thr Pro Ser Asp Ile Phe Val Ser Tyr Ser Thr Phe Pro Gly Phe Val
                        340                    345                    350

        Ser Trp Arg Asp Pro Lys Ser Gly Ser Trp Tyr Val Glu Thr Leu Asp
                355                    360                    365

        Asp Ile Phe Glu Gln Trp Ala His Ser Glu Asp Leu Gln Ser Leu Leu
                370                    375                    380

        Leu Arg Val Ala Asn Ala Val Ser Val Lys Gly Ile Tyr Lys Gln Met
        385                    390                    395                    400

        Pro Gly Cys Phe Asn Phe Leu Arg Lys Lys Leu Phe Phe Lys Thr Ser
                        405                    410                    415


        <210>  4
        <211>  168
        <212>  PRT
        <213>  Homo sapiens
```

<400> 4

```
Met Phe Gln Ile Pro Glu Phe Glu Pro Ser Gln Glu Asp Ser Ser
1               5               10              15
```

```
Ser Ala Glu Arg Gly Leu Gly Pro Ser Pro Ala Gly Asp Gly Pro Ser
            20              25              30
```

```
Gly Ser Gly Lys His His Arg Gln Ala Pro Gly Leu Leu Trp Asp Ala
            35              40              45
```

```
Ser His Gln Gln Glu Gln Pro Thr Ser Ser Ser His His Gly Gly Ala
        50              55              60
```

```
Gly Ala Val Glu Ile Arg Ser Arg His Ser Ser Tyr Pro Ala Gly Thr
65              70              75              80
```

```
Glu Asp Asp Glu Gly Met Gly Glu Glu Pro Ser Pro Phe Arg Gly Arg
                85              90              95
```

```
Ser Arg Ser Ala Pro Pro Asn Leu Trp Ala Ala Gln Arg Tyr Gly Arg
            100             105             110
```

```
Glu Leu Arg Arg Met Ser Asp Glu Phe Val Asp Ser Phe Lys Lys Gly
            115             120             125
```

```
Leu Pro Arg Pro Lys Ser Ala Gly Thr Ala Thr Gln Met Arg Gln Ser
        130             135             140
```

```
Ser Ser Trp Thr Arg Val Phe Gln Ser Trp Trp Asp Arg Asn Leu Gly
145             150             155             160
```

```
Arg Gly Ser Ser Ala Pro Ser Gln
                165
```

```
<210> 5
<211> 404
<212> PRT
<213> Homo sapiens
```

<400> 5

```
Met Ala Asp Lys Val Leu Lys Glu Lys Arg Lys Leu Phe Ile Arg Ser
1               5               10              15
```

```
Met Gly Glu Gly Thr Ile Asn Gly Leu Leu Asp Glu Leu Leu Gln Thr
            20              25              30
```

```
Arg Val Leu Asn Lys Glu Glu Met Glu Lys Val Lys Arg Glu Asn Ala
            35              40              45
```

```
Thr Val Met Asp Lys Thr Arg Ala Leu Ile Asp Ser Val Ile Pro Lys
    50              55              60

Gly Ala Gln Ala Cys Gln Ile Cys Ile Thr Tyr Ile Cys Glu Glu Asp
65              70              75              80

Ser Tyr Leu Ala Gly Thr Leu Gly Leu Ser Ala Asp Gln Thr Ser Gly
            85              90              95

Asn Tyr Leu Asn Met Gln Asp Ser Gln Gly Val Leu Ser Ser Phe Pro
            100             105             110

Ala Pro Gln Ala Val Gln Asp Asn Pro Ala Met Pro Thr Ser Ser Gly
        115             120             125

Ser Glu Gly Asn Val Lys Leu Cys Ser Leu Glu Glu Ala Gln Arg Ile
    130             135             140

Trp Lys Gln Lys Ser Ala Glu Ile Tyr Pro Ile Met Asp Lys Ser Ser
145             150             155             160

Arg Thr Arg Leu Ala Leu Ile Ile Cys Asn Glu Glu Phe Asp Ser Ile
            165             170             175

Pro Arg Arg Thr Gly Ala Glu Val Asp Ile Thr Gly Met Thr Met Leu
            180             185             190

Leu Gln Asn Leu Gly Tyr Ser Val Asp Val Lys Lys Asn Leu Thr Ala
        195             200             205

Ser Asp Met Thr Thr Glu Leu Glu Ala Phe Ala His Arg Pro Glu His
    210             215             220

Lys Thr Ser Asp Ser Thr Phe Leu Val Phe Met Ser His Gly Ile Arg
225             230             235             240

Glu Gly Ile Cys Gly Lys Lys His Ser Glu Gln Val Pro Asp Ile Leu
            245             250             255

Gln Leu Asn Ala Ile Phe Asn Met Leu Asn Thr Lys Asn Cys Pro Ser
        260             265             270

Leu Lys Asp Lys Pro Lys Val Ile Ile Ile Gln Ala Cys Arg Gly Asp
        275             280             285

Ser Pro Gly Val Val Trp Phe Lys Asp Ser Val Gly Val Ser Gly Asn
    290             295             300
```

168

```
Leu Ser Leu Pro Thr Thr Glu Glu Phe Glu Asp Asp Ala Ile Lys Lys
305             310             315             320

Ala His Ile Glu Lys Asp Phe Ile Ala Phe Cys Ser Ser Thr Pro Asp
                325             330             335

Asn Val Ser Trp Arg His Pro Thr Met Gly Ser Val Phe Ile Gly Arg
                340             345             350

Leu Ile Glu His Met Gln Glu Tyr Ala Cys Ser Cys Asp Val Glu Glu
            355             360             365

Ile Phe Arg Lys Val Arg Phe Ser Phe Glu Gln Pro Asp Gly Arg Ala
        370             375             380

Gln Met Pro Thr Thr Glu Arg Val Thr Leu Thr Arg Cys Phe Tyr Leu
385             390             395             400

Phe Pro Gly His


<210> 6
<211> 882
<212> PRT
<213> Homo sapiens

<400> 6
Met Gly Pro Trp Ser Arg Ser Leu Ser Ala Leu Leu Leu Leu Leu Gln
1               5               10              15

Val Ser Ser Trp Leu Cys Gln Glu Pro Glu Pro Cys His Pro Gly Phe
                20              25              30

Asp Ala Glu Ser Tyr Thr Phe Thr Val Pro Arg Arg His Leu Glu Arg
            35              40              45

Gly Arg Val Leu Gly Arg Val Asn Phe Glu Asp Cys Thr Gly Arg Gln
        50              55              60

Arg Thr Ala Tyr Phe Ser Leu Asp Thr Arg Phe Lys Val Gly Thr Asp
65              70              75              80

Gly Val Ile Thr Val Lys Arg Pro Leu Arg Phe His Asn Pro Gln Ile
                85              90              95

His Phe Leu Val Tyr Ala Trp Asp Ser Thr Tyr Arg Lys Phe Ser Thr
                100             105             110
```

```
Lys Val Thr Leu Asn Thr Val Gly His His His Arg Pro Pro Pro His
        115             120             125

Gln Ala Ser Val Ser Gly Ile Gln Ala Glu Leu Leu Thr Phe Pro Asn
    130             135             140

Ser Ser Pro Gly Leu Arg Arg Gln Lys Arg Asp Trp Val Ile Pro Pro
145             150             155             160

Ile Ser Cys Pro Glu Asn Glu Lys Gly Pro Phe Pro Lys Asn Leu Val
            165             170             175

Gln Ile Lys Ser Asn Lys Asp Lys Glu Gly Lys Val Phe Tyr Ser Ile
        180             185             190

Thr Gly Gln Gly Ala Asp Thr Pro Pro Val Gly Val Phe Ile Ile Glu
        195             200             205

Arg Glu Thr Gly Trp Leu Lys Val Thr Glu Pro Leu Asp Arg Glu Arg
    210             215             220

Ile Ala Thr Tyr Thr Leu Phe Ser His Ala Val Ser Ser Asn Gly Asn
225             230             235             240

Ala Val Glu Asp Pro Met Glu Ile Leu Ile Thr Val Thr Asp Gln Asn
            245             250             255

Asp Asn Lys Pro Glu Phe Thr Gln Glu Val Phe Lys Gly Ser Val Met
            260             265             270

Glu Gly Ala Leu Pro Gly Thr Ser Val Met Glu Val Thr Ala Thr Asp
        275             280             285

Ala Asp Asp Asp Val Asn Thr Tyr Asn Ala Ala Ile Ala Tyr Thr Ile
    290             295             300

Leu Ser Gln Asp Pro Glu Leu Pro Asp Lys Asn Met Phe Thr Ile Asn
305             310             315             320

Arg Asn Thr Gly Val Ile Ser Val Val Thr Thr Gly Leu Asp Arg Glu
            325             330             335

Ser Phe Pro Thr Tyr Thr Leu Val Val Gln Ala Ala Asp Leu Gln Gly
            340             345             350

Glu Gly Leu Ser Thr Thr Ala Thr Ala Val Ile Thr Val Thr Asp Thr
        355             360             365
```

```
Asn Asp Asn Pro Pro Ile Phe Asn Pro Thr Thr Tyr Lys Gly Gln Val
    370             375             380

Pro Glu Asn Glu Ala Asn Val Val Ile Thr Thr Leu Lys Val Thr Asp
385             390             395             400

Ala Asp Ala Pro Asn Thr Pro Ala Trp Glu Ala Val Tyr Thr Ile Leu
            405             410             415

Asn Asp Asp Gly Gly Gln Phe Val Val Thr Thr Asn Pro Val Asn Asn
            420             425             430

Asp Gly Ile Leu Lys Thr Ala Lys Gly Leu Asp Phe Glu Ala Lys Gln
    435             440             445

Gln Tyr Ile Leu His Val Ala Val Thr Asn Val Val Pro Phe Glu Val
    450             455             460

Ser Leu Thr Thr Ser Thr Ala Thr Val Thr Val Asp Val Leu Asp Val
465             470             475             480

Asn Glu Ala Pro Ile Phe Val Pro Pro Glu Lys Arg Val Glu Val Ser
            485             490             495

Glu Asp Phe Gly Val Gly Gln Glu Ile Thr Ser Tyr Thr Ala Gln Glu
    500             505             510

Pro Asp Thr Phe Met Glu Gln Lys Ile Thr Tyr Arg Ile Trp Arg Asp
    515             520             525

Thr Ala Asn Trp Leu Glu Ile Asn Pro Asp Thr Gly Ala Ile Ser Thr
    530             535             540

Arg Ala Glu Leu Asp Arg Glu Asp Phe Glu His Val Lys Asn Ser Thr
545             550             555             560

Tyr Thr Ala Leu Ile Ile Ala Thr Asp Asn Gly Ser Pro Val Ala Thr
            565             570             575

Gly Thr Gly Thr Leu Leu Leu Ile Leu Ser Asp Val Asn Asp Asn Ala
            580             585             590

Pro Ile Pro Glu Pro Arg Thr Ile Phe Phe Cys Glu Arg Asn Pro Lys
    595             600             605

Pro Gln Val Ile Asn Ile Ile Asp Ala Asp Leu Pro Pro Asn Thr Ser
```

171

                610                        615                        620

Pro Phe Thr Ala Glu Leu Thr His Gly Ala Ser Ala Asn Trp Thr Ile
625             630             635             640

Gln Tyr Asn Asp Pro Thr Gln Glu Ser Ile Ile Leu Lys Pro Lys Met
            645             650             655

Ala Leu Glu Val Gly Asp Tyr Lys Ile Asn Leu Lys Leu Met Asp Asn
            660             665             670

Gln Asn Lys Asp Gln Val Thr Thr Leu Glu Val Ser Val Cys Asp Cys
            675             680             685

Glu Gly Ala Ala Gly Val Cys Arg Lys Ala Gln Pro Val Glu Ala Gly
            690             695             700

Leu Gln Ile Pro Ala Ile Leu Gly Ile Leu Gly Gly Ile Leu Ala Leu
705             710             715             720

Leu Ile Leu Ile Leu Leu Leu Leu Leu Phe Leu Arg Arg Arg Ala Val
            725             730             735

Val Lys Glu Pro Leu Leu Pro Pro Glu Asp Asp Thr Arg Asp Asn Val
            740             745             750

Tyr Tyr Tyr Asp Glu Glu Gly Gly Gly Glu Glu Asp Gln Asp Phe Asp
            755             760             765

Leu Ser Gln Leu His Arg Gly Leu Asp Ala Arg Pro Glu Val Thr Arg
            770             775             780

Asn Asp Val Ala Pro Thr Leu Met Ser Val Pro Arg Tyr Leu Pro Arg
785             790             795             800

Pro Ala Asn Pro Asp Glu Ile Gly Asn Phe Ile Asp Glu Asn Leu Lys
            805             810             815

Ala Ala Asp Thr Asp Pro Thr Ala Pro Pro Tyr Asp Ser Leu Leu Val
            820             825             830

Phe Asp Tyr Glu Gly Ser Gly Ser Glu Ala Ala Ser Leu Ser Ser Leu
            835             840             845

Asn Ser Ser Glu Ser Asp Lys Asp Gln Asp Tyr Asp Tyr Leu Asn Glu
            850             855             860

Trp Gly Asn Arg Phe Lys Lys Leu Ala Asp Met Tyr Gly Gly Gly Glu
865                 870                 875                 880

Asp Asp


<210> 7
<211> 1014
<212> PRT
<213> Homo sapiens

<400> 7
Met Ala Glu Ser Ser Asp Lys Leu Tyr Arg Val Glu Tyr Ala Lys Ser
1               5                   10                  15

Gly Arg Ala Ser Cys Lys Lys Cys Ser Glu Ser Ile Pro Lys Asp Ser
            20                  25                  30

Leu Arg Met Ala Ile Met Val Gln Ser Pro Met Phe Asp Gly Lys Val
        35                  40                  45

Pro His Trp Tyr His Phe Ser Cys Phe Trp Lys Val Gly His Ser Ile
        50                  55                  60

Arg His Pro Asp Val Glu Val Asp Gly Phe Ser Glu Leu Arg Trp Asp
65                  70                  75                  80

Asp Gln Gln Lys Val Lys Lys Thr Ala Glu Ala Gly Gly Val Thr Gly
                85                  90                  95

Lys Gly Gln Asp Gly Ile Gly Ser Lys Ala Glu Lys Thr Leu Gly Asp
            100                 105                 110

Phe Ala Ala Glu Tyr Ala Lys Ser Asn Arg Ser Thr Cys Lys Gly Cys
        115                 120                 125

Met Glu Lys Ile Glu Lys Gly Gln Val Arg Leu Ser Lys Lys Met Val
    130                 135                 140

Asp Pro Glu Lys Pro Gln Leu Gly Met Ile Asp Arg Trp Tyr His Pro
145                 150                 155                 160

Gly Cys Phe Val Lys Asn Arg Glu Glu Leu Gly Phe Arg Pro Glu Tyr
            165                 170                 175

Ser Ala Ser Gln Leu Lys Gly Phe Ser Leu Leu Ala Thr Glu Asp Lys
            180                 185                 190

Glu Ala Leu Lys Lys Gln Leu Pro Gly Val Lys Ser Glu Gly Lys Arg

```
                195                        200                        205

        Lys Gly Asp Glu Val Asp Gly Val Asp Glu Val Ala Lys Lys Lys Ser
            210             215             220

        Lys Lys Glu Lys Asp Lys Asp Ser Lys Leu Glu Lys Ala Leu Lys Ala
        225             230             235             240

        Gln Asn Asp Leu Ile Trp Asn Ile Lys Asp Glu Leu Lys Lys Val Cys
                    245             250             255

        Ser Thr Asn Asp Leu Lys Glu Leu Leu Ile Phe Asn Lys Gln Gln Val
                    260             265             270

        Pro Ser Gly Glu Ser Ala Ile Leu Asp Arg Val Ala Asp Gly Met Val
                    275             280             285

        Phe Gly Ala Leu Leu Pro Cys Glu Glu Cys Ser Gly Gln Leu Val Phe
            290             295             300

        Lys Ser Asp Ala Tyr Tyr Cys Thr Gly Asp Val Thr Ala Trp Thr Lys
        305             310             315             320

        Cys Met Val Lys Thr Gln Thr Pro Asn Arg Lys Glu Trp Val Thr Pro
                    325             330             335

        Lys Glu Phe Arg Glu Ile Ser Tyr Leu Lys Lys Leu Lys Val Lys Lys
                    340             345             350

        Gln Asp Arg Ile Phe Pro Pro Glu Thr Ser Ala Ser Val Ala Ala Thr
                    355             360             365

        Pro Pro Pro Ser Thr Ala Ser Ala Pro Ala Ala Val Asn Ser Ser Ala
            370             375             380

        Ser Ala Asp Lys Pro Leu Ser Asn Met Lys Ile Leu Thr Leu Gly Lys
        385             390             395             400

        Leu Ser Arg Asn Lys Asp Glu Val Lys Ala Met Ile Glu Lys Leu Gly
                    405             410             415

        Gly Lys Leu Thr Gly Thr Ala Asn Lys Ala Ser Leu Cys Ile Ser Thr
            420             425             430

        Lys Lys Glu Val Glu Lys Met Asn Lys Lys Met Glu Glu Val Lys Glu
            435             440             445
```

```
Ala Asn Ile Arg Val Val Ser Glu Asp Phe Leu Gln Asp Val Ser Ala
    450             455             460

Ser Thr Lys Ser Leu Gln Glu Leu Phe Leu Ala His Ile Leu Ser Pro
465             470             475             480

Trp Gly Ala Glu Val Lys Ala Glu Pro Val Glu Val Val Ala Pro Arg
            485             490             495

Gly Lys Ser Gly Ala Ala Leu Ser Lys Lys Ser Lys Gly Gln Val Lys
            500             505             510

Glu Glu Gly Ile Asn Lys Ser Glu Lys Arg Met Lys Leu Thr Leu Lys
        515             520             525

Gly Gly Ala Ala Val Asp Pro Asp Ser Gly Leu Glu His Ser Ala His
    530             535             540

Val Leu Glu Lys Gly Gly Lys Val Phe Ser Ala Thr Leu Gly Leu Val
545             550             555             560

Asp Ile Val Lys Gly Thr Asn Ser Tyr Tyr Lys Leu Gln Leu Leu Glu
            565             570             575

Asp Asp Lys Glu Asn Arg Tyr Trp Ile Phe Arg Ser Trp Gly Arg Val
        580             585             590

Gly Thr Val Ile Gly Ser Asn Lys Leu Glu Gln Met Pro Ser Lys Glu
        595             600             605

Asp Ala Ile Glu His Phe Met Lys Leu Tyr Glu Glu Lys Thr Gly Asn
    610             615             620

Ala Trp His Ser Lys Asn Phe Thr Lys Tyr Pro Lys Lys Phe Tyr Pro
625             630             635             640

Leu Glu Ile Asp Tyr Gly Gln Asp Glu Glu Ala Val Lys Lys Leu Thr
            645             650             655

Val Asn Pro Gly Thr Lys Ser Lys Leu Pro Lys Pro Val Gln Asp Leu
            660             665             670

Ile Lys Met Ile Phe Asp Val Glu Ser Met Lys Lys Ala Met Val Glu
            675             680             685

Tyr Glu Ile Asp Leu Gln Lys Met Pro Leu Gly Lys Leu Ser Lys Arg
    690             695             700
```

175

```
Gln Ile Gln Ala Ala Tyr Ser Ile Leu Ser Glu Val Gln Gln Ala Val
705                 710                 715                 720

Ser Gln Gly Ser Ser Asp Ser Gln Ile Leu Asp Leu Ser Asn Arg Phe
                725                 730                 735

Tyr Thr Leu Ile Pro His Asp Phe Gly Met Lys Lys Pro Pro Leu Leu
                740                 745                 750

Asn Asn Ala Asp Ser Val Gln Ala Lys Val Glu Met Leu Asp Asn Leu
                755                 760                 765

Leu Asp Ile Glu Val Ala Tyr Ser Leu Leu Arg Gly Gly Ser Asp Asp
                770                 775                 780

Ser Ser Lys Asp Pro Ile Asp Val Asn Tyr Glu Lys Leu Lys Thr Asp
785                 790                 795                 800

Ile Lys Val Val Asp Arg Asp Ser Glu Glu Ala Glu Ile Ile Arg Lys
                805                 810                 815

Tyr Val Lys Asn Thr His Ala Thr Thr His Asn Ala Tyr Asp Leu Glu
                820                 825                 830

Val Ile Asp Ile Phe Lys Ile Glu Arg Glu Gly Glu Cys Gln Arg Tyr
                835                 840                 845

Lys Pro Phe Lys Gln Leu His Asn Arg Arg Leu Leu Trp His Gly Ser
    850                 855                 860

Arg Thr Thr Asn Phe Ala Gly Ile Leu Ser Gln Gly Leu Arg Ile Ala
865                 870                 875                 880

Pro Pro Glu Ala Pro Val Thr Gly Tyr Met Phe Gly Lys Gly Ile Tyr
                885                 890                 895

Phe Ala Asp Met Val Ser Lys Ser Ala Asn Tyr Cys His Thr Ser Gln
                900                 905                 910

Gly Asp Pro Ile Gly Leu Ile Leu Leu Gly Glu Val Ala Leu Gly Asn
                915                 920                 925

Met Tyr Glu Leu Lys His Ala Ser His Ile Ser Lys Leu Pro Lys Gly
                930                 935                 940

Lys His Ser Val Lys Gly Leu Gly Lys Thr Thr Pro Asp Pro Ser Ala
945                 950                 955                 960
```

176

```
Asn Ile Ser Leu Asp Gly Val Asp Val Pro Leu Gly Thr Gly Ile Ser
            965                 970                 975

Ser Gly Val Asn Asp Thr Ser Leu Leu Tyr Asn Glu Tyr Ile Val Tyr
            980                 985                 990

Asp Ile Ala Gln Val Asn Leu Lys  Tyr Leu Leu Lys Leu  Lys Phe Asn
            995                 1000                1005

Phe Lys  Thr Ser Leu Trp
    1010
```

<210> 8
<211> 164
<212> PRT
<213> Homo sapiens

<400> 8

```
Met Ser Glu Pro Ala Gly Asp Val Arg Gln Asn Pro Cys Gly Ser Lys
1               5               10                  15

Ala Cys Arg Arg Leu Phe Gly Pro Val Asp Ser Glu Gln Leu Ser Arg
            20                  25                  30

Asp Cys Asp Ala Leu Met Ala Gly Cys Ile Gln Glu Ala Arg Glu Arg
            35                  40                  45

Trp Asn Phe Asp Phe Val Thr Glu Thr Pro Leu Glu Gly Asp Phe Ala
    50                  55                  60

Trp Glu Arg Val Arg Gly Leu Gly Leu Pro Lys Leu Tyr Leu Pro Thr
65                  70                  75                  80

Gly Pro Arg Arg Gly Arg Asp Glu Leu Gly Gly Gly Arg Arg Pro Gly
            85                  90                  95

Thr Ser Pro Ala Leu Leu Gln Gly Thr Ala Glu Glu Asp His Val Asp
            100                 105                 110

Leu Ser Leu Ser Cys Thr Leu Val Pro Arg Ser Gly Glu Gln Ala Glu
            115                 120                 125

Gly Ser Pro Gly Gly Pro Gly Asp Ser Gln Gly Arg Lys Arg Arg Gln
            130                 135                 140

Thr Ser Met Thr Asp Phe Tyr His Ser Lys Arg Arg Leu Ile Phe Ser
145                 150                 155                 160
```

Lys Arg Lys Pro

<210> 9
<211> 784
<212> PRT
<213> Homo sapiens

<400> 9
Met Gln Arg Leu Met Met Leu Leu Ala Thr Ser Gly Ala Cys Leu Gly
1               5                   10                  15

Leu Leu Ala Val Ala Ala Val Ala Ala Ala Gly Ala Asn Pro Ala Gln
            20                  25                  30

Arg Asp Thr His Ser Leu Leu Pro Thr His Arg Arg Gln Lys Arg Asp
            35                  40                  45

Trp Ile Trp Asn Gln Met His Ile Asp Glu Glu Lys Asn Thr Ser Leu
        50                  55                  60

Pro His His Val Gly Lys Ile Lys Ser Ser Val Ser Arg Lys Asn Ala
65                  70                  75                  80

Lys Tyr Leu Leu Lys Gly Glu Tyr Val Gly Lys Val Phe Arg Val Asp
                85                  90                  95

Ala Glu Thr Gly Asp Val Phe Ala Ile Glu Arg Leu Asp Arg Glu Asn
                100                 105                 110

Ile Ser Glu Tyr His Leu Thr Ala Val Ile Val Asp Lys Asp Thr Gly
            115                 120                 125

Glu Asn Leu Glu Thr Pro Ser Ser Phe Thr Ile Lys Val His Asp Val
        130                 135                 140

Asn Asp Asn Trp Pro Val Phe Thr His Arg Leu Phe Asn Ala Ser Val
145                 150                 155                 160

Pro Glu Ser Ser Ala Val Gly Thr Ser Val Ile Ser Val Thr Ala Val
                165                 170                 175

Asp Ala Asp Asp Pro Thr Val Gly Asp His Ala Ser Val Met Tyr Gln
                180                 185                 190

Ile Leu Lys Gly Lys Glu Tyr Phe Ala Ile Asp Asn Ser Gly Arg Ile
            195                 200                 205

```
Ile Thr Ile Thr Lys Ser Leu Asp Arg Glu Lys Gln Ala Arg Tyr Glu
    210                 215                 220

Ile Val Val Glu Ala Arg Asp Ala Gln Gly Leu Arg Gly Asp Ser Gly
    225                 230                 235                 240

Thr Ala Thr Val Leu Val Thr Leu Gln Asp Ile Asn Asp Asn Phe Pro
                245                 250                 255

Phe Phe Thr Gln Thr Lys Tyr Thr Phe Val Val Pro Glu Asp Thr Arg
                260                 265                 270

Val Gly Thr Ser Val Gly Ser Leu Phe Val Glu Asp Pro Asp Glu Pro
                275                 280                 285

Gln Asn Arg Met Thr Lys Tyr Ser Ile Leu Arg Gly Asp Tyr Gln Asp
    290                 295                 300

Ala Phe Thr Ile Glu Thr Asn Pro Ala His Asn Glu Gly Ile Ile Lys
305                 310                 315                 320

Pro Met Lys Pro Leu Asp Tyr Glu Tyr Ile Gln Gln Tyr Ser Phe Ile
                325                 330                 335

Val Glu Ala Thr Asp Pro Thr Ile Asp Leu Arg Tyr Met Ser Pro Pro
                340                 345                 350

Ala Gly Asn Arg Ala Gln Val Ile Ile Asn Ile Thr Asp Val Asp Glu
                355                 360                 365

Pro Pro Ile Phe Gln Gln Pro Phe Tyr His Phe Gln Leu Lys Glu Asn
    370                 375                 380

Gln Lys Lys Pro Leu Ile Gly Thr Val Leu Ala Met Asp Pro Asp Ala
385                 390                 395                 400

Ala Arg His Ser Ile Gly Tyr Ser Ile Arg Arg Thr Ser Asp Lys Gly
                405                 410                 415

Gln Phe Phe Arg Val Thr Lys Lys Gly Asp Ile Tyr Asn Glu Lys Glu
                420                 425                 430

Leu Asp Arg Glu Val Tyr Pro Trp Tyr Asn Leu Thr Val Glu Ala Lys
                435                 440                 445

Glu Leu Asp Ser Thr Gly Thr Pro Thr Gly Lys Glu Ser Ile Val Gln
    450                 455                 460
```

Val His Ile Glu Val Leu Asp Glu Asn Asp Asn Ala Pro Glu Phe Ala
465             470             475             480

Lys Pro Tyr Gln Pro Lys Val Cys Glu Asn Ala Val His Gly Gln Leu
                485             490             495

Val Leu Gln Ile Ser Ala Ile Asp Lys Asp Ile Thr Pro Arg Asn Val
            500             505             510

Lys Phe Lys Phe Thr Leu Asn Thr Glu Asn Asn Phe Thr Leu Thr Asp
        515             520             525

Asn His Asp Asn Thr Ala Asn Ile Thr Val Lys Tyr Gly Gln Phe Asp
    530             535             540

Arg Glu His Thr Lys Val His Phe Leu Pro Val Val Ile Ser Asp Asn
545             550             555             560

Gly Met Pro Ser Arg Thr Gly Thr Ser Thr Leu Thr Val Ala Val Cys
            565             570             575

Lys Cys Asn Glu Gln Gly Glu Phe Thr Phe Cys Glu Asp Met Ala Ala
        580             585             590

Gln Val Gly Val Ser Ile Gln Ala Val Val Ala Ile Leu Leu Cys Ile
        595             600             605

Leu Thr Ile Thr Val Ile Thr Leu Leu Ile Phe Leu Arg Arg Arg Leu
    610             615             620

Arg Lys Gln Ala Arg Ala His Gly Lys Ser Val Pro Glu Ile His Glu
625             630             635             640

Gln Leu Val Thr Tyr Asp Glu Glu Gly Gly Gly Glu Met Asp Thr Thr
            645             650             655

Ser Tyr Asp Val Ser Val Leu Asn Ser Val Arg Arg Gly Gly Ala Lys
        660             665             670

Pro Pro Arg Pro Ala Leu Asp Ala Arg Pro Ser Leu Tyr Ala Gln Val
        675             680             685

Gln Lys Pro Pro Arg His Ala Pro Gly Ala His Gly Gly Pro Gly Glu
    690             695             700

Met Ala Ala Met Ile Glu Val Lys Lys Asp Glu Ala Asp His Asp Gly
705             710             715             720

180

```
Asp Gly Pro Pro Tyr Asp Thr Leu His Ile Tyr Gly Tyr Glu Gly Ser
            725             730             735

Glu Ser Ile Ala Glu Ser Leu Ser Ser Leu Gly Thr Asp Ser Ser Asp
            740             745             750

Ser Asp Val Asp Tyr Asp Phe Leu Asn Asp Trp Gly Pro Arg Phe Lys
            755             760             765

Met Leu Ala Glu Leu Tyr Gly Ser Asp Pro Arg Glu Glu Leu Leu Tyr
            770             775             780
```

<210> 10
<211> 154
<212> PRT
<213> Homo sapiens

<400> 10
```
Met Gly Leu Ser Asp Gly Glu Trp Gln Leu Val Leu Asn Val Trp Gly
1               5               10              15

Lys Val Glu Ala Asp Ile Pro Gly His Gly Gln Glu Val Leu Ile Arg
            20              25              30

Leu Phe Lys Gly His Pro Glu Thr Leu Glu Lys Phe Asp Lys Phe Lys
            35              40              45

His Leu Lys Ser Glu Asp Glu Met Lys Ala Ser Glu Asp Leu Lys Lys
            50              55              60

His Gly Ala Thr Val Leu Thr Ala Leu Gly Gly Ile Leu Lys Lys Lys
65              70              75              80

Gly His His Glu Ala Glu Ile Lys Pro Leu Ala Gln Ser His Ala Thr
            85              90              95

Lys His Lys Ile Pro Val Lys Tyr Leu Glu Phe Ile Ser Glu Cys Ile
            100             105             110

Ile Gln Val Leu Gln Ser Lys His Pro Gly Asp Phe Gly Ala Asp Ala
            115             120             125

Gln Gly Ala Met Asn Lys Ala Leu Glu Leu Phe Arg Lys Asp Met Ala
            130             135             140

Ser Asn Tyr Lys Glu Leu Gly Phe Gln Gly
145                 150
```

<210> 11
<211> 100
<212> PRT
<213> Homo sapiens

<400> 11
Met Lys Leu Leu Ala Ala Thr Val Leu Leu Leu Thr Ile Cys Ser Leu
1               5                   10                  15


Glu Gly Ala Leu Val Arg Arg Gln Ala Lys Glu Pro Cys Val Glu Ser
                20                  25                  30


Leu Val Ser Gln Tyr Phe Gln Thr Val Thr Asp Tyr Gly Lys Asp Leu
            35                  40                  45


Met Glu Lys Val Lys Ser Pro Glu Leu Gln Ala Glu Ala Lys Ser Tyr
    50                  55                  60


Phe Glu Lys Ser Lys Glu Gln Leu Thr Pro Leu Ile Lys Lys Ala Gly
65                  70                  75                  80


Thr Glu Leu Val Asn Phe Leu Ser Tyr Phe Val Glu Leu Gly Thr Gln
                85                  90                  95


Pro Ala Thr Gln
            100


<210> 12
<211> 22152
<212> PRT
<213> Homo sapiens


<220>
<221> MOD_RES
<222> (13877)..(13878)
<223> Any amino acid

<220>
<221> MOD_RES
<222> (13880)..(13880)
<223> Any amino acid

<220>
<221> MOD_RES
<222> (13887)..(13887)
<223> Any amino acid

<220>
<221> MOD_RES
<222> (13890)..(13891)
<223> Any amino acid

<220>
<221> MOD_RES

```
<222> (13893)..(13893)
<223> Any amino acid

<220>
<221> MOD_RES
<222> (13903)..(13903)
<223> Any amino acid

<220>
<221> MOD_RES
<222> (13913)..(13914)
<223> Any amino acid

<220>
<221> MOD_RES
<222> (13916)..(13916)
<223> Any amino acid

<220>
<221> MOD_RES
<222> (13928)..(13929)
<223> Any amino acid

<220>
<221> MOD_RES
<222> (13938)..(13938)
<223> Any amino acid

<220>
<221> MOD_RES
<222> (13940)..(13941)
<223> Any amino acid

<220>
<221> MOD_RES
<222> (14569)..(14571)
<223> Any amino acid

<220>
<221> MOD_RES
<222> (14575)..(14575)
<223> Any amino acid

<220>
<221> MOD_RES
<222> (14579)..(14579)
<223> Any amino acid

<220>
<221> MOD_RES
<222> (14581)..(14581)
<223> Any amino acid

<220>
<221> MOD_RES
<222> (14587)..(14591)
<223> Any amino acid

<220>
<221> MOD_RES
<222> (14593)..(14594)
<223> Any amino acid
```

```
<220>
<221> MOD_RES
<222> (14725)..(14727)
<223> Any amino acid

<220>
<221> MOD_RES
<222> (14731)..(14731)
<223> Any amino acid

<220>
<221> MOD_RES
<222> (14735)..(14735)
<223> Any amino acid

<220>
<221> MOD_RES
<222> (14737)..(14737)
<223> Any amino acid

<220>
<221> MOD_RES
<222> (14743)..(14747)
<223> Any amino acid

<220>
<221> MOD_RES
<222> (14749)..(14750)
<223> Any amino acid

<220>
<221> MOD_RES
<222> (15661)..(15663)
<223> Any amino acid

<220>
<221> MOD_RES
<222> (15667)..(15667)
<223> Any amino acid

<220>
<221> MOD_RES
<222> (15671)..(15671)
<223> Any amino acid

<220>
<221> MOD_RES
<222> (15673)..(15673)
<223> Any amino acid

<220>
<221> MOD_RES
<222> (15679)..(15683)
<223> Any amino acid

<220>
<221> MOD_RES
<222> (15685)..(15686)
<223> Any amino acid

<220>
<221> MOD_RES
<222> (15972)..(15974)
```

<223> Any amino acid

<220>
<221> MOD_RES
<222> (15978)..(15978)
<223> Any amino acid

<220>
<221> MOD_RES
<222> (15982)..(15982)
<223> Any amino acid

<220>
<221> MOD_RES
<222> (15984)..(15984)
<223> Any amino acid

<220>
<221> MOD_RES
<222> (15990)..(15994)
<223> Any amino acid

<220>
<221> MOD_RES
<222> (15996)..(15997)
<223> Any amino acid

<220>
<221> MOD_RES
<222> (16008)..(16008)
<223> Any amino acid

<220>
<221> MOD_RES
<222> (16015)..(16015)
<223> Any amino acid

<220>
<221> MOD_RES
<222> (16017)..(16017)
<223> Any amino acid

<220>
<221> MOD_RES
<222> (16021)..(16021)
<223> Any amino acid

<220>
<221> MOD_RES
<222> (16025)..(16025)
<223> Any amino acid

<220>
<221> MOD_RES
<222> (16034)..(16034)
<223> Any amino acid

<220>
<221> MOD_RES
<222> (16037)..(16037)
<223> Any amino acid

<220>

```
<221> MOD_RES
<222> (16040)..(16040)
<223> Any amino acid

<220>
<221> MOD_RES
<222> (16046)..(16046)
<223> Any amino acid

<220>
<221> MOD_RES
<222> (16051)..(16051)
<223> Any amino acid

<220>
<221> MOD_RES
<222> (16053)..(16055)
<223> Any amino acid

<220>
<221> MOD_RES
<222> (16058)..(16058)
<223> Any amino acid

<220>
<221> MOD_RES
<222> (16062)..(16063)
<223> Any amino acid

<220>
<221> MOD_RES
<222> (16065)..(16065)
<223> Any amino acid

<220>
<221> MOD_RES
<222> (16072)..(16072)
<223> Any amino acid

<220>
<221> MOD_RES
<222> (16075)..(16076)
<223> Any amino acid

<220>
<221> MOD_RES
<222> (16078)..(16078)
<223> Any amino acid

<220>
<221> MOD_RES
<222> (16088)..(16088)
<223> Any amino acid

<220>
<221> MOD_RES
<222> (16268)..(16269)
<223> Any amino acid

<220>
<221> MOD_RES
<222> (16278)..(16278)
<223> Any amino acid
```

```
<220>
<221> MOD_RES
<222> (16280)..(16281)
<223> Any amino acid

<220>
<221> MOD_RES
<222> (16373)..(16374)
<223> Any amino acid

<220>
<221> MOD_RES
<222> (16376)..(16376)
<223> Any amino acid

<220>
<221> MOD_RES
<222> (16383)..(16383)
<223> Any amino acid

<220>
<221> MOD_RES
<222> (16386)..(16387)
<223> Any amino acid

<220>
<221> MOD_RES
<222> (16389)..(16389)
<223> Any amino acid

<220>
<221> MOD_RES
<222> (16399)..(16399)
<223> Any amino acid

<220>
<221> MOD_RES
<222> (16409)..(16410)
<223> Any amino acid

<220>
<221> MOD_RES
<222> (16412)..(16412)
<223> Any amino acid

<220>
<221> MOD_RES
<222> (16424)..(16425)
<223> Any amino acid

<220>
<221> MOD_RES
<222> (16434)..(16434)
<223> Any amino acid

<220>
<221> MOD_RES
<222> (16436)..(16437)
<223> Any amino acid

<220>
<221> MOD_RES
```

```
<222> (16439)..(16441)
<223> Any amino acid

<220>
<221> MOD_RES
<222> (16445)..(16445)
<223> Any amino acid

<220>
<221> MOD_RES
<222> (16449)..(16449)
<223> Any amino acid

<220>
<221> MOD_RES
<222> (16451)..(16451)
<223> Any amino acid

<220>
<221> MOD_RES
<222> (16457)..(16461)
<223> Any amino acid

<220>
<221> MOD_RES
<222> (16463)..(16464)
<223> Any amino acid

<220>
<221> MOD_RES
<222> (16841)..(16842)
<223> Any amino acid

<220>
<221> MOD_RES
<222> (16844)..(16844)
<223> Any amino acid

<220>
<221> MOD_RES
<222> (16851)..(16851)
<223> Any amino acid

<220>
<221> MOD_RES
<222> (16854)..(16855)
<223> Any amino acid

<220>
<221> MOD_RES
<222> (16857)..(16857)
<223> Any amino acid

<220>
<221> MOD_RES
<222> (16867)..(16867)
<223> Any amino acid

<220>
<221> MOD_RES
<222> (16877)..(16878)
<223> Any amino acid
```

```
<220>
<221> MOD_RES
<222> (16880)..(16880)
<223> Any amino acid

<220>
<221> MOD_RES
<222> (16892)..(16893)
<223> Any amino acid

<220>
<221> MOD_RES
<222> (16902)..(16902)
<223> Any amino acid

<220>
<221> MOD_RES
<222> (16904)..(16905)
<223> Any amino acid

<220>
<221> MOD_RES
<222> (16907)..(16909)
<223> Any amino acid

<220>
<221> MOD_RES
<222> (16913)..(16913)
<223> Any amino acid

<220>
<221> MOD_RES
<222> (16917)..(16917)
<223> Any amino acid

<220>
<221> MOD_RES
<222> (16919)..(16919)
<223> Any amino acid

<220>
<221> MOD_RES
<222> (16925)..(16929)
<223> Any amino acid

<220>
<221> MOD_RES
<222> (16931)..(16932)
<223> Any amino acid

<220>
<221> MOD_RES
<222> (17465)..(17466)
<223> Any amino acid

<220>
<221> MOD_RES
<222> (17468)..(17468)
<223> Any amino acid

<220>
<221> MOD_RES
<222> (17475)..(17475)
```

```
<223> Any amino acid

<220>
<221> MOD_RES
<222> (17478)..(17479)
<223> Any amino acid

<220>
<221> MOD_RES
<222> (17481)..(17481)
<223> Any amino acid

<220>
<221> MOD_RES
<222> (17491)..(17491)
<223> Any amino acid

<220>
<221> MOD_RES
<222> (17501)..(17502)
<223> Any amino acid

<220>
<221> MOD_RES
<222> (17504)..(17504)
<223> Any amino acid

<220>
<221> MOD_RES
<222> (17516)..(17517)
<223> Any amino acid

<220>
<221> MOD_RES
<222> (17526)..(17526)
<223> Any amino acid

<220>
<221> MOD_RES
<222> (17528)..(17529)
<223> Any amino acid

<220>
<221> MOD_RES
<222> (17531)..(17533)
<223> Any amino acid

<220>
<221> MOD_RES
<222> (17537)..(17537)
<223> Any amino acid

<220>
<221> MOD_RES
<222> (17541)..(17541)
<223> Any amino acid

<220>
<221> MOD_RES
<222> (17543)..(17543)
<223> Any amino acid

<220>
```

```
<221> MOD_RES
<222> (17549)..(17553)
<223> Any amino acid

<220>
<221> MOD_RES
<222> (17555)..(17556)
<223> Any amino acid

<220>
<221> MOD_RES
<222> (17567)..(17567)
<223> Any amino acid

<220>
<221> MOD_RES
<222> (17574)..(17574)
<223> Any amino acid

<220>
<221> MOD_RES
<222> (17576)..(17576)
<223> Any amino acid

<220>
<221> MOD_RES
<222> (17580)..(17580)
<223> Any amino acid

<220>
<221> MOD_RES
<222> (17584)..(17584)
<223> Any amino acid

<220>
<221> MOD_RES
<222> (17593)..(17593)
<223> Any amino acid

<220>
<221> MOD_RES
<222> (17596)..(17596)
<223> Any amino acid

<220>
<221> MOD_RES
<222> (17599)..(17599)
<223> Any amino acid

<220>
<221> MOD_RES
<222> (17605)..(17605)
<223> Any amino acid

<220>
<221> MOD_RES
<222> (17610)..(17610)
<223> Any amino acid

<220>
<221> MOD_RES
<222> (17612)..(17614)
<223> Any amino acid
```

```
<220>
<221> MOD_RES
<222> (17617)..(17617)
<223> Any amino acid

<220>
<221> MOD_RES
<222> (17621)..(17622)
<223> Any amino acid

<220>
<221> MOD_RES
<222> (17624)..(17624)
<223> Any amino acid

<220>
<221> MOD_RES
<222> (17631)..(17631)
<223> Any amino acid

<220>
<221> MOD_RES
<222> (17777)..(17778)
<223> Any amino acid

<220>
<221> MOD_RES
<222> (17780)..(17780)
<223> Any amino acid

<220>
<221> MOD_RES
<222> (17787)..(17787)
<223> Any amino acid

<220>
<221> MOD_RES
<222> (17790)..(17791)
<223> Any amino acid

<220>
<221> MOD_RES
<222> (17793)..(17793)
<223> Any amino acid

<220>
<221> MOD_RES
<222> (17803)..(17803)
<223> Any amino acid

<220>
<221> MOD_RES
<222> (17813)..(17814)
<223> Any amino acid

<220>
<221> MOD_RES
<222> (17816)..(17816)
<223> Any amino acid

<220>
<221> MOD_RES
```

```
<222> (17828)..(17829)
<223> Any amino acid

<220>
<221> MOD_RES
<222> (17838)..(17838)
<223> Any amino acid

<220>
<221> MOD_RES
<222> (17840)..(17841)
<223> Any amino acid

<220>
<221> MOD_RES
<222> (17843)..(17845)
<223> Any amino acid

<220>
<221> MOD_RES
<222> (17849)..(17849)
<223> Any amino acid

<220>
<221> MOD_RES
<222> (17853)..(17853)
<223> Any amino acid

<220>
<221> MOD_RES
<222> (17855)..(17855)
<223> Any amino acid

<220>
<221> MOD_RES
<222> (17861)..(17865)
<223> Any amino acid

<220>
<221> MOD_RES
<222> (17867)..(17868)
<223> Any amino acid

<220>
<221> MOD_RES
<222> (17879)..(17879)
<223> Any amino acid

<220>
<221> MOD_RES
<222> (17886)..(17886)
<223> Any amino acid

<220>
<221> MOD_RES
<222> (17888)..(17888)
<223> Any amino acid

<220>
<221> MOD_RES
<222> (17892)..(17892)
<223> Any amino acid
```

```
<220>
<221> MOD_RES
<222> (17896)..(17896)
<223> Any amino acid

<220>
<221> MOD_RES
<222> (17905)..(17905)
<223> Any amino acid

<220>
<221> MOD_RES
<222> (17908)..(17908)
<223> Any amino acid

<220>
<221> MOD_RES
<222> (17911)..(17911)
<223> Any amino acid

<220>
<221> MOD_RES
<222> (17917)..(17917)
<223> Any amino acid

<220>
<221> MOD_RES
<222> (17922)..(17922)
<223> Any amino acid

<220>
<221> MOD_RES
<222> (17924)..(17926)
<223> Any amino acid

<220>
<221> MOD_RES
<222> (17929)..(17929)
<223> Any amino acid

<220>
<221> MOD_RES
<222> (17933)..(17934)
<223> Any amino acid

<220>
<221> MOD_RES
<222> (17936)..(17936)
<223> Any amino acid

<220>
<221> MOD_RES
<222> (17943)..(17943)
<223> Any amino acid

<220>
<221> MOD_RES
<222> (17946)..(17947)
<223> Any amino acid

<220>
<221> MOD_RES
<222> (17949)..(17949)
```

```
<223> Any amino acid

<220>
<221> MOD_RES
<222> (17959)..(17959)
<223> Any amino acid

<220>
<221> MOD_RES
<222> (18089)..(18090)
<223> Any amino acid

<220>
<221> MOD_RES
<222> (18092)..(18092)
<223> Any amino acid

<220>
<221> MOD_RES
<222> (18099)..(18099)
<223> Any amino acid

<220>
<221> MOD_RES
<222> (18102)..(18103)
<223> Any amino acid

<220>
<221> MOD_RES
<222> (18105)..(18105)
<223> Any amino acid

<220>
<221> MOD_RES
<222> (18115)..(18115)
<223> Any amino acid

<220>
<221> MOD_RES
<222> (18125)..(18126)
<223> Any amino acid

<220>
<221> MOD_RES
<222> (18128)..(18128)
<223> Any amino acid

<220>
<221> MOD_RES
<222> (18140)..(18141)
<223> Any amino acid

<220>
<221> MOD_RES
<222> (18150)..(18150)
<223> Any amino acid

<220>
<221> MOD_RES
<222> (18152)..(18153)
<223> Any amino acid

<220>
```

```
<221> MOD_RES
<222> (18155)..(18157)
<223> Any amino acid

<220>
<221> MOD_RES
<222> (18161)..(18161)
<223> Any amino acid

<220>
<221> MOD_RES
<222> (18165)..(18165)
<223> Any amino acid

<220>
<221> MOD_RES
<222> (18167)..(18167)
<223> Any amino acid

<220>
<221> MOD_RES
<222> (18173)..(18177)
<223> Any amino acid

<220>
<221> MOD_RES
<222> (18179)..(18180)
<223> Any amino acid

<220>
<221> MOD_RES
<222> (18191)..(18191)
<223> Any amino acid

<220>
<221> MOD_RES
<222> (18198)..(18198)
<223> Any amino acid

<220>
<221> MOD_RES
<222> (18200)..(18200)
<223> Any amino acid

<220>
<221> MOD_RES
<222> (18204)..(18204)
<223> Any amino acid

<220>
<221> MOD_RES
<222> (18208)..(18208)
<223> Any amino acid

<220>
<221> MOD_RES
<222> (18217)..(18217)
<223> Any amino acid

<220>
<221> MOD_RES
<222> (18220)..(18220)
<223> Any amino acid
```

```
<220>
<221> MOD_RES
<222> (18223)..(18223)
<223> Any amino acid

<220>
<221> MOD_RES
<222> (18229)..(18229)
<223> Any amino acid

<220>
<221> MOD_RES
<222> (18234)..(18234)
<223> Any amino acid

<220>
<221> MOD_RES
<222> (18236)..(18238)
<223> Any amino acid

<220>
<221> MOD_RES
<222> (18241)..(18241)
<223> Any amino acid

<220>
<221> MOD_RES
<222> (18245)..(18246)
<223> Any amino acid

<220>
<221> MOD_RES
<222> (18248)..(18248)
<223> Any amino acid

<220>
<221> MOD_RES
<222> (18255)..(18255)
<223> Any amino acid

<220>
<221> MOD_RES
<222> (18258)..(18259)
<223> Any amino acid

<220>
<221> MOD_RES
<222> (18261)..(18261)
<223> Any amino acid

<220>
<221> MOD_RES
<222> (18271)..(18271)
<223> Any amino acid

<220>
<221> MOD_RES
<222> (18401)..(18402)
<223> Any amino acid

<220>
<221> MOD_RES
```

```
<222> (18404)..(18404)
<223> Any amino acid

<220>
<221> MOD_RES
<222> (18411)..(18411)
<223> Any amino acid

<220>
<221> MOD_RES
<222> (18414)..(18415)
<223> Any amino acid

<220>
<221> MOD_RES
<222> (18417)..(18417)
<223> Any amino acid

<220>
<221> MOD_RES
<222> (18427)..(18427)
<223> Any amino acid

<220>
<221> MOD_RES
<222> (18437)..(18438)
<223> Any amino acid

<220>
<221> MOD_RES
<222> (18440)..(18440)
<223> Any amino acid

<220>
<221> MOD_RES
<222> (18452)..(18453)
<223> Any amino acid

<220>
<221> MOD_RES
<222> (18462)..(18462)
<223> Any amino acid

<220>
<221> MOD_RES
<222> (18464)..(18465)
<223> Any amino acid

<220>
<221> MOD_RES
<222> (18467)..(18469)
<223> Any amino acid

<220>
<221> MOD_RES
<222> (18473)..(18473)
<223> Any amino acid

<220>
<221> MOD_RES
<222> (18477)..(18477)
<223> Any amino acid
```

```
<220>
<221> MOD_RES
<222> (18479)..(18479)
<223> Any amino acid

<220>
<221> MOD_RES
<222> (18485)..(18489)
<223> Any amino acid

<220>
<221> MOD_RES
<222> (18491)..(18492)
<223> Any amino acid

<220>
<221> MOD_RES
<222> (18503)..(18503)
<223> Any amino acid

<220>
<221> MOD_RES
<222> (18510)..(18510)
<223> Any amino acid

<220>
<221> MOD_RES
<222> (18512)..(18512)
<223> Any amino acid

<220>
<221> MOD_RES
<222> (18516)..(18516)
<223> Any amino acid

<220>
<221> MOD_RES
<222> (18520)..(18520)
<223> Any amino acid

<220>
<221> MOD_RES
<222> (18529)..(18529)
<223> Any amino acid

<220>
<221> MOD_RES
<222> (18532)..(18532)
<223> Any amino acid

<220>
<221> MOD_RES
<222> (18535)..(18535)
<223> Any amino acid

<220>
<221> MOD_RES
<222> (18541)..(18541)
<223> Any amino acid

<220>
<221> MOD_RES
<222> (18546)..(18546)
```

```
<223> Any amino acid

<220>
<221> MOD_RES
<222> (18548)..(18550)
<223> Any amino acid

<220>
<221> MOD_RES
<222> (18553)..(18553)
<223> Any amino acid

<220>
<221> MOD_RES
<222> (18557)..(18558)
<223> Any amino acid

<220>
<221> MOD_RES
<222> (18560)..(18560)
<223> Any amino acid

<220>
<221> MOD_RES
<222> (18567)..(18567)
<223> Any amino acid

<220>
<221> MOD_RES
<222> (18570)..(18571)
<223> Any amino acid

<220>
<221> MOD_RES
<222> (18573)..(18573)
<223> Any amino acid

<220>
<221> MOD_RES
<222> (18583)..(18583)
<223> Any amino acid

<220>
<221> MOD_RES
<222> (18713)..(18714)
<223> Any amino acid

<220>
<221> MOD_RES
<222> (18716)..(18716)
<223> Any amino acid

<220>
<221> MOD_RES
<222> (18723)..(18723)
<223> Any amino acid

<220>
<221> MOD_RES
<222> (18726)..(18727)
<223> Any amino acid

<220>
```

```
<221> MOD_RES
<222> (18729)..(18729)
<223> Any amino acid

<220>
<221> MOD_RES
<222> (18739)..(18739)
<223> Any amino acid

<220>
<221> MOD_RES
<222> (18749)..(18750)
<223> Any amino acid

<220>
<221> MOD_RES
<222> (18752)..(18752)
<223> Any amino acid

<220>
<221> MOD_RES
<222> (18764)..(18765)
<223> Any amino acid

<220>
<221> MOD_RES
<222> (18774)..(18774)
<223> Any amino acid

<220>
<221> MOD_RES
<222> (18776)..(18777)
<223> Any amino acid

<220>
<221> MOD_RES
<222> (18779)..(18781)
<223> Any amino acid

<220>
<221> MOD_RES
<222> (18785)..(18785)
<223> Any amino acid

<220>
<221> MOD_RES
<222> (18789)..(18789)
<223> Any amino acid

<220>
<221> MOD_RES
<222> (18791)..(18791)
<223> Any amino acid

<220>
<221> MOD_RES
<222> (18797)..(18801)
<223> Any amino acid

<220>
<221> MOD_RES
<222> (18803)..(18804)
<223> Any amino acid
```

```
<220>
<221> MOD_RES
<222> (18815)..(18815)
<223> Any amino acid

<220>
<221> MOD_RES
<222> (18822)..(18822)
<223> Any amino acid

<220>
<221> MOD_RES
<222> (18824)..(18824)
<223> Any amino acid

<220>
<221> MOD_RES
<222> (18828)..(18828)
<223> Any amino acid

<220>
<221> MOD_RES
<222> (18832)..(18832)
<223> Any amino acid

<220>
<221> MOD_RES
<222> (18841)..(18841)
<223> Any amino acid

<220>
<221> MOD_RES
<222> (18844)..(18844)
<223> Any amino acid

<220>
<221> MOD_RES
<222> (18847)..(18847)
<223> Any amino acid

<220>
<221> MOD_RES
<222> (18853)..(18853)
<223> Any amino acid

<220>
<221> MOD_RES
<222> (18858)..(18858)
<223> Any amino acid

<220>
<221> MOD_RES
<222> (18860)..(18862)
<223> Any amino acid

<220>
<221> MOD_RES
<222> (18865)..(18865)
<223> Any amino acid

<220>
<221> MOD_RES
```

```
<222> (18869)..(18870)
<223> Any amino acid

<220>
<221> MOD_RES
<222> (18872)..(18872)
<223> Any amino acid

<220>
<221> MOD_RES
<222> (18879)..(18879)
<223> Any amino acid

<220>
<221> MOD_RES
<222> (18882)..(18883)
<223> Any amino acid

<220>
<221> MOD_RES
<222> (18885)..(18885)
<223> Any amino acid

<220>
<221> MOD_RES
<222> (18895)..(18895)
<223> Any amino acid

<220>
<221> MOD_RES
<222> (19091)..(19093)
<223> Any amino acid

<220>
<221> MOD_RES
<222> (19097)..(19097)
<223> Any amino acid

<220>
<221> MOD_RES
<222> (19101)..(19101)
<223> Any amino acid

<220>
<221> MOD_RES
<222> (19103)..(19103)
<223> Any amino acid

<220>
<221> MOD_RES
<222> (19109)..(19113)
<223> Any amino acid

<220>
<221> MOD_RES
<222> (19115)..(19116)
<223> Any amino acid

<220>
<221> MOD_RES
<222> (19127)..(19127)
<223> Any amino acid
```

```
<220>
<221> MOD_RES
<222> (19134)..(19134)
<223> Any amino acid

<220>
<221> MOD_RES
<222> (19136)..(19136)
<223> Any amino acid

<220>
<221> MOD_RES
<222> (19140)..(19140)
<223> Any amino acid

<220>
<221> MOD_RES
<222> (19144)..(19144)
<223> Any amino acid

<220>
<221> MOD_RES
<222> (19153)..(19153)
<223> Any amino acid

<220>
<221> MOD_RES
<222> (19156)..(19156)
<223> Any amino acid

<220>
<221> MOD_RES
<222> (19159)..(19159)
<223> Any amino acid

<220>
<221> MOD_RES
<222> (19165)..(19165)
<223> Any amino acid

<220>
<221> MOD_RES
<222> (19170)..(19170)
<223> Any amino acid

<220>
<221> MOD_RES
<222> (19172)..(19174)
<223> Any amino acid

<220>
<221> MOD_RES
<222> (19177)..(19177)
<223> Any amino acid

<220>
<221> MOD_RES
<222> (19181)..(19182)
<223> Any amino acid

<220>
<221> MOD_RES
<222> (19184)..(19184)
```

```
<223> Any amino acid

<220>
<221> MOD_RES
<222> (19191)..(19191)
<223> Any amino acid

<220>
<221> MOD_RES
<222> (19194)..(19195)
<223> Any amino acid

<220>
<221> MOD_RES
<222> (19197)..(19197)
<223> Any amino acid

<220>
<221> MOD_RES
<222> (19207)..(19207)
<223> Any amino acid

<220>
<221> MOD_RES
<222> (19337)..(19338)
<223> Any amino acid

<220>
<221> MOD_RES
<222> (19340)..(19340)
<223> Any amino acid

<220>
<221> MOD_RES
<222> (19347)..(19347)
<223> Any amino acid

<220>
<221> MOD_RES
<222> (19350)..(19351)
<223> Any amino acid

<220>
<221> MOD_RES
<222> (19353)..(19353)
<223> Any amino acid

<220>
<221> MOD_RES
<222> (19363)..(19363)
<223> Any amino acid

<220>
<221> MOD_RES
<222> (19373)..(19374)
<223> Any amino acid

<220>
<221> MOD_RES
<222> (19376)..(19376)
<223> Any amino acid

<220>
```

```
<221> MOD_RES
<222> (19388)..(19389)
<223> Any amino acid

<220>
<221> MOD_RES
<222> (19398)..(19398)
<223> Any amino acid

<220>
<221> MOD_RES
<222> (19400)..(19401)
<223> Any amino acid

<220>
<221> MOD_RES
<222> (19403)..(19405)
<223> Any amino acid

<220>
<221> MOD_RES
<222> (19409)..(19409)
<223> Any amino acid

<220>
<221> MOD_RES
<222> (19413)..(19413)
<223> Any amino acid

<220>
<221> MOD_RES
<222> (19415)..(19415)
<223> Any amino acid

<220>
<221> MOD_RES
<222> (19421)..(19425)
<223> Any amino acid

<220>
<221> MOD_RES
<222> (19427)..(19428)
<223> Any amino acid

<220>
<221> MOD_RES
<222> (19439)..(19439)
<223> Any amino acid

<220>
<221> MOD_RES
<222> (19446)..(19446)
<223> Any amino acid

<220>
<221> MOD_RES
<222> (19448)..(19448)
<223> Any amino acid

<220>
<221> MOD_RES
<222> (19452)..(19452)
<223> Any amino acid
```

```
<220>
<221> MOD_RES
<222> (19456)..(19456)
<223> Any amino acid

<220>
<221> MOD_RES
<222> (19465)..(19465)
<223> Any amino acid

<220>
<221> MOD_RES
<222> (19468)..(19468)
<223> Any amino acid

<220>
<221> MOD_RES
<222> (19471)..(19471)
<223> Any amino acid

<220>
<221> MOD_RES
<222> (19477)..(19477)
<223> Any amino acid

<220>
<221> MOD_RES
<222> (19482)..(19482)
<223> Any amino acid

<220>
<221> MOD_RES
<222> (19484)..(19486)
<223> Any amino acid

<220>
<221> MOD_RES
<222> (19489)..(19489)
<223> Any amino acid

<220>
<221> MOD_RES
<222> (19493)..(19494)
<223> Any amino acid

<220>
<221> MOD_RES
<222> (19496)..(19496)
<223> Any amino acid

<220>
<221> MOD_RES
<222> (19503)..(19503)
<223> Any amino acid

<220>
<221> MOD_RES
<222> (19506)..(19507)
<223> Any amino acid

<220>
<221> MOD_RES
```

```
<222> (19509)..(19509)
<223> Any amino acid

<220>
<221> MOD_RES
<222> (19519)..(19519)
<223> Any amino acid

<220>
<221> MOD_RES
<222> (19649)..(19650)
<223> Any amino acid

<220>
<221> MOD_RES
<222> (19652)..(19652)
<223> Any amino acid

<220>
<221> MOD_RES
<222> (19659)..(19659)
<223> Any amino acid

<220>
<221> MOD_RES
<222> (19662)..(19663)
<223> Any amino acid

<220>
<221> MOD_RES
<222> (19665)..(19665)
<223> Any amino acid

<220>
<221> MOD_RES
<222> (19675)..(19675)
<223> Any amino acid

<220>
<221> MOD_RES
<222> (19685)..(19686)
<223> Any amino acid

<220>
<221> MOD_RES
<222> (19688)..(19688)
<223> Any amino acid

<220>
<221> MOD_RES
<222> (19700)..(19701)
<223> Any amino acid

<220>
<221> MOD_RES
<222> (19710)..(19710)
<223> Any amino acid

<220>
<221> MOD_RES
<222> (19712)..(19713)
<223> Any amino acid
```

```
<220>
<221> MOD_RES
<222> (19715)..(19717)
<223> Any amino acid

<220>
<221> MOD_RES
<222> (19721)..(19721)
<223> Any amino acid

<220>
<221> MOD_RES
<222> (19725)..(19725)
<223> Any amino acid

<220>
<221> MOD_RES
<222> (19727)..(19727)
<223> Any amino acid

<220>
<221> MOD_RES
<222> (19733)..(19737)
<223> Any amino acid

<220>
<221> MOD_RES
<222> (19739)..(19740)
<223> Any amino acid

<220>
<221> MOD_RES
<222> (19751)..(19751)
<223> Any amino acid

<220>
<221> MOD_RES
<222> (19758)..(19758)
<223> Any amino acid

<220>
<221> MOD_RES
<222> (19760)..(19760)
<223> Any amino acid

<220>
<221> MOD_RES
<222> (19764)..(19764)
<223> Any amino acid

<220>
<221> MOD_RES
<222> (19768)..(19768)
<223> Any amino acid

<220>
<221> MOD_RES
<222> (19777)..(19777)
<223> Any amino acid

<220>
<221> MOD_RES
<222> (19780)..(19780)
```

```
<223> Any amino acid

<220>
<221> MOD_RES
<222> (19783)..(19783)
<223> Any amino acid

<220>
<221> MOD_RES
<222> (19789)..(19789)
<223> Any amino acid

<220>
<221> MOD_RES
<222> (19794)..(19794)
<223> Any amino acid

<220>
<221> MOD_RES
<222> (19796)..(19798)
<223> Any amino acid

<220>
<221> MOD_RES
<222> (19801)..(19801)
<223> Any amino acid

<220>
<221> MOD_RES
<222> (19805)..(19806)
<223> Any amino acid

<220>
<221> MOD_RES
<222> (19808)..(19808)
<223> Any amino acid

<220>
<221> MOD_RES
<222> (19815)..(19815)
<223> Any amino acid

<220>
<221> MOD_RES
<222> (19818)..(19819)
<223> Any amino acid

<220>
<221> MOD_RES
<222> (19821)..(19821)
<223> Any amino acid

<220>
<221> MOD_RES
<222> (19831)..(19831)
<223> Any amino acid

<220>
<221> MOD_RES
<222> (19960)..(19961)
<223> Any amino acid

<220>
```

```
<221> MOD_RES
<222> (19963)..(19963)
<223> Any amino acid

<220>
<221> MOD_RES
<222> (19970)..(19970)
<223> Any amino acid

<220>
<221> MOD_RES
<222> (19973)..(19974)
<223> Any amino acid

<220>
<221> MOD_RES
<222> (19976)..(19976)
<223> Any amino acid

<220>
<221> MOD_RES
<222> (19986)..(19986)
<223> Any amino acid

<220>
<221> MOD_RES
<222> (19996)..(19997)
<223> Any amino acid

<220>
<221> MOD_RES
<222> (19999)..(19999)
<223> Any amino acid

<220>
<221> MOD_RES
<222> (20011)..(20012)
<223> Any amino acid

<220>
<221> MOD_RES
<222> (20021)..(20021)
<223> Any amino acid

<220>
<221> MOD_RES
<222> (20023)..(20024)
<223> Any amino acid

<220>
<221> MOD_RES
<222> (20026)..(20028)
<223> Any amino acid

<220>
<221> MOD_RES
<222> (20032)..(20032)
<223> Any amino acid

<220>
<221> MOD_RES
<222> (20036)..(20036)
<223> Any amino acid
```

```
<220>
<221> MOD_RES
<222> (20038)..(20038)
<223> Any amino acid

<220>
<221> MOD_RES
<222> (20044)..(20048)
<223> Any amino acid

<220>
<221> MOD_RES
<222> (20050)..(20051)
<223> Any amino acid

<220>
<221> MOD_RES
<222> (20062)..(20062)
<223> Any amino acid

<220>
<221> MOD_RES
<222> (20069)..(20069)
<223> Any amino acid

<220>
<221> MOD_RES
<222> (20071)..(20071)
<223> Any amino acid

<220>
<221> MOD_RES
<222> (20075)..(20075)
<223> Any amino acid

<220>
<221> MOD_RES
<222> (20079)..(20079)
<223> Any amino acid

<220>
<221> MOD_RES
<222> (20088)..(20088)
<223> Any amino acid

<220>
<221> MOD_RES
<222> (20091)..(20091)
<223> Any amino acid

<220>
<221> MOD_RES
<222> (20094)..(20094)
<223> Any amino acid

<220>
<221> MOD_RES
<222> (20100)..(20100)
<223> Any amino acid

<220>
<221> MOD_RES
```

```
<222> (20105)..(20105)
<223> Any amino acid

<220>
<221> MOD_RES
<222> (20107)..(20109)
<223> Any amino acid

<220>
<221> MOD_RES
<222> (20112)..(20112)
<223> Any amino acid

<220>
<221> MOD_RES
<222> (20116)..(20117)
<223> Any amino acid

<220>
<221> MOD_RES
<222> (20119)..(20119)
<223> Any amino acid

<220>
<221> MOD_RES
<222> (20126)..(20126)
<223> Any amino acid

<220>
<221> MOD_RES
<222> (20129)..(20130)
<223> Any amino acid

<220>
<221> MOD_RES
<222> (20132)..(20132)
<223> Any amino acid

<220>
<221> MOD_RES
<222> (20142)..(20142)
<223> Any amino acid

<220>
<221> MOD_RES
<222> (20272)..(20273)
<223> Any amino acid

<220>
<221> MOD_RES
<222> (20275)..(20275)
<223> Any amino acid

<220>
<221> MOD_RES
<222> (20282)..(20282)
<223> Any amino acid

<220>
<221> MOD_RES
<222> (20285)..(20286)
<223> Any amino acid
```

```
<220>
<221> MOD_RES
<222> (20288)..(20288)
<223> Any amino acid

<220>
<221> MOD_RES
<222> (20298)..(20298)
<223> Any amino acid

<220>
<221> MOD_RES
<222> (20308)..(20309)
<223> Any amino acid

<220>
<221> MOD_RES
<222> (20311)..(20311)
<223> Any amino acid

<220>
<221> MOD_RES
<222> (20323)..(20324)
<223> Any amino acid

<220>
<221> MOD_RES
<222> (20333)..(20333)
<223> Any amino acid

<220>
<221> MOD_RES
<222> (20335)..(20336)
<223> Any amino acid

<220>
<221> MOD_RES
<222> (20806)..(20808)
<223> Any amino acid

<220>
<221> MOD_RES
<222> (20812)..(20812)
<223> Any amino acid

<220>
<221> MOD_RES
<222> (20816)..(20816)
<223> Any amino acid

<220>
<221> MOD_RES
<222> (20818)..(20818)
<223> Any amino acid

<220>
<221> MOD_RES
<222> (20824)..(20828)
<223> Any amino acid

<220>
<221> MOD_RES
<222> (20830)..(20831)
```

<223> Any amino acid

<400> 12

```
Met Leu Lys Pro Ser Gly Leu Pro Gly Ser Ser Ser Pro Thr Arg Ser
1               5                   10              15

Leu Met Thr Gly Ser Arg Ser Thr Lys Ala Thr Pro Glu Met Asp Ser
            20              25              30

Gly Leu Thr Gly Ala Thr Leu Ser Pro Lys Thr Ser Thr Gly Ala Ile
            35              40              45

Val Val Thr Glu His Thr Leu Pro Phe Thr Ser Pro Asp Lys Thr Leu
        50              55              60

Ala Ser Pro Thr Ser Ser Val Val Gly Arg Thr Thr Gln Ser Leu Gly
65              70              75              80

Val Met Ser Ser Ala Leu Pro Glu Ser Thr Ser Arg Gly Met Thr His
                85              90              95

Ser Glu Gln Arg Thr Ser Pro Ser Leu Ser Pro Gln Val Asn Gly Thr
            100             105             110

Pro Ser Arg Asn Tyr Pro Ala Thr Ser Met Val Ser Gly Leu Ser Ser
        115             120             125

Pro Arg Thr Arg Thr Ser Ser Thr Glu Gly Asn Phe Thr Lys Glu Ala
    130             135             140

Ser Thr Tyr Thr Leu Thr Val Glu Thr Thr Ser Gly Pro Val Thr Glu
145             150             155             160

Lys Tyr Thr Val Pro Thr Glu Thr Ser Thr Thr Glu Gly Asp Ser Thr
            165             170             175

Glu Thr Pro Trp Asp Thr Arg Tyr Ile Pro Val Lys Ile Thr Ser Pro
            180             185             190

Met Lys Thr Phe Ala Asp Ser Thr Ala Ser Lys Glu Asn Ala Pro Val
        195             200             205

Ser Met Thr Pro Ala Glu Thr Thr Val Thr Asp Ser His Thr Pro Gly
        210             215             220

Arg Thr Asn Pro Ser Phe Gly Thr Leu Tyr Ser Ser Phe Leu Asp Leu
225             230             235             240
```

```
Ser Pro Lys Gly Thr Pro Asn Ser Arg Gly Glu Thr Ser Leu Glu Leu
            245             250                 255

Ile Leu Ser Thr Thr Gly Tyr Pro Phe Ser Ser Pro Glu Pro Gly Ser
            260             265                 270

Ala Gly His Ser Arg Ile Ser Thr Ser Ala Pro Leu Ser Ser Ser Ala
            275             280                 285

Ser Val Leu Asp Asn Lys Ile Ser Glu Thr Ser Ile Phe Ser Gly Gln
    290             295                 300

Ser Leu Thr Ser Pro Leu Ser Pro Gly Val Pro Glu Ala Arg Ala Ser
305             310                 315                 320

Thr Met Pro Asn Ser Ala Ile Pro Phe Ser Met Thr Leu Ser Asn Ala
            325             330                 335

Glu Thr Ser Ala Glu Arg Val Arg Ser Thr Ile Ser Ser Leu Gly Thr
            340             345                 350

Pro Ser Ile Ser Thr Lys Gln Thr Ala Glu Thr Ile Leu Thr Phe His
            355             360                 365

Ala Phe Ala Glu Thr Met Asp Ile Pro Ser Thr His Ile Ala Lys Thr
            370             375                 380

Leu Ala Ser Glu Trp Leu Gly Ser Pro Gly Thr Leu Gly Gly Thr Ser
385             390                 395                 400

Thr Ser Ala Leu Thr Thr Thr Ser Pro Ser Thr Thr Leu Val Ser Glu
            405             410                 415

Glu Thr Asn Thr His His Ser Thr Ser Gly Lys Glu Thr Glu Gly Thr
            420             425                 430

Leu Asn Thr Ser Met Thr Pro Leu Glu Thr Ser Ala Pro Gly Glu Glu
            435             440                 445

Ser Glu Met Thr Ala Thr Leu Val Pro Thr Leu Gly Phe Thr Thr Leu
    450             455                 460

Asp Ser Lys Ile Arg Ser Pro Ser Gln Val Ser Ser Ser His Pro Thr
465             470                 475                 480

Arg Glu Leu Arg Thr Thr Gly Ser Thr Ser Gly Arg Gln Ser Ser Ser
            485             490                 495
```

216

Thr Ala Ala His Gly Ser Ser Asp Ile Leu Arg Ala Thr Thr Ser Ser
        500                 505                 510

Thr Ser Lys Ala Ser Ser Trp Thr Ser Glu Ser Thr Ala Gln Gln Phe
        515                 520                 525

Ser Glu Pro Gln His Thr Gln Trp Val Glu Thr Ser Pro Ser Met Lys
        530                 535                 540

Thr Glu Arg Pro Pro Ala Ser Thr Ser Val Ala Ala Pro Ile Thr Thr
545                 550                 555                 560

Ser Val Pro Ser Val Val Ser Gly Phe Thr Thr Leu Lys Thr Ser Ser
                565                 570                 575

Thr Lys Gly Ile Trp Leu Glu Glu Thr Ser Ala Asp Thr Leu Ile Gly
                580                 585                 590

Glu Ser Thr Ala Gly Pro Thr Thr His Gln Phe Ala Val Pro Thr Gly
                595                 600                 605

Ile Ser Met Thr Gly Gly Ser Ser Thr Arg Gly Ser Gln Gly Thr Thr
        610                 615                 620

His Leu Leu Thr Arg Ala Thr Ala Ser Ser Glu Thr Ser Ala Asp Leu
625                 630                 635                 640

Thr Leu Ala Thr Asn Gly Val Pro Val Ser Val Ser Pro Ala Val Ser
                645                 650                 655

Lys Thr Ala Ala Gly Ser Ser Pro Pro Gly Gly Thr Lys Pro Ser Tyr
                660                 665                 670

Thr Met Val Ser Ser Val Ile Pro Glu Thr Ser Ser Leu Gln Ser Ser
                675                 680                 685

Ala Phe Arg Glu Gly Thr Ser Leu Gly Leu Thr Pro Leu Asn Thr Arg
        690                 695                 700

His Pro Phe Ser Ser Pro Glu Pro Asp Ser Ala Gly His Thr Lys Ile
705                 710                 715                 720

Ser Thr Ser Ile Pro Leu Leu Ser Ser Ala Ser Val Leu Glu Asp Lys
                725                 730                 735

Val Ser Ala Thr Ser Thr Phe Ser His His Lys Ala Thr Ser Ser Ile
                740                 745                 750

```
Thr Thr Gly Thr Pro Glu Ile Ser Thr Lys Thr Lys Pro Ser Ser Ala
        755             760             765

Val Leu Ser Ser Met Thr Leu Ser Asn Ala Ala Thr Ser Pro Glu Arg
        770             775             780

Val Arg Asn Ala Thr Ser Pro Leu Thr His Pro Ser Pro Ser Gly Glu
785             790             795             800

Glu Thr Ala Gly Ser Val Leu Thr Leu Ser Thr Ser Ala Glu Thr Thr
            805             810             815

Asp Ser Pro Asn Ile His Pro Thr Gly Thr Leu Thr Ser Glu Ser Ser
            820             825             830

Glu Ser Pro Ser Thr Leu Ser Leu Pro Ser Val Ser Gly Val Lys Thr
        835             840             845

Thr Phe Ser Ser Ser Thr Pro Ser Thr His Leu Phe Thr Ser Gly Glu
    850             855             860

Glu Thr Glu Glu Thr Ser Asn Pro Ser Val Ser Gln Pro Glu Thr Ser
865             870             875             880

Val Ser Arg Val Arg Thr Thr Leu Ala Ser Thr Ser Val Pro Thr Pro
            885             890             895

Val Phe Pro Thr Met Asp Thr Trp Pro Thr Arg Ser Ala Gln Phe Ser
        900             905             910

Ser Ser His Leu Val Ser Glu Leu Arg Ala Thr Ser Ser Thr Ser Val
        915             920             925

Thr Asn Ser Thr Gly Ser Ala Leu Pro Lys Ile Ser His Leu Thr Gly
    930             935             940

Thr Ala Thr Met Ser Gln Thr Asn Arg Asp Thr Phe Asn Asp Ser Ala
945             950             955             960

Ala Pro Gln Ser Thr Thr Trp Pro Glu Thr Ser Pro Arg Phe Lys Thr
            965             970             975

Gly Leu Pro Ser Ala Thr Thr Thr Val Ser Thr Ser Ala Thr Ser Leu
            980             985             990

Ser Ala Thr Val Met Val Ser Lys  Phe Thr Ser Pro Ala  Thr Ser Ser
```

995                           1000                            1005

Met Glu  Ala Thr Ser Ile Arg  Glu Pro Ser Thr Thr  Ile Leu Thr
     1010             1015             1020

Thr Glu  Thr Thr Asn Gly Pro  Gly Ser Met Ala Val  Ala Ser Thr
     1025             1030             1035

Asn Ile  Pro Ile Gly Lys Gly  Tyr Ile Thr Glu Gly  Arg Leu Asp
     1040             1045             1050

Thr Ser  His Leu Pro Ile Gly  Thr Thr Ala Ser Ser  Glu Thr Ser
     1055             1060             1065

Met Asp  Phe Thr Met Ala Lys  Glu Ser Val Ser Met  Ser Val Ser
     1070             1075             1080

Pro Ser  Gln Ser Met Asp Ala  Ala Gly Ser Ser Thr  Pro Gly Arg
     1085             1090             1095

Thr Ser  Gln Phe Val Asp Thr  Phe Ser Asp Asp Val  Tyr His Leu
     1100             1105             1110

Thr Ser  Arg Glu Ile Thr Ile  Pro Arg Asp Gly Thr  Ser Ser Ala
     1115             1120             1125

Leu Thr  Pro Gln Met Thr Ala  Thr His Pro Pro Ser  Pro Asp Pro
     1130             1135             1140

Gly Ser  Ala Arg Ser Thr Trp  Leu Gly Ile Leu Ser  Ser Ser Pro
     1145             1150             1155

Ser Ser  Pro Thr Pro Lys Val  Thr Met Ser Ser Thr  Phe Ser Thr
     1160             1165             1170

Gln Arg  Val Thr Thr Ser Met  Ile Met Asp Thr Val  Glu Thr Ser
     1175             1180             1185

Arg Trp  Asn Met Pro Asn Leu  Pro Ser Thr Thr Ser  Leu Thr Pro
     1190             1195             1200

Ser Asn  Ile Pro Thr Ser Gly  Ala Ile Gly Lys Ser  Thr Leu Val
     1205             1210             1215

Pro Leu  Asp Thr Pro Ser Pro  Ala Thr Ser Leu Glu  Ala Ser Glu
     1220             1225             1230

```
Gly Gly Leu Pro Thr Leu Ser  Thr Tyr Pro Glu Ser  Thr Asn Thr
    1235                1240             1245

Pro Ser Ile His Leu Gly Ala  His Ala Ser Ser Glu  Ser Pro Ser
    1250                1255             1260

Thr Ile Lys Leu Thr Met Ala  Ser Val Val Lys Pro  Gly Ser Tyr
    1265                1270             1275

Thr Pro Leu Thr Phe Pro Ser  Ile Glu Thr His Ile  His Val Ser
    1280                1285             1290

Thr Ala Arg Met Ala Tyr Ser  Ser Gly Ser Ser Pro  Glu Met Thr
    1295                1300             1305

Ala Pro Gly Glu Thr Asn Thr  Gly Ser Thr Trp Asp  Pro Thr Thr
    1310                1315             1320

Tyr Ile Thr Thr Thr Asp Pro  Lys Asp Thr Ser Ser  Ala Gln Val
    1325                1330             1335

Ser Thr Pro His Ser Val Arg  Thr Leu Arg Thr Thr  Glu Asn His
    1340                1345             1350

Pro Lys Thr Glu Ser Ala Thr  Pro Ala Ala Tyr Ser  Gly Ser Pro
    1355                1360             1365

Lys Ile Ser Ser Ser Pro Asn  Leu Thr Ser Pro Ala  Thr Lys Ala
    1370                1375             1380

Trp Thr Ile Thr Asp Thr Thr  Glu His Ser Thr Gln  Leu His Tyr
    1385                1390             1395

Thr Lys Leu Ala Glu Lys Ser  Ser Gly Phe Glu Thr  Gln Ser Ala
    1400                1405             1410

Pro Gly Pro Val Ser Val Val  Ile Pro Thr Ser Pro  Thr Ile Gly
    1415                1420             1425

Ser Ser Thr Leu Glu Leu Thr  Ser Asp Val Pro Gly  Glu Pro Leu
    1430                1435             1440

Val Leu Ala Pro Ser Glu Gln  Thr Thr Ile Thr Leu  Pro Met Ala
    1445                1450             1455

Thr Trp Leu Ser Thr Ser Leu  Thr Glu Glu Met Ala  Ser Thr Asp
    1460                1465             1470
```

```
Leu Asp Ile Ser Ser Pro Ser   Ser Pro Met Ser Thr   Phe Ala Ile
    1475                1480                1485

Phe Pro Pro Met Ser Thr Pro   Ser His Glu Leu Ser   Lys Ser Glu
    1490                1495                1500

Ala Asp Thr Ser Ala Ile Arg   Asn Thr Asp Ser Thr   Thr Leu Asp
    1505                1510                1515

Gln His Leu Gly Ile Arg Ser   Leu Gly Arg Thr Gly   Asp Leu Thr
    1520                1525                1530

Thr Val Pro Ile Thr Pro Leu   Thr Thr Thr Trp Thr   Ser Val Ile
    1535                1540                1545

Glu His Ser Thr Gln Ala Gln   Asp Thr Leu Ser Ala   Thr Met Ser
    1550                1555                1560

Pro Thr His Val Thr Gln Ser   Leu Lys Asp Gln Thr   Ser Ile Pro
    1565                1570                1575

Ala Ser Ala Ser Pro Ser His   Leu Thr Glu Val Tyr   Pro Glu Leu
    1580                1585                1590

Gly Thr Gln Gly Arg Ser Ser   Ser Glu Ala Thr Thr   Phe Trp Lys
    1595                1600                1605

Pro Ser Thr Asp Thr Leu Ser   Arg Glu Ile Glu Thr   Gly Pro Thr
    1610                1615                1620

Asn Ile Gln Ser Thr Pro Pro   Met Asp Asn Thr Thr   Thr Gly Ser
    1625                1630                1635

Ser Ser Ser Gly Val Thr Leu   Gly Ile Ala His Leu   Pro Ile Gly
    1640                1645                1650

Thr Ser Ser Pro Ala Glu Thr   Ser Thr Asn Met Ala   Leu Glu Arg
    1655                1660                1665

Arg Ser Ser Thr Ala Thr Val   Ser Met Ala Gly Thr   Met Gly Leu
    1670                1675                1680

Leu Val Thr Ser Ala Pro Gly   Arg Ser Ile Ser Gln   Ser Leu Gly
    1685                1690                1695

Arg Val Ser Ser Val Leu Ser   Glu Ser Thr Thr Glu   Gly Val Thr
    1700                1705                1710
```

221

```
Asp Ser  Ser Lys Gly Ser Ser  Pro Arg Leu Asn Thr  Gln Gly Asn
    1715             1720               1725

Thr Ala  Leu Ser Ser Ser Leu  Glu Pro Ser Tyr Ala  Glu Gly Ser
    1730             1735               1740

Gln Met  Ser Thr Ser Ile Pro  Leu Thr Ser Ser Pro  Thr Thr Pro
    1745             1750               1755

Asp Val  Glu Phe Ile Gly Gly  Ser Thr Phe Trp Thr  Lys Glu Val
    1760             1765               1770

Thr Thr  Val Met Thr Ser Asp  Ile Ser Lys Ser Ser  Ala Arg Thr
    1775             1780               1785

Glu Ser  Ser Ser Ala Thr Leu  Met Ser Thr Ala Leu  Gly Ser Thr
    1790             1795               1800

Glu Asn  Thr Gly Lys Glu Lys  Leu Arg Thr Ala Ser  Met Asp Leu
    1805             1810               1815

Pro Ser  Pro Thr Pro Ser Met  Glu Val Thr Pro Trp  Ile Ser Leu
    1820             1825               1830

Thr Leu  Ser Asn Ala Pro Asn  Thr Thr Asp Ser Leu  Asp Leu Ser
    1835             1840               1845

His Gly  Val His Thr Ser Ser  Ala Gly Thr Leu Ala  Thr Asp Arg
    1850             1855               1860

Ser Leu  Asn Thr Gly Val Thr  Arg Ala Ser Arg Leu  Glu Asn Gly
    1865             1870               1875

Ser Asp  Thr Ser Ser Lys Ser  Leu Ser Met Gly Asn  Ser Thr His
    1880             1885               1890

Thr Ser  Met Thr Asp Thr Glu  Lys Ser Glu Val Ser  Ser Ser Ile
    1895             1900               1905

His Pro  Arg Pro Glu Thr Ser  Ala Pro Gly Ala Glu  Thr Thr Leu
    1910             1915               1920

Thr Ser  Thr Pro Gly Asn Arg  Ala Ile Ser Leu Thr  Leu Pro Phe
    1925             1930               1935

Ser Ser  Ile Pro Val Glu Glu  Val Ile Ser Thr Gly  Ile Thr Ser
```

222

1940                        1945                          1950

Gly Pro Asp Ile Asn Ser Ala  Pro Met Thr His Ser  Pro Ile Thr
    1955                1960                1965

Pro Pro Thr Ile Val Trp Thr  Ser Thr Gly Thr Ile  Glu Gln Ser
    1970                1975                1980

Thr Gln Pro Leu His Ala Val  Ser Ser Glu Lys Val  Ser Val Gln
    1985                1990                1995

Thr Gln Ser Thr Pro Tyr Val  Asn Ser Val Ala Val  Ser Ala Ser
    2000                2005                2010

Pro Thr His Glu Asn Ser Val  Ser Ser Gly Ser Ser  Thr Ser Ser
    2015                2020                2025

Pro Tyr Ser Ser Ala Ser Leu  Glu Ser Leu Asp Ser  Thr Ile Ser
    2030                2035                2040

Arg Arg Asn Ala Ile Thr Ser  Trp Leu Trp Asp Leu  Thr Thr Ser
    2045                2050                2055

Leu Pro Thr Thr Thr Trp Pro  Ser Thr Ser Leu Ser  Glu Ala Leu
    2060                2065                2070

Ser Ser Gly His Ser Gly Val  Ser Asn Pro Ser Ser  Thr Thr Thr
    2075                2080                2085

Glu Phe Pro Leu Phe Ser Ala  Ala Ser Thr Ser Ala  Ala Lys Gln
    2090                2095                2100

Arg Asn Pro Glu Thr Glu Thr  His Gly Pro Gln Asn  Thr Ala Ala
    2105                2110                2115

Ser Thr Leu Asn Thr Asp Ala  Ser Ser Val Thr Gly  Leu Ser Glu
    2120                2125                2130

Thr Pro Val Gly Ala Ser Ile  Ser Ser Glu Val Pro  Leu Pro Met
    2135                2140                2145

Ala Ile Thr Ser Arg Ser Asp  Val Ser Gly Leu Thr  Ser Glu Ser
    2150                2155                2160

Thr Ala Asn Pro Ser Leu Gly  Thr Ala Ser Ser Ala  Gly Thr Lys
    2165                2170                2175

```
Leu Thr  Arg Thr Ile Ser Leu  Pro Thr Ser Glu Ser  Leu Val Ser
    2180            2185            2190

Phe Arg  Met Asn Lys Asp Pro  Trp Thr Val Ser Ile  Pro Leu Gly
    2195            2200            2205

Ser His  Pro Thr Thr Asn Thr  Glu Thr Ser Ile Pro  Val Asn Ser
    2210            2215            2220

Ala Gly  Pro Pro Gly Leu Ser  Thr Val Ala Ser Asp  Val Ile Asp
    2225            2230            2235

Thr Pro  Ser Asp Gly Ala Glu  Ser Ile Pro Thr Val  Ser Phe Ser
    2240            2245            2250

Pro Ser  Pro Asp Thr Glu Val  Thr Thr Ile Ser His  Phe Pro Glu
    2255            2260            2265

Lys Thr  Thr His Ser Phe Arg  Thr Ile Ser Ser Leu  Thr His Glu
    2270            2275            2280

Leu Thr  Ser Arg Val Thr Pro  Ile Pro Gly Asp Trp  Met Ser Ser
    2285            2290            2295

Ala Met  Ser Thr Lys Pro Thr  Gly Ala Ser Pro Ser  Ile Thr Leu
    2300            2305            2310

Gly Glu  Arg Arg Thr Ile Thr  Ser Ala Ala Pro Thr  Thr Ser Pro
    2315            2320            2325

Ile Val  Leu Thr Ala Ser Phe  Thr Glu Thr Ser Thr  Val Ser Leu
    2330            2335            2340

Asp Asn  Glu Thr Thr Val Lys  Thr Ser Asp Ile Leu  Asp Ala Arg
    2345            2350            2355

Lys Thr  Asn Glu Leu Pro Ser  Asp Ser Ser Ser Ser  Ser Asp Leu
    2360            2365            2370

Ile Asn  Thr Ser Ile Ala Ser  Ser Thr Met Asp Val  Thr Lys Thr
    2375            2380            2385

Ala Ser  Ile Ser Pro Thr Ser  Ile Ser Gly Met Thr  Ala Ser Ser
    2390            2395            2400

Ser Pro  Ser Leu Phe Ser Ser  Asp Arg Pro Gln Val  Pro Thr Ser
    2405            2410            2415
```

224

Thr Thr Glu Thr Asn Thr Ala Thr Ser Pro Ser Val Ser Ser Asn
2420 2425 2430

Thr Tyr Ser Leu Asp Gly Gly Ser Asn Val Gly Gly Thr Pro Ser
2435 2440 2445

Thr Leu Pro Pro Phe Thr Ile Thr His Pro Val Glu Thr Ser Ser
2450 2455 2460

Ala Leu Leu Ala Trp Ser Arg Pro Val Arg Thr Phe Ser Thr Met
2465 2470 2475

Val Ser Thr Asp Thr Ala Ser Gly Glu Asn Pro Thr Ser Ser Asn
2480 2485 2490

Ser Val Val Thr Ser Val Pro Ala Pro Gly Thr Trp Ala Ser Val
2495 2500 2505

Gly Ser Thr Thr Asp Leu Pro Ala Met Gly Phe Leu Lys Thr Ser
2510 2515 2520

Pro Ala Gly Glu Ala His Ser Leu Leu Ala Ser Thr Ile Glu Pro
2525 2530 2535

Ala Thr Ala Phe Thr Pro His Leu Ser Ala Ala Val Val Thr Gly
2540 2545 2550

Ser Ser Ala Thr Ser Glu Ala Ser Leu Leu Thr Thr Ser Glu Ser
2555 2560 2565

Lys Ala Ile His Ser Ser Pro Gln Thr Pro Thr Thr Pro Thr Ser
2570 2575 2580

Gly Ala Asn Trp Glu Thr Ser Ala Thr Pro Glu Ser Leu Leu Val
2585 2590 2595

Val Thr Glu Thr Ser Asp Thr Thr Leu Thr Ser Lys Ile Leu Val
2600 2605 2610

Thr Asp Thr Ile Leu Phe Ser Thr Val Ser Thr Pro Pro Ser Lys
2615 2620 2625

Phe Pro Ser Thr Gly Thr Leu Ser Gly Ala Ser Phe Pro Thr Leu
2630 2635 2640

Leu Pro Asp Thr Pro Ala Ile Pro Leu Thr Ala Thr Glu Pro Thr
2645 2650 2655

Ser Ser Leu Ala Thr Ser Phe Asp Ser Thr Pro Leu Val Thr Ile
2660                2665                2670

Ala Ser Asp Ser Leu Gly Thr Val Pro Glu Thr Thr Leu Thr Met
2675                2680                2685

Ser Glu Thr Ser Asn Gly Asp Ala Leu Val Leu Lys Thr Val Ser
2690                2695                2700

Asn Pro Asp Arg Ser Ile Pro Gly Ile Thr Ile Gln Gly Val Thr
2705                2710                2715

Glu Ser Pro Leu His Pro Ser Ser Thr Ser Pro Ser Lys Ile Val
2720                2725                2730

Ala Pro Arg Asn Thr Thr Tyr Glu Gly Ser Ile Thr Val Ala Leu
2735                2740                2745

Ser Thr Leu Pro Ala Gly Thr Thr Gly Ser Leu Val Phe Ser Gln
2750                2755                2760

Ser Ser Glu Asn Ser Glu Thr Thr Ala Leu Val Asp Ser Ser Ala
2765                2770                2775

Gly Leu Glu Arg Ala Ser Val Met Pro Leu Thr Thr Gly Ser Gln
2780                2785                2790

Gly Met Ala Ser Ser Gly Gly Ile Arg Ser Gly Ser Thr His Ser
2795                2800                2805

Thr Gly Thr Lys Thr Phe Ser Ser Leu Pro Leu Thr Met Asn Pro
2810                2815                2820

Gly Glu Val Thr Ala Met Ser Glu Ile Thr Thr Asn Arg Leu Thr
2825                2830                2835

Ala Thr Gln Ser Thr Ala Pro Lys Gly Ile Pro Val Lys Pro Thr
2840                2845                2850

Ser Ala Glu Ser Gly Leu Leu Thr Pro Val Ser Ala Ser Ser Ser
2855                2860                2865

Pro Ser Lys Ala Phe Ala Ser Leu Thr Thr Ala Pro Pro Ser Thr
2870                2875                2880

Trp Gly Ile Pro Gln Ser Thr Leu Thr Phe Glu Phe Ser Glu Val

```
        2885                    2890                    2895


        Pro Ser  Leu Asp Thr Lys Ser  Ala Ser Leu Pro  Thr  Pro Gly Gln
            2900                2905                2910


        Ser Leu  Asn Thr Ile Pro Asp  Ser Asp Ala Ser  Thr  Ala Ser Ser
            2915                2920                2925


        Ser Leu  Ser Lys Ser Pro Glu  Lys Asn Pro Arg  Ala  Arg Met Met
            2930                2935                2940


        Thr Ser  Thr Lys Ala Ile Ser  Ala Ser Ser Phe  Gln  Ser Thr Gly
            2945                2950                2955


        Phe Thr  Glu Thr Pro Glu Gly  Ser Ala Ser Pro  Ser  Met Ala Gly
            2960                2965                2970


        His Glu  Pro Arg Val Pro Thr  Ser Gly Thr Gly  Asp  Pro Arg Tyr
            2975                2980                2985


        Ala Ser  Glu Ser Met Ser Tyr  Pro Asp Pro Ser  Lys  Ala Ser Ser
            2990                2995                3000


        Ala Met  Thr Ser Thr Ser Leu  Ala Ser Lys Leu  Thr  Thr Leu Phe
            3005                3010                3015


        Ser Thr  Gly Gln Ala Ala Arg  Ser Gly Ser Ser  Ser  Ser Pro Ile
            3020                3025                3030


        Ser Leu  Ser Thr Glu Lys Glu  Thr Ser Phe Leu  Ser  Pro Thr Ala
            3035                3040                3045


        Ser Thr  Ser Arg Lys Thr Ser  Leu Phe Leu Gly  Pro  Ser Met Ala
            3050                3055                3060


        Arg Gln  Pro Asn Ile Leu Val  His Leu Gln Thr  Ser  Ala Leu Thr
            3065                3070                3075


        Leu Ser  Pro Thr Ser Thr Leu  Asn Met Ser Gln  Glu  Glu Pro Pro
            3080                3085                3090


        Glu Leu  Thr Ser Ser Gln Thr  Ile Ala Glu Glu  Glu  Gly Thr Thr
            3095                3100                3105


        Ala Glu  Thr Gln Thr Leu Thr  Phe Thr Pro Ser  Glu  Thr Pro Thr
            3110                3115                3120
```

```
Ser Leu Leu Pro Val Ser Ser Pro Thr Glu Pro Thr Ala Arg Arg
    3125                3130            3135

Lys Ser Ser Pro Glu Thr Trp Ala Ser Ser Ile Ser Val Pro Ala
    3140                3145            3150

Lys Thr Ser Leu Val Glu Thr Thr Asp Gly Thr Leu Val Thr Thr
    3155                3160            3165

Ile Lys Met Ser Ser Gln Ala Ala Gln Gly Asn Ser Thr Trp Pro
    3170                3175            3180

Ala Pro Ala Glu Glu Thr Gly Thr Ser Pro Ala Gly Thr Ser Pro
    3185                3190            3195

Gly Ser Pro Glu Val Ser Thr Thr Leu Lys Ile Met Ser Ser Lys
    3200                3205            3210

Glu Pro Ser Ile Ser Pro Glu Ile Arg Ser Thr Val Arg Asn Ser
    3215                3220            3225

Pro Trp Lys Thr Pro Glu Thr Thr Val Pro Met Glu Thr Thr Val
    3230                3235            3240

Glu Pro Val Thr Leu Gln Ser Thr Ala Leu Gly Ser Gly Ser Thr
    3245                3250            3255

Ser Ile Ser His Leu Pro Thr Gly Thr Thr Ser Pro Thr Lys Ser
    3260                3265            3270

Pro Thr Glu Asn Met Leu Ala Thr Glu Arg Val Ser Leu Ser Pro
    3275                3280            3285

Ser Pro Pro Glu Ala Trp Thr Asn Leu Tyr Ser Gly Thr Pro Gly
    3290                3295            3300

Gly Thr Arg Gln Ser Leu Ala Thr Met Ser Ser Val Ser Leu Glu
    3305                3310            3315

Ser Pro Thr Ala Arg Ser Ile Thr Gly Thr Gly Gln Gln Ser Ser
    3320                3325            3330

Pro Glu Leu Val Ser Lys Thr Thr Gly Met Glu Phe Ser Met Trp
    3335                3340            3345

His Gly Ser Thr Gly Gly Thr Thr Gly Asp Thr His Val Ser Leu
    3350                3355            3360
```

228

```
Ser Thr Ser Ser Asn Ile Leu  Glu Asp Pro Val Thr  Ser Pro Asn
    3365             3370              3375

Ser Val Ser Ser Leu Thr Asp  Lys Ser Lys His Lys  Thr Glu Thr
    3380             3385              3390

Trp Val Ser Thr Thr Ala Ile  Pro Ser Thr Val Leu  Asn Asn Lys
    3395             3400              3405

Ile Met Ala Ala Glu Gln Gln  Thr Ser Arg Ser Val  Asp Glu Ala
    3410             3415              3420

Tyr Ser Ser Thr Ser Ser Trp  Ser Asp Gln Thr Ser  Gly Ser Asp
    3425             3430              3435

Ile Thr Leu Gly Ala Ser Pro  Asp Val Thr Asn Thr  Leu Tyr Ile
    3440             3445              3450

Thr Ser Thr Ala Gln Thr Thr  Ser Leu Val Ser Leu  Pro Ser Gly
    3455             3460              3465

Asp Gln Gly Ile Thr Ser Leu  Thr Asn Pro Ser Gly  Gly Lys Thr
    3470             3475              3480

Ser Ser Ala Ser Ser Val Thr  Ser Pro Ser Ile Gly  Leu Glu Thr
    3485             3490              3495

Leu Arg Ala Asn Val Ser Ala  Val Lys Ser Asp Ile  Ala Pro Thr
    3500             3505              3510

Ala Gly His Leu Ser Gln Thr  Ser Ser Pro Ala Glu  Val Ser Ile
    3515             3520              3525

Leu Asp Val Thr Thr Ala Pro  Thr Pro Gly Ile Ser  Thr Thr Ile
    3530             3535              3540

Thr Thr Met Gly Thr Asn Ser  Ile Ser Thr Thr Thr  Pro Asn Pro
    3545             3550              3555

Glu Val Gly Met Ser Thr Met  Asp Ser Thr Pro Ala  Thr Glu Arg
    3560             3565              3570

Arg Thr Thr Ser Thr Glu His  Pro Ser Thr Trp Ser  Ser Thr Ala
    3575             3580              3585

Ala Ser Asp Ser Trp Thr Val  Thr Asp Met Thr Ser  Asn Leu Lys
    3590             3595              3600
```

```
Val Ala  Arg Ser Pro Gly Thr  Ile Ser Thr Met His  Thr Thr Ser
    3605            3610           3615
```

```
Phe Leu  Ala Ser Ser Thr Glu  Leu Asp Ser Met Ser  Thr Pro His
    3620            3625           3630
```

```
Gly Arg  Ile Thr Val Ile Gly  Thr Ser Leu Val Thr  Pro Ser Ser
    3635            3640           3645
```

```
Asp Ala  Ser Ala Val Lys Thr  Glu Thr Ser Thr Ser  Glu Arg Thr
    3650            3655           3660
```

```
Leu Ser  Pro Ser Asp Thr Thr  Ala Ser Thr Pro Ile  Ser Thr Phe
    3665            3670           3675
```

```
Ser Arg  Val Gln Arg Met Ser  Ile Ser Val Pro Asp  Ile Leu Ser
    3680            3685           3690
```

```
Thr Ser  Trp Thr Pro Ser Ser  Thr Glu Ala Glu Asp  Val Pro Val
    3695            3700           3705
```

```
Ser Met  Val Ser Thr Asp His  Ala Ser Thr Lys Thr  Asp Pro Asn
    3710            3715           3720
```

```
Thr Pro  Leu Ser Thr Phe Leu  Phe Asp Ser Leu Ser  Thr Leu Asp
    3725            3730           3735
```

```
Trp Asp  Thr Gly Arg Ser Leu  Ser Ser Ala Thr Ala  Thr Thr Ser
    3740            3745           3750
```

```
Ala Pro  Gln Gly Ala Thr Thr  Pro Gln Glu Leu Thr  Leu Glu Thr
    3755            3760           3765
```

```
Met Ile  Ser Pro Ala Thr Ser  Gln Leu Pro Phe Ser  Ile Gly His
    3770            3775           3780
```

```
Ile Thr  Ser Ala Val Thr Pro  Ala Ala Met Ala Arg  Ser Ser Gly
    3785            3790           3795
```

```
Val Thr  Phe Ser Arg Pro Asp  Pro Thr Ser Lys Lys  Ala Glu Gln
    3800            3805           3810
```

```
Thr Ser  Thr Gln Leu Pro Thr  Thr Thr Ser Ala His  Pro Gly Gln
    3815            3820           3825
```

```
Val Pro  Arg Ser Ala Ala Thr  Thr Leu Asp Val Ile  Pro His Thr
```

230

3830               3835             3840

Ala Lys Thr Pro Asp Ala Thr Phe Gln Arg Gln Gly Gln Thr Ala
    3845             3850             3855

Leu Thr Thr Glu Ala Arg Ala Thr Ser Asp Ser Trp Asn Glu Lys
    3860             3865             3870

Glu Lys Ser Thr Pro Ser Ala Pro Trp Ile Thr Glu Met Met Asn
    3875             3880             3885

Ser Val Ser Glu Asp Thr Ile Lys Glu Val Thr Ser Ser Ser Ser
    3890             3895             3900

Val Leu Lys Asp Pro Glu Tyr Ala Gly His Lys Leu Gly Ile Trp
    3905             3910             3915

Asp Asp Phe Ile Pro Lys Phe Gly Lys Ala Ala His Met Arg Glu
    3920             3925             3930

Leu Pro Leu Leu Ser Pro Pro Gln Asp Lys Glu Ala Ile His Pro
    3935             3940             3945

Ser Thr Asn Thr Val Glu Thr Thr Gly Trp Val Thr Ser Ser Glu
    3950             3955             3960

His Ala Ser His Ser Thr Ile Pro Ala His Ser Ala Ser Ser Lys
    3965             3970             3975

Leu Thr Ser Pro Val Val Thr Thr Ser Thr Arg Glu Gln Ala Ile
    3980             3985             3990

Val Ser Met Ser Thr Thr Thr Trp Pro Glu Ser Thr Arg Ala Arg
    3995             4000             4005

Thr Glu Pro Asn Ser Phe Leu Thr Ile Glu Leu Arg Asp Val Ser
    4010             4015             4020

Pro Tyr Met Asp Thr Ser Ser Thr Thr Gln Thr Ser Ile Ile Ser
    4025             4030             4035

Ser Pro Gly Ser Thr Ala Ile Thr Lys Gly Pro Arg Thr Glu Ile
    4040             4045             4050

Thr Ser Ser Lys Arg Ile Ser Ser Ser Phe Leu Ala Gln Ser Met
    4055             4060             4065

```
Arg Ser  Ser Asp Ser Pro Ser  Glu Ala Ile Thr Arg  Leu Ser Asn
    4070             4075             4080

Phe Pro  Ala Met Thr Glu Ser  Gly Gly Met Ile Leu  Ala Met Gln
    4085             4090             4095

Thr Ser  Pro Pro Gly Ala Thr  Ser Leu Ser Ala Pro  Thr Leu Asp
    4100             4105             4110

Thr Ser  Ala Thr Ala Ser Trp  Thr Gly Thr Pro Leu  Ala Thr Thr
    4115             4120             4125

Gln Arg  Phe Thr Tyr Ser Glu  Lys Thr Thr Leu Phe  Ser Lys Gly
    4130             4135             4140

Pro Glu  Asp Thr Ser Gln Pro  Ser Pro Pro Ser Val  Glu Glu Thr
    4145             4150             4155

Ser Ser  Ser Ser Ser Leu Val  Pro Ile His Ala Thr  Thr Ser Pro
    4160             4165             4170

Ser Asn  Ile Leu Leu Thr Ser  Gln Gly His Ser Pro  Ser Ser Thr
    4175             4180             4185

Pro Pro  Val Thr Ser Val Phe  Leu Ser Glu Thr Ser  Gly Leu Gly
    4190             4195             4200

Lys Thr  Thr Asp Met Ser Arg  Ile Ser Leu Glu Pro  Gly Thr Ser
    4205             4210             4215

Leu Pro  Pro Asn Leu Ser Ser  Thr Ala Gly Glu Ala  Leu Ser Thr
    4220             4225             4230

Tyr Glu  Ala Ser Arg Asp Thr  Lys Ala Ile His His  Ser Ala Asp
    4235             4240             4245

Thr Ala  Val Thr Asn Met Glu  Ala Thr Ser Ser Glu  Tyr Ser Pro
    4250             4255             4260

Ile Pro  Gly His Thr Lys Pro  Ser Lys Ala Thr Ser  Pro Leu Val
    4265             4270             4275

Thr Ser  His Ile Met Gly Asp  Ile Thr Ser Ser Thr  Ser Val Phe
    4280             4285             4290

Gly Ser  Ser Glu Thr Thr Glu  Ile Glu Thr Val Ser  Ser Val Asn
    4295             4300             4305
```

232

```
Gln Gly Leu Gln Glu Arg Ser   Thr Ser Gln Val Ala   Ser Ser Ala
    4310                4315               4320

Thr Glu Thr Ser Thr Val Ile   Thr His Val Ser Ser   Gly Asp Ala
    4325                4330               4335

Thr Thr His Val Thr Lys Thr   Gln Ala Thr Phe Ser   Ser Gly Thr
    4340                4345               4350

Ser Ile Ser Ser Pro His Gln   Phe Ile Thr Ser Thr   Asn Thr Phe
    4355                4360               4365

Thr Asp Val Ser Thr Asn Pro   Ser Thr Ser Leu Ile   Met Thr Glu
    4370                4375               4380

Ser Ser Gly Val Thr Ile Thr   Thr Gln Thr Gly Pro   Thr Gly Ala
    4385                4390               4395

Ala Thr Gln Gly Pro Tyr Leu   Leu Asp Thr Ser Thr   Met Pro Tyr
    4400                4405               4410

Leu Thr Glu Thr Pro Leu Ala   Val Thr Pro Asp Phe   Met Gln Ser
    4415                4420               4425

Glu Lys Thr Thr Leu Ile Ser   Lys Gly Pro Lys Asp   Val Thr Trp
    4430                4435               4440

Thr Ser Pro Pro Ser Val Ala   Glu Thr Ser Tyr Pro   Ser Ser Leu
    4445                4450               4455

Thr Pro Phe Leu Val Thr Thr   Ile Pro Pro Ala Thr   Ser Thr Leu
    4460                4465               4470

Gln Gly Gln His Thr Ser Ser   Pro Val Ser Ala Thr   Ser Val Leu
    4475                4480               4485

Thr Ser Gly Leu Val Lys Thr   Thr Asp Met Leu Asn   Thr Ser Met
    4490                4495               4500

Glu Pro Val Thr Asn Ser Pro   Gln Asn Leu Asn Asn   Pro Ser Asn
    4505                4510               4515

Glu Ile Leu Ala Thr Leu Ala   Ala Thr Thr Asp Ile   Glu Thr Ile
    4520                4525               4530

His Pro Ser Ile Asn Lys Ala   Val Thr Asn Met Gly   Thr Ala Ser
    4535                4540               4545
```

233

```
Ser Ala His Val Leu His Ser  Thr Leu Pro Val Ser  Ser Glu Pro
    4550            4555            4560

Ser Thr Ala Thr Ser Pro Met  Val Pro Ala Ser Ser  Met Gly Asp
    4565            4570            4575

Ala Leu Ala Ser Ile Ser Ile  Pro Gly Ser Glu Thr  Thr Asp Ile
    4580            4585            4590

Glu Gly Glu Pro Thr Ser Ser  Leu Thr Ala Gly Arg  Lys Glu Asn
    4595            4600            4605

Ser Thr Leu Gln Glu Met Asn  Ser Thr Thr Glu Ser  Asn Ile Ile
    4610            4615            4620

Leu Ser Asn Val Ser Val Gly  Ala Ile Thr Glu Ala  Thr Lys Met
    4625            4630            4635

Glu Val Pro Ser Phe Asp Ala  Thr Phe Ile Pro Thr  Pro Ala Gln
    4640            4645            4650

Ser Thr Lys Phe Pro Asp Ile  Phe Ser Val Ala Ser  Ser Arg Leu
    4655            4660            4665

Ser Asn Ser Pro Pro Met Thr  Ile Ser Thr His Met  Thr Thr Thr
    4670            4675            4680

Gln Thr Gly Ser Ser Gly Ala  Thr Ser Lys Ile Pro  Leu Ala Leu
    4685            4690            4695

Asp Thr Ser Thr Leu Glu Thr  Ser Ala Gly Thr Pro  Ser Val Val
    4700            4705            4710

Thr Glu Gly Phe Ala His Ser  Lys Ile Thr Thr Ala  Met Asn Asn
    4715            4720            4725

Asp Val Lys Asp Val Ser Gln  Thr Asn Pro Pro Phe  Gln Asp Glu
    4730            4735            4740

Ala Ser Ser Pro Ser Ser Gln  Ala Pro Val Leu Val  Thr Thr Leu
    4745            4750            4755

Pro Ser Ser Val Ala Phe Thr  Pro Gln Trp His Ser  Thr Ser Ser
    4760            4765            4770

Pro Val Ser Met Ser Ser Val  Leu Thr Ser Ser Leu  Val Lys Thr
```

4775     4780     4785

Ala Gly Lys Val Asp Thr Ser Leu Glu Thr Val Thr Ser Ser Pro
  4790     4795     4800

Gln Ser Met Ser Asn Thr Leu Asp Asp Ile Ser Val Thr Ser Ala
  4805     4810     4815

Ala Thr Thr Asp Ile Glu Thr Thr His Pro Ser Ile Asn Thr Val
  4820     4825     4830

Val Thr Asn Val Gly Thr Thr Gly Ser Ala Phe Glu Ser His Ser
  4835     4840     4845

Thr Val Ser Ala Tyr Pro Glu Pro Ser Lys Val Thr Ser Pro Asn
  4850     4855     4860

Val Thr Thr Ser Thr Met Glu Asp Thr Thr Ile Ser Arg Ser Ile
  4865     4870     4875

Pro Lys Ser Ser Lys Thr Thr Arg Thr Glu Thr Glu Thr Thr Ser
  4880     4885     4890

Ser Leu Thr Pro Lys Leu Arg Glu Thr Ser Ile Ser Gln Glu Ile
  4895     4900     4905

Thr Ser Ser Thr Glu Thr Ser Thr Val Pro Tyr Lys Glu Leu Thr
  4910     4915     4920

Gly Ala Thr Thr Glu Val Ser Arg Thr Asp Val Thr Ser Ser Ser
  4925     4930     4935

Ser Thr Ser Phe Pro Gly Pro Asp Gln Ser Thr Val Ser Leu Asp
  4940     4945     4950

Ile Ser Thr Glu Thr Asn Thr Arg Leu Ser Thr Ser Pro Ile Met
  4955     4960     4965

Thr Glu Ser Ala Glu Ile Thr Ile Thr Thr Gln Thr Gly Pro His
  4970     4975     4980

Gly Ala Thr Ser Gln Asp Thr Phe Thr Met Asp Pro Ser Asn Thr
  4985     4990     4995

Thr Pro Gln Ala Gly Ile His Ser Ala Met Thr His Gly Phe Ser
  5000     5005     5010

Gln Leu Asp Val Thr Thr Leu Met Ser Arg Ile Pro Gln Asp Val

Ser Trp Thr Ser Pro Pro Ser Val Asp Lys Thr Ser Ser Pro Ser

Ser Phe Leu Ser Ser Pro Ala Met Thr Thr Pro Ser Leu Ile Ser

Ser Thr Leu Pro Glu Asp Lys Leu Ser Ser Pro Met Thr Ser Leu

Leu Thr Ser Gly Leu Val Lys Ile Thr Asp Ile Leu Arg Thr Arg

Leu Glu Pro Val Thr Ser Ser Leu Pro Asn Phe Ser Ser Thr Ser

Asp Lys Ile Leu Ala Thr Ser Lys Asp Ser Lys Asp Thr Lys Glu

Ile Phe Pro Ser Ile Asn Thr Glu Glu Thr Asn Val Lys Ala Asn

Asn Ser Gly His Glu Ser His Ser Pro Ala Leu Ala Asp Ser Glu

Thr Pro Lys Ala Thr Thr Gln Met Val Ile Thr Thr Thr Val Gly

Asp Pro Ala Pro Ser Thr Ser Met Pro Val His Gly Ser Ser Glu

Thr Thr Asn Ile Lys Arg Glu Pro Thr Tyr Phe Leu Thr Pro Arg

Leu Arg Glu Thr Ser Thr Ser Gln Glu Ser Ser Phe Pro Thr Asp

Thr Ser Phe Leu Leu Ser Lys Val Pro Thr Gly Thr Ile Thr Glu

Val Ser Ser Thr Gly Val Asn Ser Ser Ser Lys Ile Ser Thr Pro

Asp His Asp Lys Ser Thr Val Pro Pro Asp Thr Phe Thr Gly Glu

```
Ile Pro  Arg Val Phe Thr Ser  Ser Ile Lys Thr Lys  Ser Ala Glu
    5255             5260             5265

Met Thr  Ile Thr Thr Gln Ala  Ser Pro Pro Glu Ser  Ala Ser His
    5270             5275             5280

Ser Thr  Leu Pro Leu Asp Thr  Ser Thr Thr Leu Ser  Gln Gly Gly
    5285             5290             5295

Thr His  Ser Thr Val Thr Gln  Gly Phe Pro Tyr Ser  Glu Val Thr
    5300             5305             5310

Thr Leu  Met Gly Met Gly Pro  Gly Asn Val Ser Trp  Met Thr Thr
    5315             5320             5325

Pro Pro  Val Glu Glu Thr Ser  Ser Val Ser Ser Leu  Met Ser Ser
    5330             5335             5340

Pro Ala  Met Thr Ser Pro Ser  Pro Val Ser Ser Thr  Ser Pro Gln
    5345             5350             5355

Ser Ile  Pro Ser Ser Pro Leu  Pro Val Thr Ala Leu  Pro Thr Ser
    5360             5365             5370

Val Leu  Val Thr Thr Thr Asp  Val Leu Gly Thr Thr  Ser Pro Glu
    5375             5380             5385

Ser Val  Thr Ser Ser Pro Pro  Asn Leu Ser Ser Ile  Thr His Glu
    5390             5395             5400

Arg Pro  Ala Thr Tyr Lys Asp  Thr Ala His Thr Glu  Ala Ala Met
    5405             5410             5415

His His  Ser Thr Asn Thr Ala  Val Thr Asn Val Gly  Thr Ser Gly
    5420             5425             5430

Ser Gly  His Lys Ser Gln Ser  Ser Val Leu Ala Asp  Ser Glu Thr
    5435             5440             5445

Ser Lys  Ala Thr Pro Leu Met  Ser Thr Thr Ser Thr  Leu Gly Asp
    5450             5455             5460

Thr Ser  Val Ser Thr Ser Thr  Pro Asn Ile Ser Gln  Thr Asn Gln
    5465             5470             5475

Ile Gln  Thr Glu Pro Thr Ala  Ser Leu Ser Pro Arg  Leu Arg Glu
    5480             5485             5490
```

237

```
Ser Ser Thr Ser Glu Lys Thr   Ser Ser Thr Thr Glu   Thr Asn Thr
    5495                5500                5505

Ala Phe Ser Tyr Val Pro Thr   Gly Ala Ile Thr Gln   Ala Ser Arg
    5510                5515                5520

Thr Glu Ile Ser Ser Ser Arg   Thr Ser Ile Ser Asp   Leu Asp Arg
    5525                5530                5535

Pro Thr Ile Ala Pro Asp Ile   Ser Thr Gly Met Ile   Thr Arg Leu
    5540                5545                5550

Phe Thr Ser Pro Ile Met Thr   Lys Ser Ala Glu Met   Thr Val Thr
    5555                5560                5565

Thr Gln Thr Thr Thr Pro Gly   Ala Thr Ser Gln Gly   Ile Leu Pro
    5570                5575                5580

Trp Asp Thr Ser Thr Thr Leu   Phe Gln Gly Gly Thr   His Ser Thr
    5585                5590                5595

Val Ser Gln Gly Phe Pro His   Ser Glu Ile Thr Thr   Leu Arg Ser
    5600                5605                5610

Arg Thr Pro Gly Asp Val Ser   Trp Met Thr Thr Pro   Pro Val Glu
    5615                5620                5625

Glu Thr Ser Ser Gly Phe Ser   Leu Met Ser Pro Ser   Met Thr Ser
    5630                5635                5640

Pro Ser Pro Val Ser Ser Thr   Ser Pro Glu Ser Ile   Pro Ser Ser
    5645                5650                5655

Pro Leu Pro Val Thr Ala Leu   Leu Thr Ser Val Leu   Val Thr Thr
    5660                5665                5670

Thr Asn Val Leu Gly Thr Thr   Ser Pro Glu Thr Val   Thr Ser Ser
    5675                5680                5685

Pro Pro Asn Leu Ser Ser Pro   Thr Gln Glu Arg Leu   Thr Thr Tyr
    5690                5695                5700

Lys Asp Thr Ala His Thr Glu   Ala Met His Ala Ser   Met His Thr
    5705                5710                5715

Asn Thr Ala Val Ala Asn Val   Gly Thr Ser Ile Ser   Gly His Glu
```

5720                     5725                     5730

Ser Gln Ser Ser Val Pro Ala Asp Ser His Thr Ser Lys Ala Thr
        5735            5740            5745

Ser Pro Met Gly Ile Thr Phe Ala Met Gly Asp Thr Ser Val Ser
        5750            5755            5760

Thr Ser Thr Pro Ala Phe Phe Glu Thr Arg Ile Gln Thr Glu Ser
        5765            5770            5775

Thr Ser Ser Leu Ile Pro Gly Leu Arg Asp Thr Arg Thr Ser Glu
        5780            5785            5790

Glu Ile Asn Thr Val Thr Glu Thr Ser Thr Val Leu Ser Glu Val
        5795            5800            5805

Pro Thr Thr Thr Thr Thr Glu Val Ser Arg Thr Glu Val Ile Thr
        5810            5815            5820

Ser Ser Arg Thr Thr Ile Ser Gly Pro Asp His Ser Lys Met Ser
        5825            5830            5835

Pro Tyr Ile Ser Thr Glu Thr Ile Thr Arg Leu Ser Thr Phe Pro
        5840            5845            5850

Phe Val Thr Gly Ser Thr Glu Met Ala Ile Thr Asn Gln Thr Gly
        5855            5860            5865

Pro Ile Gly Thr Ile Ser Gln Ala Thr Leu Thr Leu Asp Thr Ser
        5870            5875            5880

Ser Thr Ala Ser Trp Glu Gly Thr His Ser Pro Val Thr Gln Arg
        5885            5890            5895

Phe Pro His Ser Glu Glu Thr Thr Thr Met Ser Arg Ser Thr Lys
        5900            5905            5910

Gly Val Ser Trp Gln Ser Pro Pro Ser Val Glu Glu Thr Ser Ser
        5915            5920            5925

Pro Ser Ser Pro Val Pro Leu Pro Ala Ile Thr Ser His Ser Ser
        5930            5935            5940

Leu Tyr Ser Ala Val Ser Gly Ser Ser Pro Thr Ser Ala Leu Pro
        5945            5950            5955

```
Val Thr Ser Leu Leu Thr Ser   Gly Arg Arg Lys Thr   Ile Asp Met
    5960                5965             5970

Leu Asp Thr His Ser Glu Leu   Val Thr Ser Ser Leu   Pro Ser Ala
    5975                5980             5985

Ser Ser Phe Ser Gly Glu Ile   Leu Thr Ser Glu Ala   Ser Thr Asn
    5990                5995             6000

Thr Glu Thr Ile His Phe Ser   Glu Asn Thr Ala Glu   Thr Asn Met
    6005                6010             6015

Gly Thr Thr Asn Ser Met His   Lys Leu His Ser Ser   Val Ser Ile
    6020                6025             6030

His Ser Gln Pro Ser Gly His   Thr Pro Pro Lys Val   Thr Gly Ser
    6035                6040             6045

Met Met Glu Asp Ala Ile Val   Ser Thr Ser Thr Pro   Gly Ser Pro
    6050                6055             6060

Glu Thr Lys Asn Val Asp Arg   Asp Ser Thr Ser Pro   Leu Thr Pro
    6065                6070             6075

Glu Leu Lys Glu Asp Ser Thr   Ala Leu Val Met Asn   Ser Thr Thr
    6080                6085             6090

Glu Ser Asn Thr Val Phe Ser   Ser Val Ser Leu Asp   Ala Ala Thr
    6095                6100             6105

Glu Val Ser Arg Ala Glu Val   Thr Tyr Tyr Asp Pro   Thr Phe Met
    6110                6115             6120

Pro Ala Ser Ala Gln Ser Thr   Lys Ser Pro Asp Ile   Ser Pro Glu
    6125                6130             6135

Ala Ser Ser Ser His Ser Asn   Ser Pro Pro Leu Thr   Ile Ser Thr
    6140                6145             6150

His Lys Thr Ile Ala Thr Gln   Thr Gly Pro Ser Gly   Val Thr Ser
    6155                6160             6165

Leu Gly Gln Leu Thr Leu Asp   Thr Ser Thr Ile Ala   Thr Ser Ala
    6170                6175             6180

Gly Thr Pro Ser Ala Arg Thr   Gln Asp Phe Val Asp   Ser Glu Thr
    6185                6190             6195
```

```
Thr Ser Val Met Asn Asn Asp  Leu Asn Asp Val Leu  Lys Thr Ser
    6200             6205             6210

Pro Phe Ser Ala Glu Glu Ala  Asn Ser Leu Ser Ser  Gln Ala Pro
    6215             6220             6225

Leu Leu Val Thr Thr Ser Pro  Ser Pro Val Thr Ser  Thr Leu Gln
    6230             6235             6240

Glu His Ser Thr Ser Ser Leu  Val Ser Val Thr Ser  Val Pro Thr
    6245             6250             6255

Pro Thr Leu Ala Lys Ile Thr  Asp Met Asp Thr Asn  Leu Glu Pro
    6260             6265             6270

Val Thr Arg Ser Pro Gln Asn  Leu Arg Asn Thr Leu  Ala Thr Ser
    6275             6280             6285

Glu Ala Thr Thr Asp Thr His  Thr Met His Pro Ser  Ile Asn Thr
    6290             6295             6300

Ala Met Ala Asn Val Gly Thr  Thr Ser Ser Pro Asn  Glu Phe Tyr
    6305             6310             6315

Phe Thr Val Ser Pro Asp Ser  Asp Pro Tyr Lys Ala  Thr Ser Ala
    6320             6325             6330

Val Val Ile Thr Ser Thr Ser  Gly Asp Ser Ile Val  Ser Thr Ser
    6335             6340             6345

Met Pro Arg Ser Ser Ala Met  Lys Lys Ile Glu Ser  Glu Thr Thr
    6350             6355             6360

Phe Ser Leu Ile Phe Arg Leu  Arg Glu Thr Ser Thr  Ser Gln Lys
    6365             6370             6375

Ile Gly Ser Ser Ser Asp Thr  Ser Thr Val Phe Asp  Lys Ala Phe
    6380             6385             6390

Thr Ala Ala Thr Thr Glu Val  Ser Arg Thr Glu Leu  Thr Ser Ser
    6395             6400             6405

Ser Arg Thr Ser Ile Gln Gly  Thr Glu Lys Pro Thr  Met Ser Pro
    6410             6415             6420

Asp Thr Ser Thr Arg Ser Val  Thr Met Leu Ser Thr  Phe Ala Gly
    6425             6430             6435
```

241

```
Leu Thr   Lys Ser Glu Glu Arg   Thr Ile Ala Thr Gln   Thr Gly Pro
    6440              6445                6450

His Arg   Ala Thr Ser Gln Gly   Thr Leu Thr Trp Asp   Thr Ser Ile
    6455              6460                6465

Thr Thr   Ser Gln Ala Gly Thr   His Ser Ala Met Thr   His Gly Phe
    6470              6475                6480

Ser Gln   Leu Asp Leu Ser Thr   Leu Thr Ser Arg Val   Pro Glu Tyr
    6485              6490                6495

Ile Ser   Gly Thr Ser Pro Pro   Ser Val Glu Lys Thr   Ser Ser Ser
    6500              6505                6510

Ser Ser   Leu Leu Ser Leu Pro   Ala Ile Thr Ser Pro   Ser Pro Val
    6515              6520                6525

Pro Thr   Thr Leu Pro Glu Ser   Arg Pro Ser Ser Pro   Val His Leu
    6530              6535                6540

Thr Ser   Leu Pro Thr Ser Gly   Leu Val Lys Thr Thr   Asp Met Leu
    6545              6550                6555

Ala Ser   Val Ala Ser Leu Pro   Pro Asn Leu Gly Ser   Thr Ser His
    6560              6565                6570

Lys Ile   Pro Thr Thr Ser Glu   Asp Ile Lys Asp Thr   Glu Lys Met
    6575              6580                6585

Tyr Pro   Ser Thr Asn Ile Ala   Val Thr Asn Val Gly   Thr Thr Thr
    6590              6595                6600

Ser Glu   Lys Glu Ser Tyr Ser   Ser Val Pro Ala Tyr   Ser Glu Pro
    6605              6610                6615

Pro Lys   Val Thr Ser Pro Met   Val Thr Ser Phe Asn   Ile Arg Asp
    6620              6625                6630

Thr Ile   Val Ser Thr Ser Met   Pro Gly Ser Ser Glu   Ile Thr Arg
    6635              6640                6645

Ile Glu   Met Glu Ser Thr Phe   Ser Val Ala His Gly   Leu Lys Gly
    6650              6655                6660

Thr Ser   Thr Ser Gln Asp Pro   Ile Val Ser Thr Glu   Lys Ser Ala
```

6665 6670 6675

Val Leu His Lys Leu Thr Thr Gly Ala Thr Glu Thr Ser Arg Thr
6680 6685 6690

Glu Val Ala Ser Ser Arg Arg Thr Ser Ile Pro Gly Pro Asp His
6695 6700 6705

Ser Thr Glu Ser Pro Asp Ile Ser Thr Glu Val Ile Pro Ser Leu
6710 6715 6720

Pro Ile Ser Leu Gly Ile Thr Glu Ser Ser Asn Met Thr Ile Ile
6725 6730 6735

Thr Arg Thr Gly Pro Pro Leu Gly Ser Thr Ser Gln Gly Thr Phe
6740 6745 6750

Thr Leu Asp Thr Pro Thr Thr Ser Ser Arg Ala Gly Thr His Ser
6755 6760 6765

Met Ala Thr Gln Glu Phe Pro His Ser Glu Met Thr Thr Val Met
6770 6775 6780

Asn Lys Asp Pro Glu Ile Leu Ser Trp Thr Ile Pro Pro Ser Ile
6785 6790 6795

Glu Lys Thr Ser Phe Ser Ser Ser Leu Met Pro Ser Pro Ala Met
6800 6805 6810

Thr Ser Pro Pro Val Ser Ser Thr Leu Pro Lys Thr Ile His Thr
6815 6820 6825

Thr Pro Ser Pro Met Thr Ser Leu Leu Thr Pro Ser Leu Val Met
6830 6835 6840

Thr Thr Asp Thr Leu Gly Thr Ser Pro Glu Pro Thr Thr Ser Ser
6845 6850 6855

Pro Pro Asn Leu Ser Ser Thr Ser His Val Ile Leu Thr Thr Asp
6860 6865 6870

Glu Asp Thr Thr Ala Ile Glu Ala Met His Pro Ser Thr Ser Thr
6875 6880 6885

Ala Ala Thr Asn Val Glu Thr Thr Cys Ser Gly His Gly Ser Gln
6890 6895 6900

```
Ser Ser Val Leu Thr Asp Ser Glu Lys Thr Lys Ala Thr Ala Pro
    6905                6910              6915

Met Asp Thr Thr Ser Thr Met Gly His Thr Thr Val Ser Thr Ser
    6920                6925              6930

Met Ser Val Ser Ser Glu Thr Thr Lys Ile Lys Arg Glu Ser Thr
    6935                6940              6945

Tyr Ser Leu Thr Pro Gly Leu Arg Glu Thr Ser Ile Ser Gln Asn
    6950                6955              6960

Ala Ser Phe Ser Thr Asp Thr Ser Ile Val Leu Ser Glu Val Pro
    6965                6970              6975

Thr Gly Thr Thr Ala Glu Val Ser Arg Thr Glu Val Thr Ser Ser
    6980                6985              6990

Gly Arg Thr Ser Ile Pro Gly Pro Ser Gln Ser Thr Val Leu Pro
    6995                7000              7005

Glu Ile Ser Thr Arg Thr Met Thr Arg Leu Phe Ala Ser Pro Thr
    7010                7015              7020

Met Thr Glu Ser Ala Glu Met Thr Ile Pro Thr Gln Thr Gly Pro
    7025                7030              7035

Ser Gly Ser Thr Ser Gln Asp Thr Leu Thr Leu Asp Thr Ser Thr
    7040                7045              7050

Thr Lys Ser Gln Ala Lys Thr His Ser Thr Leu Thr Gln Arg Phe
    7055                7060              7065

Pro His Ser Glu Met Thr Thr Leu Met Ser Arg Gly Pro Gly Asp
    7070                7075              7080

Met Ser Trp Gln Ser Ser Pro Ser Leu Glu Asn Pro Ser Ser Leu
    7085                7090              7095

Pro Ser Leu Leu Ser Leu Pro Ala Thr Thr Ser Pro Pro Pro Ile
    7100                7105              7110

Ser Ser Thr Leu Pro Val Thr Ile Ser Ser Ser Pro Leu Pro Val
    7115                7120              7125

Thr Ser Leu Leu Thr Ser Ser Pro Val Thr Thr Thr Asp Met Leu
    7130                7135              7140
```

244

```
His Thr Ser Pro Glu Leu Val   Thr Ser Ser Pro Pro   Lys Leu Ser
    7145             7150                   7155

His Thr Ser Asp Glu Arg Leu   Thr Thr Gly Lys Asp   Thr Thr Asn
    7160             7165                   7170

Thr Glu Ala Val His Pro Ser   Thr Asn Thr Ala Ala   Ser Asn Val
    7175             7180                   7185

Glu Ile Pro Ser Phe Gly His   Glu Ser Pro Ser Ser   Ala Leu Ala
    7190             7195                   7200

Asp Ser Glu Thr Ser Lys Ala   Thr Ser Pro Met Phe   Ile Thr Ser
    7205             7210                   7215

Thr Gln Glu Asp Thr Thr Val   Ala Ile Ser Thr Pro   His Phe Leu
    7220             7225                   7230

Glu Thr Ser Arg Ile Gln Lys   Glu Ser Ile Ser Ser   Leu Ser Pro
    7235             7240                   7245

Lys Leu Arg Glu Thr Gly Ser   Ser Val Glu Thr Ser   Ser Ala Ile
    7250             7255                   7260

Glu Thr Ser Ala Val Leu Ser   Glu Val Ser Ile Gly   Ala Thr Thr
    7265             7270                   7275

Glu Ile Ser Arg Thr Glu Val   Thr Ser Ser Ser Arg   Thr Ser Ile
    7280             7285                   7290

Ser Gly Ser Ala Glu Ser Thr   Met Leu Pro Glu Ile   Ser Thr Thr
    7295             7300                   7305

Arg Lys Ile Ile Lys Phe Pro   Thr Ser Pro Ile Leu   Ala Glu Ser
    7310             7315                   7320

Ser Glu Met Thr Ile Lys Thr   Gln Thr Ser Pro Pro   Gly Ser Thr
    7325             7330                   7335

Ser Glu Ser Thr Phe Thr Leu   Asp Thr Ser Thr Thr   Pro Ser Leu
    7340             7345                   7350

Val Ile Thr His Ser Thr Met   Thr Gln Arg Leu Pro   His Ser Glu
    7355             7360                   7365

Ile Thr Thr Leu Val Ser Arg   Gly Ala Gly Asp Val   Pro Arg Pro
    7370             7375                   7380
```

245

```
Ser Ser  Leu Pro Val Glu Glu  Thr Ser Pro Pro Ser  Ser Gln Leu
    7385             7390              7395

Ser Leu  Ser Ala Met Ile Ser  Pro Ser Pro Val Ser  Ser Thr Leu
    7400             7405              7410

Pro Ala  Ser Ser His Ser Ser  Ser Ala Ser Val Thr  Ser Pro Leu
    7415             7420              7425

Thr Pro  Gly Gln Val Lys Thr  Thr Glu Val Leu Asp  Ala Ser Ala
    7430             7435              7440

Glu Pro  Glu Thr Ser Ser Pro  Pro Ser Leu Ser Ser  Thr Ser Val
    7445             7450              7455

Glu Ile  Leu Ala Thr Ser Glu  Val Thr Thr Asp Thr  Glu Lys Ile
    7460             7465              7470

His Pro  Phe Pro Asn Thr Ala  Val Thr Lys Val Gly  Thr Ser Ser
    7475             7480              7485

Ser Gly  His Glu Ser Pro Ser  Ser Val Leu Pro Asp  Ser Glu Thr
    7490             7495              7500

Thr Lys  Ala Thr Ser Ala Met  Gly Thr Ile Ser Ile  Met Gly Asp
    7505             7510              7515

Thr Ser  Val Ser Thr Leu Thr  Pro Ala Leu Ser Asn  Thr Arg Lys
    7520             7525              7530

Ile Gln  Ser Glu Pro Ala Ser  Ser Leu Thr Thr Arg  Leu Arg Glu
    7535             7540              7545

Thr Ser  Thr Ser Glu Glu Thr  Ser Leu Ala Thr Glu  Ala Asn Thr
    7550             7555              7560

Val Leu  Ser Lys Val Ser Thr  Gly Ala Thr Thr Glu  Val Ser Arg
    7565             7570              7575

Thr Glu  Ala Ile Ser Phe Ser  Arg Thr Ser Met Ser  Gly Pro Glu
    7580             7585              7590

Gln Ser  Thr Met Ser Gln Asp  Ile Ser Ile Gly Thr  Ile Pro Arg
    7595             7600              7605

Ile Ser  Ala Ser Ser Val Leu  Thr Glu Ser Ala Lys  Met Thr Ile
```

246

```
        7610                    7615                        7620


Thr Thr Gln Thr Gly Pro Ser  Glu Ser Thr Leu Glu  Ser Thr Leu
        7625                    7630                        7635


Asn Leu Asn Thr Ala Thr Thr  Pro Ser Trp Val Glu  Thr His Ser
        7640                    7645                        7650


Ile Val Ile Gln Gly Phe Pro  His Pro Glu Met Thr  Thr Ser Met
        7655                    7660                        7665


Gly Arg Gly Pro Gly Gly Val  Ser Trp Pro Ser Pro  Pro Phe Val
        7670                    7675                        7680


Lys Glu Thr Ser Pro Pro Ser  Ser Pro Leu Ser Leu  Pro Ala Val
        7685                    7690                        7695


Thr Ser Pro His Pro Val Ser  Thr Thr Phe Leu Ala  His Ile Pro
        7700                    7705                        7710


Pro Ser Pro Leu Pro Val Thr  Ser Leu Leu Thr Ser  Gly Pro Ala
        7715                    7720                        7725


Thr Thr Thr Asp Ile Leu Gly  Thr Ser Thr Glu Pro  Gly Thr Ser
        7730                    7735                        7740


Ser Ser Ser Ser Leu Ser Thr  Thr Ser His Glu Arg  Leu Thr Thr
        7745                    7750                        7755


Tyr Lys Asp Thr Ala His Thr  Glu Ala Val His Pro  Ser Thr Asn
        7760                    7765                        7770


Thr Gly Gly Thr Asn Val Ala  Thr Thr Ser Ser Gly  Tyr Lys Ser
        7775                    7780                        7785


Gln Ser Ser Val Leu Ala Asp  Ser Ser Pro Met Cys  Thr Thr Ser
        7790                    7795                        7800


Thr Met Gly Asp Thr Ser Val  Leu Thr Ser Thr Pro  Ala Phe Leu
        7805                    7810                        7815


Glu Thr Arg Arg Ile Gln Thr  Glu Leu Ala Ser Ser  Leu Thr Pro
        7820                    7825                        7830


Gly Leu Arg Glu Ser Ser Gly  Ser Glu Gly Thr Ser  Ser Gly Thr
        7835                    7840                        7845
```

```
Lys Met Ser Thr Val Leu Ser   Lys Val Pro Thr Gly   Ala Thr Thr
    7850                7855                 7860

Glu Ile Ser Lys Glu Asp Val   Thr Ser Ile Pro Gly   Pro Ala Gln
    7865                7870                 7875

Ser Thr Ile Ser Pro Asp Ile   Ser Thr Arg Thr Val   Ser Trp Phe
    7880                7885                 7890

Ser Thr Ser Pro Val Met Thr   Glu Ser Ala Glu Ile   Thr Met Asn
    7895                7900                 7905

Thr His Thr Ser Pro Leu Gly   Ala Thr Thr Gln Gly   Thr Ser Thr
    7910                7915                 7920

Leu Ala Thr Ser Ser Thr Thr   Ser Leu Thr Met Thr   His Ser Thr
    7925                7930                 7935

Ile Ser Gln Gly Phe Ser His   Ser Gln Met Ser Thr   Leu Met Arg
    7940                7945                 7950

Arg Gly Pro Glu Asp Val Ser   Trp Met Ser Pro Pro   Leu Leu Glu
    7955                7960                 7965

Lys Thr Arg Pro Ser Phe Ser   Leu Met Ser Ser Pro   Ala Thr Thr
    7970                7975                 7980

Ser Pro Ser Pro Val Ser Ser   Thr Leu Pro Glu Ser   Ile Ser Ser
    7985                7990                 7995

Ser Pro Leu Pro Val Thr Ser   Leu Leu Thr Ser Gly   Leu Ala Lys
    8000                8005                 8010

Thr Thr Asp Met Leu His Lys   Ser Ser Glu Pro Val   Thr Asn Ser
    8015                8020                 8025

Pro Ala Asn Leu Ser Ser Thr   Ser Val Glu Ile Leu   Ala Thr Ser
    8030                8035                 8040

Glu Val Thr Thr Asp Thr Glu   Lys Thr His Pro Ser   Ser Asn Arg
    8045                8050                 8055

Thr Val Thr Asp Val Gly Thr   Ser Ser Ser Gly His   Glu Ser Thr
    8060                8065                 8070

Ser Phe Val Leu Ala Asp Ser   Gln Thr Ser Lys Val   Thr Ser Pro
    8075                8080                 8085
```

248

```
Met Val Ile Thr Ser Thr Met  Glu Asp Thr Ser Val  Ser Thr Ser
    8090                8095                8100

Thr Pro Gly Phe Phe Glu Thr  Ser Arg Ile Gln Thr  Glu Pro Thr
    8105                8110                8115

Ser Ser Leu Thr Leu Gly Leu  Arg Lys Thr Ser Ser  Ser Glu Gly
    8120                8125                8130

Thr Ser Leu Ala Thr Glu Met  Ser Thr Val Leu Ser  Gly Val Pro
    8135                8140                8145

Thr Gly Ala Thr Ala Glu Val  Ser Arg Thr Glu Val  Thr Ser Ser
    8150                8155                8160

Ser Arg Thr Ser Ile Ser Gly  Phe Ala Gln Leu Thr  Val Ser Pro
    8165                8170                8175

Glu Thr Ser Thr Glu Thr Ile  Thr Arg Leu Pro Thr  Ser Ser Ile
    8180                8185                8190

Met Thr Glu Ser Ala Glu Met  Met Ile Lys Thr Gln  Thr Asp Pro
    8195                8200                8205

Pro Gly Ser Thr Pro Glu Ser  Thr His Thr Val Asp  Ile Ser Thr
    8210                8215                8220

Thr Pro Asn Trp Val Glu Thr  His Ser Thr Val Thr  Gln Arg Phe
    8225                8230                8235

Ser His Ser Glu Met Thr Thr  Leu Val Ser Arg Ser  Pro Gly Asp
    8240                8245                8250

Met Leu Trp Pro Ser Gln Ser  Ser Val Glu Glu Thr  Ser Ser Ala
    8255                8260                8265

Ser Ser Leu Leu Ser Leu Pro  Ala Thr Thr Ser Pro  Ser Pro Val
    8270                8275                8280

Ser Ser Thr Leu Val Glu Asp  Phe Pro Ser Ala Ser  Leu Pro Val
    8285                8290                8295

Thr Ser Leu Leu Thr Pro Gly  Leu Val Ile Thr Thr  Asp Arg Met
    8300                8305                8310

Gly Ile Ser Arg Glu Pro Gly  Thr Ser Ser Thr Ser  Asn Leu Ser
    8315                8320                8325
```

```
Ser Thr  Ser His Glu Arg Leu  Thr Thr Leu Glu Asp  Thr Val Asp
    8330                 8335                 8340


Thr Glu  Asp Met Gln Pro Ser  Thr His Thr Ala Val  Thr Asn Val
    8345                 8350                 8355


Arg Thr  Ser Ile Ser Gly His  Glu Ser Gln Ser Ser  Val Leu Ser
    8360                 8365                 8370


Asp Ser  Glu Thr Pro Lys Ala  Thr Ser Pro Met Gly  Thr Thr Tyr
    8375                 8380                 8385


Thr Met  Gly Glu Thr Ser Val  Ser Ile Ser Thr Ser  Asp Phe Phe
    8390                 8395                 8400


Glu Thr  Ser Arg Ile Gln Ile  Glu Pro Thr Ser Ser  Leu Thr Ser
    8405                 8410                 8415


Gly Leu  Arg Glu Thr Ser Ser  Ser Glu Arg Ile Ser  Ser Ala Thr
    8420                 8425                 8430


Glu Gly  Ser Thr Val Leu Ser  Glu Val Pro Ser Gly  Ala Thr Thr
    8435                 8440                 8445


Glu Val  Ser Arg Thr Glu Val  Ile Ser Ser Arg Gly  Thr Ser Met
    8450                 8455                 8460


Ser Gly  Pro Asp Gln Phe Thr  Ile Ser Pro Asp Ile  Ser Thr Glu
    8465                 8470                 8475


Ala Ile  Thr Arg Leu Ser Thr  Ser Pro Ile Met Thr  Glu Ser Ala
    8480                 8485                 8490


Glu Ser  Ala Ile Thr Ile Glu  Thr Gly Ser Pro Gly  Ala Thr Ser
    8495                 8500                 8505


Glu Gly  Thr Leu Thr Leu Asp  Thr Ser Thr Thr Thr  Phe Trp Ser
    8510                 8515                 8520


Gly Thr  His Ser Thr Ala Ser  Pro Gly Phe Ser His  Ser Glu Met
    8525                 8530                 8535


Thr Thr  Leu Met Ser Arg Thr  Pro Gly Asp Val Pro  Trp Pro Ser
    8540                 8545                 8550


Leu Pro  Ser Val Glu Glu Ala  Ser Ser Val Ser Ser  Ser Leu Ser
```

250

```
                 8555                        8560                        8565


         Ser Pro  Ala Met Thr Ser Thr  Ser Phe Phe Ser Ala  Leu Pro Glu
             8570                 8575                 8580

         Ser Ile  Ser Ser Ser Pro His  Pro Val Thr Ala Leu  Leu Thr Leu
             8585                 8590                 8595

         Gly Pro  Val Lys Thr Thr Asp  Met Leu Arg Thr Ser  Ser Glu Pro
             8600                 8605                 8610

         Glu Thr  Ser Ser Pro Pro Asn  Leu Ser Ser Thr Ser  Ala Glu Ile
             8615                 8620                 8625

         Leu Ala  Thr Ser Glu Val Thr  Lys Asp Arg Glu Lys  Ile His Pro
             8630                 8635                 8640

         Ser Ser  Asn Thr Pro Val Val  Asn Val Gly Thr Val  Ile Tyr Lys
             8645                 8650                 8655

         His Leu  Ser Pro Ser Ser Val  Leu Ala Asp Leu Val  Thr Thr Lys
             8660                 8665                 8670

         Pro Thr  Ser Pro Met Ala Thr  Thr Ser Thr Leu Gly  Asn Thr Ser
             8675                 8680                 8685

         Val Ser  Thr Ser Thr Pro Ala  Phe Pro Glu Thr Met  Met Thr Gln
             8690                 8695                 8700

         Pro Thr  Ser Ser Leu Thr Ser  Gly Leu Arg Glu Ile  Ser Thr Ser
             8705                 8710                 8715

         Gln Glu  Thr Ser Ser Ala Thr  Glu Arg Ser Ala Ser  Leu Ser Gly
             8720                 8725                 8730

         Met Pro  Thr Gly Ala Thr Thr  Lys Val Ser Arg Thr  Glu Ala Leu
             8735                 8740                 8745

         Ser Leu  Gly Arg Thr Ser Thr  Pro Gly Pro Ala Gln  Ser Thr Ile
             8750                 8755                 8760

         Ser Pro  Glu Ile Ser Thr Glu  Thr Ile Thr Arg Ile  Ser Thr Pro
             8765                 8770                 8775

         Leu Thr  Thr Thr Gly Ser Ala  Glu Met Thr Ile Thr  Pro Lys Thr
             8780                 8785                 8790
```

```
Gly His Ser Gly Ala Ser Ser  Gln Gly Thr Phe Thr  Leu Asp Thr
    8795             8800             8805

Ser Ser Arg Ala Ser Trp Pro  Gly Thr His Ser Ala  Ala Thr His
    8810             8815             8820

Arg Ser Pro His Ser Gly Met  Thr Thr Pro Met Ser  Arg Gly Pro
    8825             8830             8835

Glu Asp Val Ser Trp Pro Ser  Arg Pro Ser Val Glu  Lys Thr Ser
    8840             8845             8850

Pro Pro Ser Ser Leu Val Ser  Leu Ser Ala Val Thr  Ser Pro Ser
    8855             8860             8865

Pro Leu Tyr Ser Thr Pro Ser  Glu Ser Ser His Ser  Ser Pro Leu
    8870             8875             8880

Arg Val Thr Ser Leu Phe Thr  Pro Val Met Met Lys  Thr Thr Asp
    8885             8890             8895

Met Leu Asp Thr Ser Leu Glu  Pro Val Thr Thr Ser  Pro Pro Ser
    8900             8905             8910

Met Asn Ile Thr Ser Asp Glu  Ser Leu Ala Thr Ser  Lys Ala Thr
    8915             8920             8925

Met Glu Thr Glu Ala Ile Gln  Leu Ser Glu Asn Thr  Ala Val Thr
    8930             8935             8940

Gln Met Gly Thr Ile Ser Ala  Arg Gln Glu Phe Tyr  Ser Ser Tyr
    8945             8950             8955

Pro Gly Leu Pro Glu Pro Ser  Lys Val Thr Ser Pro  Val Val Thr
    8960             8965             8970

Ser Ser Thr Ile Lys Asp Ile  Val Ser Thr Thr Ile  Pro Ala Ser
    8975             8980             8985

Ser Glu Ile Thr Arg Ile Glu  Met Glu Ser Thr Ser  Thr Leu Thr
    8990             8995             9000

Pro Thr Pro Arg Glu Thr Ser  Thr Ser Gln Glu Ile  His Ser Ala
    9005             9010             9015

Thr Lys Pro Ser Thr Val Pro  Tyr Lys Ala Leu Thr  Ser Ala Thr
    9020             9025             9030
```

252

```
Ile Glu Asp Ser Met Thr Gln  Val Met Ser Ser Ser  Arg Gly Pro
    9035                9040            9045

Ser Pro Asp Gln Ser Thr Met  Ser Gln Asp Ile Ser  Ser Glu Val
    9050                9055            9060

Ile Thr Arg Leu Ser Thr Ser  Pro Ile Lys Ala Glu  Ser Thr Glu
    9065                9070            9075

Met Thr Ile Thr Thr Gln Thr  Gly Ser Pro Gly Ala  Thr Ser Arg
    9080                9085            9090

Gly Thr Leu Thr Leu Asp Thr  Ser Thr Thr Phe Met  Ser Gly Thr
    9095                9100            9105

His Ser Thr Ala Ser Gln Gly  Phe Ser His Ser Gln  Met Thr Ala
    9110                9115            9120

Leu Met Ser Arg Thr Pro Gly  Asp Val Pro Trp Leu  Ser His Pro
    9125                9130            9135

Ser Val Glu Glu Ala Ser Ser  Ala Ser Phe Ser Leu  Ser Ser Pro
    9140                9145            9150

Val Met Thr Ser Ser Ser Pro  Val Ser Ser Thr Leu  Pro Asp Ser
    9155                9160            9165

Ile His Ser Ser Ser Leu Pro  Val Thr Ser Leu Leu  Thr Ser Gly
    9170                9175            9180

Leu Val Lys Thr Thr Glu Leu  Leu Gly Thr Ser Ser  Glu Pro Glu
    9185                9190            9195

Thr Ser Ser Pro Pro Asn Leu  Ser Ser Thr Ser Ala  Glu Ile Leu
    9200                9205            9210

Ala Thr Thr Glu Val Thr Thr  Asp Thr Glu Lys Leu  Glu Met Thr
    9215                9220            9225

Asn Val Val Thr Ser Gly Tyr  Thr His Glu Ser Pro  Ser Ser Val
    9230                9235            9240

Leu Ala Asp Ser Val Thr Thr  Lys Ala Thr Ser Ser  Met Gly Ile
    9245                9250            9255

Thr Tyr Pro Thr Gly Asp Thr  Asn Val Leu Thr Ser  Thr Pro Ala
    9260                9265            9270
```

253

Phe Ser Asp Thr Ser Arg Ile Gln Thr Lys Ser Lys Leu Ser Leu
9275 9280 9285

Thr Pro Gly Leu Met Glu Thr Ser Ile Ser Glu Glu Thr Ser Ser
9290 9295 9300

Ala Thr Glu Lys Ser Thr Val Leu Ser Ser Val Pro Thr Gly Ala
9305 9310 9315

Thr Thr Glu Val Ser Arg Thr Glu Ala Ile Ser Ser Ser Arg Thr
9320 9325 9330

Ser Ile Pro Gly Pro Ala Gln Ser Thr Met Ser Ser Asp Thr Ser
9335 9340 9345

Met Glu Thr Ile Thr Arg Ile Ser Thr Pro Leu Thr Arg Lys Glu
9350 9355 9360

Ser Thr Asp Met Ala Ile Thr Pro Lys Thr Gly Pro Ser Gly Ala
9365 9370 9375

Thr Ser Gln Gly Thr Phe Thr Leu Asp Ser Ser Ser Thr Ala Ser
9380 9385 9390

Trp Pro Gly Thr His Ser Ala Thr Thr Gln Arg Phe Pro Gln Ser
9395 9400 9405

Val Val Thr Thr Pro Met Ser Arg Gly Pro Glu Asp Val Ser Trp
9410 9415 9420

Pro Ser Pro Leu Ser Val Glu Lys Asn Ser Pro Pro Ser Ser Leu
9425 9430 9435

Val Ser Ser Ser Ser Val Thr Ser Pro Ser Pro Leu Tyr Ser Thr
9440 9445 9450

Pro Ser Gly Ser Ser His Ser Ser Pro Val Pro Val Thr Ser Leu
9455 9460 9465

Phe Thr Ser Ile Met Met Lys Ala Thr Asp Met Leu Asp Ala Ser
9470 9475 9480

Leu Glu Pro Glu Thr Thr Ser Ala Pro Asn Met Asn Ile Thr Ser
9485 9490 9495

Asp Glu Ser Leu Ala Thr Ser Lys Ala Thr Thr Glu Thr Glu Ala

```
          9500                      9505                      9510


     Ile His  Val Phe Glu Asn Thr  Ala Ala Ser His Val  Glu Thr Thr
         9515                  9520                  9525


     Ser Ala  Thr Glu Glu Leu Tyr  Ser Ser Ser Pro Gly  Phe Ser Glu
         9530                  9535                  9540


     Pro Thr  Lys Val Ile Ser Pro  Val Val Thr Ser Ser  Ser Ile Arg
         9545                  9550                  9555


     Asp Asn  Met Val Ser Thr Thr  Met Pro Gly Ser Ser  Gly Ile Thr
         9560                  9565                  9570


     Arg Ile  Glu Ile Glu Ser Met  Ser Ser Leu Thr Pro  Gly Leu Arg
         9575                  9580                  9585


     Glu Thr  Arg Thr Ser Gln Asp  Ile Thr Ser Ser Thr  Glu Thr Ser
         9590                  9595                  9600


     Thr Val  Leu Tyr Lys Met Ser  Ser Gly Ala Thr Pro  Glu Val Ser
         9605                  9610                  9615


     Arg Thr  Glu Val Met Pro Ser  Ser Arg Thr Ser Ile  Pro Gly Pro
         9620                  9625                  9630


     Ala Gln  Ser Thr Met Ser Leu  Asp Ile Ser Asp Glu  Val Val Thr
         9635                  9640                  9645


     Arg Leu  Ser Thr Ser Pro Ile  Met Thr Glu Ser Ala  Glu Ile Thr
         9650                  9655                  9660


     Ile Thr  Thr Gln Thr Gly Tyr  Ser Leu Ala Thr Ser  Gln Val Thr
         9665                  9670                  9675


     Leu Pro  Leu Gly Thr Ser Met  Thr Phe Leu Ser Gly  Thr His Ser
         9680                  9685                  9690


     Thr Met  Ser Gln Gly Leu Ser  His Ser Glu Met Thr  Asn Leu Met
         9695                  9700                  9705


     Ser Arg  Gly Pro Glu Ser Leu  Ser Trp Thr Ser Pro  Arg Phe Val
         9710                  9715                  9720


     Glu Thr  Thr Arg Ser Ser Ser  Ser Leu Thr Ser Leu  Pro Leu Thr
         9725                  9730                  9735
```

```
Thr Ser  Leu Ser Pro Val Ser  Ser Thr Leu Leu Asp  Ser Ser Pro
    9740             9745                9750

Ser Ser  Pro Leu Pro Val Thr  Ser Leu Ile Leu Pro  Gly Leu Val
    9755             9760                9765

Lys Thr  Thr Glu Val Leu Asp  Thr Ser Ser Glu Pro  Lys Thr Ser
    9770             9775                9780

Ser Ser  Pro Asn Leu Ser Ser  Thr Ser Val Glu Ile  Pro Ala Thr
    9785             9790                9795

Ser Glu  Ile Met Thr Asp Thr  Glu Lys Ile His Pro  Ser Ser Asn
    9800             9805                9810

Thr Ala  Val Ala Lys Val Arg  Thr Ser Ser Ser Val  His Glu Ser
    9815             9820                9825

His Ser  Ser Val Leu Ala Asp  Ser Glu Thr Thr Ile  Thr Ile Pro
    9830             9835                9840

Ser Met  Gly Ile Thr Ser Ala  Val Asp Asp Thr Thr  Val Phe Thr
    9845             9850                9855

Ser Asn  Pro Ala Phe Ser Glu  Thr Arg Arg Ile Pro  Thr Glu Pro
    9860             9865                9870

Thr Phe  Ser Leu Thr Pro Gly  Phe Arg Glu Thr Ser  Thr Ser Glu
    9875             9880                9885

Glu Thr  Thr Ser Ile Thr Glu  Thr Ser Ala Val Leu  Tyr Gly Val
    9890             9895                9900

Pro Thr  Ser Ala Thr Thr Glu  Val Ser Met Thr Glu  Ile Met Ser
    9905             9910                9915

Ser Asn  Arg Thr His Ile Pro  Asp Ser Asp Gln Ser  Thr Met Ser
    9920             9925                9930

Pro Asp  Ile Ile Thr Glu Val  Ile Thr Arg Leu Ser  Ser Ser Ser
    9935             9940                9945

Met Met  Ser Glu Ser Thr Gln  Met Thr Ile Thr Thr  Gln Lys Ser
    9950             9955                9960

Ser Pro  Gly Ala Thr Ala Gln  Ser Thr Leu Thr Leu  Ala Thr Thr
    9965             9970                9975
```

256

```
Thr Ala  Pro Leu Ala Arg Thr  His Ser Thr Val Pro  Pro Arg Phe
    9980              9985              9990


Leu His  Ser Glu Met Thr Thr  Leu Met Ser Arg Ser  Pro Glu Asn
    9995              10000             10005


Pro Ser  Trp Lys Ser Ser Pro  Phe Val Glu Lys Thr  Ser Ser Ser
    10010             10015             10020


Ser Ser  Leu Leu Ser Leu Pro  Val Thr Thr Ser Pro  Ser Val Ser
    10025             10030             10035


Ser Thr  Leu Pro Gln Ser Ile  Pro Ser Ser Ser Phe  Ser Val Thr
    10040             10045             10050


Ser Leu  Leu Thr Pro Gly Met  Val Lys Thr Thr Asp  Thr Ser Thr
    10055             10060             10065


Glu Pro  Gly Thr Ser Leu Ser  Pro Asn Leu Ser Gly  Thr Ser Val
    10070             10075             10080


Glu Ile  Leu Ala Ala Ser Glu  Val Thr Thr Asp Thr  Glu Lys Ile
    10085             10090             10095


His Pro  Ser Ser Ser Met Ala  Val Thr Asn Val Gly  Thr Thr Ser
    10100             10105             10110


Ser Gly  His Glu Leu Tyr Ser  Ser Val Ser Ile His  Ser Glu Pro
    10115             10120             10125


Ser Lys  Ala Thr Tyr Pro Val  Gly Thr Pro Ser Ser  Met Ala Glu
    10130             10135             10140


Thr Ser  Ile Ser Thr Ser Met  Pro Ala Asn Phe Glu  Thr Thr Gly
    10145             10150             10155


Phe Glu  Ala Glu Pro Phe Ser  His Leu Thr Ser Gly  Phe Arg Lys
    10160             10165             10170


Thr Asn  Met Ser Leu Asp Thr  Ser Ser Val Thr Pro  Thr Asn Thr
    10175             10180             10185


Pro Ser  Ser Pro Gly Ser Thr  His Leu Leu Gln Ser  Ser Lys Thr
    10190             10195             10200


Asp Phe  Thr Ser Ser Ala Lys  Thr Ser Ser Pro Asp  Trp Pro Pro
    10205             10210             10215
```

```
Ala  Ser  Gln  Tyr  Thr  Glu  Ile    Pro  Val  Asp  Ile  Ile    Thr  Pro  Phe
     10220                  10225                    10230

Asn  Ala  Ser  Pro  Ser  Ile  Thr    Glu  Ser  Thr  Gly  Ile    Thr  Ser  Phe
     10235                  10240                    10245

Pro  Glu  Ser  Arg  Phe  Thr  Met    Ser  Val  Thr  Glu  Ser    Thr  His  His
     10250                  10255                    10260

Leu  Ser  Thr  Asp  Leu  Leu  Pro    Ser  Ala  Glu  Thr  Ile    Ser  Thr  Gly
     10265                  10270                    10275

Thr  Val  Met  Pro  Ser  Leu  Ser    Glu  Ala  Met  Thr  Ser    Phe  Ala  Thr
     10280                  10285                    10290

Thr  Gly  Val  Pro  Arg  Ala  Ile    Ser  Gly  Ser  Gly  Ser    Pro  Phe  Ser
     10295                  10300                    10305

Arg  Thr  Glu  Ser  Gly  Pro  Gly    Asp  Ala  Thr  Leu  Ser    Thr  Ile  Ala
     10310                  10315                    10320

Glu  Ser  Leu  Pro  Ser  Ser  Thr    Pro  Val  Pro  Phe  Ser    Ser  Ser  Thr
     10325                  10330                    10335

Phe  Thr  Thr  Thr  Asp  Ser  Ser    Thr  Ile  Pro  Ala  Leu    His  Glu  Ile
     10340                  10345                    10350

Thr  Ser  Ser  Ser  Ala  Thr  Pro    Tyr  Arg  Val  Asp  Thr    Ser  Leu  Gly
     10355                  10360                    10365

Thr  Glu  Ser  Ser  Thr  Thr  Glu    Gly  Arg  Leu  Val  Met    Val  Ser  Thr
     10370                  10375                    10380

Leu  Asp  Thr  Ser  Ser  Gln  Pro    Gly  Arg  Thr  Ser  Ser    Thr  Pro  Ile
     10385                  10390                    10395

Leu  Asp  Thr  Arg  Met  Thr  Glu    Ser  Val  Glu  Leu  Gly    Thr  Val  Thr
     10400                  10405                    10410

Ser  Ala  Tyr  Gln  Val  Pro  Ser    Leu  Ser  Thr  Arg  Leu    Thr  Arg  Thr
     10415                  10420                    10425

Asp  Gly  Ile  Met  Glu  His  Ile    Thr  Lys  Ile  Pro  Asn    Glu  Ala  Ala
     10430                  10435                    10440

His  Arg  Gly  Thr  Ile  Arg  Pro    Val  Lys  Gly  Pro  Gln    Thr  Ser  Thr
```

10445       10450       10455

Ser Pro Ala Ser Pro Lys Gly Leu His Thr Gly Gly Thr Lys Arg
   10460      10465      10470

Met Glu Thr Thr Thr Thr Ala Leu Lys Thr Thr Thr Thr Ala Leu
   10475      10480      10485

Lys Thr Thr Ser Arg Ala Thr Leu Thr Thr Ser Val Tyr Thr Pro
   10490      10495      10500

Thr Leu Gly Thr Leu Thr Pro Leu Asn Ala Ser Arg Gln Met Ala
   10505      10510      10515

Ser Thr Ile Leu Thr Glu Met Met Ile Thr Thr Pro Tyr Val Phe
   10520      10525      10530

Pro Asp Val Pro Glu Thr Thr Ser Ser Leu Ala Thr Ser Leu Gly
   10535      10540      10545

Ala Glu Thr Ser Thr Ala Leu Pro Arg Thr Thr Pro Ser Val Leu
   10550      10555      10560

Asn Arg Glu Ser Glu Thr Thr Ala Ser Leu Val Ser Arg Ser Gly
   10565      10570      10575

Ala Glu Arg Ser Pro Val Ile Gln Thr Leu Asp Val Ser Ser Ser
   10580      10585      10590

Glu Pro Asp Thr Thr Ala Ser Trp Val Ile His Pro Ala Glu Thr
   10595      10600      10605

Ile Pro Thr Val Ser Lys Thr Thr Pro Asn Phe Phe His Ser Glu
   10610      10615      10620

Leu Asp Thr Val Ser Ser Thr Ala Thr Ser His Gly Ala Asp Val
   10625      10630      10635

Ser Ser Ala Ile Pro Thr Asn Ile Ser Pro Ser Glu Leu Asp Ala
   10640      10645      10650

Leu Thr Pro Leu Val Thr Ile Ser Gly Thr Asp Thr Ser Thr Thr
   10655      10660      10665

Phe Pro Thr Leu Thr Lys Ser Pro His Glu Thr Glu Thr Arg Thr
   10670      10675      10680

EP 3 151 005 A1

Thr Trp   Leu Thr His Pro Ala   Glu Thr Ser Ser Thr   Ile Pro Arg
    10685                 10690               10695

Thr Ile   Pro Asn Phe Ser His   His Glu Ser Asp Ala   Thr Pro Ser
    10700                 10705               10710

Ile Ala   Thr Ser Pro Gly Ala   Glu Thr Ser Ser Ala   Ile Pro Ile
    10715                 10720               10725

Met Thr   Val Ser Pro Gly Ala   Glu Asp Leu Val Thr   Ser Gln Val
    10730                 10735               10740

Thr Ser   Ser Gly Thr Asp Arg   Asn Met Thr Ile Pro   Thr Leu Thr
    10745                 10750               10755

Leu Ser   Pro Gly Glu Pro Lys   Thr Ile Ala Ser Leu   Val Thr His
    10760                 10765               10770

Pro Glu   Ala Gln Thr Ser Ser   Ala Ile Pro Thr Ser   Thr Ile Ser
    10775                 10780               10785

Pro Ala   Val Ser Arg Leu Val   Thr Ser Met Val Thr   Ser Leu Ala
    10790                 10795               10800

Ala Lys   Thr Ser Thr Thr Asn   Arg Ala Leu Thr Asn   Ser Pro Gly
    10805                 10810               10815

Glu Pro   Ala Thr Thr Val Ser   Leu Val Thr His Pro   Ala Gln Thr
    10820                 10825               10830

Ser Pro   Thr Val Pro Trp Thr   Thr Ser Ile Phe Phe   His Ser Lys
    10835                 10840               10845

Ser Asp   Thr Thr Pro Ser Met   Thr Thr Ser His Gly   Ala Glu Ser
    10850                 10855               10860

Ser Ser   Ala Val Pro Thr Pro   Thr Val Ser Thr Glu   Val Pro Gly
    10865                 10870               10875

Val Val   Thr Pro Leu Val Thr   Ser Ser Arg Ala Val   Ile Ser Thr
    10880                 10885               10890

Thr Ile   Pro Ile Leu Thr Leu   Ser Pro Gly Glu Pro   Glu Thr Thr
    10895                 10900               10905

Pro Ser   Met Ala Thr Ser His   Gly Glu Glu Ala Ser   Ser Ala Ile
    10910                 10915               10920

260

```
Pro Thr   Pro Thr Val Ser Pro   Gly Val Pro Gly Val   Val Thr Ser
    10925             10930                10935

Leu Val   Thr Ser Ser Arg Ala   Val Thr Ser Thr Thr   Ile Pro Ile
    10940             10945                10950

Leu Thr   Phe Ser Leu Gly Glu   Pro Glu Thr Thr Pro   Ser Met Ala
    10955             10960                10965

Thr Ser   His Gly Thr Glu Ala   Gly Ser Ala Val Pro   Thr Val Leu
    10970             10975                10980

Pro Glu   Val Pro Gly Met Val   Thr Ser Leu Val Ala   Ser Ser Arg
    10985             10990                10995

Ala Val   Thr Ser Thr Thr Leu   Pro Thr Leu Thr Leu   Ser Pro Gly
    11000             11005                11010

Glu Pro   Glu Thr Thr Pro Ser   Met Ala Thr Ser His   Gly Ala Glu
    11015             11020                11025

Ala Ser   Ser Thr Val Pro Thr   Val Ser Pro Glu Val   Pro Gly Val
    11030             11035                11040

Val Thr   Ser Leu Val Thr Ser   Ser Ser Gly Val Asn   Ser Thr Ser
    11045             11050                11055

Ile Pro   Thr Leu Ile Leu Ser   Pro Gly Glu Leu Glu   Thr Thr Pro
    11060             11065                11070

Ser Met   Ala Thr Ser His Gly   Ala Glu Ala Ser Ser   Ala Val Pro
    11075             11080                11085

Thr Pro   Thr Val Ser Pro Gly   Val Ser Gly Val Val   Thr Pro Leu
    11090             11095                11100

Val Thr   Ser Ser Arg Ala Val   Thr Ser Thr Thr Ile   Pro Ile Leu
    11105             11110                11115

Thr Leu   Ser Ser Ser Glu Pro   Glu Thr Thr Pro Ser   Met Ala Thr
    11120             11125                11130

Ser His   Gly Val Glu Ala Ser   Ser Ala Val Leu Thr   Val Ser Pro
    11135             11140                11145

Glu Val   Pro Gly Met Val Thr   Ser Leu Val Thr Ser   Ser Arg Ala
    11150             11155                11160
```

```
Val Thr   Ser Thr Thr Ile Pro   Thr Leu Thr Ile Ser   Ser Asp Glu
    11165             11170               11175

Pro Glu   Thr Thr Thr Ser Leu   Val Thr His Ser Glu   Ala Lys Met
    11180             11185               11190

Ile Ser   Ala Ile Pro Thr Leu   Ala Val Ser Pro Thr   Val Gln Gly
    11195             11200               11205

Leu Val   Thr Ser Leu Val Thr   Ser Ser Gly Ser Glu   Thr Ser Ala
    11210             11215               11220

Phe Ser   Asn Leu Thr Val Ala   Ser Ser Gln Pro Glu   Thr Ile Asp
    11225             11230               11235

Ser Trp   Val Ala His Pro Gly   Thr Glu Ala Ser Ser   Val Val Pro
    11240             11245               11250

Thr Leu   Thr Val Ser Thr Gly   Glu Pro Phe Thr Asn   Ile Ser Leu
    11255             11260               11265

Val Thr   His Pro Ala Glu Ser   Ser Ser Thr Leu Pro   Arg Thr Thr
    11270             11275               11280

Ser Arg   Phe Ser His Ser Glu   Leu Asp Thr Met Pro   Ser Thr Val
    11285             11290               11295

Thr Ser   Pro Glu Ala Glu Ser   Ser Ser Ala Ile Ser   Thr Thr Ile
    11300             11305               11310

Ser Pro   Gly Ile Pro Gly Val   Leu Thr Ser Leu Val   Thr Ser Ser
    11315             11320               11325

Gly Arg   Asp Ile Ser Ala Thr   Phe Pro Thr Val Pro   Glu Ser Pro
    11330             11335               11340

His Glu   Ser Glu Ala Thr Ala   Ser Trp Val Thr His   Pro Ala Val
    11345             11350               11355

Thr Ser   Thr Thr Val Pro Arg   Thr Thr Pro Asn Tyr   Ser His Ser
    11360             11365               11370

Glu Pro   Asp Thr Thr Pro Ser   Ile Ala Thr Ser Pro   Gly Ala Glu
    11375             11380               11385

Ala Thr   Ser Asp Phe Pro Thr   Ile Thr Val Ser Pro   Asp Val Pro
```

11390 11395 11400

Asp Met Val Thr Ser Gln Val Thr Ser Ser Gly Thr Asp Thr Ser
11405 11410 11415

Ile Thr Ile Pro Thr Leu Thr Leu Ser Ser Gly Glu Pro Glu Thr
11420 11425 11430

Thr Thr Ser Phe Ile Thr Tyr Ser Glu Thr His Thr Ser Ser Ala
11435 11440 11445

Ile Pro Thr Leu Pro Val Ser Pro Gly Ala Ser Lys Met Leu Thr
11450 11455 11460

Ser Leu Val Ile Ser Ser Gly Thr Asp Ser Thr Thr Thr Phe Pro
11465 11470 11475

Thr Leu Thr Glu Thr Pro Tyr Glu Pro Glu Thr Thr Ala Ile Gln
11480 11485 11490

Leu Ile His Pro Ala Glu Thr Asn Thr Met Val Pro Lys Thr Thr
11495 11500 11505

Pro Lys Phe Ser His Ser Lys Ser Asp Thr Thr Leu Pro Val Ala
11510 11515 11520

Ile Thr Ser Pro Gly Pro Glu Ala Ser Ser Ala Val Ser Thr Thr
11525 11530 11535

Thr Ile Ser Pro Asp Met Ser Asp Leu Val Thr Ser Leu Val Pro
11540 11545 11550

Ser Ser Gly Thr Asp Thr Ser Thr Thr Phe Pro Thr Leu Ser Glu
11555 11560 11565

Thr Pro Tyr Glu Pro Glu Thr Thr Val Thr Trp Leu Thr His Pro
11570 11575 11580

Ala Glu Thr Ser Thr Thr Val Ser Gly Thr Ile Pro Asn Phe Ser
11585 11590 11595

His Arg Gly Ser Asp Thr Ala Pro Ser Met Val Thr Ser Pro Gly
11600 11605 11610

Val Asp Thr Arg Ser Gly Val Pro Thr Thr Thr Ile Pro Pro Ser
11615 11620 11625

```
Ile Pro Gly Val Val Thr Ser     Gln Val Thr Ser Ser     Ala Thr Asp
    11630               11635               11640

Thr Ser Thr Ala Ile Pro Thr     Leu Thr Pro Ser Pro     Gly Glu Pro
    11645               11650               11655

Glu Thr Thr Ala Ser Ser Ala     Thr His Pro Gly Thr     Gln Thr Gly
    11660               11665               11670

Phe Thr Val Pro Ile Arg Thr     Val Pro Ser Ser Glu     Pro Asp Thr
    11675               11680               11685

Met Ala Ser Trp Val Thr His     Pro Pro Gln Thr Ser     Thr Pro Val
    11690               11695               11700

Ser Arg Thr Thr Ser Ser Phe     Ser His Ser Ser Pro     Asp Ala Thr
    11705               11710               11715

Pro Val Met Ala Thr Ser Pro     Arg Thr Glu Ala Ser     Ser Ala Val
    11720               11725               11730

Leu Thr Thr Ile Ser Pro Gly     Ala Pro Glu Met Val     Thr Ser Gln
    11735               11740               11745

Ile Thr Ser Ser Gly Ala Ala     Thr Ser Thr Thr Val     Pro Thr Leu
    11750               11755               11760

Thr His Ser Pro Gly Met Pro     Glu Thr Thr Ala Leu     Leu Ser Thr
    11765               11770               11775

His Pro Arg Thr Gly Thr Ser     Lys Thr Phe Pro Ala     Ser Thr Val
    11780               11785               11790

Phe Pro Gln Val Ser Glu Thr     Thr Ala Ser Leu Thr     Ile Arg Pro
    11795               11800               11805

Gly Ala Glu Thr Ser Thr Ala     Leu Pro Thr Gln Thr     Thr Ser Ser
    11810               11815               11820

Leu Phe Thr Leu Leu Val Thr     Gly Thr Ser Arg Val     Asp Leu Ser
    11825               11830               11835

Pro Thr Ala Ser Pro Gly Val     Ser Ala Lys Thr Ala     Pro Leu Ser
    11840               11845               11850

Thr His Pro Gly Thr Glu Thr     Ser Thr Met Ile Pro     Thr Ser Thr
    11855               11860               11865
```

264

```
Leu Ser     Leu Gly Leu Leu Glu     Thr Thr Gly Leu Leu     Ala Thr Ser
    11870                   11875             11880

Ser Ser     Ala Glu Thr Ser Thr     Ser Thr Leu Thr Leu     Thr Val Ser
    11885                   11890             11895

Pro Ala     Val Ser Gly Leu Ser     Ser Ala Ser Ile Thr     Thr Asp Lys
    11900                   11905             11910

Pro Gln     Thr Val Thr Ser Trp     Asn Thr Glu Thr Ser     Pro Ser Val
    11915                   11920             11925

Thr Ser     Val Gly Pro Pro Glu     Phe Ser Arg Thr Val     Thr Gly Thr
    11930                   11935             11940

Thr Met     Thr Leu Ile Pro Ser     Glu Met Pro Thr Pro     Pro Lys Thr
    11945                   11950             11955

Ser His     Gly Glu Gly Val Ser     Pro Thr Thr Ile Leu     Arg Thr Thr
    11960                   11965             11970

Met Val     Glu Ala Thr Asn Leu     Ala Thr Thr Gly Ser     Ser Pro Thr
    11975                   11980             11985

Val Ala     Lys Thr Thr Thr Thr     Phe Asn Thr Leu Ala     Gly Ser Leu
    11990                   11995             12000

Phe Thr     Pro Leu Thr Thr Pro     Gly Met Ser Thr Leu     Ala Ser Glu
    12005                   12010             12015

Ser Val     Thr Ser Arg Thr Ser     Tyr Asn His Arg Ser     Trp Ile Ser
    12020                   12025             12030

Thr Thr     Ser Ser Tyr Asn Arg     Arg Tyr Trp Thr Pro     Ala Thr Ser
    12035                   12040             12045

Thr Pro     Val Thr Ser Thr Phe     Ser Pro Gly Ile Ser     Thr Ser Ser
    12050                   12055             12060

Ile Pro     Ser Ser Thr Ala Ala     Thr Val Pro Phe Met     Val Pro Phe
    12065                   12070             12075

Thr Leu     Asn Phe Thr Ile Thr     Asn Leu Gln Tyr Glu     Glu Asp Met
    12080                   12085             12090

Arg His     Pro Gly Ser Arg Lys     Phe Asn Ala Thr Glu     Arg Glu Leu
    12095                   12100             12105
```

Gln Gly   Leu Leu Lys Pro Leu   Phe Arg Asn Ser Ser   Leu Glu Tyr
12110              12115             12120

Leu Tyr   Ser Gly Cys Arg Leu   Ala Ser Leu Arg Pro   Glu Lys Asp
12125              12130             12135

Ser Ser   Ala Met Ala Val Asp   Ala Ile Cys Thr His   Arg Pro Asp
12140              12145             12150

Pro Glu   Asp Leu Gly Leu Asp   Arg Glu Arg Leu Tyr   Trp Glu Leu
12155              12160             12165

Ser Asn   Leu Thr Asn Gly Ile   Gln Glu Leu Gly Pro   Tyr Thr Leu
12170              12175             12180

Asp Arg   Asn Ser Leu Tyr Val   Asn Gly Phe Thr His   Arg Ser Ser
12185              12190             12195

Met Pro   Thr Thr Ser Thr Pro   Gly Thr Ser Thr Val   Asp Val Gly
12200              12205             12210

Thr Ser   Gly Thr Pro Ser Ser   Ser Pro Ser Pro Thr   Ala Ala Gly
12215              12220             12225

Pro Leu   Leu Met Pro Phe Thr   Leu Asn Phe Thr Ile   Thr Asn Leu
12230              12235             12240

Gln Tyr   Glu Glu Asp Met Arg   Arg Thr Gly Ser Arg   Lys Phe Asn
12245              12250             12255

Thr Met   Glu Ser Val Leu Gln   Gly Leu Leu Lys Pro   Leu Phe Lys
12260              12265             12270

Asn Thr   Ser Val Gly Pro Leu   Tyr Ser Gly Cys Arg   Leu Thr Leu
12275              12280             12285

Leu Arg   Pro Glu Lys Asp Gly   Ala Ala Thr Gly Val   Asp Ala Ile
12290              12295             12300

Cys Thr   His Arg Leu Asp Pro   Lys Ser Pro Gly Leu   Asn Arg Glu
12305              12310             12315

Gln Leu   Tyr Trp Glu Leu Ser   Lys Leu Thr Asn Asp   Ile Glu Glu
12320              12325             12330

Leu Gly   Pro Tyr Thr Leu Asp   Arg Asn Ser Leu Tyr   Val Asn Gly

12335 12340 12345

Phe Thr His Gln Ser Ser Val Ser Thr Thr Ser Thr Pro Gly Thr
12350 12355 12360

Ser Thr Val Asp Leu Arg Thr Ser Gly Thr Pro Ser Ser Leu Ser
12365 12370 12375

Ser Pro Thr Ile Met Ala Ala Gly Pro Leu Leu Val Pro Phe Thr
12380 12385 12390

Leu Asn Phe Thr Ile Thr Asn Leu Gln Tyr Gly Glu Asp Met Gly
12395 12400 12405

His Pro Gly Ser Arg Lys Phe Asn Thr Thr Glu Arg Val Leu Gln
12410 12415 12420

Gly Leu Leu Gly Pro Ile Phe Lys Asn Thr Ser Val Gly Pro Leu
12425 12430 12435

Tyr Ser Gly Cys Arg Leu Thr Ser Leu Arg Ser Glu Lys Asp Gly
12440 12445 12450

Ala Ala Thr Gly Val Asp Ala Ile Cys Ile His His Leu Asp Pro
12455 12460 12465

Lys Ser Pro Gly Leu Asn Arg Glu Arg Leu Tyr Trp Glu Leu Ser
12470 12475 12480

Gln Leu Thr Asn Gly Ile Lys Glu Leu Gly Pro Tyr Thr Leu Asp
12485 12490 12495

Arg Asn Ser Leu Tyr Val Asn Gly Phe Thr His Arg Thr Ser Val
12500 12505 12510

Pro Thr Thr Ser Thr Pro Gly Thr Ser Thr Val Asp Leu Gly Thr
12515 12520 12525

Ser Gly Thr Pro Phe Ser Leu Pro Ser Pro Ala Thr Ala Gly Pro
12530 12535 12540

Leu Leu Val Leu Phe Thr Leu Asn Phe Thr Ile Thr Asn Leu Lys
12545 12550 12555

Tyr Glu Glu Asp Met His Arg Pro Gly Ser Arg Lys Phe Asn Thr
12560 12565 12570

```
Thr Glu   Arg Val Leu Gln Thr   Leu Leu Gly Pro Met   Phe Lys Asn
    12575                 12580                 12585

Thr Ser   Val Gly Leu Leu Tyr   Ser Gly Cys Arg Leu   Thr Leu Leu
    12590                 12595                 12600

Arg Ser   Glu Lys Asp Gly Ala   Ala Thr Gly Val Asp   Ala Ile Cys
    12605                 12610                 12615

Thr His   Arg Leu Asp Pro Lys   Ser Pro Gly Leu Asp   Arg Glu Gln
    12620                 12625                 12630

Leu Tyr   Trp Glu Leu Ser Gln   Leu Thr Asn Gly Ile   Lys Glu Leu
    12635                 12640                 12645

Gly Pro   Tyr Thr Leu Asp Arg   Asn Ser Leu Tyr Val   Asn Gly Phe
    12650                 12655                 12660

Thr His   Trp Ile Pro Val Pro   Thr Ser Ser Thr Pro   Gly Thr Ser
    12665                 12670                 12675

Thr Val   Asp Leu Gly Ser Gly   Thr Pro Ser Ser Leu   Pro Ser Pro
    12680                 12685                 12690

Thr Ala   Ala Gly Pro Leu Leu   Val Pro Phe Thr Leu   Asn Phe Thr
    12695                 12700                 12705

Ile Thr   Asn Leu Gln Tyr Glu   Glu Asp Met His His   Pro Gly Ser
    12710                 12715                 12720

Arg Lys   Phe Asn Thr Thr Glu   Arg Val Leu Gln Gly   Leu Leu Gly
    12725                 12730                 12735

Pro Met   Phe Lys Asn Thr Ser   Val Gly Leu Leu Tyr   Ser Gly Cys
    12740                 12745                 12750

Arg Leu   Thr Leu Leu Arg Ser   Glu Lys Asp Gly Ala   Ala Thr Gly
    12755                 12760                 12765

Val Asp   Ala Ile Cys Thr His   Arg Leu Asp Pro Lys   Ser Pro Gly
    12770                 12775                 12780

Val Asp   Arg Glu Gln Leu Tyr   Trp Glu Leu Ser Gln   Leu Thr Asn
    12785                 12790                 12795

Gly Ile   Lys Glu Leu Gly Pro   Tyr Thr Leu Asp Arg   Asn Ser Leu
    12800                 12805                 12810
```

```
Tyr Val   Asn Gly Phe Thr His   Gln Thr Ser Ala Pro   Asn Thr Ser
    12815               12820                 12825

Thr Pro   Gly Thr Ser Thr Val   Asp Leu Gly Thr Ser   Gly Thr Pro
    12830               12835                 12840

Ser Ser   Leu Pro Ser Pro Thr   Ser Ala Gly Pro Leu   Leu Val Pro
    12845               12850                 12855

Phe Thr   Leu Asn Phe Thr Ile   Thr Asn Leu Gln Tyr   Glu Glu Asp
    12860               12865                 12870

Met Arg   His Pro Gly Ser Arg   Lys Phe Asn Thr Thr   Glu Arg Val
    12875               12880                 12885

Leu Gln   Gly Leu Leu Lys Pro   Leu Phe Lys Ser Thr   Ser Val Gly
    12890               12895                 12900

Pro Leu   Tyr Ser Gly Cys Arg   Leu Thr Leu Leu Arg   Ser Glu Lys
    12905               12910                 12915

Asp Gly   Ala Ala Thr Gly Val   Asp Ala Ile Cys Thr   His Arg Leu
    12920               12925                 12930

Asp Pro   Lys Ser Pro Gly Val   Asp Arg Glu Gln Leu   Tyr Trp Glu
    12935               12940                 12945

Leu Ser   Gln Leu Thr Asn Gly   Ile Lys Glu Leu Gly   Pro Tyr Thr
    12950               12955                 12960

Leu Asp   Arg Asn Ser Leu Tyr   Val Asn Gly Phe Thr   His Gln Thr
    12965               12970                 12975

Ser Ala   Pro Asn Thr Ser Thr   Pro Gly Thr Ser Thr   Val Asp Leu
    12980               12985                 12990

Gly Thr   Ser Gly Thr Pro Ser   Ser Leu Pro Ser Pro   Thr Ser Ala
    12995               13000                 13005

Gly Pro   Leu Leu Val Pro Phe   Thr Leu Asn Phe Thr   Ile Thr Asn
    13010               13015                 13020

Leu Gln   Tyr Glu Glu Asp Met   His His Pro Gly Ser   Arg Lys Phe
    13025               13030                 13035

Asn Thr   Thr Glu Arg Val Leu   Gln Gly Leu Leu Gly   Pro Met Phe
    13040               13045                 13050
```

269

Lys Asn    Thr Ser Val Gly Leu    Leu Tyr Ser Gly Cys    Arg Leu Thr
13055                13060                13065

Leu Leu    Arg Pro Glu Lys Asn    Gly Ala Ala Thr Gly    Met Asp Ala
13070                13075                13080

Ile Cys    Ser His Arg Leu Asp    Pro Lys Ser Pro Gly    Leu Asn Arg
13085                13090                13095

Glu Gln    Leu Tyr Trp Glu Leu    Ser Gln Leu Thr His    Gly Ile Lys
13100                13105                13110

Glu Leu    Gly Pro Tyr Thr Leu    Asp Arg Asn Ser Leu    Tyr Val Asn
13115                13120                13125

Gly Phe    Thr His Arg Ser Ser    Val Ala Pro Thr Ser    Thr Pro Gly
13130                13135                13140

Thr Ser    Thr Val Asp Leu Gly    Thr Ser Gly Thr Pro    Ser Ser Leu
13145                13150                13155

Pro Ser    Pro Thr Thr Ala Val    Pro Leu Leu Val Pro    Phe Thr Leu
13160                13165                13170

Asn Phe    Thr Ile Thr Asn Leu    Gln Tyr Gly Glu Asp    Met Arg His
13175                13180                13185

Pro Gly    Ser Arg Lys Phe Asn    Thr Thr Glu Arg Val    Leu Gln Gly
13190                13195                13200

Leu Leu    Gly Pro Leu Phe Lys    Asn Ser Ser Val Gly    Pro Leu Tyr
13205                13210                13215

Ser Gly    Cys Arg Leu Ile Ser    Leu Arg Ser Glu Lys    Asp Gly Ala
13220                13225                13230

Ala Thr    Gly Val Asp Ala Ile    Cys Thr His His Leu    Asn Pro Gln
13235                13240                13245

Ser Pro    Gly Leu Asp Arg Glu    Gln Leu Tyr Trp Gln    Leu Ser Gln
13250                13255                13260

Met Thr    Asn Gly Ile Lys Glu    Leu Gly Pro Tyr Thr    Leu Asp Arg
13265                13270                13275

Asn Ser    Leu Tyr Val Asn Gly    Phe Thr His Arg Ser    Ser Gly Leu

13280                    13285                         13290

Thr Thr  Ser Thr Pro Trp Thr   Ser Thr Val Asp Leu   Gly Thr Ser
     13295                 13300                13305

Gly Thr  Pro Ser Pro Val Pro   Ser Pro Thr Thr Ala   Gly Pro Leu
     13310                 13315                13320

Leu Val  Pro Phe Thr Leu Asn   Phe Thr Ile Thr Asn   Leu Gln Tyr
     13325                 13330                13335

Glu Glu  Asp Met His Arg Pro   Gly Ser Arg Lys Phe   Asn Thr Thr
     13340                 13345                13350

Glu Arg  Val Leu Gln Gly Leu   Leu Ser Pro Ile Phe   Lys Asn Ser
     13355                 13360                13365

Ser Val  Gly Pro Leu Tyr Ser   Gly Cys Arg Leu Thr   Ser Leu Arg
     13370                 13375                13380

Pro Glu  Lys Asp Gly Ala Ala   Thr Gly Met Asp Ala   Val Cys Leu
     13385                 13390                13395

Tyr His  Pro Asn Pro Lys Arg   Pro Gly Leu Asp Arg   Glu Gln Leu
     13400                 13405                13410

Tyr Trp  Glu Leu Ser Gln Leu   Thr His Asn Ile Thr   Glu Leu Gly
     13415                 13420                13425

Pro Tyr  Ser Leu Asp Arg Asp   Ser Leu Tyr Val Asn   Gly Phe Thr
     13430                 13435                13440

His Gln  Asn Ser Val Pro Thr   Thr Ser Thr Pro Gly   Thr Ser Thr
     13445                 13450                13455

Val Tyr  Trp Ala Thr Thr Gly   Thr Pro Ser Ser Phe   Pro Gly His
     13460                 13465                13470

Thr Glu  Pro Gly Pro Leu Leu   Ile Pro Phe Thr Phe   Asn Phe Thr
     13475                 13480                13485

Ile Thr  Asn Leu His Tyr Glu   Glu Asn Met Gln His   Pro Gly Ser
     13490                 13495                13500

Arg Lys  Phe Asn Thr Thr Glu   Arg Val Leu Gln Gly   Leu Leu Lys
     13505                 13510                13515

271

```
Pro Leu   Phe Lys Asn Thr Ser   Val Gly Pro Leu Tyr   Ser Gly Cys
    13520                 13525                 13530

Arg Leu   Thr Ser Leu Arg Pro   Glu Lys Asp Gly Ala   Ala Thr Gly
    13535                 13540                 13545

Met Asp   Ala Val Cys Leu Tyr   His Pro Asn Pro Lys   Arg Pro Gly
    13550                 13555                 13560

Leu Asp   Arg Glu Gln Leu Tyr   Trp Glu Leu Ser Gln   Leu Thr His
    13565                 13570                 13575

Asn Ile   Thr Glu Leu Gly Pro   Tyr Ser Leu Asp Arg   Asp Ser Leu
    13580                 13585                 13590

Tyr Val   Asn Gly Phe Thr His   Gln Asn Ser Val Pro   Thr Thr Ser
    13595                 13600                 13605

Thr Pro   Gly Thr Ser Thr Val   Tyr Trp Ala Thr Thr   Gly Thr Pro
    13610                 13615                 13620

Ser Ser   Phe Pro Gly His Thr   Glu Pro Gly Pro Leu   Leu Ile Pro
    13625                 13630                 13635

Phe Thr   Phe Asn Phe Thr Ile   Thr Asn Leu His Tyr   Glu Glu Asn
    13640                 13645                 13650

Met Gln   His Pro Gly Ser Arg   Lys Phe Asn Thr Thr   Glu Arg Val
    13655                 13660                 13665

Leu Gln   Gly Leu Leu Lys Pro   Leu Phe Lys Asn Thr   Ser Val Gly
    13670                 13675                 13680

Pro Leu   Tyr Ser Gly Cys Arg   Leu Thr Leu Leu Arg   Pro Glu Lys
    13685                 13690                 13695

His Glu   Ala Ala Thr Gly Val   Asp Thr Ile Cys Thr   His Arg Val
    13700                 13705                 13710

Asp Pro   Ile Gly Pro Gly Leu   Asp Arg Glu Arg Leu   Tyr Trp Glu
    13715                 13720                 13725

Leu Ser   Gln Leu Thr Asn Ser   Ile Thr Glu Leu Gly   Pro Tyr Thr
    13730                 13735                 13740

Leu Asp   Arg Asp Ser Leu Tyr   Val Asn Gly Phe Asn   Pro Arg Ser
    13745                 13750                 13755
```

272

```
Ser Val Pro Thr Thr Ser Thr     Pro Gly Thr Ser Thr     Val His Leu
    13760                 13765                 13770

Ala Thr Ser Gly Thr Pro Ser     Ser Leu Pro Gly His     Thr Ala Pro
    13775                 13780                 13785

Val Pro Leu Leu Ile Pro Phe     Thr Leu Asn Phe Thr     Ile Thr Asn
    13790                 13795                 13800

Leu His Tyr Glu Glu Asn Met     Gln His Pro Gly Ser     Arg Lys Phe
    13805                 13810                 13815

Asn Thr Thr Glu Arg Val Leu     Gln Gly Leu Leu Lys     Pro Leu Phe
    13820                 13825                 13830

Lys Asn Thr Ser Val Gly Pro     Leu Tyr Ser Gly Cys     Arg Leu Thr
    13835                 13840                 13845

Leu Leu Arg Pro Glu Lys His     Glu Ala Ala Thr Gly     Val Asp Thr
    13850                 13855                 13860

Ile Cys Thr His Arg Val Asp     Pro Ile Gly Pro Gly     Leu Xaa Xaa
    13865                 13870                 13875

Glu Xaa Leu Tyr Trp Glu Leu     Ser Xaa Leu Thr Xaa     Xaa Ile Xaa
    13880                 13885                 13890

Glu Leu Gly Pro Tyr Thr Leu     Asp Arg Xaa Ser Leu     Tyr Val Asn
    13895                 13900                 13905

Gly Phe Thr His Xaa Xaa Ser     Xaa Pro Thr Thr Ser     Thr Pro Gly
    13910                 13915                 13920

Thr Ser Thr Val Xaa Xaa Gly     Thr Ser Gly Thr Pro     Ser Ser Xaa
    13925                 13930                 13935

Pro Xaa Xaa Thr Ser Ala Gly     Pro Leu Leu Val Pro     Phe Thr Leu
    13940                 13945                 13950

Asn Phe Thr Ile Thr Asn Leu     Gln Tyr Glu Glu Asp     Met His His
    13955                 13960                 13965

Pro Gly Ser Arg Lys Phe Asn     Thr Thr Glu Arg Val     Leu Gln Gly
    13970                 13975                 13980

Leu Leu Gly Pro Met Phe Lys     Asn Thr Ser Val Gly     Leu Leu Tyr
    13985                 13990                 13995
```

273

Ser Gly Cys Arg Leu Thr Leu Leu Arg Pro Glu Lys Asn Gly Ala
14000 14005 14010

Ala Thr Gly Met Asp Ala Ile Cys Ser His Arg Leu Asp Pro Lys
14015 14020 14025

Ser Pro Gly Leu Asp Arg Glu Gln Leu Tyr Trp Glu Leu Ser Gln
14030 14035 14040

Leu Thr His Gly Ile Lys Glu Leu Gly Pro Tyr Thr Leu Asp Arg
14045 14050 14055

Asn Ser Leu Tyr Val Asn Gly Phe Thr His Arg Ser Ser Val Ala
14060 14065 14070

Pro Thr Ser Thr Pro Gly Thr Ser Thr Val Asp Leu Gly Thr Ser
14075 14080 14085

Gly Thr Pro Ser Ser Leu Pro Ser Pro Thr Thr Ala Val Pro Leu
14090 14095 14100

Leu Val Pro Phe Thr Leu Asn Phe Thr Ile Thr Asn Leu Gln Tyr
14105 14110 14115

Gly Glu Asp Met Arg His Pro Gly Ser Arg Lys Phe Asn Thr Thr
14120 14125 14130

Glu Arg Val Leu Gln Gly Leu Leu Gly Pro Leu Phe Lys Asn Ser
14135 14140 14145

Ser Val Gly Pro Leu Tyr Ser Gly Cys Arg Leu Ile Ser Leu Arg
14150 14155 14160

Ser Glu Lys Asp Gly Ala Ala Thr Gly Val Asp Ala Ile Cys Thr
14165 14170 14175

His His Leu Asn Pro Gln Ser Pro Gly Leu Asp Arg Glu Gln Leu
14180 14185 14190

Tyr Trp Gln Leu Ser Gln Met Thr Asn Gly Ile Lys Glu Leu Gly
14195 14200 14205

Pro Tyr Thr Leu Asp Arg Asn Ser Leu Tyr Val Asn Gly Phe Thr
14210 14215 14220

His Arg Ser Ser Gly Leu Thr Thr Ser Thr Pro Trp Thr Ser Thr

14225                      14230                      14235

Val Asp  Leu Gly Thr Ser Gly  Thr Pro Ser Pro Val  Pro Ser Pro
     14240                14245                14250

Thr Thr  Ala Gly Pro Leu Leu  Val Pro Phe Thr Leu  Asn Phe Thr
     14255                14260                14265

Ile Thr  Asn Leu Gln Tyr Glu  Glu Asp Met His Arg  Pro Gly Ser
     14270                14275                14280

Arg Lys  Phe Asn Ala Thr Glu  Arg Val Leu Gln Gly  Leu Leu Ser
     14285                14290                14295

Pro Ile  Phe Lys Asn Ser Ser  Val Gly Pro Leu Tyr  Ser Gly Cys
     14300                14305                14310

Arg Leu  Thr Ser Leu Arg Pro  Glu Lys Asp Gly Ala  Ala Thr Gly
     14315                14320                14325

Met Asp  Ala Val Cys Leu Tyr  His Pro Asn Pro Lys  Arg Pro Gly
     14330                14335                14340

Leu Asp  Arg Glu Gln Leu Tyr  Trp Glu Leu Ser Gln  Leu Thr His
     14345                14350                14355

Asn Ile  Thr Glu Leu Gly Pro  Tyr Ser Leu Asp Arg  Asp Ser Leu
     14360                14365                14370

Tyr Val  Asn Gly Phe Thr His  Gln Ser Ser Met Thr  Thr Thr Arg
     14375                14380                14385

Thr Pro  Asp Thr Ser Thr Met  His Leu Ala Thr Ser  Arg Thr Pro
     14390                14395                14400

Ala Ser  Leu Ser Gly Pro Thr  Thr Ala Ser Pro Leu  Leu Val Leu
     14405                14410                14415

Phe Thr  Ile Asn Cys Thr Ile  Thr Asn Leu Gln Tyr  Glu Glu Asp
     14420                14425                14430

Met Arg  Arg Thr Gly Ser Arg  Lys Phe Asn Thr Met  Glu Ser Val
     14435                14440                14445

Leu Gln  Gly Leu Leu Lys Pro  Leu Phe Lys Asn Thr  Ser Val Gly
     14450                14455                14460

Pro Leu Tyr Ser Gly Cys Arg     Leu Thr Leu Leu Arg     Pro Lys Lys
    14465               14470                   14475

Asp Gly Ala Ala Thr Gly Val     Asp Ala Ile Cys Thr     His Arg Leu
    14480               14485                   14490

Asp Pro Lys Ser Pro Gly Leu     Asn Arg Glu Gln Leu     Tyr Trp Glu
    14495               14500                   14505

Leu Ser Lys Leu Thr Asn Asp     Ile Glu Glu Leu Gly     Pro Tyr Thr
    14510               14515                   14520

Leu Asp Arg Asn Ser Leu Tyr     Val Asn Gly Phe Thr     His Gln Ser
    14525               14530                   14535

Ser Val Ser Thr Thr Ser Thr     Pro Gly Thr Ser Thr     Val Asp Leu
    14540               14545                   14550

Arg Thr Ser Gly Thr Pro Ser     Ser Leu Ser Ser Pro     Thr Ile Met
    14555               14560                   14565

Xaa Xaa Xaa Pro Leu Leu Xaa     Pro Phe Thr Xaa Asn     Xaa Thr Ile
    14570               14575                   14580

Thr Asn Leu Xaa Xaa Xaa Xaa     Xaa Met Xaa Xaa Pro     Gly Ser Arg
    14585               14590                   14595

Lys Phe Asn Thr Thr Glu Arg     Val Leu Gln Gly Leu     Leu Arg Pro
    14600               14605                   14610

Leu Phe Lys Asn Thr Ser Val     Ser Ser Leu Tyr Ser     Gly Cys Arg
    14615               14620                   14625

Leu Thr Leu Leu Arg Pro Glu     Lys Asp Gly Ala Ala     Thr Arg Val
    14630               14635                   14640

Asp Ala Ala Cys Thr Tyr Arg     Pro Asp Pro Lys Ser     Pro Gly Leu
    14645               14650                   14655

Asp Arg Glu Gln Leu Tyr Trp     Glu Leu Ser Gln Leu     Thr His Ser
    14660               14665                   14670

Ile Thr Glu Leu Gly Pro Tyr     Thr Leu Asp Arg Val     Ser Leu Tyr
    14675               14680                   14685

Val Asn Gly Phe Asn Pro Arg     Ser Ser Val Pro Thr     Thr Ser Thr
    14690               14695                   14700

276

```
Pro Gly  Thr Ser Thr Val His  Leu Ala Thr Ser Gly  Thr Pro Ser
    14705                14710                14715

Ser Leu  Pro Gly His Thr Xaa  Xaa Xaa Pro Leu Leu  Xaa Pro Phe
    14720                14725                14730

Thr Xaa  Asn Xaa Thr Ile Thr  Asn Leu Xaa Xaa Xaa  Xaa Xaa Met
    14735                14740                14745

Xaa Xaa  Pro Gly Ser Arg Lys  Phe Asn Thr Thr Glu  Arg Val Leu
    14750                14755                14760

Gln Gly  Leu Leu Lys Pro Leu  Phe Arg Asn Ser Ser  Leu Glu Tyr
    14765                14770                14775

Leu Tyr  Ser Gly Cys Arg Leu  Ala Ser Leu Arg Pro  Glu Lys Asp
    14780                14785                14790

Ser Ser  Ala Met Ala Val Asp  Ala Ile Cys Thr His  Arg Pro Asp
    14795                14800                14805

Pro Glu  Asp Leu Gly Leu Asp  Arg Glu Arg Leu Tyr  Trp Glu Leu
    14810                14815                14820

Ser Asn  Leu Thr Asn Gly Ile  Gln Glu Leu Gly Pro  Tyr Thr Leu
    14825                14830                14835

Asp Arg  Asn Ser Leu Tyr Val  Asn Gly Phe Thr His  Arg Ser Ser
    14840                14845                14850

Gly Leu  Thr Thr Ser Thr Pro  Trp Thr Ser Thr Val  Asp Leu Gly
    14855                14860                14865

Thr Ser  Gly Thr Pro Ser Pro  Val Pro Ser Pro Thr  Thr Ala Gly
    14870                14875                14880

Pro Leu  Leu Val Pro Phe Thr  Leu Asn Phe Thr Ile  Thr Asn Leu
    14885                14890                14895

Gln Tyr  Glu Glu Asp Met His  Arg Pro Gly Ser Arg  Arg Phe Asn
    14900                14905                14910

Thr Thr  Glu Arg Val Leu Gln  Gly Leu Leu Thr Pro  Leu Phe Lys
    14915                14920                14925

Asn Thr  Ser Val Gly Pro Leu  Tyr Ser Gly Cys Arg  Leu Thr Leu
    14930                14935                14940
```

277

Leu Arg Pro Glu Lys Gln Glu    Ala Ala Thr Gly Val    Asp Thr Ile
14945                14950                14955

Cys Thr His Arg Val Asp Pro    Ile Gly Pro Gly Leu    Asp Arg Glu
14960                14965                14970

Arg Leu Tyr Trp Glu Leu Ser    Gln Leu Thr Asn Ser    Ile Thr Glu
14975                14980                14985

Leu Gly Pro Tyr Thr Leu Asp    Arg Asp Ser Leu Tyr    Val Asn Gly
14990                14995                15000

Phe Asn Pro Trp Ser Ser Val    Pro Thr Thr Ser Thr    Pro Gly Thr
15005                15010                15015

Ser Thr Val His Leu Ala Thr    Ser Gly Thr Pro Ser    Ser Leu Pro
15020                15025                15030

Gly His Thr Ala Pro Val Pro    Leu Leu Ile Pro Phe    Thr Leu Asn
15035                15040                15045

Phe Thr Ile Thr Asp Leu His    Tyr Glu Glu Asn Met    Gln His Pro
15050                15055                15060

Gly Ser Arg Lys Phe Asn Thr    Thr Glu Arg Val Leu    Gln Gly Leu
15065                15070                15075

Leu Lys Pro Leu Phe Lys Ser    Thr Ser Val Gly Pro    Leu Tyr Ser
15080                15085                15090

Gly Cys Arg Leu Thr Leu Leu    Arg Pro Glu Lys His    Gly Ala Ala
15095                15100                15105

Thr Gly Val Asp Ala Ile Cys    Thr Leu Arg Leu Asp    Pro Thr Gly
15110                15115                15120

Pro Gly Leu Asp Arg Glu Arg    Leu Tyr Trp Glu Leu    Ser Gln Leu
15125                15130                15135

Thr Asn Ser Val Thr Glu Leu    Gly Pro Tyr Thr Leu    Asp Arg Asp
15140                15145                15150

Ser Leu Tyr Val Asn Gly Phe    Thr His Arg Ser Ser    Val Pro Thr
15155                15160                15165

Thr Ser    Ile Pro Gly Thr Ser    Ala Val His Leu Glu    Thr Ser Gly

278

```
            15170                  15175                    15180

        Thr Pro   Ala Ser Leu Pro Gly   His Thr Ala Pro Gly   Pro Leu Leu
            15185                  15190                    15195

        Val Pro   Phe Thr Leu Asn Phe   Thr Ile Thr Asn Leu   Gln Tyr Glu
            15200                  15205                    15210

        Glu Asp   Met Arg His Pro Gly   Ser Arg Lys Phe Ser   Thr Thr Glu
            15215                  15220                    15225

        Arg Val   Leu Gln Gly Leu Leu   Lys Pro Leu Phe Lys   Asn Thr Ser
            15230                  15235                    15240

        Val Ser   Ser Leu Tyr Ser Gly   Cys Arg Leu Thr Leu   Leu Arg Pro
            15245                  15250                    15255

        Glu Lys   Asp Gly Ala Ala Thr   Arg Val Asp Ala Val   Cys Thr His
            15260                  15265                    15270

        Arg Pro   Asp Pro Lys Ser Pro   Gly Leu Asp Arg Glu   Arg Leu Tyr
            15275                  15280                    15285

        Trp Lys   Leu Ser Gln Leu Thr   His Gly Ile Thr Glu   Leu Gly Pro
            15290                  15295                    15300

        Tyr Thr   Leu Asp Arg His Ser   Leu Tyr Val Asn Gly   Phe Thr His
            15305                  15310                    15315

        Gln Ser   Ser Met Thr Thr Thr   Arg Thr Pro Asp Thr   Ser Thr Met
            15320                  15325                    15330

        His Leu   Ala Thr Ser Arg Thr   Pro Ala Ser Leu Ser   Gly Pro Thr
            15335                  15340                    15345

        Thr Ala   Ser Pro Leu Leu Val   Leu Phe Thr Ile Asn   Phe Thr Ile
            15350                  15355                    15360

        Thr Asn   Leu Arg Tyr Glu Glu   Asn Met His His Pro   Gly Ser Arg
            15365                  15370                    15375

        Lys Phe   Asn Thr Thr Glu Arg   Val Leu Gln Gly Leu   Leu Arg Pro
            15380                  15385                    15390

        Val Phe   Lys Asn Thr Ser Val   Gly Pro Leu Tyr Ser   Gly Cys Arg
            15395                  15400                    15405
```

279

Leu Thr    Thr Leu Arg Pro Lys    Lys Asp Gly Ala Ala    Thr Lys Val
    15410                15415                15420

Asp Ala    Ile Cys Thr Tyr Arg    Pro Asp Pro Lys Ser    Pro Gly Leu
    15425                15430                15435

Asp Arg    Glu Gln Leu Tyr Trp    Glu Leu Ser Gln Leu    Thr His Ser
    15440                15445                15450

Ile Thr    Glu Leu Gly Pro Tyr    Thr Gln Asp Arg Asp    Ser Leu Tyr
    15455                15460                15465

Val Asn    Gly Phe Thr His Arg    Ser Ser Val Pro Thr    Thr Ser Ile
    15470                15475                15480

Pro Gly    Thr Ser Ala Val His    Leu Glu Thr Ser Gly    Thr Pro Ala
    15485                15490                15495

Ser Leu    Pro Gly His Thr Ala    Pro Gly Pro Leu Leu    Val Pro Phe
    15500                15505                15510

Thr Leu    Asn Phe Thr Ile Thr    Asn Leu Gln Tyr Glu    Glu Asp Met
    15515                15520                15525

Arg His    Pro Gly Ser Arg Lys    Phe Asn Thr Thr Glu    Arg Val Leu
    15530                15535                15540

Gln Gly    Leu Leu Lys Pro Leu    Phe Lys Ser Thr Ser    Val Gly Pro
    15545                15550                15555

Leu Tyr    Ser Gly Cys Arg Leu    Thr Leu Leu Arg Pro    Glu Lys Arg
    15560                15565                15570

Gly Ala    Ala Thr Gly Val Asp    Thr Ile Cys Thr His    Arg Leu Asp
    15575                15580                15585

Pro Leu    Asn Pro Gly Leu Asp    Arg Glu Gln Leu Tyr    Trp Glu Leu
    15590                15595                15600

Ser Lys    Leu Thr Arg Gly Ile    Ile Glu Leu Gly Pro    Tyr Leu Leu
    15605                15610                15615

Asp Arg    Gly Ser Leu Tyr Val    Asn Gly Phe Thr His    Arg Thr Ser
    15620                15625                15630

Val Pro    Thr Thr Ser Thr Pro    Gly Thr Ser Thr Val    Asp Leu Gly
    15635                15640                15645

Thr Ser Gly Thr Pro Phe Ser     Leu Pro Ser Pro Ala     Xaa Xaa Xaa
    15650               15655               15660

Pro Leu Leu Xaa Pro Phe Thr     Xaa Asn Xaa Thr Ile     Thr Asn Leu
    15665               15670               15675

Xaa Xaa Xaa Xaa Xaa Met Xaa     Xaa Pro Gly Ser Arg     Lys Phe Asn
    15680               15685               15690

Thr Thr Glu Arg Val Leu Gln     Thr Leu Leu Gly Pro     Met Phe Lys
    15695               15700               15705

Asn Thr Ser Val Gly Leu Leu     Tyr Ser Gly Cys Arg     Leu Thr Leu
    15710               15715               15720

Leu Arg Ser Glu Lys Asp Gly     Ala Ala Thr Gly Val     Asp Ala Ile
    15725               15730               15735

Cys Thr His Arg Leu Asp Pro     Lys Ser Pro Gly Val     Asp Arg Glu
    15740               15745               15750

Gln Leu Tyr Trp Glu Leu Ser     Gln Leu Thr Asn Gly     Ile Lys Glu
    15755               15760               15765

Leu Gly Pro Tyr Thr Leu Asp     Arg Asn Ser Leu Tyr     Val Asn Gly
    15770               15775               15780

Phe Thr His Trp Ile Pro Val     Pro Thr Ser Ser Thr     Pro Gly Thr
    15785               15790               15795

Ser Thr Val Asp Leu Gly Ser     Gly Thr Pro Ser Ser     Leu Pro Ser
    15800               15805               15810

Pro Thr Thr Ala Gly Pro Leu     Leu Val Pro Phe Thr     Leu Asn Phe
    15815               15820               15825

Thr Ile Thr Asn Leu Lys Tyr     Glu Glu Asp Met His     Cys Pro Gly
    15830               15835               15840

Ser Arg Lys Phe Asn Thr Thr     Glu Arg Val Leu Gln     Ser Leu Leu
    15845               15850               15855

Gly Pro Met Phe Lys Asn Thr     Ser Val Gly Pro Leu     Tyr Ser Gly
    15860               15865               15870

Cys Arg Leu Thr Leu Leu Arg     Ser Glu Lys Asp Gly     Ala Ala Thr
    15875               15880               15885

281

Gly Val Asp Ala Ile Cys Thr His Arg Leu Asp Pro Lys Ser Pro
15890                15895           15900

Gly Val Asp Arg Glu Gln Leu Tyr Trp Glu Leu Ser Gln Leu Thr
15905                15910           15915

Asn Gly Ile Lys Glu Leu Gly Pro Tyr Thr Leu Asp Arg Asn Ser
15920                15925           15930

Leu Tyr Val Asn Gly Phe Thr His Gln Thr Ser Ala Pro Asn Thr
15935                15940           15945

Ser Thr Pro Gly Thr Ser Thr Val Asp Leu Gly Thr Ser Gly Thr
15950                15955           15960

Pro Ser Ser Leu Pro Ser Pro Thr Xaa Xaa Xaa Pro Leu Leu Xaa
15965                15970           15975

Pro Phe Thr Xaa Asn Xaa Thr Ile Thr Asn Leu Xaa Xaa Xaa Xaa
15980                15985           15990

Xaa Met Xaa Xaa Pro Gly Ser Arg Lys Phe Asn Thr Thr Glu Xaa
15995                16000           16005

Val Leu Gln Gly Leu Leu Xaa Pro Xaa Phe Lys Asn Xaa Ser Val
16010                16015           16020

Gly Xaa Leu Tyr Ser Gly Cys Arg Leu Thr Xaa Leu Arg Xaa Glu
16025                16030           16035

Lys Xaa Gly Ala Ala Thr Gly Xaa Asp Ala Ile Cys Xaa His Xaa
16040                16045           16050

Xaa Xaa Pro Lys Xaa Pro Gly Leu Xaa Xaa Glu Xaa Leu Tyr Trp
16055                16060           16065

Glu Leu Ser Xaa Leu Thr Xaa Xaa Ile Xaa Glu Leu Gly Pro Tyr
16070                16075           16080

Thr Leu Asp Arg Xaa Ser Leu Tyr Val Asn Gly Phe Thr His Trp
16085                16090           16095

Ile Pro Val Pro Thr Ser Ser Thr Pro Gly Thr Ser Thr Val Asp
16100                16105           16110

Leu Gly Ser Gly Thr Pro Ser Ser Leu Pro Ser Pro Thr Thr Ala

16115                16120                16125

Gly Pro Leu Leu Val Pro Phe  Thr Leu Asn Phe Thr  Ile Thr Asn
16130             16135            16140

Leu Lys Tyr Glu Glu Asp Met  His Cys Pro Gly Ser  Arg Lys Phe
16145             16150            16155

Asn Thr Thr Glu Arg Val Leu  Gln Ser Leu Leu Gly  Pro Met Phe
16160             16165            16170

Lys Asn Thr Ser Val Gly Pro  Leu Tyr Ser Gly Cys  Arg Leu Thr
16175             16180            16185

Ser Leu Arg Ser Glu Lys Asp  Gly Ala Ala Thr Gly  Val Asp Ala
16190             16195            16200

Ile Cys Thr His Arg Val Asp  Pro Lys Ser Pro Gly  Val Asp Arg
16205             16210            16215

Glu Gln Leu Tyr Trp Glu Leu  Ser Gln Leu Thr Asn  Gly Ile Lys
16220             16225            16230

Glu Leu Gly Pro Tyr Thr Leu  Asp Arg Asn Ser Leu  Tyr Val Asn
16235             16240            16245

Gly Phe Thr His Gln Thr Ser  Ala Pro Asn Thr Ser  Thr Pro Gly
16250             16255            16260

Thr Ser Thr Val Xaa Xaa Gly  Thr Ser Gly Thr Pro  Ser Ser Xaa
16265             16270            16275

Pro Xaa Xaa Thr Ser Ala Gly  Pro Leu Leu Val Pro  Phe Thr Leu
16280             16285            16290

Asn Phe Thr Ile Thr Asn Leu  Gln Tyr Glu Glu Asp  Met His His
16295             16300            16305

Pro Gly Ser Arg Lys Phe Asn  Thr Thr Glu Arg Val  Leu Gln Gly
16310             16315            16320

Leu Leu Gly Pro Met Phe Lys  Asn Thr Ser Val Gly  Leu Leu Tyr
16325             16330            16335

Ser Gly Cys Arg Leu Thr Leu  Leu Arg Pro Glu Lys  Asn Gly Ala
16340             16345            16350

```
Thr Thr   Gly Met Asp Ala Ile   Cys Thr His Arg Leu   Asp Pro Lys
    16355                 16360              16365

Ser Pro   Gly Leu Xaa Xaa Glu   Xaa Leu Tyr Trp Glu   Leu Ser Xaa
    16370                 16375              16380

Leu Thr   Xaa Xaa Ile Xaa Glu   Leu Gly Pro Tyr Thr   Leu Asp Arg
    16385                 16390              16395

Xaa Ser   Leu Tyr Val Asn Gly   Phe Thr His Xaa Xaa   Ser Xaa Pro
    16400                 16405              16410

Thr Thr   Ser Thr Pro Gly Thr   Ser Thr Val Xaa Xaa   Gly Thr Ser
    16415                 16420              16425

Gly Thr   Pro Ser Ser Xaa Pro   Xaa Xaa Thr Xaa Xaa   Xaa Pro Leu
    16430                 16435              16440

Leu Xaa   Pro Phe Thr Xaa Asn   Xaa Thr Ile Thr Asn   Leu Xaa Xaa
    16445                 16450              16455

Xaa Xaa   Xaa Met Xaa Xaa Pro   Gly Ser Arg Lys Phe   Asn Thr Thr
    16460                 16465              16470

Glu Arg   Val Leu Gln Gly Leu   Leu Lys Pro Leu Phe   Arg Asn Ser
    16475                 16480              16485

Ser Leu   Glu Tyr Leu Tyr Ser   Gly Cys Arg Leu Ala   Ser Leu Arg
    16490                 16495              16500

Pro Glu   Lys Asp Ser Ser Ala   Met Ala Val Asp Ala   Ile Cys Thr
    16505                 16510              16515

His Arg   Pro Asp Pro Glu Asp   Leu Gly Leu Asp Arg   Glu Arg Leu
    16520                 16525              16530

Tyr Trp   Glu Leu Ser Asn Leu   Thr Asn Gly Ile Gln   Glu Leu Gly
    16535                 16540              16545

Pro Tyr   Thr Leu Asp Arg Asn   Ser Leu Tyr Val Asn   Gly Phe Thr
    16550                 16555              16560

His Arg   Ser Ser Met Pro Thr   Thr Ser Thr Pro Gly   Thr Ser Thr
    16565                 16570              16575

Val Asp   Val Gly Thr Ser Gly   Thr Pro Ser Ser Ser   Pro Ser Pro
    16580                 16585              16590
```

284

Thr Thr Ala Gly Pro Leu Leu Ile Pro Phe Thr Leu Asn Phe Thr
16595 16600 16605

Ile Thr Asn Leu Gln Tyr Gly Glu Asp Met Gly His Pro Gly Ser
16610 16615 16620

Arg Lys Phe Asn Thr Thr Glu Arg Val Leu Gln Gly Leu Leu Gly
16625 16630 16635

Pro Ile Phe Lys Asn Thr Ser Val Gly Pro Leu Tyr Ser Gly Cys
16640 16645 16650

Arg Leu Thr Ser Leu Arg Ser Glu Lys Asp Gly Ala Ala Thr Gly
16655 16660 16665

Val Asp Ala Ile Cys Ile His His Leu Asp Pro Lys Ser Pro Gly
16670 16675 16680

Leu Asn Arg Glu Arg Leu Tyr Trp Glu Leu Ser Gln Leu Thr Asn
16685 16690 16695

Gly Ile Lys Glu Leu Gly Pro Tyr Thr Leu Asp Arg Asn Ser Leu
16700 16705 16710

Tyr Val Asn Gly Phe Thr His Arg Thr Ser Val Pro Thr Thr Ser
16715 16720 16725

Thr Pro Gly Thr Ser Thr Val Asp Leu Gly Thr Ser Gly Thr Pro
16730 16735 16740

Phe Ser Leu Pro Ser Pro Ala Thr Ala Gly Pro Leu Leu Val Leu
16745 16750 16755

Phe Thr Leu Asn Phe Thr Ile Thr Asn Leu Lys Tyr Glu Glu Asp
16760 16765 16770

Met His Arg Pro Gly Ser Arg Lys Phe Asn Thr Thr Glu Arg Val
16775 16780 16785

Leu Gln Thr Leu Leu Gly Pro Met Phe Lys Asn Thr Ser Val Gly
16790 16795 16800

Leu Leu Tyr Ser Gly Cys Arg Leu Thr Leu Leu Arg Ser Glu Lys
16805 16810 16815

Asp Gly Ala Ala Thr Gly Val Asp Ala Ile Cys Thr His Arg Leu
16820 16825 16830

285

Asp Pro Lys Ser Pro Gly Leu Xaa Xaa Glu Xaa Leu Tyr Trp Glu
    16835              16840              16845

Leu Ser Xaa Leu Thr Xaa Xaa Ile Xaa Glu Leu Gly Pro Tyr Thr
    16850              16855              16860

Leu Asp Arg Xaa Ser Leu Tyr Val Asn Gly Phe Thr His Xaa Xaa
    16865              16870              16875

Ser Xaa Pro Thr Thr Ser Thr Pro Gly Thr Ser Thr Val Xaa Xaa
    16880              16885              16890

Gly Thr Ser Gly Thr Pro Ser Ser Xaa Pro Xaa Xaa Thr Xaa Xaa
    16895              16900              16905

Xaa Pro Leu Leu Xaa Pro Phe Thr Xaa Asn Xaa Thr Ile Thr Asn
    16910              16915              16920

Leu Xaa Xaa Xaa Xaa Xaa Met Xaa Xaa Pro Gly Ser Arg Lys Phe
    16925              16930              16935

Asn Thr Thr Glu Arg Val Leu Gln Gly Leu Leu Arg Pro Val Phe
    16940              16945              16950

Lys Asn Thr Ser Val Gly Pro Leu Tyr Ser Gly Cys Arg Leu Thr
    16955              16960              16965

Leu Leu Arg Pro Lys Lys Asp Gly Ala Ala Thr Lys Val Asp Ala
    16970              16975              16980

Ile Cys Thr Tyr Arg Pro Asp Pro Lys Ser Pro Gly Leu Asp Arg
    16985              16990              16995

Glu Gln Leu Tyr Trp Glu Leu Ser Gln Leu Thr His Ser Ile Thr
    17000              17005              17010

Glu Leu Gly Pro Tyr Thr Gln Asp Arg Asp Ser Leu Tyr Val Asn
    17015              17020              17025

Gly Phe Thr His Arg Ser Ser Val Pro Thr Thr Ser Ile Pro Gly
    17030              17035              17040

Thr Ser Ala Val His Leu Glu Thr Thr Gly Thr Pro Ser Ser Phe
    17045              17050              17055

Pro Gly His Thr Glu Pro Gly Pro Leu Leu Ile Pro Phe Thr Phe

286

17060                    17065                         17070

Asn Phe  Thr Ile Thr Asn Leu  Arg Tyr Glu Glu Asn  Met Gln His
         17075                 17080                 17085

Pro Gly  Ser Arg Lys Phe Asn  Thr Thr Glu Arg Val  Leu Gln Gly
         17090                 17095                 17100

Leu Leu  Thr Pro Leu Phe Lys  Asn Thr Ser Val Gly  Pro Leu Tyr
         17105                 17110                 17115

Ser Gly  Cys Arg Leu Thr Leu  Leu Arg Pro Glu Lys  Gln Glu Ala
         17120                 17125                 17130

Ala Thr  Gly Val Asp Thr Ile  Cys Thr His Arg Val  Asp Pro Ile
         17135                 17140                 17145

Gly Pro  Gly Leu Asp Arg Glu  Arg Leu Tyr Trp Glu  Leu Ser Gln
         17150                 17155                 17160

Leu Thr  Asn Ser Ile Thr Glu  Leu Gly Pro Tyr Thr  Leu Asp Arg
         17165                 17170                 17175

Asp Ser  Leu Tyr Val Asp Gly  Phe Asn Pro Trp Ser  Ser Val Pro
         17180                 17185                 17190

Thr Thr  Ser Thr Pro Gly Thr  Ser Thr Val His Leu  Ala Thr Ser
         17195                 17200                 17205

Gly Thr  Pro Ser Pro Leu Pro  Gly His Thr Ala Pro  Val Pro Leu
         17210                 17215                 17220

Leu Ile  Pro Phe Thr Leu Asn  Phe Thr Ile Thr Asp  Leu His Tyr
         17225                 17230                 17235

Glu Glu  Asn Met Gln His Pro  Gly Ser Arg Lys Phe  Asn Thr Thr
         17240                 17245                 17250

Glu Arg  Val Leu Gln Gly Leu  Leu Lys Pro Leu Phe  Lys Ser Thr
         17255                 17260                 17265

Ser Val  Gly Pro Leu Tyr Ser  Gly Cys Arg Leu Thr  Leu Leu Arg
         17270                 17275                 17280

Pro Glu  Lys His Gly Ala Ala  Thr Gly Val Asp Ala  Ile Cys Thr
         17285                 17290                 17295

```
Leu Arg   Leu Asp Pro Thr Gly   Pro Gly Leu Asp Arg   Glu Arg Leu
    17300                 17305                 17310

Tyr Trp   Glu Leu Ser Gln Leu   Thr Asn Ser Ile Thr   Glu Leu Gly
    17315                 17320                 17325

Pro Tyr   Thr Leu Asp Arg Asp   Ser Leu Tyr Val Asn   Gly Phe Asn
    17330                 17335                 17340

Pro Trp   Ser Ser Val Pro Thr   Thr Ser Thr Pro Gly   Thr Ser Thr
    17345                 17350                 17355

Val His   Leu Ala Thr Ser Gly   Thr Pro Ser Ser Leu   Pro Gly His
    17360                 17365                 17370

Thr Thr   Ala Gly Pro Leu Leu   Val Pro Phe Thr Leu   Asn Phe Thr
    17375                 17380                 17385

Ile Thr   Asn Leu Lys Tyr Glu   Glu Asp Met His Cys   Pro Gly Ser
    17390                 17395                 17400

Arg Lys   Phe Asn Thr Thr Glu   Arg Val Leu Gln Ser   Leu His Gly
    17405                 17410                 17415

Pro Met   Phe Lys Asn Thr Ser   Val Gly Pro Leu Tyr   Ser Gly Cys
    17420                 17425                 17430

Arg Leu   Thr Leu Leu Arg Ser   Glu Lys Asp Gly Ala   Ala Thr Gly
    17435                 17440                 17445

Val Asp   Ala Ile Cys Thr His   Arg Leu Asp Pro Lys   Ser Pro Gly
    17450                 17455                 17460

Leu Xaa   Xaa Glu Xaa Leu Tyr   Trp Glu Leu Ser Xaa   Leu Thr Xaa
    17465                 17470                 17475

Xaa Ile   Xaa Glu Leu Gly Pro   Tyr Thr Leu Asp Arg   Xaa Ser Leu
    17480                 17485                 17490

Tyr Val   Asn Gly Phe Thr His   Xaa Xaa Ser Xaa Pro   Thr Thr Ser
    17495                 17500                 17505

Thr Pro   Gly Thr Ser Thr Val   Xaa Xaa Gly Thr Ser   Gly Thr Pro
    17510                 17515                 17520

Ser Ser   Xaa Pro Xaa Xaa Thr   Xaa Xaa Xaa Pro Leu   Leu Xaa Pro
    17525                 17530                 17535
```

```
Phe Thr Xaa Asn Xaa Thr Ile   Thr Asn Leu Xaa Xaa   Xaa Xaa Xaa
    17540           17545                   17550

Met Xaa Xaa Pro Gly Ser Arg   Lys Phe Asn Thr Thr   Glu Xaa Val
    17555           17560                   17565

Leu Gln Gly Leu Leu Xaa Pro   Xaa Phe Lys Asn Xaa   Ser Val Gly
    17570           17575                   17580

Xaa Leu Tyr Ser Gly Cys Arg   Leu Thr Xaa Leu Arg   Xaa Glu Lys
    17585           17590                   17595

Xaa Gly Ala Ala Thr Gly Xaa   Asp Ala Ile Cys Xaa   His Xaa Xaa
    17600           17605                   17610

Xaa Pro Lys Xaa Pro Gly Leu   Xaa Xaa Glu Xaa Leu   Tyr Trp Glu
    17615           17620                   17625

Leu Ser Xaa Leu Thr Asn Ser   Ile Thr Glu Leu Gly   Pro Tyr Thr
    17630           17635                   17640

Leu Asp Arg Asp Ser Leu Tyr   Val Asn Gly Phe Thr   His Arg Ser
    17645           17650                   17655

Ser Met Pro Thr Thr Ser Ile   Pro Gly Thr Ser Ala   Val His Leu
    17660           17665                   17670

Glu Thr Ser Gly Thr Pro Ala   Ser Leu Pro Gly His   Thr Ala Pro
    17675           17680                   17685

Gly Pro Leu Leu Val Pro Phe   Thr Leu Asn Phe Thr   Ile Thr Asn
    17690           17695                   17700

Leu Gln Tyr Glu Glu Asp Met   Arg His Pro Gly Ser   Arg Lys Phe
    17705           17710                   17715

Asn Thr Thr Glu Arg Val Leu   Gln Gly Leu Leu Lys   Pro Leu Phe
    17720           17725                   17730

Lys Ser Thr Ser Val Gly Pro   Leu Tyr Ser Gly Cys   Arg Leu Thr
    17735           17740                   17745

Leu Leu Arg Pro Glu Lys Arg   Gly Ala Ala Thr Gly   Val Asp Thr
    17750           17755                   17760

Ile Cys Thr His Arg Leu Asp   Pro Leu Asn Pro Gly   Leu Xaa Xaa
    17765           17770                   17775
```

```
Glu Xaa   Leu Tyr Trp Glu Leu   Ser Xaa Leu Thr Xaa   Xaa Ile Xaa
    17780                 17785                 17790

Glu Leu   Gly Pro Tyr Thr Leu   Asp Arg Xaa Ser Leu   Tyr Val Asn
    17795                 17800                 17805

Gly Phe   Thr His Xaa Xaa Ser   Xaa Pro Thr Thr Ser   Thr Pro Gly
    17810                 17815                 17820

Thr Ser   Thr Val Xaa Xaa Gly   Thr Ser Gly Thr Pro   Ser Ser Xaa
    17825                 17830                 17835

Pro Xaa   Xaa Thr Xaa Xaa Xaa   Pro Leu Leu Xaa Pro   Phe Thr Xaa
    17840                 17845                 17850

Asn Xaa   Thr Ile Thr Asn Leu   Xaa Xaa Xaa Xaa Xaa   Met Xaa Xaa
    17855                 17860                 17865

Pro Gly   Ser Arg Lys Phe Asn   Thr Thr Glu Xaa Val   Leu Gln Gly
    17870                 17875                 17880

Leu Leu   Xaa Pro Xaa Phe Lys   Asn Xaa Ser Val Gly   Xaa Leu Tyr
    17885                 17890                 17895

Ser Gly   Cys Arg Leu Thr Xaa   Leu Arg Xaa Glu Lys   Xaa Gly Ala
    17900                 17905                 17910

Ala Thr   Gly Xaa Asp Ala Ile   Cys Xaa His Xaa Xaa   Xaa Pro Lys
    17915                 17920                 17925

Xaa Pro   Gly Leu Xaa Xaa Glu   Xaa Leu Tyr Trp Glu   Leu Ser Xaa
    17930                 17935                 17940

Leu Thr   Xaa Xaa Ile Xaa Glu   Leu Gly Pro Tyr Thr   Leu Asp Arg
    17945                 17950                 17955

Xaa Ser   Leu Tyr Val Asn Gly   Phe His Pro Arg Ser   Ser Val Pro
    17960                 17965                 17970

Thr Thr   Ser Thr Pro Gly Thr   Ser Thr Val His Leu   Ala Thr Ser
    17975                 17980                 17985

Gly Thr   Pro Ser Ser Leu Pro   Gly His Thr Ala Pro   Val Pro Leu
    17990                 17995                 18000

Leu Ile   Pro Phe Thr Leu Asn   Phe Thr Ile Thr Asn   Leu His Tyr
```

18005      18010      18015

Glu Glu Asn Met Gln His Pro Gly Ser Arg Lys Phe Asn Thr Thr
  18020      18025      18030

Glu Arg Val Leu Gln Gly Leu Leu Gly Pro Met Phe Lys Asn Thr
  18035      18040      18045

Ser Val Gly Leu Leu Tyr Ser Gly Cys Arg Leu Thr Leu Leu Arg
  18050      18055      18060

Pro Glu Lys Asn Gly Ala Ala Thr Gly Met Asp Ala Ile Cys Ser
  18065      18070      18075

His Arg Leu Asp Pro Lys Ser Pro Gly Leu Xaa Xaa Glu Xaa Leu
  18080      18085      18090

Tyr Trp Glu Leu Ser Xaa Leu Thr Xaa Xaa Ile Xaa Glu Leu Gly
  18095      18100      18105

Pro Tyr Thr Leu Asp Arg Xaa Ser Leu Tyr Val Asn Gly Phe Thr
  18110      18115      18120

His Xaa Xaa Ser Xaa Pro Thr Thr Ser Thr Pro Gly Thr Ser Thr
  18125      18130      18135

Val Xaa Xaa Gly Thr Ser Gly Thr Pro Ser Ser Xaa Pro Xaa Xaa
  18140      18145      18150

Thr Xaa Xaa Xaa Pro Leu Leu Xaa Pro Phe Thr Xaa Asn Xaa Thr
  18155      18160      18165

Ile Thr Asn Leu Xaa Xaa Xaa Xaa Xaa Met Xaa Xaa Pro Gly Ser
  18170      18175      18180

Arg Lys Phe Asn Thr Thr Glu Xaa Val Leu Gln Gly Leu Leu Xaa
  18185      18190      18195

Pro Xaa Phe Lys Asn Xaa Ser Val Gly Xaa Leu Tyr Ser Gly Cys
  18200      18205      18210

Arg Leu Thr Xaa Leu Arg Xaa Glu Lys Xaa Gly Ala Ala Thr Gly
  18215      18220      18225

Xaa Asp Ala Ile Cys Xaa His Xaa Xaa Xaa Pro Lys Xaa Pro Gly
  18230      18235      18240

```
Leu Xaa   Xaa Glu Xaa Leu Tyr   Trp Glu Leu Ser Xaa   Leu Thr Xaa
    18245                 18250              18255

Xaa Ile   Xaa Glu Leu Gly Pro   Tyr Thr Leu Asp Arg   Xaa Ser Leu
    18260                 18265              18270

Tyr Val   Asn Gly Phe Thr His   Gln Asn Ser Val Pro   Thr Thr Ser
    18275                 18280              18285

Thr Pro   Gly Thr Ser Thr Val   Tyr Trp Ala Thr Thr   Gly Thr Pro
    18290                 18295              18300

Ser Ser   Phe Pro Gly His Thr   Glu Pro Gly Pro Leu   Leu Ile Pro
    18305                 18310              18315

Phe Thr   Phe Asn Phe Thr Ile   Thr Asn Leu His Tyr   Glu Glu Asn
    18320                 18325              18330

Met Gln   His Pro Gly Ser Arg   Lys Phe Asn Thr Thr   Glu Arg Val
    18335                 18340              18345

Leu Gln   Gly Leu Leu Thr Pro   Leu Phe Lys Asn Thr   Ser Val Gly
    18350                 18355              18360

Pro Leu   Tyr Ser Gly Cys Arg   Leu Thr Leu Leu Arg   Pro Glu Lys
    18365                 18370              18375

Gln Glu   Ala Ala Thr Gly Val   Asp Thr Ile Cys Thr   His Arg Val
    18380                 18385              18390

Asp Pro   Ile Gly Pro Gly Leu   Xaa Xaa Glu Xaa Leu   Tyr Trp Glu
    18395                 18400              18405

Leu Ser   Xaa Leu Thr Xaa Xaa   Ile Xaa Glu Leu Gly   Pro Tyr Thr
    18410                 18415              18420

Leu Asp   Arg Xaa Ser Leu Tyr   Val Asn Gly Phe Thr   His Xaa Xaa
    18425                 18430              18435

Ser Xaa   Pro Thr Thr Ser Thr   Pro Gly Thr Ser Thr   Val Xaa Xaa
    18440                 18445              18450

Gly Thr   Ser Gly Thr Pro Ser   Ser Xaa Pro Xaa Xaa   Thr Xaa Xaa
    18455                 18460              18465

Xaa Pro   Leu Leu Xaa Pro Phe   Thr Xaa Asn Xaa Thr   Ile Thr Asn
    18470                 18475              18480
```

292

```
Leu Xaa Xaa Xaa Xaa Xaa Met     Xaa Xaa Pro Gly Ser     Arg Lys Phe
    18485               18490             18495

Asn Thr Thr Glu Xaa Val Leu     Gln Gly Leu Leu Xaa     Pro Xaa Phe
    18500               18505             18510

Lys Asn Xaa Ser Val Gly Xaa     Leu Tyr Ser Gly Cys     Arg Leu Thr
    18515               18520             18525

Xaa Leu Arg Xaa Glu Lys Xaa     Gly Ala Ala Thr Gly     Xaa Asp Ala
    18530               18535             18540

Ile Cys Xaa His Xaa Xaa Xaa     Pro Lys Xaa Pro Gly     Leu Xaa Xaa
    18545               18550             18555

Glu Xaa Leu Tyr Trp Glu Leu     Ser Xaa Leu Thr Xaa     Xaa Ile Xaa
    18560               18565             18570

Glu Leu Gly Pro Tyr Thr Leu     Asp Arg Xaa Ser Leu     Tyr Val Asn
    18575               18580             18585

Gly Phe Thr His Arg Ser Ser     Val Pro Thr Thr Ser     Ser Pro Gly
    18590               18595             18600

Thr Ser Thr Val His Leu Ala     Thr Ser Gly Thr Pro     Ser Ser Leu
    18605               18610             18615

Pro Gly His Thr Ala Pro Val     Pro Leu Leu Ile Pro     Phe Thr Leu
    18620               18625             18630

Asn Phe Thr Ile Thr Asn Leu     His Tyr Glu Glu Asn     Met Gln His
    18635               18640             18645

Pro Gly Ser Arg Lys Phe Asn     Thr Thr Glu Arg Val     Leu Gln Gly
    18650               18655             18660

Leu Leu Lys Pro Leu Phe Lys     Ser Thr Ser Val Gly     Pro Leu Tyr
    18665               18670             18675

Ser Gly Cys Arg Leu Thr Leu     Leu Arg Pro Glu Lys     His Gly Ala
    18680               18685             18690

Ala Thr Gly Val Asp Ala Ile     Cys Thr Leu Arg Leu     Asp Pro Thr
    18695               18700             18705

Gly Pro Gly Leu Xaa Xaa Glu     Xaa Leu Tyr Trp Glu     Leu Ser Xaa
    18710               18715             18720
```

293

```
Leu Thr Xaa Xaa Ile Xaa Glu    Leu Gly Pro Tyr Thr    Leu Asp Arg
    18725               18730            18735

Xaa Ser Leu Tyr Val Asn Gly    Phe Thr His Xaa Xaa    Ser Xaa Pro
    18740               18745            18750

Thr Thr Ser Thr Pro Gly Thr    Ser Thr Val Xaa Xaa    Gly Thr Ser
    18755               18760            18765

Gly Thr Pro Ser Ser Xaa Pro    Xaa Xaa Thr Xaa Xaa    Xaa Pro Leu
    18770               18775            18780

Leu Xaa Pro Phe Thr Xaa Asn    Xaa Thr Ile Thr Asn    Leu Xaa Xaa
    18785               18790            18795

Xaa Xaa Xaa Met Xaa Xaa Pro    Gly Ser Arg Lys Phe    Asn Thr Thr
    18800               18805            18810

Glu Xaa Val Leu Gln Gly Leu    Leu Xaa Pro Xaa Phe    Lys Asn Xaa
    18815               18820            18825

Ser Val Gly Xaa Leu Tyr Ser    Gly Cys Arg Leu Thr    Xaa Leu Arg
    18830               18835            18840

Xaa Glu Lys Xaa Gly Ala Ala    Thr Gly Xaa Asp Ala    Ile Cys Xaa
    18845               18850            18855

His Xaa Xaa Xaa Pro Lys Xaa    Pro Gly Leu Xaa Xaa    Glu Xaa Leu
    18860               18865            18870

Tyr Trp Glu Leu Ser Xaa Leu    Thr Xaa Xaa Ile Xaa    Glu Leu Gly
    18875               18880            18885

Pro Tyr Thr Leu Asp Arg Xaa    Ser Leu Tyr Val Asn    Gly Phe Thr
    18890               18895            18900

His Arg Thr Ser Val Pro Thr    Thr Ser Thr Pro Gly    Thr Ser Thr
    18905               18910            18915

Val His Leu Ala Thr Ser Gly    Thr Pro Ser Ser Leu    Pro Gly His
    18920               18925            18930

Thr Ala Pro Val Pro Leu Leu    Ile Pro Phe Thr Leu    Asn Phe Thr
    18935               18940            18945

Ile Thr Asn Leu Gln Tyr Glu    Glu Asp Met His Arg    Pro Gly Ser
```

18950                    18955                         18960


Arg Lys    Phe Asn Thr Thr Glu    Arg Val Leu Gln Gly    Leu Leu Ser
    18965                  18970                  18975


Pro Ile    Phe Lys Asn Ser Ser    Val Gly Pro Leu Tyr    Ser Gly Cys
    18980                  18985                  18990


Arg Leu    Thr Ser Leu Arg Pro    Glu Lys Asp Gly Ala    Ala Thr Gly
    18995                  19000                  19005


Met Asp    Ala Val Cys Leu Tyr    His Pro Asn Pro Lys    Arg Pro Gly
    19010                  19015                  19020


Leu Asp    Arg Glu Gln Leu Tyr    Cys Glu Leu Ser Gln    Leu Thr His
    19025                  19030                  19035


Asn Ile    Thr Glu Leu Gly Pro    Tyr Ser Leu Asp Arg    Asp Ser Leu
    19040                  19045                  19050


Tyr Val    Asn Gly Phe Thr His    Gln Asn Ser Val Pro    Thr Thr Ser
    19055                  19060                  19065


Thr Pro    Gly Thr Ser Thr Val    Tyr Trp Ala Thr Thr    Gly Thr Pro
    19070                  19075                  19080


Ser Ser    Phe Pro Gly His Thr    Xaa Xaa Xaa Pro Leu    Leu Xaa Pro
    19085                  19090                  19095


Phe Thr    Xaa Asn Xaa Thr Ile    Thr Asn Leu Xaa Xaa    Xaa Xaa Xaa
    19100                  19105                  19110


Met Xaa    Xaa Pro Gly Ser Arg    Lys Phe Asn Thr Thr    Glu Xaa Val
    19115                  19120                  19125


Leu Gln    Gly Leu Leu Xaa Pro    Xaa Phe Lys Asn Xaa    Ser Val Gly
    19130                  19135                  19140


Xaa Leu    Tyr Ser Gly Cys Arg    Leu Thr Xaa Leu Arg    Xaa Glu Lys
    19145                  19150                  19155


Xaa Gly    Ala Ala Thr Gly Xaa    Asp Ala Ile Cys Xaa    His Xaa Xaa
    19160                  19165                  19170


Xaa Pro    Lys Xaa Pro Gly Leu    Xaa Xaa Glu Xaa Leu    Tyr Trp Glu
    19175                  19180                  19185

```
Leu Ser Xaa Leu Thr Xaa Xaa     Ile Xaa Glu Leu Gly     Pro Tyr Thr
    19190                 19195                 19200

Leu Asp Arg Xaa Ser Leu Tyr     Val Asn Gly Phe Thr     His Trp Ser
    19205                 19210                 19215

Ser Gly Leu Thr Thr Ser Thr     Pro Trp Thr Ser Thr     Val Asp Leu
    19220                 19225                 19230

Gly Thr Ser Gly Thr Pro Ser     Pro Val Pro Ser Pro     Thr Thr Ala
    19235                 19240                 19245

Gly Pro Leu Leu Val Pro Phe     Thr Leu Asn Phe Thr     Ile Thr Asn
    19250                 19255                 19260

Leu Gln Tyr Glu Glu Asp Met     His Arg Pro Gly Ser     Arg Lys Phe
    19265                 19270                 19275

Asn Ala Thr Glu Arg Val Leu     Gln Gly Leu Leu Ser     Pro Ile Phe
    19280                 19285                 19290

Lys Asn Thr Ser Val Gly Pro     Leu Tyr Ser Gly Cys     Arg Leu Thr
    19295                 19300                 19305

Leu Leu Arg Pro Glu Lys Gln     Glu Ala Ala Thr Gly     Val Asp Thr
    19310                 19315                 19320

Ile Cys Thr His Arg Val Asp     Pro Ile Gly Pro Gly     Leu Xaa Xaa
    19325                 19330                 19335

Glu Xaa Leu Tyr Trp Glu Leu     Ser Xaa Leu Thr Xaa     Xaa Ile Xaa
    19340                 19345                 19350

Glu Leu Gly Pro Tyr Thr Leu     Asp Arg Xaa Ser Leu     Tyr Val Asn
    19355                 19360                 19365

Gly Phe Thr His Xaa Xaa Ser     Xaa Pro Thr Thr Ser     Thr Pro Gly
    19370                 19375                 19380

Thr Ser Thr Val Xaa Xaa Gly     Thr Ser Gly Thr Pro     Ser Ser Xaa
    19385                 19390                 19395

Pro Xaa Xaa Thr Xaa Xaa Xaa     Pro Leu Leu Xaa Pro     Phe Thr Xaa
    19400                 19405                 19410

Asn Xaa Thr Ile Thr Asn Leu     Xaa Xaa Xaa Xaa Xaa     Met Xaa Xaa
    19415                 19420                 19425
```

```
Pro Gly   Ser Arg Lys Phe Asn   Thr Thr Glu Xaa Val   Leu Gln Gly
    19430                 19435                 19440

Leu Leu   Xaa Pro Xaa Phe Lys   Asn Xaa Ser Val Gly   Xaa Leu Tyr
    19445                 19450                 19455

Ser Gly   Cys Arg Leu Thr Xaa   Leu Arg Xaa Glu Lys   Xaa Gly Ala
    19460                 19465                 19470

Ala Thr   Gly Xaa Asp Ala Ile   Cys Xaa His Xaa Xaa   Xaa Pro Lys
    19475                 19480                 19485

Xaa Pro   Gly Leu Xaa Xaa Glu   Xaa Leu Tyr Trp Glu   Leu Ser Xaa
    19490                 19495                 19500

Leu Thr   Xaa Xaa Ile Xaa Glu   Leu Gly Pro Tyr Thr   Leu Asp Arg
    19505                 19510                 19515

Xaa Ser   Leu Tyr Val Asn Gly   Phe Thr His Arg Ser   Phe Gly Leu
    19520                 19525                 19530

Thr Thr   Ser Thr Pro Trp Thr   Ser Thr Val Asp Leu   Gly Thr Ser
    19535                 19540                 19545

Gly Thr   Pro Ser Pro Val Pro   Ser Pro Thr Thr Ala   Gly Pro Leu
    19550                 19555                 19560

Leu Val   Pro Phe Thr Leu Asn   Phe Thr Ile Thr Asn   Leu Gln Tyr
    19565                 19570                 19575

Glu Glu   Asp Met His Arg Pro   Gly Ser Arg Lys Phe   Asn Thr Thr
    19580                 19585                 19590

Glu Arg   Val Leu Gln Gly Leu   Leu Thr Pro Leu Phe   Arg Asn Thr
    19595                 19600                 19605

Ser Val   Ser Ser Leu Tyr Ser   Gly Cys Arg Leu Thr   Leu Leu Arg
    19610                 19615                 19620

Pro Glu   Lys Asp Gly Ala Ala   Thr Arg Val Asp Ala   Val Cys Thr
    19625                 19630                 19635

His Arg   Pro Asp Pro Lys Ser   Pro Gly Leu Xaa Xaa   Glu Xaa Leu
    19640                 19645                 19650

Tyr Trp   Glu Leu Ser Xaa Leu   Thr Xaa Xaa Ile Xaa   Glu Leu Gly
    19655                 19660                 19665
```

297

```
Pro Tyr   Thr Leu Asp Arg Xaa   Ser Leu Tyr Val Asn   Gly Phe Thr
    19670                19675                19680

His Xaa   Xaa Ser Xaa Pro Thr   Thr Ser Thr Pro Gly   Thr Ser Thr
    19685                19690                19695

Val Xaa   Xaa Gly Thr Ser Gly   Thr Pro Ser Ser Xaa   Pro Xaa Xaa
    19700                19705                19710

Thr Xaa   Xaa Xaa Pro Leu Leu   Xaa Pro Phe Thr Xaa   Asn Xaa Thr
    19715                19720                19725

Ile Thr   Asn Leu Xaa Xaa Xaa   Xaa Xaa Met Xaa Xaa   Pro Gly Ser
    19730                19735                19740

Arg Lys   Phe Asn Thr Thr Glu   Xaa Val Leu Gln Gly   Leu Leu Xaa
    19745                19750                19755

Pro Xaa   Phe Lys Asn Xaa Ser   Val Gly Xaa Leu Tyr   Ser Gly Cys
    19760                19765                19770

Arg Leu   Thr Xaa Leu Arg Xaa   Glu Lys Xaa Gly Ala   Ala Thr Gly
    19775                19780                19785

Xaa Asp   Ala Ile Cys Xaa His   Xaa Xaa Xaa Pro Lys   Xaa Pro Gly
    19790                19795                19800

Leu Xaa   Xaa Glu Xaa Leu Tyr   Trp Glu Leu Ser Xaa   Leu Thr Xaa
    19805                19810                19815

Xaa Ile   Xaa Glu Leu Gly Pro   Tyr Thr Leu Asp Arg   Xaa Ser Leu
    19820                19825                19830

Tyr Val   Asn Gly Phe Thr His   Trp Ile Pro Val Pro   Thr Ser Ser
    19835                19840                19845

Thr Pro   Gly Thr Ser Thr Val   Asp Leu Gly Ser Gly   Thr Pro Ser
    19850                19855                19860

Ser Leu   Pro Ser Pro Thr Thr   Ala Gly Pro Leu Leu   Val Pro Phe
    19865                19870                19875

Thr Leu   Asn Phe Thr Ile Thr   Asn Leu Gln Tyr Gly   Glu Asp Met
    19880                19885                19890

Gly His   Pro Gly Ser Arg Lys   Phe Asn Thr Thr Glu   Arg Val Leu
```

298

19895                    19900                    19905

Gln Gly   Leu Leu Gly Pro Ile   Phe Lys Asn Thr Ser   Val Gly Pro
    19910                 19915                 19920

Leu Tyr   Ser Gly Cys Arg Leu   Thr Ser Leu Arg Ser   Glu Lys Asp
    19925                 19930                 19935

Gly Ala   Ala Thr Gly Val Asp   Ala Ile Cys Ile His   His Leu Asp
    19940                 19945                 19950

Pro Lys   Ser Pro Gly Leu Xaa   Xaa Glu Xaa Leu Tyr   Trp Glu Leu
    19955                 19960                 19965

Ser Xaa   Leu Thr Xaa Xaa Ile   Xaa Glu Leu Gly Pro   Tyr Thr Leu
    19970                 19975                 19980

Asp Arg   Xaa Ser Leu Tyr Val   Asn Gly Phe Thr His   Xaa Xaa Ser
    19985                 19990                 19995

Xaa Pro   Thr Thr Ser Thr Pro   Gly Thr Ser Thr Val   Xaa Xaa Gly
    20000                 20005                 20010

Thr Ser   Gly Thr Pro Ser Ser   Xaa Pro Xaa Xaa Thr   Xaa Xaa Xaa
    20015                 20020                 20025

Pro Leu   Leu Xaa Pro Phe Thr   Xaa Asn Xaa Thr Ile   Thr Asn Leu
    20030                 20035                 20040

Xaa Xaa   Xaa Xaa Xaa Met Xaa   Xaa Pro Gly Ser Arg   Lys Phe Asn
    20045                 20050                 20055

Thr Thr   Glu Xaa Val Leu Gln   Gly Leu Leu Xaa Pro   Xaa Phe Lys
    20060                 20065                 20070

Asn Xaa   Ser Val Gly Xaa Leu   Tyr Ser Gly Cys Arg   Leu Thr Xaa
    20075                 20080                 20085

Leu Arg   Xaa Glu Lys Xaa Gly   Ala Ala Thr Gly Xaa   Asp Ala Ile
    20090                 20095                 20100

Cys Xaa   His Xaa Xaa Xaa Pro   Lys Xaa Pro Gly Leu   Xaa Xaa Glu
    20105                 20110                 20115

Xaa Leu   Tyr Trp Glu Leu Ser   Xaa Leu Thr Xaa Xaa   Ile Xaa Glu
    20120                 20125                 20130

299

```
Leu Gly  Pro Tyr Thr Leu Asp   Arg Xaa Ser Leu Tyr   Val Asn Gly
    20135                20140                20145

Phe Thr  His Gln Thr Phe Ala   Pro Asn Thr Ser Thr   Pro Gly Thr
    20150                20155                20160

Ser Thr  Val Asp Leu Gly Thr   Ser Gly Thr Pro Ser   Ser Leu Pro
    20165                20170                20175

Ser Pro  Thr Ser Ala Gly Pro   Leu Leu Val Pro Phe   Thr Leu Asn
    20180                20185                20190

Phe Thr  Ile Thr Asn Leu Gln   Tyr Glu Glu Asp Met   His His Pro
    20195                20200                20205

Gly Ser  Arg Lys Phe Asn Thr   Thr Glu Arg Val Leu   Gln Gly Leu
    20210                20215                20220

Leu Gly  Pro Met Phe Lys Asn   Thr Ser Val Gly Leu   Leu Tyr Ser
    20225                20230                20235

Gly Cys  Arg Leu Thr Leu Leu   Arg Pro Glu Lys Asn   Gly Ala Ala
    20240                20245                20250

Thr Arg  Val Asp Ala Val Cys   Thr His Arg Pro Asp   Pro Lys Ser
    20255                20260                20265

Pro Gly  Leu Xaa Xaa Glu Xaa   Leu Tyr Trp Glu Leu   Ser Xaa Leu
    20270                20275                20280

Thr Xaa  Xaa Ile Xaa Glu Leu   Gly Pro Tyr Thr Leu   Asp Arg Xaa
    20285                20290                20295

Ser Leu  Tyr Val Asn Gly Phe   Thr His Xaa Xaa Ser   Xaa Pro Thr
    20300                20305                20310

Thr Ser  Thr Pro Gly Thr Ser   Thr Val Xaa Xaa Gly   Thr Ser Gly
    20315                20320                20325

Thr Pro  Ser Ser Xaa Pro Xaa   Xaa Thr Ala Pro Val   Pro Leu Leu
    20330                20335                20340

Ile Pro  Phe Thr Leu Asn Phe   Thr Ile Thr Asn Leu   His Tyr Glu
    20345                20350                20355

Glu Asn  Met Gln His Pro Gly   Ser Arg Lys Phe Asn   Thr Thr Glu
    20360                20365                20370
```

300

Arg Val Leu Gln Gly Leu Leu Lys Pro Leu Phe Lys Ser Thr Ser
20375 20380 20385

Val Gly Pro Leu Tyr Ser Gly Cys Arg Leu Thr Leu Leu Arg Pro
20390 20395 20400

Glu Lys His Gly Ala Ala Thr Gly Val Asp Ala Ile Cys Thr Leu
20405 20410 20415

Arg Leu Asp Pro Thr Gly Pro Gly Leu Asp Arg Glu Arg Leu Tyr
20420 20425 20430

Trp Glu Leu Ser Gln Leu Thr Asn Ser Val Thr Glu Leu Gly Pro
20435 20440 20445

Tyr Thr Leu Asp Arg Asp Ser Leu Tyr Val Asn Gly Phe Thr Gln
20450 20455 20460

Arg Ser Ser Val Pro Thr Thr Ser Ile Pro Gly Thr Ser Ala Val
20465 20470 20475

His Leu Glu Thr Ser Gly Thr Pro Ala Ser Leu Pro Gly His Thr
20480 20485 20490

Ala Pro Gly Pro Leu Leu Val Pro Phe Thr Leu Asn Phe Thr Ile
20495 20500 20505

Thr Asn Leu Gln Tyr Glu Val Asp Met Arg His Pro Gly Ser Arg
20510 20515 20520

Lys Phe Asn Thr Thr Glu Arg Val Leu Gln Gly Leu Leu Lys Pro
20525 20530 20535

Leu Phe Lys Ser Thr Ser Val Gly Pro Leu Tyr Ser Gly Cys Arg
20540 20545 20550

Leu Thr Leu Leu Arg Pro Glu Lys Arg Gly Ala Ala Thr Gly Val
20555 20560 20565

Asp Thr Ile Cys Thr His Arg Leu Asp Pro Leu Asn Pro Gly Leu
20570 20575 20580

Asp Arg Glu Gln Leu Tyr Trp Glu Leu Ser Lys Leu Thr Arg Gly
20585 20590 20595

Ile Ile Glu Leu Gly Pro Tyr Leu Leu Asp Arg Gly Ser Leu Tyr
20600 20605 20610

Val Asn   Gly Phe Thr His Arg   Asn Phe Val Pro Ile   Thr Ser Thr
    20615                 20620           20625

Pro Gly   Thr Ser Thr Val His   Leu Gly Thr Ser Glu   Thr Pro Ser
    20630                 20635           20640

Ser Leu   Pro Arg Pro Ile Val   Pro Gly Pro Leu Leu   Val Pro Phe
    20645                 20650           20655

Thr Leu   Asn Phe Thr Ile Thr   Asn Leu Gln Tyr Glu   Glu Ala Met
    20660                 20665           20670

Arg His   Pro Gly Ser Arg Lys   Phe Asn Thr Thr Glu   Arg Val Leu
    20675                 20680           20685

Gln Gly   Leu Leu Arg Pro Leu   Phe Lys Asn Thr Ser   Ile Gly Pro
    20690                 20695           20700

Leu Tyr   Ser Ser Cys Arg Leu   Thr Leu Leu Arg Pro   Glu Lys Asp
    20705                 20710           20715

Lys Ala   Ala Thr Arg Val Asp   Ala Ile Cys Thr His   His Pro Asp
    20720                 20725           20730

Pro Gln   Ser Pro Gly Leu Asn   Arg Glu Gln Leu Tyr   Trp Glu Leu
    20735                 20740           20745

Ser Gln   Leu Thr His Gly Ile   Thr Glu Leu Gly Pro   Tyr Thr Leu
    20750                 20755           20760

Asp Arg   Asp Ser Leu Tyr Val   Asp Gly Phe Thr His   Trp Ser Pro
    20765                 20770           20775

Ile Pro   Thr Thr Ser Thr Pro   Gly Thr Ser Ile Val   Asn Leu Gly
    20780                 20785           20790

Thr Ser   Gly Ile Pro Pro Ser   Leu Pro Glu Thr Thr   Xaa Xaa Xaa
    20795                 20800           20805

Pro Leu   Leu Xaa Pro Phe Thr   Xaa Asn Xaa Thr Ile   Thr Asn Leu
    20810                 20815           20820

Xaa Xaa   Xaa Xaa Xaa Met Xaa   Xaa Pro Gly Ser Arg   Lys Phe Asn
    20825                 20830           20835

Thr Thr   Glu Arg Val Leu Gln   Gly Leu Leu Lys Pro   Leu Phe Lys

```
         20840                    20845                      20850


Ser Thr   Ser Val Gly Pro Leu   Tyr Ser Gly Cys Arg   Leu Thr Leu
     20855                  20860                  20865


Leu Arg   Pro Glu Lys Asp Gly   Val Ala Thr Arg Val   Asp Ala Ile
     20870                  20875                  20880


Cys Thr   His Arg Pro Asp Pro   Lys Ile Pro Gly Leu   Asp Arg Gln
     20885                  20890                  20895


Gln Leu   Tyr Trp Glu Leu Ser   Gln Leu Thr His Ser   Ile Thr Glu
     20900                  20905                  20910


Leu Gly   Pro Tyr Thr Leu Asp   Arg Asp Ser Leu Tyr   Val Asn Gly
     20915                  20920                  20925


Phe Thr   Gln Arg Ser Ser Val   Pro Thr Thr Ser Thr   Pro Gly Thr
     20930                  20935                  20940


Phe Thr   Val Gln Pro Glu Thr   Ser Glu Thr Pro Ser   Ser Leu Pro
     20945                  20950                  20955


Gly Pro   Thr Ala Thr Gly Pro   Val Leu Leu Pro Phe   Thr Leu Asn
     20960                  20965                  20970


Phe Thr   Ile Thr Asn Leu Gln   Tyr Glu Glu Asp Met   His Arg Pro
     20975                  20980                  20985


Gly Ser   Arg Lys Phe Asn Thr   Thr Glu Arg Val Leu   Gln Gly Leu
     20990                  20995                  21000


Leu Met   Pro Leu Phe Lys Asn   Thr Ser Val Ser Ser   Leu Tyr Ser
     21005                  21010                  21015


Gly Cys   Arg Leu Thr Leu Leu   Arg Pro Glu Lys Asp   Gly Ala Ala
     21020                  21025                  21030


Thr Arg   Val Asp Ala Val Cys   Thr His Arg Pro Asp   Pro Lys Ser
     21035                  21040                  21045


Pro Gly   Leu Asp Arg Glu Arg   Leu Tyr Trp Lys Leu   Ser Gln Leu
     21050                  21055                  21060


Thr His   Gly Ile Thr Glu Leu   Gly Pro Tyr Thr Leu   Asp Arg His
     21065                  21070                  21075
```

Ser Leu Tyr Val Asn Gly Phe Thr His Gln Ser Ser Met Thr Thr
21080 21085 21090

Thr Arg Thr Pro Asp Thr Ser Thr Met His Leu Ala Thr Ser Arg
21095 21100 21105

Thr Pro Ala Ser Leu Ser Gly Pro Thr Thr Ala Ser Pro Leu Leu
21110 21115 21120

Val Leu Phe Thr Ile Asn Phe Thr Ile Thr Asn Leu Arg Tyr Glu
21125 21130 21135

Glu Asn Met His His Pro Gly Ser Arg Lys Phe Asn Thr Thr Glu
21140 21145 21150

Arg Val Leu Gln Gly Leu Leu Arg Pro Val Phe Lys Asn Thr Ser
21155 21160 21165

Val Gly Pro Leu Tyr Ser Gly Cys Arg Leu Thr Leu Leu Arg Pro
21170 21175 21180

Lys Lys Asp Gly Ala Ala Thr Lys Val Asp Ala Ile Cys Thr Tyr
21185 21190 21195

Arg Pro Asp Pro Lys Ser Pro Gly Leu Asp Arg Glu Gln Leu Tyr
21200 21205 21210

Trp Glu Leu Ser Gln Leu Thr His Ser Ile Thr Glu Leu Gly Pro
21215 21220 21225

Tyr Thr Leu Asp Arg Asp Ser Leu Tyr Val Asn Gly Phe Thr Gln
21230 21235 21240

Arg Ser Ser Val Pro Thr Thr Ser Ile Pro Gly Thr Pro Thr Val
21245 21250 21255

Asp Leu Gly Thr Ser Gly Thr Pro Val Ser Lys Pro Gly Pro Ser
21260 21265 21270

Ala Ala Ser Pro Leu Leu Val Leu Phe Thr Leu Asn Phe Thr Ile
21275 21280 21285

Thr Asn Leu Arg Tyr Glu Glu Asn Met Gln His Pro Gly Ser Arg
21290 21295 21300

Lys Phe Asn Thr Thr Glu Arg Val Leu Gln Gly Leu Leu Arg Ser
21305 21310 21315

304

Leu Phe Lys Ser Thr Ser Val Gly Pro Leu Tyr Ser Gly Cys Arg
21320 21325 21330

Leu Thr Leu Leu Arg Pro Glu Lys Asp Gly Thr Ala Thr Gly Val
21335 21340 21345

Asp Ala Ile Cys Thr His His Pro Asp Pro Lys Ser Pro Arg Leu
21350 21355 21360

Asp Arg Glu Gln Leu Tyr Trp Glu Leu Ser Gln Leu Thr His Asn
21365 21370 21375

Ile Thr Glu Leu Gly His Tyr Ala Leu Asp Asn Asp Ser Leu Phe
21380 21385 21390

Val Asn Gly Phe Thr His Arg Ser Ser Val Ser Thr Thr Ser Thr
21395 21400 21405

Pro Gly Thr Pro Thr Val Tyr Leu Gly Ala Ser Lys Thr Pro Ala
21410 21415 21420

Ser Ile Phe Gly Pro Ser Ala Ala Ser His Leu Leu Ile Leu Phe
21425 21430 21435

Thr Leu Asn Phe Thr Ile Thr Asn Leu Arg Tyr Glu Glu Asn Met
21440 21445 21450

Trp Pro Gly Ser Arg Lys Phe Asn Thr Thr Glu Arg Val Leu Gln
21455 21460 21465

Gly Leu Leu Arg Pro Leu Phe Lys Asn Thr Ser Val Gly Pro Leu
21470 21475 21480

Tyr Ser Gly Ser Arg Leu Thr Leu Leu Arg Pro Glu Lys Asp Gly
21485 21490 21495

Glu Ala Thr Gly Val Asp Ala Ile Cys Thr His Arg Pro Asp Pro
21500 21505 21510

Thr Gly Pro Gly Leu Asp Arg Glu Gln Leu Tyr Leu Glu Leu Ser
21515 21520 21525

Gln Leu Thr His Ser Ile Thr Glu Leu Gly Pro Tyr Thr Leu Asp
21530 21535 21540

Arg Asp Ser Leu Tyr Val Asn Gly Phe Thr His Arg Ser Ser Val
21545 21550 21555

305

Pro Thr Thr Ser Thr Gly Val Val Ser Glu Glu Pro Phe Thr Leu
21560 21565 21570

Asn Phe Thr Ile Asn Asn Leu Arg Tyr Met Ala Asp Met Gly Gln
21575 21580 21585

Pro Gly Ser Leu Lys Phe Asn Ile Thr Asp Asn Val Met Lys His
21590 21595 21600

Leu Leu Ser Pro Leu Phe Gln Arg Ser Ser Leu Gly Ala Arg Tyr
21605 21610 21615

Thr Gly Cys Arg Val Ile Ala Leu Arg Ser Val Lys Asn Gly Ala
21620 21625 21630

Glu Thr Arg Val Asp Leu Leu Cys Thr Tyr Leu Gln Pro Leu Ser
21635 21640 21645

Gly Pro Gly Leu Pro Ile Lys Gln Val Phe His Glu Leu Ser Gln
21650 21655 21660

Gln Thr His Gly Ile Thr Arg Leu Gly Pro Tyr Ser Leu Asp Lys
21665 21670 21675

Asp Ser Leu Tyr Leu Asn Gly Tyr Asn Glu Pro Gly Leu Asp Glu
21680 21685 21690

Pro Pro Thr Thr Pro Lys Pro Ala Thr Thr Phe Leu Pro Pro Leu
21695 21700 21705

Ser Glu Ala Thr Thr Ala Met Gly Tyr His Leu Lys Thr Leu Thr
21710 21715 21720

Leu Asn Phe Thr Ile Ser Asn Leu Gln Tyr Ser Pro Asp Met Gly
21725 21730 21735

Lys Gly Ser Ala Thr Phe Asn Ser Thr Glu Gly Val Leu Gln His
21740 21745 21750

Leu Leu Arg Pro Leu Phe Gln Lys Ser Ser Met Gly Pro Phe Tyr
21755 21760 21765

Leu Gly Cys Gln Leu Ile Ser Leu Arg Pro Glu Lys Asp Gly Ala
21770 21775 21780

Ala Thr Gly Val Asp Thr Thr Cys Thr Tyr His Pro Asp Pro Val

21785       21790       21795

Gly Pro Gly Leu Asp Ile Gln   Gln Leu Tyr Trp Glu   Leu Ser Gln
    21800          21805          21810

Leu Thr His Gly Val Thr Gln   Leu Gly Phe Tyr Val   Leu Asp Arg
    21815          21820          21825

Asp Ser Leu Phe Ile Asn Gly   Tyr Ala Pro Gln Asn   Leu Ser Ile
    21830          21835          21840

Arg Gly Glu Tyr Gln Ile Asn   Phe His Ile Val Asn   Trp Asn Leu
    21845          21850          21855

Ser Asn Pro Asp Pro Thr Ser   Ser Glu Tyr Ile Thr   Leu Leu Arg
    21860          21865          21870

Asp Ile Gln Asp Lys Val Thr   Thr Leu Tyr Lys Gly   Ser Gln Leu
    21875          21880          21885

His Asp Thr Phe Arg Phe Cys   Leu Val Thr Asn Leu   Thr Met Asp
    21890          21895          21900

Ser Val Leu Val Thr Val Lys   Ala Leu Phe Ser Ser   Asn Leu Asp
    21905          21910          21915

Pro Ser Leu Val Glu Gln Val   Phe Leu Asp Lys Thr   Leu Asn Ala
    21920          21925          21930

Ser Phe His Trp Leu Gly Ser   Thr Tyr Gln Leu Val   Asp Ile His
    21935          21940          21945

Val Thr Glu Met Glu Ser Ser   Val Tyr Gln Pro Thr   Ser Ser Ser
    21950          21955          21960

Ser Thr Gln His Phe Tyr Leu   Asn Phe Thr Ile Thr   Asn Leu Pro
    21965          21970          21975

Tyr Ser Gln Asp Lys Ala Gln   Pro Gly Thr Thr Asn   Tyr Gln Arg
    21980          21985          21990

Asn Lys Arg Asn Ile Glu Asp   Ala Leu Asn Gln Leu   Phe Arg Asn
    21995          22000          22005

Ser Ser Ile Lys Ser Tyr Phe   Ser Asp Cys Gln Val   Ser Thr Phe
    22010          22015          22020

```
Arg Ser    Val Pro Asn Arg His    His Thr Gly Val Asp    Ser Leu Cys
    22025                22030                22035

Asn Phe    Ser Pro Leu Ala Arg    Arg Val Asp Arg Val    Ala Ile Tyr
    22040                22045                22050

Glu Glu    Phe Leu Arg Met Thr    Arg Asn Gly Thr Gln    Leu Gln Asn
    22055                22060                22065

Phe Thr    Leu Asp Arg Ser Ser    Val Leu Val Asp Gly    Tyr Ser Pro
    22070                22075                22080

Asn Arg    Asn Glu Pro Leu Thr    Gly Asn Ser Asp Leu    Pro Phe Trp
    22085                22090                22095

Ala Val    Ile Leu Ile Gly Leu    Ala Gly Leu Leu Gly    Leu Ile Thr
    22100                22105                22110

Cys Leu    Ile Cys Gly Val Leu    Val Thr Thr Arg Arg    Arg Lys Lys
    22115                22120                22125

Glu Gly    Glu Tyr Asn Val Gln    Gln Gln Cys Pro Gly    Tyr Tyr Gln
    22130                22135                22140

Ser His    Leu Asp Leu Glu Asp    Leu Gln
    22145                22150


<210> 13
<211> 702
<212> PRT
<213> Homo sapiens

<400> 13
Met Glu Ser Pro Ser Ala Pro Pro His Arg Trp Cys Ile Pro Trp Gln
1               5               10              15

Arg Leu Leu Leu Thr Ala Ser Leu Leu Thr Phe Trp Asn Pro Pro Thr
            20              25              30

Thr Ala Lys Leu Thr Ile Glu Ser Thr Pro Phe Asn Val Ala Glu Gly
        35              40              45

Lys Glu Val Leu Leu Leu Val His Asn Leu Pro Gln His Leu Phe Gly
        50              55              60

Tyr Ser Trp Tyr Lys Gly Glu Arg Val Asp Gly Asn Arg Gln Ile Ile
65              70              75              80

Gly Tyr Val Ile Gly Thr Gln Gln Ala Thr Pro Gly Pro Ala Tyr Ser
```

```
                    85                      90                      95

        Gly Arg Glu Ile Ile Tyr Pro Asn Ala Ser Leu Leu Ile Gln Asn Ile
                100             105             110

        Ile Gln Asn Asp Thr Gly Phe Tyr Thr Leu His Val Ile Lys Ser Asp
                115             120             125

        Leu Val Asn Glu Glu Ala Thr Gly Gln Phe Arg Val Tyr Pro Glu Leu
                130             135             140

        Pro Lys Pro Ser Ile Ser Ser Asn Asn Ser Lys Pro Val Glu Asp Lys
        145             150             155             160

        Asp Ala Val Ala Phe Thr Cys Glu Pro Glu Thr Gln Asp Ala Thr Tyr
                        165             170             175

        Leu Trp Trp Val Asn Asn Gln Ser Leu Pro Val Ser Pro Arg Leu Gln
                    180             185             190

        Leu Ser Asn Gly Asn Arg Thr Leu Thr Leu Phe Asn Val Thr Arg Asn
                195             200             205

        Asp Thr Ala Ser Tyr Lys Cys Glu Thr Gln Asn Pro Val Ser Ala Arg
                210             215             220

        Arg Ser Asp Ser Val Ile Leu Asn Val Leu Tyr Gly Pro Asp Ala Pro
        225             230             235             240

        Thr Ile Ser Pro Leu Asn Thr Ser Tyr Arg Ser Gly Glu Asn Leu Asn
                    245             250             255

        Leu Ser Cys His Ala Ala Ser Asn Pro Pro Ala Gln Tyr Ser Trp Phe
                    260             265             270

        Val Asn Gly Thr Phe Gln Gln Ser Thr Gln Glu Leu Phe Ile Pro Asn
                275             280             285

        Ile Thr Val Asn Asn Ser Gly Ser Tyr Thr Cys Gln Ala His Asn Ser
                290             295             300

        Asp Thr Gly Leu Asn Arg Thr Thr Val Thr Thr Ile Thr Val Tyr Ala
        305             310             315             320

        Glu Pro Pro Lys Pro Phe Ile Thr Ser Asn Asn Ser Asn Pro Val Glu
                    325             330             335
```

309

```
Asp Glu Asp Ala Val Ala Leu Thr Cys Glu Pro Glu Ile Gln Asn Thr
        340                 345             350

Thr Tyr Leu Trp Trp Val Asn Asn Gln Ser Leu Pro Val Ser Pro Arg
        355                 360             365

Leu Gln Leu Ser Asn Asp Asn Arg Thr Leu Thr Leu Leu Ser Val Thr
        370                 375             380

Arg Asn Asp Val Gly Pro Tyr Glu Cys Gly Ile Gln Asn Glu Leu Ser
385                 390             395                 400

Val Asp His Ser Asp Pro Val Ile Leu Asn Val Leu Tyr Gly Pro Asp
                405                 410             415

Asp Pro Thr Ile Ser Pro Ser Tyr Thr Tyr Tyr Arg Pro Gly Val Asn
            420                 425             430

Leu Ser Leu Ser Cys His Ala Ala Ser Asn Pro Pro Ala Gln Tyr Ser
        435                 440             445

Trp Leu Ile Asp Gly Asn Ile Gln Gln His Thr Gln Glu Leu Phe Ile
    450                 455             460

Ser Asn Ile Thr Glu Lys Asn Ser Gly Leu Tyr Thr Cys Gln Ala Asn
465                 470             475                 480

Asn Ser Ala Ser Gly His Ser Arg Thr Thr Val Lys Thr Ile Thr Val
                485                 490             495

Ser Ala Glu Leu Pro Lys Pro Ser Ile Ser Ser Asn Asn Ser Lys Pro
        500                 505             510

Val Glu Asp Lys Asp Ala Val Ala Phe Thr Cys Glu Pro Glu Ala Gln
        515                 520             525

Asn Thr Thr Tyr Leu Trp Trp Val Asn Gly Gln Ser Leu Pro Val Ser
    530                 535             540

Pro Arg Leu Gln Leu Ser Asn Gly Asn Arg Thr Leu Thr Leu Phe Asn
545                 550             555                 560

Val Thr Arg Asn Asp Ala Arg Ala Tyr Val Cys Gly Ile Gln Asn Ser
                565                 570             575

Val Ser Ala Asn Arg Ser Asp Pro Val Thr Leu Asp Val Leu Tyr Gly
        580                 585             590
```

```
Pro Asp Thr Pro Ile Ile Ser Pro Pro Asp Ser Ser Tyr Leu Ser Gly
        595             600             605

Ala Asn Leu Asn Leu Ser Cys His Ser Ala Ser Asn Pro Ser Pro Gln
    610             615             620

Tyr Ser Trp Arg Ile Asn Gly Ile Pro Gln Gln His Thr Gln Val Leu
625             630             635                 640

Phe Ile Ala Lys Ile Thr Pro Asn Asn Asn Gly Thr Tyr Ala Cys Phe
            645             650                 655

Val Ser Asn Leu Ala Thr Gly Arg Asn Asn Ser Ile Val Lys Ser Ile
        660             665             670

Thr Val Ser Ala Ser Gly Thr Ser Pro Gly Leu Ser Ala Gly Ala Thr
        675             680             685

Val Gly Ile Met Ile Gly Val Leu Val Gly Val Ala Leu Ile
    690             695             700
```

```
<210> 14
<211> 393
<212> PRT
<213> Homo sapiens

<400> 14
Met Glu Glu Pro Gln Ser Asp Pro Ser Val Glu Pro Pro Leu Ser Gln
1               5               10              15

Glu Thr Phe Ser Asp Leu Trp Lys Leu Leu Pro Glu Asn Asn Val Leu
        20              25              30

Ser Pro Leu Pro Ser Gln Ala Met Asp Asp Leu Met Leu Ser Pro Asp
        35              40              45

Asp Ile Glu Gln Trp Phe Thr Glu Asp Pro Gly Pro Asp Glu Ala Pro
    50              55              60

Arg Met Pro Glu Ala Ala Pro Arg Val Ala Pro Ala Pro Ala Ala Pro
65              70              75              80

Thr Pro Ala Ala Pro Ala Pro Ala Pro Ser Trp Pro Leu Ser Ser Ser
            85              90              95

Val Pro Ser Gln Lys Thr Tyr Gln Gly Ser Tyr Gly Phe Arg Leu Gly
        100             105             110
```

Phe Leu His Ser Gly Thr Ala Lys Ser Val Thr Cys Thr Tyr Ser Pro
    115          120         125

Ala Leu Asn Lys Met Phe Cys Gln Leu Ala Lys Thr Cys Pro Val Gln
    130          135         140

Leu Trp Val Asp Ser Thr Pro Pro Pro Gly Thr Arg Val Arg Ala Met
145           150         155         160

Ala Ile Tyr Lys Gln Ser Gln His Met Thr Glu Val Val Arg Arg Cys
         165         170         175

Pro His His Glu Arg Cys Ser Asp Ser Asp Gly Leu Ala Pro Pro Gln
         180         185         190

His Leu Ile Arg Val Glu Gly Asn Leu Arg Val Glu Tyr Leu Asp Asp
         195         200         205

Arg Asn Thr Phe Arg His Ser Val Val Val Pro Tyr Glu Pro Pro Glu
    210          215         220

Val Gly Ser Asp Cys Thr Thr Ile His Tyr Asn Tyr Met Cys Asn Ser
225           230         235         240

Ser Cys Met Gly Gly Met Asn Arg Arg Pro Ile Leu Thr Ile Ile Thr
         245         250         255

Leu Glu Asp Ser Ser Gly Asn Leu Leu Gly Arg Asn Ser Phe Glu Val
         260         265         270

Arg Val Cys Ala Cys Pro Gly Arg Asp Arg Arg Thr Glu Glu Glu Asn
         275         280         285

Leu Arg Lys Lys Gly Glu Pro His His Glu Leu Pro Pro Gly Ser Thr
    290          295         300

Lys Arg Ala Leu Pro Asn Asn Thr Ser Ser Ser Pro Gln Pro Lys Lys
305           310         315         320

Lys Pro Leu Asp Gly Glu Tyr Phe Thr Leu Gln Ile Arg Gly Arg Glu
         325         330         335

Arg Phe Glu Met Phe Arg Glu Leu Asn Glu Ala Leu Glu Leu Lys Asp
         340         345         350

Ala Gln Ala Gly Lys Glu Pro Gly Gly Ser Arg Ala His Ser Ser His
    355          360         365

EP 3 151 005 A1

```
        Leu Lys Ser Lys Lys Gly Gln Ser Thr Ser Arg His Lys Lys Leu Met
            370                 375                 380


        Phe Lys Thr Glu Gly Pro Asp Ser Asp
        385                 390



        <210> 15
        <211> 10
        <212> PRT
        <213> Homo sapiens

        <400> 15
        Ile Arg Gly Arg Glu Arg Phe Glu Met Phe
        1               5                   10



        <210> 16
        <211> 10
        <212> PRT
        <213> Artificial Sequence

        <220>
        <223> Description of Artificial Sequence: Synthetic
              peptide

        <400> 16
        Asp Gly Thr Ser Phe Gln Lys Glu Asn Cys
        1               5                   10
```

**Description**

The present invention claims priority to U.S. provisional patent applications 61/238,115 filed 8/28/2009, 61/238,118 filed 8/28/2009, 61/238,120 filed 8/28/2009, 61/238,121 filed 8/28/2009, 61/238,123 filed 8/28/2009, 61/238,125 filed 8/28/2009, 61/238,127 filed 8/28/2009, 61/238,129 filed 8/28/2009, 61/238,134 filed 8/28/2009, 61/243,991 filed 9/18/2009, 61/243,997 filed 9/18/2009, 61/244,002 filed 9/18/2009, 61/243,993 filed 9/18/2009, and 61/238,128 filed 8/28/2009, each of which is hereby incorporated in its entirety including all tables, figures and claims.

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830, which are hereby incorporated by reference in their entirety. Renal disease and/or injury may be acute or chronic. Acute and chronic kidney disease are described as follows (from Current Medical Diagnosis & Treatment 2008, 47th Ed, McGraw Hill, New York, pages 785-815, which are hereby incorporated by reference in their entirety): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222, and which is hereby incorporated by reference in their entirety:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |

(continued)

| Intrinsic Renal | |
|---|---|
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulo interstitial nephritis | Drug reaction (eg, $\beta$-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005, which, with the references listed therein, are hereby incorporated by reference in their entirety. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004, hereby incorporated by reference in its entirety) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, which is hereby incorporated by reference in its entirety, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, each hereby incorporated by reference in its entirety, the RIFLE criteria provide a uniform definition of AKI which has been validated in numerous studies.

More recently, Mehta et al., Crit. Care 11:R31 (doi:10.1186.cc5713), 2007, hereby incorporated by reference in its entirety, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4):177-197, hereby incorporated by reference in its entirety) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. As described herein, measurement of one or more biomarkers selected from the group consisting of Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 (referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

The kidney injury markers of the present invention may be used, individually or in panels comprising a plurality of kidney injury markers, for risk stratification (that is, to identify subjects at risk for a future injury to renal function, for future progression to reduced renal function, for future progression to ARF, for future improvement in renal function, *etc.*); for diagnosis of existing disease (that is, to identify subjects who have suffered an injury to renal function, who have progressed to reduced renal function, who have progressed to ARF, *etc.*)*; for monitoring for deterioration or improvement of renal function; and for predicting a future medical outcome, such as improved or worsening renal function, a decreased or increased mortality risk, a decreased or increased risk that a subject will require renal replacement therapy (*i.e.,* hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present invention relates to methods for evaluating renal status in a subject. These methods comprise performing an assay method that is configured to detect one or more biomarkers selected from the group consisting of Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 in a body fluid sample obtained from the subject. The assay result(s), for example measured concentration(s) of one or more biomarkers selected from the group consisting of Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example measured concentration(s) of one or more biomarkers selected from the group consisting of Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example measured concentration(s) of one or more biomarkers selected from the group consisting of Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example measured concentration(s) of one or more biomarkers selected from the group consisting of Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, *etc.),* by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/-5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, *etc.*) as well as molecules that may be indirectly detected by production of a detectable reaction product (e.g., enzymes such as horseradish peroxidase, alkaline phosphatase, *etc.)* or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of one or more biomarkers selected from the group consisting of Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "tumor necrosis factor receptor superfamily member 10B" refers to one or more polypeptides present in a biological sample that are derived from the tumor necrosis factor receptor superfamily member 10B precursor (Swiss-Prot 014763 (SEQ ID NO: 1)).

```
          10          20          30          40          50          60

MEQRGQNAPA  ASGARKRHGP  GPREARGARP  GPRVPKTLVL  VVAAVLLLVS  AESALITQQD

          70          80          90         100         110         120

LAPQQRAAPQ  QKRSSPSEGL  CPPGHHISED  GRDCISCKYG  QDYSTHWNDL  LFCLRCTRCD

         130         140         150         160         170         180

SGEVELSPCT  TTRNTVCQCE  EGTFREEDSP  EMCRKCRTGC  PRGMVKVGDC  TPWSDIECVH

         190         200         210         220         230         240

KESGTKHSGE  APAVEETVTS  SPGTPASPCS  LSGIIIGVTV  AAVVLIVAVF  VCKSLLWKKV

         250         260         270         280         290         300

LPYLKGICSG  GGGDPERVDR  SSQRPGAEDN  VLNEIVSILQ  PTQVPEQEME  VQEPAEPTGV

         310         320         330         340         350         360

NMLSPGESEH  LLEPAEAERS  QRRRLLVPAN  EGDPTETLRQ  CFDDFADLVP  FDSWEPLMRK

         370         380         390         400         410         420

LGLMDNEIKV  AKAEAAGHRD  TLYTMLIKWV  NKTGRDASVH  TLLDALETLG  ERLAKQKIED

         430         440

HLLSSGKFMY  LEGNADSAMS
```

Most preferably, the tumor necrosis factor receptor superfamily member 10B assay detects one or more soluble forms of tumor necrosis factor receptor superfamily member 10B. Tumor necrosis factor receptor superfamily member 10B is a single-pass type I membrane protein having a large extracellular domain, most or all of which is present in soluble forms of tumor necrosis factor receptor superfamily member 10B generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in tumor necrosis factor receptor superfamily member 10B:

| Residues | Length | Domain ID |
|---|---|---|
| 1-55 | 55 | signal sequence |
| 56-440 | 385 | tumor necrosis factor receptor superfamily member 10B |
| 56-210 | 155 | extracellular |
| 211-231 | 21 | transmembrane |
| 232-440 | 209 | cytoplasmic |

As used herein, the term "cadherin-16" refers to one or more polypeptides present in a biological sample that are derived from the cadherin-16 precursor (Swiss-Prot 075309 (SEQ ID NO: 2)).

```
                 10          20          30          40          50          60

      MVPAWLWLLC  VSVPQALPKA  QPAELSVEVP  ENYGGNFPLY  LTKLPLPREG  AEGQIVLSGD

                 70          80          90         100         110         120

      SGKATEGPFA  MDPDSGFLLV  TRALDREEQA  EYQLQVTLEM  QDGHVLWGPQ  PVLVHVKDEN

                130         140         150         160         170         180

      DQVPHFSQAI  YRARLSRGTR  PGIPFLFLEA  SDRDEPGTAN  SDLRFHILSQ  APAQPSPDMF

                190         200         210         220         230         240
```

```
QLEPRLGALA  LSPKGSTSLD  HALERTYQLL  VQVKDMGDQA  SGHQATATVE  VSIIESTWVS
       250         260         270         280         290         300

LEPIHLAENL  KVLYPHHMAQ  VHWSGGDVHY  HLESHPPGPF  EVNAEGNLYV  TRELDREAQA
       310         320         330         340         350         360

EYLLQVRAQN  SHGEDYAAPL  ELHVLVMDEN  DNVPICPPRD  PTVSIPELSP  PGTEVTRLSA
       370         380         390         400         410         420

EDADAPGSPN  SHVVYQLLSP  EPEDGVEGRA  FQVDPTSGSV  TLGVLPLRAG  QNILLLVLAM
       430         440         450         460         470         480

DLAGAEGGFS  STCEVEVAVT  DINDHAPEFI  TSQIGPISLP  EDVEPGTLVA  MLTAIDADLE
       490         500         510         520         530         540

PAFRLMDFAI  ERGDTEGTFG  LDWEPDSGHV  RLRLCKNLSY  EAAPSHEVVV  VVQSVAKLVG
       550         560         570         580         590         600

PGPGPGATAT  VTVLVERVMP  PPKLDQESYE  ASVPISAPAG  SFLLTIQPSD  PISRTLRFSL
       610         620         630         640         650         660

VNDSEGWLCI  EKFSGEVHTA  QSLQGAQPGD  TYTVLVEAQD  TDEPRLSASA  PLVIHFLKAP
       670         680         690         700         710         720

PAPALTLAPV  PSQYLCTPRQ  DHGLIVSGPS  KDPDLASGHG  PYSFTLGPNP  TVQRDWRLQT
       730         740         750         760         770         780

LNGSHAYLTL  ALHWVEPREH  IIPVVSHNA  QMWQLLVRVI  VCRCNVEGQC  MRKVGRMKGM
       790         800         810         820

PTKLSAVGIL  VGTLVAIGIF  LILIFTHWTM  SRKKDPDQPA  DSVPLKATV
```

Most preferably, the cadherin-16 assay detects one or more soluble forms of cadherin-16. Cadherin-16 is a single-pass type I membrane protein having a large extracellular domain, most or all of which is present in soluble forms of cadherin-16 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in cadherin-16:

| Residues | Length | Domain ID |
|---|---|---|
| 1-18 | 18 | signal sequence |
| 19 | 829 | cadherin-16 |
| 19-786 | 768 | extracellular |
| 787-807 | 22 | transmembrane |

(continued)

| Residues | Length | Domain ID |
|---|---|---|
| 808-829 | 22 | cytoplasmic |

As used herein, the term "caspase-9" refers to one or more polypeptides present in a biological sample that are derived from the caspase-9 precursor (Swiss-Prot P55211 (SEQ ID NO: 3)).

```
           10         20         30         40         50         60

 MDEADRRLLR RCRLRLVEEL QVDQLWDALL SRELFRPHMI EDIQRAGSGS RRDQARQLII

           70         80         90        100        110        120

 DLETRGSQAL PLFISCLEDT GQDMLASFLR TNRQAAKLSK PTLENLTPVV LRPEIRKPEV

          130        140        150        160        170        180

 LRPETPRPVD IGSGGFGDVG ALESLRGNAD LAYILSMEPC GHCLIINNVN FCRESGLRTR

          190        200        210        220        230        240

 TGSNIDCEKL RRRFSSLHFM VEVKGDLTAK KMVLALLELA QQDHGALDCC VVVILSHGCQ

          250        260        270        280        290        300

 ASHLQFPGAV YGTDGCPVSV EKIVNIFNGT SCPSLGGKPK LFFIQACGGE QKDHGFEVAS

          310        320        330        340        350        360

 TSPEDESPGS NPEPDATPFQ EGLRTFDQLD AISSLPTPSD IFVSYSTFPG FVSWRDPKSG

          370        380        390        400        410

 SWYVETLDDI FEQWAHSEDL QSLLLRVANA VSVKGIYKQM PGCFNFLRKK LFFKTS
```

The following domains have been identified in caspase-9:

| Residues | Length | Domain ID |
|---|---|---|
| 1-315 | 315 | caspase-9 p35 subunit |
| 316-330 | 15 | pro-peptide |
| 331-416 | 86 | caspase-9 subunit p10 |

As used herein, the term "Bcl2 antagonist of cell death" refers to one or more polypeptides present in a biological sample that are derived from the Bcl2 antagonist of cell death precursor (Swiss-Prot Q92934 (SEQ ID NO: 4)).

```
            10          20          30          40          50          60

  MFQIPEFEPS  EQEDSSSAER  GLGPSPAGDG  PSGSGKHHRQ  APGLLWDASH  QQEQPTSSSH

            70          80          90         100         110         120

  HGGAGAVEIR  SRHSSYPAGT  EDDEGMGEEP  SPFRGRSRSA  PPNLWAAQRY  GRELRRMSDE

           130         140         150         160

  FVDSFKKGLP  RPKSAGTATQ  MRQSSSWTRV  FQSWWDRNLG  RGSSAPSQ
```

As used herein, the term "caspase-1" refers to one or more polypeptides present in a biological sample that are derived from the caspase-1 precursor (Swiss-Prot P29466 (SEQ ID NO: 5)).

```
            10          20          30          40          50          60

  MADKVLKEKR  KLFIRSMGEG  TINGLLDELL  QTRVLNKEEM  EKVKRENATV  MDKTRALIDS

            70          80          90         100         110         120

  VIPKGAQACQ  ICITYICEED  SYLAGTLGLS  ADQTSGNYLN  MQDSQGVLSS  FPAPQAVQDN

           130         140         150         160         170         180

  PAMPTSSGSE  GNVKLCSLEE  AQRIWKQKSA  EIYPIMDKSS  RTRLALIICN  EEFDSIPRRT

           190         200         210         220         230         240

  GAEVDITGMT  MLLQNLGYSV  DVKKNLTASD  MTTELEAFAH  RPEHKTSDST  FLVFMSHGIR

           250         260         270         280         290         300

  EGICGKKHSE  QVPDILQLNA  IFNMLNTKNC  PSLKDKPKVI  IIQACRGDSP  GVVWFKDSVG

           310         320         330         340         350         360

  VSGNLSLPTT  EEFEDDAIKK  AHIEKDFIAF  CSSTPDNVSW  RHPTMGSVFI  GRLIEHMQEY

           370         380         390         400

  ACSCDVEEIF  RKVRFSFEQP  DGRAQMPTTE  RVTLTRCFYL  FPGH
```

The following domains have been identified in caspase-1:

| Residues | Length | Domain ID |
|---|---|---|
| 1-119 | 119 | pro-peptide |
| 120-297 | 178 | caspase-1 p20 subunit |
| 298-316 | 19 | pro-peptide |
| 317-404 | 88 | caspase-1 p10 subunit |

As used herein, the terms "epithelial cadherin" or "Cadherin-1" refers to one or more polypeptides present in a biological sample that are derived from the epithelial cadherin precursor (Swiss-Prot P12830 (SEQ ID NO: 6)).

```
            10          20          30          40          50          60

MGPWSRSLSA  LLLLLQVSSW  LCQEPEPCHP  GFDAESYTFT  VPRRHLERGR  VLGRVNFEDC

            70          80          90         100         110         120

TGRQRTAYFS  LDTRFKVGTD  GVITVKRPLR  FHNPQIHFLV  YAWDSTYRKF  STKVTLNTVG

           130         140         150         160         170         180

HHHRPPPHQA  SVSGIQAELL  TFPNSSPGLR  RQKRDWVIPP  ISCPENEKGP  FPKNLVQIKS

           190         200         210         220         230         240

NKDKEGKVFY  SITGQGADTP  PVGVFIIERE  TGWLKVTEPL  DRERIATYTL  FSHAVSSNGN

           250         260         270         280         290         300

AVEDPMEILI  TVTDQNDNKP  EFTQEVFKGS  VMEGALPGTS  VMEVTATDAD  DDVNTYNAAI

           310         320         330         340         350         360

AYTILSQDPE  LPDKNMFTIN  RNTGVISVVT  TGLDRESFPT  YTLVVQAADL  QGEGLSTTAT

           370         380         390         400         410         420

AVITVTDTND  NPPIFNPTTY  KGQVPENEAN  VVITTLKVTD  ADAPNTPAWE  AVYTILNDDG

           430         440         450         460         470         480

GQFVVTTNPV  NNDGILKTAK  GLDFEAKQQY  ILHVAVTNVV  PFEVSLTTST  ATVTVDVLDV

           490         500         510         520         530         540

NEAPIFVPPE  KRVEVSEDFG  VGQEITSYTA  QEPDTFMEQK  ITYRIWRDTA  NWLEINPDTG

           550         560         570         580         590         600

AISTRAELDR  EDFEHVKNST  YTALIIATDN  GSPVATGTGT  LLLILSDVND  NAPIPEPRTI

           610         620         630         640         650         660

FFCERNPKPQ  VINIIDADLP  PNTSPFTAEL  THGASANWTI  QYNDPTQESI  ILKPKMALEV
```

```
        670         680         690         700         710         720
GDYKINLKLM DNQNKDQVTT LEVSVCDCEG AAGVCRKAQP VEAGLQIPAI LGILGGILAL
        730         740         750         760         770         780
LILILLLLLF LRRRAVVKEP LLPPEDDTRD NVYYYDEEGG GEEDQDFDLS QLHRGLDARP
        790         800         810         820         830         840
EVTRNDVAPT LMSVPRYLPR PANPDEIGNF IDENLKAADT DPTAPPYDSL LVFDYEGSGS
        850         860         870         880
EAASLSSLNS SESDKDQDYD YLNEWGNRFK KLADMYGGGE DD
```

Most preferably, the epithelial cadherin assay detects one or more soluble forms of epithelial cadherin. Epithelial cadherin is a single-pass type I membrane protein having a large extracellular domain, most or all of which is present in soluble forms of epithelial cadherin generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in epithelial cadherin:

| Residues | Length | Domain ID |
| --- | --- | --- |
| 1-22 | 22 | signal sequence |
| 23-154 | 132 | pro-peptide |
| 155-882 | 728 | epithelial cadherin |
| 155-709 | 555 | extracellular |
| 710-730 | 21 | transmembrane |
| 731-882 | 152 | cytoplasmic |

As used herein, the term "poly [ADP-ribose] polymerase 1" refers to one or more polypeptides present in a biological sample that are derived from the poly [ADP-ribose] polymerase 1 precursor (Swiss-Prot 09874 (SEQ ID NO: 7)).

```
         10          20          30          40          50          60
MAESSDKLYR VEYAKSGRAS CKKCSESIPK DSLRMAIMVQ SPMFDGKVPH WYHFSCFWKV
         70          80          90         100         110         120
```

```
GHSIRHPDVE VDGFSELRWD DQQKVKKTAE AGGVTGKGQD GIGSKAEKTL GDFAAEYAKS
           130        140        150        160        170        180

NRSTCKGCME KIEKGQVRLS KKMVDPEKPQ LGMIDRWYHP GCFVKNREEL GFRPEYSASQ
           190        200        210        220        230        240

LKGFSLLATE DKEALKKQLP GVKSEGKRKG DEVDGVDEVA KKKSKKEKDK DSKLEKALKA
           250        260        270        280        290        300

QNDLIWNIKD ELKKVCSTND LKELLIFNKQ QVPSGESAIL DRVADGMVFG ALLPCEECSG
           310        320        330        340        350        360

QLVFKSDAYY CTGDVTAWTK CMVKTQTPNR KEWVTPKEFR EISYLKKLKV KKQDRIFPPE
           370        380        390        400        410        420

TSASVAATPP PSTASAPAAV NSSASADKPL SNMKILTLGK LSRNKDEVKA MIEKLGGKLT
           430        440        450        460        470        480

GTANKASLCI STKKEVEKMN KKMEEVKEAN IRVVSEDFLQ DVSASTKSLQ ELFLAHILSP
           490        500        510        520        530        540

WGAEVKAEPV EVVAPRGKSG AALSKKSKGQ VKEEGINKSE KRMKLTLKGG AAVDPDSGLE
           550        560        570        580        590        600

HSAHVLEKGG KVFSATLGLV DIVKGTNSYY KLQLLEDDKE NRYWIFRSWG RVGTVIGSNK
           610        620        630        640        650        660

LEQMPSKEDA IEHFMKLYEE KTGNAWHSKN FTKYPKKFYP LEIDYGQDEE AVKKLTVNPG
           670        680        690        700        710        720

TKSKLPKPVQ DLIKMIFDVE SMKKAMVEYE IDLQKMPLGK LSKRQIQAAY SILSEVQQAV
           730        740        750        760        770        780

SQGSSDSQIL DLSNRFYTLI PHDFGMKKPP LLNNADSVQA KVEMLDNLLD IEVAYSLLRG
           790        800        810        820        830        840

GSDDSSKDPI DVNYEKLKTD IKVVDRDSEE AEIIRKYVKN THATTHNAYD LEVIDIFKIE
           850        860        870        880        890        900

REGECQRYKP FKQLHNRRLL WHGSRTTNFA GILSQGLRIA PPEAPVTGYM FGKGIYFADM
           910        920        930        940        950        960
```

```
VSKSANYCHT  SQGDPIGLIL  LGEVALGNMY  ELKHASHISK  LPKGKHSVKG  LGKTTPDPSA

       970         980         990        1000        1010

NISLDGVDVP  LGTGISSGVN  DTSLLYNEYI  VYDIAQVNLK  YLLKLKFNFK  TSLW
```

The following domains have been identified in poly [ADP-ribose] polymerase 1:

| Residues | Length | Domain ID |
|---|---|---|
| 1 | 1 | initiator methionine |
| 2-1014 | 1013 | poly [ADP-ribose] polymerase 1 |

Poly [ADP-ribose] polymerase 1 can be cleaved by many caspases in vitro and is one of the main cleavage targets of caspase-3 in vivo. The cleavage occurs between Asp(214) and Gly(215), which separates PARP's N-terminal DNA binding domain (24 kDa) from its C-terminal catalytic domain (89 kDa). Suitable assays may recognize only the large fragment of poly [ADP-ribose] polymerase 1 (89 kDa) but not the full length poly [ADP-ribose] polymerase 1, may recognize only the small fragment of poly [ADP-ribose] polymerase 1 (24 kDa) but not the full length poly [ADP-ribose] polymerase 1, may recognize only full length poly [ADP-ribose] polymerase 1, or may recognize one fragment and the full length full length poly [ADP-ribose] polymerase 1.

As used herein, the term "cyclin-dependent kinase inhibitor 1" refers to one or more polypeptides present in a biological sample that are derived from the cyclin-dependent kinase inhibitor 1 precursor (Swiss-Prot P38936 (SEQ ID NO: 8)).

```
        10          20          30          40          50          60

MSEPAGDVRQ  NPCGSKACRR  LFGPVDSEQL  SRDCDALMAG  CIQEARERWN  FDFVTETPLE

        70          80          90         100         110         120

GDFAWERVRG  LGLPKLYLPT  GPRRGRDELG  GGRRPGTSPA  LLQGTAEEDH  VDLSLSCTLV

       130         140         150         160

PRSGEQAEGS  PGGPGDSQGR  KRRQTSMTDF  YHSKRRLIFS  KRKP
```

The following domains have been identified in cyclin-dependent kinase inhibitor 1:

| Residues | Length | Domain |
|---|---|---|
| 1 | 1 | initiator methionine |
| 2-164 | 163 | cyclin-dependent kinase inhibitor 1 |

As used herein, the term "cadherin-5" refers to one or more polypeptides present in a biological sample that are derived from the cadherin-5 precursor (Swiss-Prot P33151 (SEQ ID NO: 9)).

```
            10          20          30          40          50          60

MQRLMMLLAT  SGACLGLLAV  AAVAAAGANP  AQRDTHSLLP  THRRQKRDWI  WNQMHIDEEK

            70          80          90         100         110         120

NTSLPHHVGK  IKSSVSRKNA  KYLLKGEYVG  KVFRVDAETG  DVFAIERLDR  ENISEYHLTA

           130         140         150         160         170         180

VIVDKDTGEN  LETPSSFTIK  VHDVNDNWPV  FTHRLFNASV  PESSAVGTSV  ISVTAVDADD

           190         200         210         220         230         240

PTVGDHASVM  YQILKGKEYF  AIDNSGRIIT  ITKSLDREKQ  ARYEIVVEAR  DAQGLRGDSG

           250         260         270         280         290         300

TATVLVTLQD  INDNFPFFTQ  TKYTFVVPED  TRVGTSVGSL  FVEDPDEPQN  RMTKYSILRG

           310         320         330         340         350         360

DYQDAFTIET  NPAHNEGIIK  PMKPLDYEYI  QQYSFIVEAT  DPTIDLRYMS  PPAGNRAQVI

           370         380         390         400         410         420

INITDVDEPP  IFQQPFYHFQ  LKENQKKPLI  GTVLAMDPDA  ARHSIGYSIR  RTSDKGQFFR

           430         440         450         460         470         480

VTKKGDIYNE  KELDREVYPW  YNLTVEAKEL  DSTGTPTGKE  SIVQVHIEVL  DENDNAPEFA

           490         500         510         520         530         540

KPYQPKVCEN  AVHGQLVLQI  SAIDKDITPR  NVKFKFTLNT  ENNFTLTDNH  DNTANITVKY

           550         560         570         580         590         600

GQFDREHTKV  HFLPVVISDN  GMPSRTGTST  LTVAVCKCNE  QGEFTFCEDM  AAQVGVSIQA

           610         620         630         640         650         660

VVAILLCILT  ITVITLLIFL  RRRLRKQARA  HGKSVPEIHE  QLVTYDEEGG  GEMDTTSYDV

           670         680         690         700         710         720
```

```
SVLNSVRRGG AKPPRPALDA RPSLYAQVQK PPRHAPGAHG GPGEMAAMIE VKKDEADHDG

        730        740        750        760        770        780

DGPPYDTLHI YGYEGSESIA ESLSSLGTDS SDSDVDYDFL NDWGPRFKML AELYGSDPRE


ELLY
```

Most preferably, the cadherin-5 assay detects one or more soluble forms of cadherin-5. Cadherin-5 is a single-pass type I membrane protein having a large extracellular domain, most or all of which is present in soluble forms of cadherin-5 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in cadherin-5:

| Residues | Length | Domain ID |
| --- | --- | --- |
| 1-25 | 251 | signal sequence |
| 26-47 | 22 | pro-peptide |
| 48-784 | 737 | cadherin-5 |
| 48-599 | 522 | extracellular |
| 600-620 | 21 | transmembrane |
| 621-784 | 164 | cytoplasmic |

As used herein, the term "Myoglobin" refers to one or polypeptides present in a biological sample that are derived from the Myoglobin precursor (Swiss-Prot P02144 (SEQ ID NO: 10)).

```
        10         20         30         40         50         60

MGLSDGEWQL VLNVWGKVEA DIPGHGQEVL IRLFKGHPET LEKFDKFKHL KSEDEMKASE

        70         80         90        100        110        120

DLKKHGATVL TALGGILKKK GHHEAEIKPL AQSHATKHKI PVKYLEFISE CIIQVLQSKH

       130        140        150

PGDFGADAQG AMNKALELFR KDMASNYKEL GFQG
```

The following domains have been identified in Myoglobin:

| Residues | Length | Domain ID |
| --- | --- | --- |
| 1 | 1 | Initiator Methionine |
| 2-154 | 153 | Myoglobin |

As used herein, the term "Apolipoprotein A-II" refers to one or polypeptides present in a biological sample that are derived from the Apolipoprotein A-II precursor (Swiss-Prot P02652 (SEQ ID NO: 11)).

```
        10          20          30          40          50          60

MKLLAATVLL  LTICSLEGAL  VRRQAKEPCV  ESLVSQYFQT  VTDYGKDLME  KVKSPELQAE

        70          80          90         100

AKSYFEKSKE  QLTPLIKKAG  TELVNFLSYF  VELGTQPATQ
```

The following domains have been identified in Apolipoprotein A-II:

| Residues | Length | Domain ID |
|---|---|---|
| 1-18 | 18 | Signal sequence |
| 19-23 | 5 | Propeptide |
| 24-100 | 77 | Apolipoprotein A-II |
| 24-99 | 76 | Apolipoprotein A-II(1-76) |

As used herein, the term "Mucin-16" refers to one or polypeptides present in a biological sample that are derived from the Mucin-16 precursor (Swiss-Prot Q8WXI7 (SEQ ID NO: 12)).

```
        10          20          30          40          50          60

MLKPSGLPGS  SSPTRSLMTG  SRSTKATPEM  DSGLTGATLS  PKTSTGAIVV  TEHTLPFTSP

        70          80          90         100         110         120

DKTLASPTSS  VVGRTTQSLG  VMSSALPEST  SRGMTHSEQR  TSPSLSPQVN  GTPSRNYPAT

       130         140         150         160         170         180

SMVSGLSSPR  TRTSSTEGNF  TKEASTYTLT  VETTSGPVTE  KYTVPTETST  TEGDSTETPW
```

|  | 190 | 200 | 210 | 220 | 230 | 240 |
|---|---|---|---|---|---|---|
|  | DTRYIPVKIT | SPMKTFADST | ASKENAPVSM | TPAETTVTDS | HTPGRTNPSF | GTLYSSFLDL |

|  | 250 | 260 | 270 | 280 | 290 | 300 |
|---|---|---|---|---|---|---|
|  | SPKGTPNSRG | ETSLELILST | TGYPFSSPEP | GSAGHSRIST | SAPLSSSASV | LDNKISETSI |

|  | 310 | 320 | 330 | 340 | 350 | 360 |
|---|---|---|---|---|---|---|
|  | FSGQSLTSPL | SPGVPEARAS | TMPNSAIPFS | MTLSNAETSA | ERVRSTISSL | GTPSISTKQT |

|  | 370 | 380 | 390 | 400 | 410 | 420 |
|---|---|---|---|---|---|---|
|  | AETILTFHAF | AETMDIPSTH | IAKTLASEWL | GSPGTLGGTS | TSALTTTSPS | TTLVSEETNT |

|  | 430 | 440 | 450 | 460 | 470 | 480 |
|---|---|---|---|---|---|---|
|  | HHSTSGKETE | GTLNTSMTPL | ETSAPGEESE | MTATLVPTLG | FTTLDSKIRS | PSQVSSSHPT |

|  | 490 | 500 | 510 | 520 | 530 | 540 |
|---|---|---|---|---|---|---|
|  | RELRTTGSTS | GRQSSSTAAH | GSSDILRATT | SSTSKASSWT | SESTAQQFSE | PQHTQWVETS |

|  | 550 | 560 | 570 | 580 | 590 | 600 |
|---|---|---|---|---|---|---|
|  | PSMKTERPPA | STSVAAPITT | SVPSVVSGFT | TLKTSSTKGI | WLEETSADTL | IGESTAGPTT |

|  | 610 | 620 | 630 | 640 | 650 | 660 |
|---|---|---|---|---|---|---|
|  | HQFAVPTGIS | MTGGSSTRGS | QGTTHLLTRA | TASSETSADL | TLATNGVPVS | VSPAVSKTAA |

|  | 670 | 680 | 690 | 700 | 710 | 720 |
|---|---|---|---|---|---|---|
|  | GSSPPGGTKP | SYTMVSSVIP | ETSSLQSSAF | REGTSLGLTP | LNTRHPFSSP | EPDSAGHTKI |

|  | 730 | 740 | 750 | 760 | 770 | 780 |
|---|---|---|---|---|---|---|
|  | STSIPLLSSA | SVLEDKVSAT | STFSHHKATS | SITTGTPEIS | TKTKPSSAVL | SSMTLSNAAT |

|  | 790 | 800 | 810 | 820 | 830 | 840 |
|---|---|---|---|---|---|---|
|  | SPERVRNATS | PLTHPSPSGE | ETAGSVLTLS | TSAETTDSPN | IHPTGTLTSE | SSESPSTLSL |

|  | 850 | 860 | 870 | 880 | 890 | 900 |
|---|---|---|---|---|---|---|
|  | PSVSGVKTTF | SSSTPSTHLF | TSGEETEETS | NPSVSQPETS | VSRVRTTLAS | TSVPTPVFPT |

|  | 910 | 920 | 930 | 940 | 950 | 960 |
|---|---|---|---|---|---|---|
|  | MDTWPTRSAQ | FSSSHLVSEL | RATSSTSVTN | STGSALPKIS | HLTGTATMSQ | TNRDTFNDSA |

|  | 970 | 980 | 990 | 1000 | 1010 | 1020 |
|---|---|---|---|---|---|---|
|  | APQSTTWPET | SPRFKTGLPS | ATTTVSTSAT | SLSATVMVSK | FTSPATSSME | ATSIREPSTT |

```
        1030       1040       1050       1060       1070       1080
ILTTETTNGP GSMAVASTNI PIGKGYITEG RLDTSHLPIG TTASSETSMD FTMAKESVSM

        1090       1100       1110       1120       1130       1140
SVSPSQSMDA AGSSTPGRTS QFVDTFSDDV YHLTSREITI PRDGTSSALT PQMTATHPPS

        1150       1160       1170       1180       1190       1200
PDPGSARSTW LGILSSSPSS PTPKVTMSST FSTQRVTTSM IMDTVETSRW NMPNLPSTTS

        1210       1220       1230       1240       1250       1260
LTPSNIPTSG AIGKSTLVPL DTPSPATSLE ASEGGLPTLS TYPESTNTPS IHLGAHASSE

        1270       1280       1290       1300       1310       1320
SPSTIKLTMA SVVKPGSYTP LTFPSIETHI HVSTARMAYS SGSSPEMTAP GETNTGSTWD

        1330       1340       1350       1360       1370       1380
PTTYITTTDP KDTSSAQVST PHSVRTLRTT ENHPKTESAT PAAYSGSPKI SSSPNLTSPA

        1390       1400       1410       1420       1430       1440
TKAWTITDTT EHSTQLHYTK LAEKSSGFET QSAPGPVSVV IPTSPTIGSS TLELTSDVPG

        1450       1460       1470       1480       1490       1500
EPLVLAPSEQ TTITLPMATW LSTSLTEEMA STDLDISSPS SPMSTFAIFP PMSTPSHELS

        1510       1520       1530       1540       1550       1560
KSEADTSAIR NTDSTTLDQH LGIRSLGRTG DLTTVPITPL TTTWTSVIEH STQAQDTLSA

        1570       1580       1590       1600       1610       1620
TMSPTHVTQS LKDQTSIPAS ASPSHLTEVY PELGTQGRSS SEATTFWKPS TDTLSREIET

        1630       1640       1650       1660       1670       1680
GPTNIQSTPP MDNTTTGSSS SGVTLGIAHL PIGTSSPAET STNMALERRS STATVSMAGT

        1690       1700       1710       1720       1730       1740
MGLLVTSAPG RSISQSLGRV SSVLSESTTE GVTDSSKGSS PRLNTQGNTA LSSSLEPSYA

        1750       1760       1770       1780       1790       1800
EGSQMSTSIP LTSSPTTPDV EFIGGSTFWT KEVTTVMTSD ISKSSARTES SSATLMSTAL

        1810       1820       1830       1840       1850       1860
GSTENTGKEK LRTASMDLPS PTPSMEVTPW ISLTLSNAPN TTDSLDLSHG VHTSSAGTLA
```

```
        1870        1880        1890        1900        1910        1920

TDRSLNTGVT RASRLENGSD TSSKSLSMGN STHTSMTDTE KSEVSSSIHP RPETSAPGAE

        1930        1940        1950        1960        1970        1980

TTLTSTPGNR AISLTLPFSS IPVEEVISTG ITSGPDINSA PMTHSPITPP TIVWTSTGTI

        1990        2000        2010        2020        2030        2040

EQSTQPLHAV SSEKVSVQTQ STPYVNSVAV SASPTHENSV SSGSSTSSPY SSASLESLDS

        2050        2060        2070        2080        2090        2100

TISRRNAITS WLWDLTTSLP TTTWPSTSLS EALSSGHSGV SNPSSTTTEF PLFSAASTSA

        2110        2120        2130        2140        2150        2160

AKQRNPETET HGPQNTAAST LNTDASSVTG LSETPVGASI SSEVPLPMAI TSRSDVSGLT

        2170        2180        2190        2200        2210        2220

SESTANPSLG TASSAGTKLT RTISLPTSES LVSFRMNKDP WTVSIPLGSH PTTNTETSIP

        2230        2240        2250        2260        2270        2280

VNSAGPPGLS TVASDVIDTP SDGAESIPTV SFSPSPDTEV TTISHFPEKT THSFRTISSL

        2290        2300        2310        2320        2330        2340

THELTSRVTP IPGDWMSSAM STKPTGASPS ITLGERRTIT SAAPTTSPIV LTASFTETST

        2350        2360        2370        2380        2390        2400

VSLDNETTVK TSDILDARKT NELPSDSSSS SDLINTSIAS STMDVTKTAS ISPTSISGMT

        2410        2420        2430        2440        2450        2460

ASSSPSLFSS DRPQVPTSTT ETNTATSPSV SSNTYSLDGG SNVGGTPSTL PPFTITHPVE

        2470        2480        2490        2500        2510        2520

TSSALLAWSR PVRTFSTMVS TDTASGENPT SSNSVVTSVP APGTWASVGS TTDLPAMGFL

        2530        2540        2550        2560        2570        2580

KTSPAGEAHS LLASTIEPAT AFTPHLSAAV VTGSSATSEA SLLTTSESKA IHSSPQTPTT

        2590        2600        2610        2620        2630        2640

PTSGANWETS ATPESLLVVT ETSDTTLTSK ILVTDTILFS TVSTPPSKFP STGTLSGASF

        2650        2660        2670        2680        2690        2700

PTLLPDTPAI PLTATEPTSS LATSFDSTPL VTIASDSLGT VPETTLTMSE TSNGDALVLK
```

```
        2710        2720        2730        2740        2750        2760

TVSNPDRSIP GITIQGVTES PLHPSSTSPS KIVAPRNTTY EGSITVALST LPAGTTGSLV

        2770        2780        2790        2800        2810        2820

FSQSSENSET TALVDSSAGL ERASVMPLTT GSQGMASSGG IRSGSTHSTG TKTFSSLPLT

        2830        2840        2850        2860        2870        2880

MNPGEVTAMS EITTNRLTAT QSTAPKGIPV KPTSAESGLL TPVSASSSPS KAFASLTTAP

        2890        2900        2910        2920        2930        2940

PSTWGIPQST LTFEFSEVPS LDTKSASLPT PGQSLNTIPD SDASTASSSL SKSPEKNPRA

        2950        2960        2970        2980        2990        3000

RMMTSTKAIS ASSFQSTGFT ETPEGSASPS MAGHEPRVPT SGTGDPRYAS ESMSYPDPSK

        3010        3020        3030        3040        3050        3060

ASSAMTSTSL ASKLTTLFST GQAARSGSSS SPISLSTEKE TSFLSPTAST SRKTSLFLGP

        3070        3080        3090        3100        3110        3120

SMARQPNILV HLQTSALTLS PTSTLNMSQE EPPELTSSQT IAEEEGTTAE TQTLTFTPSE

        3130        3140        3150        3160        3170        3180

TPTSLLPVSS PTEPTARRKS SPETWASSIS VPAKTSLVET TDGTLVTTIK MSSQAAQGNS

        3190        3200        3210        3220        3230        3240

TWPAPAEETG TSPAGTSPGS PEVSTTLKIM SSKEPSISPE IRSTVRNSPW KTPETTVPME

        3250        3260        3270        3280        3290        3300

TTVEPVTLQS TALGSGSTSI SHLPTGTTSP TKSPTENMLA TERVSLSPSP PEAWTNLYSG

        3310        3320        3330        3340        3350        3360

TPGGTRQSLA TMSSVSLESP TARSITGTGQ QSSPELVSKT TGMEFSMWHG STGGTTGDTH

        3370        3380        3390        3400        3410        3420

VSLSTSSNIL EDPVTSPNSV SSLTDKSKHK TETWVSTTAI PSTVLNNKIM AAEQQTSRSV

        3430        3440        3450        3460        3470        3480

DEAYSSTSSW SDQTSGSDIT LGASPDVTNT LYITSTAQTT SLVSLPSGDQ GITSLTNPSG

        3490        3500        3510        3520        3530        3540

GKTSSASSVT SPSIGLETLR ANVSAVKSDI APTAGHLSQT SSPAEVSILD VTTAPTPGIS
```

```
       3550        3560        3570        3580        3590        3600
TTITTMGTNS  ISTTTPNPEV  GMSTMDSTPA  TERRTTSTEH  PSTWSSTAAS  DSWTVTDMTS

       3610        3620        3630        3640        3650        3660
NLKVARSPGT  ISTMHTTSFL  ASSTELDSMS  TPHGRITVIG  TSLVTPSSDA  SAVKTETSTS

       3670        3680        3690        3700        3710        3720
ERTLSPSDTT  ASTPISTFSR  VQRMSISVPD  ILSTSWTPSS  TEAEDVPVSM  VSTDHASTKT

       3730        3740        3750        3760        3770        3780
DPNTPLSTFL  FDSLSTLDWD  TGRSLSSATA  TTSAPQGATT  PQELTLETMI  SPATSQLPFS

       3790        3800        3810        3820        3830        3840
IGHITSAVTP  AAMARSSGVT  FSRPDPTSKK  AEQTSTQLPT  TTSAHPGQVP  RSAATTLDVI

       3850        3860        3870        3880        3890        3900
PHTAKTPDAT  FQRQGQTALT  TEARATSDSW  NEKEKSTPSA  PWITEMMNSV  SEDTIKEVTS

       3910        3920        3930        3940        3950        3960
SSSVLKDPEY  AGHKLGIWDD  FIPKFGKAAH  MRELPLLSPP  QDKEAIHPST  NTVETTGWVT

       3970        3980        3990        4000        4010        4020
SSEHASHSTI  PAHSASSKLT  SPVVTTSTRE  QAIVSMSTTT  WPESTRARTE  PNSFLTIELR

       4030        4040        4050        4060        4070        4080
DVSPYMDTSS  TTQTSIISSP  GSTAITKGPR  TEITSSKRIS  SSFLAQSMRS  SDSPSEAITR

       4090        4100        4110        4120        4130        4140
LSNFPAMTES  GGMILAMQTS  PPGATSLSAP  TLDTSATASW  TGTPLATTQR  FTYSEKTTLF

       4150        4160        4170        4180        4190        4200
SKGPEDTSQP  SPPSVEETSS  SSSLVPIHAT  TSPSNILLTS  QGHSPSSTPP  VTSVFLSETS

       4210        4220        4230        4240        4250        4260
GLGKTTDMSR  ISLEPGTSLP  PNLSSTAGEA  LSTYEASRDT  KAIHHSADTA  VTNMEATSSE

       4270        4280        4290        4300        4310        4320
YSPIPGHTKP  SKATSPLVTS  HIMGDITSST  SVFGSSETTE  IETVSSVNQG  LQERSTSQVA

       4330        4340        4350        4360        4370        4380
SSATETSTVI  THVSSGDATT  HVTKTQATFS  SGTSISSPHQ  FITSTNTFTD  VSTNPSTSLI
```

```
         4390        4400        4410        4420        4430        4440

MTESSGVTIT TQTGPTGAAT QGPYLLDTST MPYLTETPLA VTPDFMQSEK TTLISKGPKD

         4450        4460        4470        4480        4490        4500

VTWTSPPSVA ETSYPSSLTP FLVTTIPPAT STLQGQHTSS PVSATSVLTS GLVKTTDMLN

         4510        4520        4530        4540        4550        4560

TSMEPVTNSP QNLNNPSNEI LATLAATTDI ETIHPSINKA VTNMGTASSA HVLHSTLPVS

         4570        4580        4590        4600        4610        4620

SEPSTATSPM VPASSMGDAL ASISIPGSET TDIEGEPTSS LTAGRKENST LQEMNSTTES

         4630        4640        4650        4660        4670        4680

NIILSNVSVG AITEATKMEV PSFDATFIPT PAQSTKFPDI FSVASSRLSN SPPMTISTHM

         4690        4700        4710        4720        4730        4740

TTTQTGSSGA TSKIPLALDT STLETSAGTP SVVTEGFAHS KITTAMNNDV KDVSQTNPPF

         4750        4760        4770        4780        4790        4800

QDEASSPSSQ APVLVTTLPS SVAFTPQWHS TSSPVSMSSV LTSSLVKTAG KVDTSLETVT

         4810        4820        4830        4840        4850        4860

SSPQSMSNTL DDISVTSAAT TDIETTHPSI NTVVTNVGTT GSAFESHSTV SAYPEPSKVT

         4870        4880        4890        4900        4910        4920

SPNVTTSTME DTTISRSIPK SSKTTRTETE TTSSLTPKLR ETSISQEITS STETSTVPYK

         4930        4940        4950        4960        4970        4980

ELTGATTEVS RTDVTSSSST SFPGPDQSTV SLDISTETNT RLSTSPIMTE SAEITITTQT

         4990        5000        5010        5020        5030        5040

GPHGATSQDT FTMDPSNTTP QAGIHSAMTH GFSQLDVTTL MSRIPQDVSW TSPPSVDKTS

         5050        5060        5070        5080        5090        5100

SPSSFLSSPA MTTPSLISST LPEDKLSSPM TSLLTSGLVK ITDILRTRLE PVTSSLPNFS

         5110        5120        5130        5140        5150        5160

STSDKILATS KDSKDTKEIF PSINTEETNV KANNSGHESH SPALADSETP KATTQMVITT

         5170        5180        5190        5200        5210        5220

TVGDPAPSTS MPVHGSSETT NIKREPTYFL TPRLRETSTS QESSFPTDTS FLLSKVPTGT
```

```
        5230      5240      5250      5260      5270      5280
ITEVSSTGVN SSSKISTPDH DKSTVPPDTF TGEIPRVFTS SIKTKSAEMT ITTQASPPES

        5290      5300      5310      5320      5330      5340
ASHSTLPLDT STTLSQGGTH STVTQGFPYS EVTTLMGMGP GNVSWMTTPP VEETSSVSSL

        5350      5360      5370      5380      5390      5400
MSSPAMTSPS PVSSTSPQSI PSSPLPVTAL PTSVLVTTTD VLGTTSPESV TSSPPNLSSI

        5410      5420      5430      5440      5450      5460
THERPATYKD TAHTEAAMHH STNTAVTNVG TSGSGHKSQS SVLADSETSK ATPLMSTTST

        5470      5480      5490      5500      5510      5520
LGDTSVSTST PNISQTNQIQ TEPTASLSPR LRESSTSEKT SSTTETNTAF SYVPTGAITQ

        5530      5540      5550      5560      5570      5580
ASRTEISSSR TSISDLDRPT IAPDISTGMI TRLFTSPIMT KSAEMTVTTQ TTTPGATSQG

        5590      5600      5610      5620      5630      5640
ILPWDTSTTL FQGGTHSTVS QGFPHSEITT LRSRTPGDVS WMTTPPVEET SSGFSLMSPS

        5650      5660      5670      5680      5690      5700
MTSPSPVSST SPESIPSSPL PVTALLTSVL VTTTNVLGTT SPETVTSSPP NLSSPTQERL

        5710      5720      5730      5740      5750      5760
TTYKDTAHTE AMHASMHTNT AVANVGTSIS GHESQSSVPA DSHTSKATSP MGITFAMGDT

        5770      5780      5790      5800      5810      5820
SVSTSTPAFF ETRIQTESTS SLIPGLRDTR TSEEINTVTE TSTVLSEVPT TTTTEVSRTE

        5830      5840      5850      5860      5870      5880
VITSSRTTIS GPDHSKMSPY ISTETITRLS TFPFVTGSTE MAITNQTGPI GTISQATLTL

        5890      5900      5910      5920      5930      5940
DTSSTASWEG THSPVTQRFP HSEETTMSR STKGVSWQSP PSVEETSSPS SPVPLPAITS

        5950      5960      5970      5980      5990      6000
HSSLYSAVSG SSPTSALPVT SLLTSGRRKT IDMLDTHSEL VTSSLPSASS FSGEILTSEA

        6010      6020      6030      6040      6050      6060
STNTETIHFS ENTAETNMGT TNSMHKLHSS VSIHSQPSGH TPPKVTGSMM EDAIVSTSTP
```

```
        6070        6080        6090        6100        6110        6120

GSPETKNVDR  DSTSPLTPEL  KEDSTALVMN  STTESNTVFS  SVSLDAATEV  SRAEVTYYDP

        6130        6140        6150        6160        6170        6180

TFMPASAQST  KSPDISPEAS  SSHSNSPPLT  ISTHKTIATQ  TGPSGVTSLG  QLTLDTSTIA

        6190        6200        6210        6220        6230        6240

TSAGTPSART  QDFVDSETTS  VMNNDLNDVL  KTSPFSAEEA  NSLSSQAPLL  VTTSPSPVTS

        6250        6260        6270        6280        6290        6300

TLQEHSTSSL  VSVTSVPTPT  LAKITDMDTN  LEPVTRSPQN  LRNTLATSEA  TTDTHTMHPS

        6310        6320        6330        6340        6350        6360

INTAMANVGT  TSSPNEFYFT  VSPDSDPYKA  TSAVVITSTS  GDSIVSTSMP  RSSAMKKIES

        6370        6380        6390        6400        6410        6420

ETTFSLIFRL  RETSTSQKIG  SSSDTSTVFD  KAFTAATTEV  SRTELTSSSR  TSIQGTEKPT

        6430        6440        6450        6460        6470        6480

MSPDTSTRSV  TMLSTFAGLT  KSEERTIATQ  TGPHRATSQG  TLTWDTSITT  SQAGTHSAMT

        6490        6500        6510        6520        6530        6540

HGFSQLDLST  LTSRVPEYIS  GTSPPSVEKT  SSSSSLLSLP  AITSPSPVPT  TLPESRPSSP

        6550        6560        6570        6580        6590        6600

VHLTSLPTSG  LVKTTDMLAS  VASLPPNLGS  TSHKIPTTSE  DIKDTEKMYP  STNIAVTNVG

        6610        6620        6630        6640        6650        6660

TTTSEKESYS  SVPAYSEPPK  VTSPMVTSFN  IRDTIVSTSM  PGSSEITRIE  MESTFSVAHG

        6670        6680        6690        6700        6710        6720

LKGTSTSQDP  IVSTEKSAVL  HKLTTGATET  SRTEVASSRR  TSIPGPDHST  ESPDISTEVI

        6730        6740        6750        6760        6770        6780

PSLPISLGIT  ESSNMTIITR  TGPPLGSTSQ  GTFTLDTPTT  SSRAGTHSMA  TQEFPHSEMT

        6790        6800        6810        6820        6830        6840

TVMNKDPEIL  SWTIPPSIEK  TSFSSSLMPS  PAMTSPPVSS  TLPKTIHTTP  SPMTSLLTPS

        6850        6860        6870        6880        6890        6900

LVMTTDTLGT  SPEPTTSSPP  NLSSTSHVIL  TTDEDTTAIE  AMHPSTSTAA  TNVETTCSGH
```

```
          6910       6920       6930       6940       6950       6960

GSQSSVLTDS EKTKATAPMD TTSTMGHTTV STSMSVSSET TKIKRESTYS LTPGLRETSI

          6970       6980       6990       7000       7010       7020

SQNASFSTDT SIVLSEVPTG TTAEVSRTEV TSSGRTSIPG PSQSTVLPEI STRTMTRLFA

          7030       7040       7050       7060       7070       7080

SPTMTESAEM TIPTQTGPSG STSQDTLTLD TSTTKSQAKT HSTLTQRFPH SEMTTLMSRG

          7090       7100       7110       7120       7130       7140

PGDMSWQSSP SLENPSSLPS LLSLPATTSP PPISSTLPVT ISSSPLPVTS LLTSSPVTTT

          7150       7160       7170       7180       7190       7200

DMLHTSPELV TSSPPKLSHT SDERLTTGKD TTNTEAVHPS TNTAASNVEI PSFGHESPSS

          7210       7220       7230       7240       7250       7260

ALADSETSKA TSPMFITSTQ EDTTVAISTP HFLETSRIQK ESISSLSPKL RETGSSVETS

          7270       7280       7290       7300       7310       7320

SAIETSAVLS EVSIGATTEI SRTEVTSSSR TSISGSAEST MLPEISTTRK IIKFPTSPIL

          7330       7340       7350       7360       7370       7380

AESSEMTIKT QTSPPGSTSE STFTLDTSTT PSLVITHSTM TQRLPHSEIT TLVSRGAGDV

          7390       7400       7410       7420       7430       7440

PRPSSLPVEE TSPPSSQLSL SAMISPSPVS STLPASSHSS SASVTSPLTP GQVKTTEVLD

          7450       7460       7470       7480       7490       7500

ASAEPETSSP PSLSSTSVEI LATSEVTTDT EKIHPFPNTA VTKVGTSSSG HESPSSVLPD

          7510       7520       7530       7540       7550       7560

SETTKATSAM GTISIMGDTS VSTLTPALSN TRKIQSEPAS SLTTRLRETS TSEETSLATE

          7570       7580       7590       7600       7610       7620

ANTVLSKVST GATTEVSRTE AISFSRTSMS GPEQSTMSQD ISIGTIPRIS ASSVLTESAK

          7630       7640       7650       7660       7670       7680

MTITTQTGPS ESTLESTLNL NTATTPSWVE THSIVIQGFP HPEMTTSMGR GPGGVSWPSP

          7690       7700       7710       7720       7730       7740

PFVKETSPPS SPLSLPAVTS PHPVSTTFLA HIPPSPLPVT SLLTSGPATT TDILGTSTEP
```

```
        7750       7760       7770       7780       7790       7800
GTSSSSSLST TSHERLTTYK DTAHTEAVHP STNTGGTNVA TTSSGYKSQS SVLADSSPMC

        7810       7820       7830       7840       7850       7860
TTSTMGDTSV LTSTPAFLET RRIQTELASS LTPGLRESSG SEGTSSGTKM STVLSKVPTG

        7870       7880       7890       7900       7910       7920
ATTEISKEDV TSIPGPAQST ISPDISTRTV SWFSTSPVMT ESAEITMNTH TSPLGATTQG

        7930       7940       7950       7960       7970       7980
TSTLATSSTT SLTMTHSTIS QGFSHSQMST LMRRGPEDVS WMSPPLLEKT RPSFSLMSSP

        7990       8000       8010       8020       8030       8040
ATTSPSPVSS TLPESISSSP LPVTSLLTSG LAKTTDMLHK SSEPVTNSPA NLSSTSVEIL

        8050       8060       8070       8080       8090       8100
ATSEVTTDTE KTHPSSNRTV TDVGTSSSGH ESTSFVLADS QTSKVTSPMV ITSTMEDTSV

        8110       8120       8130       8140       8150       8160
STSTPGFFET SRIQTEPTSS LTLGLRKTSS SEGTSLATEM STVLSGVPTG ATAEVSRTEV

        8170       8180       8190       8200       8210       8220
TSSSRTSISG FAQLTVSPET STETITRLPT SSIMTESAEM MIKTQTDPPG STPESTHTVD

        8230       8240       8250       8260       8270       8280
ISTTPNWVET HSTVTQRFSH SEMTTLVSRS PGDMLWPSQS SVEETSSASS LLSLPATTSP

        8290       8300       8310       8320       8330       8340
SPVSSTLVED FPSASLPVTS LLTPGLVITT DRMGISREPG TSSTSNLSST SHERLTTLED

        8350       8360       8370       8380       8390       8400
TVDTEDMQPS THTAVTNVRT SISGHESQSS VLSDSETPKA TSPMGTTYTM GETSVSISTS

        8410       8420       8430       8440       8450       8460
DFFETSRIQI EPTSSLTSGL RETSSSERIS SATEGSTVLS EVPSGATTEV SRTEVISSRG

        8470       8480       8490       8500       8510       8520
TSMSGPDQFT ISPDISTEAI TRLSTSPIMT ESAESAITIE TGSPGATSEG TLTLDTSTTT

        8530       8540       8550       8560       8570       8580
FWSGTHSTAS PGFSHSEMTT LMSRTPGDVP WPSLPSVEEA SSVSSSLSSP AMTSTSFFSA
```

```
        8590        8600        8610        8620        8630        8640

LPESISSSPH  PVTALLTLGP  VKTTDMLRTS  SEPETSSPPN  LSSTSAEILA  TSEVTKDREK

        8650        8660        8670        8680        8690        8700

IHPSSNTPVV  NVGTVIYKHL  SPSSVLADLV  TTKPTSPMAT  TSTLGNTSVS  TSTPAFPETM

        8710        8720        8730        8740        8750        8760

MTQPTSSLTS  GLREISTSQE  TSSATERSAS  LSGMPTGATT  KVSRTEALSL  GRTSTPGPAQ

        8770        8780        8790        8800        8810        8820

STISPEISTE  TITRISTPLT  TTGSAEMTIT  PKTGHSGASS  QGTFTLDTSS  RASWPGTHSA

        8830        8840        8850        8860        8870        8880

ATHRSPHSGM  TTPMSRGPED  VSWPSRPSVE  KTSPPSSLVS  LSAVTSPSPL  YSTPSESSHS

        8890        8900        8910        8920        8930        8940

SPLRVTSLFT  PVMMKTTDML  DTSLEPVTTS  PPSMNITSDE  SLATSKATME  TEAIQLSENT

        8950        8960        8970        8980        8990        9000

AVTQMGTISA  RQEFYSSYPG  LPEPSKVTSP  VVTSSTIKDI  VSTTIPASSE  ITRIEMESTS

        9010        9020        9030        9040        9050        9060

TLTPTPRETS  TSQEIHSATK  PSTVPYKALT  SATIEDSMTQ  VMSSSRGPSP  DQSTMSQDIS

        9070        9080        9090        9100        9110        9120

SEVITRLSTS  PIKAESTEMT  ITTQTGSPGA  TSRGTLTLDT  STTFMSGTHS  TASQGFSHSQ

        9130        9140        9150        9160        9170        9180

MTALMSRTPG  DVPWLSHPSV  EEASSASFSL  SSPVMTSSSP  VSSTLPDSIH  SSSLPVTSLL

        9190        9200        9210        9220        9230        9240

TSGLVKTTEL  LGTSSEPETS  SPPNLSSTSA  EILATTEVTT  DTEKLEMTNV  VTSGYTHESP

        9250        9260        9270        9280        9290        9300

SSVLADSVTT  KATSSMGITY  PTGDTNVLTS  TPAFSDTSRI  QTKSKLSLTP  GLMETSISEE

        9310        9320        9330        9340        9350        9360

TSSATEKSTV  LSSVPTGATT  EVSRTEAISS  SRTSIPGPAQ  STMSSDTSME  TITRISTPLT

        9370        9380        9390        9400        9410        9420

RKESTDMAIT  PKTGPSGATS  QGTFTLDSSS  TASWPGTHSA  TTQRFPQSVV  TTPMSRGPED
```

```
        9430       9440       9450       9460       9470       9480

VSWPSPLSVE KNSPPSSLVS SSSVTSPSPL YSTPSGSSHS SPVPVTSLFT SIMMKATDML

        9490       9500       9510       9520       9530       9540

DASLEPETTS APNMNITSDE SLATSKATTE TEAIHVFENT AASHVETTSA TEELYSSSPG

        9550       9560       9570       9580       9590       9600

FSEPTKVISP VVTSSSIRDN MVSTTMPGSS GITRIEIESM SSLTPGLRET RTSQDITSST

        9610       9620       9630       9640       9650       9660

ETSTVLYKMS SGATPEVSRT EVMPSSRTSI PGPAQSTMSL DISDEVVTRL STSPIMTESA

        9670       9680       9690       9700       9710       9720

EITITTQTGY SLATSQVTLP LGTSMTFLSG THSTMSQGLS HSEMTNLMSR GPESLSWTSP

        9730       9740       9750       9760       9770       9780

RFVETTRSSS SLTSLPLTTS LSPVSSTLLD SSPSSPLPVT SLILPGLVKT TEVLDTSSEP

        9790       9800       9810       9820       9830       9840

KTSSSPNLSS TSVEIPATSE IMTDTEKIHP SSNTAVAKVR TSSSVHESHS SVLADSETTI

        9850       9860       9870       9880       9890       9900

TIPSMGITSA VDDTTVFTSN PAFSETRRIP TEPTFSLTPG FRETSTSEET TSITETSAVL

        9910       9920       9930       9940       9950       9960

YGVPTSATTE VSMTEIMSSN RTHIPDSDQS TMSPDIITEV ITRLSSSSMM SESTQMTITT

        9970       9980       9990      10000      10010      10020

QKSSPGATAQ STLTLATTTA PLARTHSTVP PRFLHSEMTT LMSRSPENPS WKSSPFVEKT

       10030      10040      10050      10060      10070      10080

SSSSSLLSLP VTTSPSVSST LPQSIPSSSF SVTSLLTPGM VKTTDTSTEP GTSLSPNLSG

       10090      10100      10110      10120      10130      10140

TSVEILAASE VTTDTEKIHP SSSMAVTNVG TTSSGHELYS SVSIHSEPSK ATYPVGTPSS

       10150      10160      10170      10180      10190      10200

MAETSISTSM PANFETTGFE AEPFSHLTSG FRKTNMSLDT SSVTPTNTPS SPGSTHLLQS

       10210      10220      10230      10240      10250      10260

SKTDFTSSAK TSSPDWPPAS QYTEIPVDII TPFNASPSIT ESTGITSFPE SRFTMSVTES
```

```
         10270        10280        10290        10300        10310        10320

THHLSTDLLP   SAETISTGTV   MPSLSEAMTS   FATTGVPRAI   SGSGSPFSRT   ESGPGDATLS

         10330        10340        10350        10360        10370        10380

TIAESLPSST   PVPFSSSTFT   TTDSSTIPAL   HEITSSSATP   YRVDTSLGTE   SSTTEGRLVM

         10390        10400        10410        10420        10430        10440

VSTLDTSSQP   GRTSSTPILD   TRMTESVELG   TVTSAYQVPS   LSTRLTRTDG   IMEHITKIPN

         10450        10460        10470        10480        10490        10500

EAAHRGTIRP   VKGPQTSTSP   ASPKGLHTGG   TKRMETTTTA   LKTTTTALKT   TSRATLTTSV

         10510        10520        10530        10540        10550        10560

YTPTLGTLTP   LNASRQMAST   ILTEMMITTP   YVFPDVPETT   SSLATSLGAE   TSTALPRTTP

         10570        10580        10590        10600        10610        10620

SVLNRESETT   ASLVSRSGAE   RSPVIQTLDV   SSSEPDTTAS   WVIHPAETIP   TVSKTTPNFF

         10630        10640        10650        10660        10670        10680

HSELDTVSST   ATSHGADVSS   AIPTNISPSE   LDALTPLVTI   SGTDTSTTFP   TLTKSPHETE

         10690        10700        10710        10720        10730        10740

TRTTWLTHPA   ETSSTIPRTI   PNFSHHESDA   TPSIATSPGA   ETSSAIPIMT   VSPGAEDLVT

         10750        10760        10770        10780        10790        10800

SQVTSSGTDR   NMTIPTLTLS   PGEPKTIASL   VTHPEAQTSS   AIPTSTISPA   VSRLVTSMVT

         10810        10820        10830        10840        10850        10860

SLAAKTSTTN   RALTNSPGEP   ATTVSLVTHP   AQTSPTVPWT   TSIFFHSKSD   TTPSMTTSHG

         10870        10880        10890        10900        10910        10920

AESSSAVPTP   TVSTEVPGVV   TPLVTSSRAV   ISTTIPILTL   SPGEPETTPS   MATSHGEEAS

         10930        10940        10950        10960        10970        10980

SAIPTPTVSP   GVPGVVTSLV   TSSRAVTSTT   IPILTFSLGE   PETTPSMATS   HGTEAGSAVP

         10990        11000        11010        11020        11030        11040

TVLPEVPGMV   TSLVASSRAV   TSTTLPTLTL   SPGEPETTPS   MATSHGAEAS   STVPTVSPEV

         11050        11060        11070        11080        11090        11100

PGVVTSLVTS   SSGVNSTSIP   TLILSPGELE   TTPSMATSHG   AEASSAVPTP   TVSPGVSGVV
```

```
        11110      11120      11130      11140      11150      11160
TPLVTSSRAV TSTTIPILTL SSSEPETTPS MATSHGVEAS SAVLTVSPEV PGMVTSLVTS
        11170      11180      11190      11200      11210      11220
SRAVTSTTIP TLTISSDEPE TTTSLVTHSE AKMISAIPTL AVSPTVQGLV TSLVTSSGSE
        11230      11240      11250      11260      11270      11280
TSAFSNLTVA SSQPETIDSW VAHPGTEASS VVPTLTVSTG EPFTNISLVT HPAESSSTLP
        11290      11300      11310      11320      11330      11340
RTTSRFSHSE LDTMPSTVTS PEAESSSAIS TTISPGIPGV LTSLVTSSGR DISATFPTVP
        11350      11360      11370      11380      11390      11400
ESPHESEATA SWVTHPAVTS TTVPRTTPNY SHSEPDTTPS IATSPGAEAT SDFPTITVSP
        11410      11420      11430      11440      11450      11460
DVPDMVTSQV TSSGTDTSIT IPTLTLSSGE PETTTSFITY SETHTSSAIP TLPVSPGASK
        11470      11480      11490      11500      11510      11520
MLTSLVISSG TDSTTTFPTL TETPYEPETT AIQLIHPAET NTMVPKTTPK FSHSKSDTTL
        11530      11540      11550      11560      11570      11580
PVAITSPGPE ASSAVSTTTI SPDMSDLVTS LVPSSGTDTS TTFPTLSETP YEPETTVTWL
        11590      11600      11610      11620      11630      11640
THPAETSTTV SGTIPNFSHR GSDTAPSMVT SPGVDTRSGV PTTTIPPSIP GVVTSQVTSS
        11650      11660      11670      11680      11690      11700
ATDTSTAIPT LTPSPGEPET TASSATHPGT QTGFTVPIRT VPSSEPDTMA SWVTHPPQTS
        11710      11720      11730      11740      11750      11760
TPVSRTTSSF SHSSPDATPV MATSPRTEAS SAVLTTISPG APEMVTSQIT SSGAATSTTV
        11770      11780      11790      11800      11810      11820
PTLTHSPGMP ETTALLSTHP RTGTSKTFPA STVFPQVSET TASLTIRPGA ETSTALPTQT
        11830      11840      11850      11860      11870      11880
TSSLFTLLVT GTSRVDLSPT ASPGVSAKTA PLSTHPGTET STMIPTSTLS LGLLETTGLL
        11890      11900      11910      11920      11930      11940
ATSSSAETST STLTLTVSPA VSGLSSASIT TDKPQTVTSW NTETSPSVTS VGPPEFSRTV
```

|       |       |       |       |       |       |
|-------|-------|-------|-------|-------|-------|
| 11950 | 11960 | 11970 | 11980 | 11990 | 12000 |

TGTTMTLIPS EMPTPPKTSH GEGVSPTTIL RTTMVEATNL ATTGSSPTVA KTTTTFNTLA

|       |       |       |       |       |       |
|-------|-------|-------|-------|-------|-------|
| 12010 | 12020 | 12030 | 12040 | 12050 | 12060 |

GSLFTPLTTP GMSTLASESV TSRTSYNHRS WISTTSSYNR RYWTPATSTP VTSTFSPGIS

|       |       |       |       |       |       |
|-------|-------|-------|-------|-------|-------|
| 12070 | 12080 | 12090 | 12100 | 12110 | 12120 |

TSSIPSSTAA TVPFMVPFTL NFTITNLQYE EDMRHPGSRK FNATERELQG LLKPLFRNSS

|       |       |       |       |       |       |
|-------|-------|-------|-------|-------|-------|
| 12130 | 12140 | 12150 | 12160 | 12170 | 12180 |

LEYLYSGCRL ASLRPEKDSS AMAVDAICTH RPDPEDLGLD RERLYWELSN LTNGIQELGP

|       |       |       |       |       |       |
|-------|-------|-------|-------|-------|-------|
| 12190 | 12200 | 12210 | 12220 | 12230 | 12240 |

YTLDRNSLYV NGFTHRSSMP TTSTPGTSTV DVGTSGTPSS SPSPTAAGPL LMPFTLNFTI

|       |       |       |       |       |       |
|-------|-------|-------|-------|-------|-------|
| 12250 | 12260 | 12270 | 12280 | 12290 | 12300 |

TNLQYEEDMR RTGSRKFNTM ESVLQGLLKP LFKNTSVGPL YSGCRLTLLR PEKDGAATGV

|       |       |       |       |       |       |
|-------|-------|-------|-------|-------|-------|
| 12310 | 12320 | 12330 | 12340 | 12350 | 12360 |

DAICTHRLDP KSPGLNREQL YWELSKLTND IEELGPYTLD RNSLYVNGFT HQSSVSTTST

|       |       |       |       |       |       |
|-------|-------|-------|-------|-------|-------|
| 12370 | 12380 | 12390 | 12400 | 12410 | 12420 |

PGTSTVDLRT SGTPSSLSSP TIMAAGPLLV PFTLNFTITN LQYGEDMGHP GSRKFNTTER

|       |       |       |       |       |       |
|-------|-------|-------|-------|-------|-------|
| 12430 | 12440 | 12450 | 12460 | 12470 | 12480 |

VLQGLLGPIF KNTSVGPLYS GCRLTSLRSE KDGAATGVDA ICIHHLDPKS PGLNRERLYW

|       |       |       |       |       |       |
|-------|-------|-------|-------|-------|-------|
| 12490 | 12500 | 12510 | 12520 | 12530 | 12540 |

ELSQLTNGIK ELGPYTLDRN SLYVNGFTHR TSVPTTSTPG TSTVDLGTSG TPFSLPSPAT

|       |       |       |       |       |       |
|-------|-------|-------|-------|-------|-------|
| 12550 | 12560 | 12570 | 12580 | 12590 | 12600 |

AGPLLVLFTL NFTITNLKYE EDMHRPGSRK FNTTERVLQT LLGPMFKNTS VGLLYSGCRL

|       |       |       |       |       |       |
|-------|-------|-------|-------|-------|-------|
| 12610 | 12620 | 12630 | 12640 | 12650 | 12660 |

TLLRSEKDGA ATGVDAICTH RLDPKSPGLD REQLYWELSQ LTNGIKELGP YTLDRNSLYV

|       |       |       |       |       |       |
|-------|-------|-------|-------|-------|-------|
| 12670 | 12680 | 12690 | 12700 | 12710 | 12720 |

NGFTHWIPVP TSSTPGTSTV DLGSGTPSSL PSPTAAGPLL VPFTLNFTIT NLQYEEDMHH

|       |       |       |       |       |       |
|-------|-------|-------|-------|-------|-------|
| 12730 | 12740 | 12750 | 12760 | 12770 | 12780 |

PGSRKFNTTE RVLQGLLGPM FKNTSVGLLY SGCRLTLLRS EKDGAATGVD AICTHRLDPK

```
        12790         12800         12810         12820         12830         12840

SPGVDREQLY  WELSQLTNGI  KELGPYTLDR  NSLYVNGFTH  QTSAPNTSTP  GTSTVDLGTS

        12850         12860         12870         12880         12890         12900

GTPSSLPSPT  SAGPLLVPFT  LNFTITNLQY  EEDMRHPGSR  KFNTTERVLQ  GLLKPLFKST

        12910         12920         12930         12940         12950         12960

SVGPLYSGCR  LTLLRSEKDG  AATGVDAICT  HRLDPKSPGV  DREQLYWELS  QLTNGIKELG

        12970         12980         12990         13000         13010         13020

PYTLDRNSLY  VNGFTHQTSA  PNTSTPGTST  VDLGTSGTPS  SLPSPTSAGP  LLVPFTLNFT

        13030         13040         13050         13060         13070         13080

ITNLQYEEDM  HHPGSRKFNT  TERVLQGLLG  PMFKNTSVGL  LYSGCRLTLL  RPEKNGAATG

        13090         13100         13110         13120         13130         13140

MDAICSHRLD  PKSPGLNREQ  LYWELSQLTH  GIKELGPYTL  DRNSLYVNGF  THRSSVAPTS

        13150         13160         13170         13180         13190         13200

TPGTSTVDLG  TSGTPSSLPS  PTTAVPLLVP  FTLNFTITNL  QYGEDMRHPG  SRKFNTTERV

        13210         13220         13230         13240         13250         13260

LQGLLGPLFK  NSSVGPLYSG  CRLISLRSEK  DGAATGVDAI  CTHHLNPQSP  GLDREQLYWQ

        13270         13280         13290         13300         13310         13320

LSQMTNGIKE  LGPYTLDRNS  LYVNGFTHRS  SGLTTSTPWT  STVDLGTSGT  PSPVPSPTTA

        13330         13340         13350         13360         13370         13380

GPLLVPFTLN  FTITNLQYEE  DMHRPGSRKF  NTTERVLQGL  LSPIFKNSSV  GPLYSGCRLT

        13390         13400         13410         13420         13430         13440

SLRPEKDGAA  TGMDAVCLYH  PNPKRPGLDR  EQLYWELSQL  THNITELGPY  SLDRDSLYVN

        13450         13460         13470         13480         13490         13500

GFTHQNSVPT  TSTPGTSTVY  WATTGTPSSF  PGHTEPGPLL  IPFTFNFTIT  NLHYEENMQH

        13510         13520         13530         13540         13550         13560

PGSRKFNTTE  RVLQGLLKPL  FKNTSVGPLY  SGCRLTSLRP  EKDGAATGMD  AVCLYHPNPK

        13570         13580         13590         13600         13610         13620

RPGLDREQLY  WELSQLTHNI  TELGPYSLDR  DSLYVNGFTH  QNSVPTTSTP  GTSTVYWATT
```

GTPSSFPGHT EPGPLLIPFT FNFTITNLHY EENMQHPGSR KFNTTERVLQ GLLKPLFKNT

SVGPLYSGCR LTLLRPEKHE AATGVDTICT HRVDPIGPGL DRERLYWELS QLTNSITELG

PYTLDRDSLY VNGFNPRSSV PTTSTPGTST VHLATSGTPS SLPGHTAPVP LLIPFTLNFT

ITNLHYEENM QHPGSRKFNT TERVLQGLLK PLFKNTSVGP LYSGCRLTLL RPEKHEAATG

VDTICTHRVD PIGPGLXXEX LYWELSXLTX XIXELGPYTL DRXSLYVNGF THXXSXPTTS

TPGTSTVXXG TSGTPSSXPX XTSAGPLLVP FTLNFTITNL QYEEDMHHPG SRKFNTTERV

LQGLLGPMFK NTSVGLLYSG CRLTLLRPEK NGAATGMDAI CSHRLDPKSP GLDREQLYWE

LSQLTHGIKE LGPYTLDRNS LYVNGFTHRS SVAPTSTPGT STVDLGTSGT PSSLPSPTTA

VPLLVPFTLN FTITNLQYGE DMRHPGSRKF NTTERVLQGL LGPLFKNSSV GPLYSGCRLI

SLRSEKDGAA TGVDAICTHH LNPQSPGLDR EQLYWQLSQM TNGIKELGPY TLDRNSLYVN

GFTHRSSGLT TSTPWTSTVD LGTSGTPSPV PSPTTAGPLL VPFTLNFTIT NLQYEEDMHR

PGSRKFNATE RVLQGLLSPI FKNSSVGPLY SGCRLTSLRP EKDGAATGMD AVCLYHPNPK

RPGLDREQLY WELSQLTHNI TELGPYSLDR DSLYVNGFTH QSSMTTTRTP DTSTMHLATS

RTPASLSGPT TASPLLVLFT INCTITNLQY EEDMRRTGSR KFNTMESVLQ GLLKPLFKNT

```
          14470        14480        14490        14500        14510        14520
SVGPLYSGCR   LTLLRPKKDG   AATGVDAICT   HRLDPKSPGL   NREQLYWELS   KLTNDIEELG
          14530        14540        14550        14560        14570        14580
PYTLDRNSLY   VNGFTHQSSV   STTSTPGTST   VDLRTSGTPS   SLSSPTIMXX   XPLLXPFTXN
          14590        14600        14610        14620        14630        14640
XTITNLXXXX   XMXXPGSRKF   NTTERVLQGL   LRPLFKNTSV   SSLYSGCRLT   LLRPEKDGAA
          14650        14660        14670        14680        14690        14700
TRVDAACTYR   PDPKSPGLDR   EQLYWELSQL   THSITELGPY   TLDRVSLYVN   GFNPRSSVPT
          14710        14720        14730        14740        14750        14760
TSTPGTSTVH   LATSGTPSSL   PGHTXXXPLL   XPFTXNXTIT   NLXXXXXMXX   PGSRKFNTTE
          14770        14780        14790        14800        14810        14820
RVLQGLLKPL   FRNSSLEYLY   SGCRLASLRP   EKDSSAMAVD   AICTHRPDPE   DLGLDRERLY
          14830        14840        14850        14860        14870        14880
WELSNLTNGI   QELGPYTLDR   NSLYVNGFTH   RSSGLTTSTP   WTSTVDLGTS   GTPSPVPSPT
          14890        14900        14910        14920        14930        14940
TAGPLLVPFT   LNFTITNLQY   EEDMHRPGSR   RFNTTERVLQ   GLLTPLFKNT   SVGPLYSGCR
          14950        14960        14970        14980        14990        15000
LTLLRPEKQE   AATGVDTICT   HRVDPIGPGL   DRERLYWELS   QLTNSITELG   PYTLDRDSLY
          15010        15020        15030        15040        15050        15060
VNGFNPWSSV   PTTSTPGTST   VHLATSGTPS   SLPGHTAPVP   LLIPFTLNFT   ITDLHYEENM
          15070        15080        15090        15100        15110        15120
QHPGSRKFNT   TERVLQGLLK   PLFKSTSVGP   LYSGCRLTLL   RPEKHGAATG   VDAICTLRLD
          15130        15140        15150        15160        15170        15180
PTGPGLDRER   LYWELSQLTN   SVTELGPYTL   DRDSLYVNGF   THRSSVPTTS   IPGTSAVHLE
          15190        15200        15210        15220        15230        15240
TSGTPASLPG   HTAPGPLLVP   FTLNFTITNL   QYEEDMRHPG   SRKFSTTERV   LQGLLKPLFK
          15250        15260        15270        15280        15290        15300
NTSVSSLYSG   CRLTLLRPEK   DGAATRVDAV   CTHRPDPKSP   GLDRERLYWK   LSQLTHGITE
```

```
         15310        15320        15330        15340        15350        15360

LGPYTLDRHS   LYVNGFTHQS   SMTTTRTPDT   STMHLATSRT   PASLSGPTTA   SPLLVLFTIN

         15370        15380        15390        15400        15410        15420

FTITNLRYEE   NMHHPGSRKF   NTTERVLQGL   LRPVFKNTSV   GPLYSGCRLT   TLRPKKDGAA

         15430        15440        15450        15460        15470        15480

TKVDAICTYR   PDPKSPGLDR   EQLYWELSQL   THSITELGPY   TQDRDSLYVN   GFTHRSSVPT

         15490        15500        15510        15520        15530        15540

TSIPGTSAVH   LETSGTPASL   PGHTAPGPLL   VPFTLNFTIT   NLQYEEDMRH   PGSRKFNTTE

         15550        15560        15570        15580        15590        15600

RVLQGLLKPL   FKSTSVGPLY   SGCRLTLLRP   EKRGAATGVD   TICTHRLDPL   NPGLDREQLY

         15610        15620        15630        15640        15650        15660

WELSKLTRGI   IELGPYLLDR   GSLYVNGFTH   RTSVPTTSTP   GTSTVDLGTS   GTPFSLPSPA

         15670        15680        15690        15700        15710        15720

XXXPLLXPFT   XNXTITNLXX   XXXMXXPGSR   KFNTTERVLQ   TLLGPMFKNT   SVGLLYSGCR

         15730        15740        15750        15760        15770        15780

LTLLRSEKDG   AATGVDAICT   HRLDPKSPGV   DREQLYWELS   QLTNGIKELG   PYTLDRNSLY

         15790        15800        15810        15820        15830        15840

VNGFTHWIPV   PTSSTPGTST   VDLGSGTPSS   LPSPTTAGPL   LVPFTLNFTI   TNLKYEEDMH

         15850        15860        15870        15880        15890        15900

CPGSRKFNTT   ERVLQSLLGP   MFKNTSVGPL   YSGCRLTLLR   SEKDGAATGV   DAICTHRLDP

         15910        15920        15930        15940        15950        15960

KSPGVDREQL   YWELSQLTNG   IKELGPYTLD   RNSLYVNGFT   HQTSAPNTST   PGTSTVDLGT

         15970        15980        15990        16000        16010        16020

SGTPSSLPSP   TXXXPLLXPF   TXNXTITNLX   XXXXMXXPGS   RKFNTTEXVL   QGLLXPXFKN

         16030        16040        16050        16060        16070        16080

XSVGXLYSGC   RLTXLRXEKX   GAATGXDAIC   XHXXXPKXPG   LXXEXLYWEL   SXLTXXIXEL

         16090        16100        16110        16120        16130        16140

GPYTLDRXSL   YVNGFTHWIP   VPTSSTPGTS   TVDLGSGTPS   SLPSPTTAGP   LLVPFTLNFT
```

```
      16150      16160      16170      16180      16190      16200

ITNLKYEEDM HCPGSRKFNT TERVLQSLLG PMFKNTSVGP LYSGCRLTSL RSEKDGAATG

      16210      16220      16230      16240      16250      16260

VDAICTHRVD PKSPGVDREQ LYWELSQLTN GIKELGPYTL DRNSLYVNGF THQTSAPNTS

      16270      16280      16290      16300      16310      16320

TPGTSTVXXG TSGTPSSXPX XTSAGPLLVP FTLNFTITNL QYEEDMHHPG SRKFNTTERV

      16330      16340      16350      16360      16370      16380

LQGLLGPMFK NTSVGLLYSG CRLTLLRPEK NGATTGMDAI CTHRLDPKSP GLXXEXLYWE

      16390      16400      16410      16420      16430      16440

LSXLTXXIXE LGPYTLDRXS LYVNGFTHXX SXPTTSTPGT STVXXGTSGT PSSXPXXTXX

      16450      16460      16470      16480      16490      16500

XPLLXPFTXN XTITNLXXXX XMXXPGSRKF NTTERVLQGL LKPLFRNSSL EYLYSGCRLA

      16510      16520      16530      16540      16550      16560

SLRPEKDSSA MAVDAICTHR PDPEDLGLDR ERLYWELSNL TNGIQELGPY TLDRNSLYVN

      16570      16580      16590      16600      16610      16620

GFTHRSSMPT TSTPGTSTVD VGTSGTPSSS PSPTTAGPLL IPFTLNFTIT NLQYGEDMGH

      16630      16640      16650      16660      16670      16680

PGSRKFNTTE RVLQGLLGPI FKNTSVGPLY SGCRLTSLRS EKDGAATGVD AICIHHLDPK

      16690      16700      16710      16720      16730      16740

SPGLNRERLY WELSQLTNGI KELGPYTLDR NSLYVNGFTH RTSVPTTSTP GTSTVDLGTS

      16750      16760      16770      16780      16790      16800

GTPFSLPSPA TAGPLLVLFT LNFTITNLKY EEDMHRPGSR KFNTTERVLQ TLLGPMFKNT

      16810      16820      16830      16840      16850      16860

SVGLLYSGCR LTLLRSEKDG AATGVDAICT HRLDPKSPGL XXEXLYWELS XLTXXIXELG

      16870      16880      16890      16900      16910      16920

PYTLDRXSLY VNGFTHXXSX PTTSTPGTST VXXGTSGTPS SXPXXTXXXP LLXPFTXNXT

      16930      16940      16950      16960      16970      16980

ITNLXXXXXM XXPGSRKFNT TERVLQGLLR PVFKNTSVGP LYSGCRLTLL RPKKDGAATK
```

```
        16990      17000      17010      17020      17030      17040
VDAICTYRPD PKSPGLDREQ LYWELSQLTH SITELGPYTQ DRDSLYVNGF THRSSVPTTS
        17050      17060      17070      17080      17090      17100
IPGTSAVHLE TTGTPSSFPG HTEPGPLLIP FTFNFTITNL RYEENMQHPG SRKFNTTERV
        17110      17120      17130      17140      17150      17160
LQGLLTPLFK NTSVGPLYSG CRLTLLRPEK QEAATGVDTI CTHRVDPIGP GLDRERLYWE
        17170      17180      17190      17200      17210      17220
LSQLTNSITE LGPYTLDRDS LYVDGFNPWS SVPTTSTPGT STVHLATSGT PSPLPGHTAP
        17230      17240      17250      17260      17270      17280
VPLLIPFTLN FTITDLHYEE NMQHPGSRKF NTTERVLQGL LKPLFKSTSV GPLYSGCRLT
        17290      17300      17310      17320      17330      17340
LLRPEKHGAA TGVDAICTLR LDPTGPGLDR ERLYWELSQL TNSITELGPY TLDRDSLYVN
        17350      17360      17370      17380      17390      17400
GFNPWSSVPT TSTPGTSTVH LATSGTPSSL PGHTTAGPLL VPFTLNFTIT NLKYEEDMHC
        17410      17420      17430      17440      17450      17460
PGSRKFNTTE RVLQSLHGPM FKNTSVGPLY SGCRLTLLRS EKDGAATGVD AICTHRLDPK
        17470      17480      17490      17500      17510      17520
SPGLXXEXLY WELSXLTXXI XELGPYTLDR XSLYVNGFTH XXSXPTTSTP GTSTVXXGTS
        17530      17540      17550      17560      17570      17580
GTPSSXPXXT XXXPLLXPFT XNXTITNLXX XXXMXXPGSR KFNTTEXVLQ GLLXPXFKNX
        17590      17600      17610      17620      17630      17640
SVGXLYSGCR LTXLRXEKXG AATGXDAICX HXXXPKXPGL XXEXLYWELS XLTNSITELG
        17650      17660      17670      17680      17690      17700
PYTLDRDSLY VNGFTHRSSM PTTSIPGTSA VHLETSGTPA SLPGHTAPGP LLVPFTLNFT
        17710      17720      17730      17740      17750      17760
ITNLQYEEDM RHPGSRKFNT TERVLQGLLK PLFKSTSVGP LYSGCRLTLL RPEKRGAATG
        17770      17780      17790      17800      17810      17820
VDTICTHRLD PLNPGLXXEX LYWELSXLTX XIXELGPYTL DRXSLYVNGF THXXSXPTTS
```

```
    17830      17840      17850      17860      17870      17880

TPGTSTVXXG TSGTPSSXPX XTXXXPLLXP FTXNXTITNL XXXXXMXXPG SRKFNTTEXV

    17890      17900      17910      17920      17930      17940

LQGLLXPXFK NXSVGXLYSG CRLTXLRXEK XGAATGXDAI CXHXXXPKXP GLXXEXLYWE

    17950      17960      17970      17980      17990      18000

LSXLTXXIXE LGPYTLDRXS LYVNGFHPRS SVPTTSTPGT STVHLATSGT PSSLPGHTAP

    18010      18020      18030      18040      18050      18060

VPLLIPFTLN FTITNLHYEE NMQHPGSRKF NTTERVLQGL LGPMFKNTSV GLLYSGCRLT

    18070      18080      18090      18100      18110      18120

LLRPEKNGAA TGMDAICSHR LDPKSPGLXX EXLYWELSXL TXXIXELGPY TLDRXSLYVN

    18130      18140      18150      18160      18170      18180

GFTHXXSXPT TSTPGTSTVX XGTSGTPSSX PXXTXXXPLL XPFTXNXTIT NLXXXXXMXX

    18190      18200      18210      18220      18230      18240

PGSRKFNTTE XVLQGLLXPX FKNXSVGXLY SGCRLTXLRX EKXGAATGXD AICXHXXXPK

    18250      18260      18270      18280      18290      18300

XPGLXXEXLY WELSXLTXXI XELGPYTLDR XSLYVNGFTH QNSVPTTSTP GTSTVYWATT

    18310      18320      18330      18340      18350      18360

GTPSSFPGHT EPGPLLIPFT FNFTITNLHY EENMQHPGSR KFNTTERVLQ GLLTPLFKNT

    18370      18380      18390      18400      18410      18420

SVGPLYSGCR LTLLRPEKQE AATGVDTICT HRVDPIGPGL XXEXLYWELS XLTXXIXELG

    18430      18440      18450      18460      18470      18480

PYTLDRXSLY VNGFTHXXSX PTTSTPGTST VXXGTSGTPS SXPXXTXXXP LLXPFTXNXT

    18490      18500      18510      18520      18530      18540

ITNLXXXXXM XXPGSRKFNT TEXVLQGLLX PXFKNXSVGX LYSGCRLTXL RXEKXGAATG

    18550      18560      18570      18580      18590      18600

XDAICXHXXX PKXPGLXXEX LYWELSXLTX XIXELGPYTL DRXSLYVNGF THRSSVPTTS

    18610      18620      18630      18640      18650      18660

SPGTSTVHLA TSGTPSSLPG HTAPVPLLIP FTLNFTITNL HYEENMQHPG SRKFNTTERV
```

```
        18670        18680        18690        18700        18710        18720

LQGLLKPLFK   STSVGPLYSG   CRLTLLRPEK   HGAATGVDAI   CTLRLDPTGP   GLXXEXLYWE

        18730        18740        18750        18760        18770        18780

LSXLTXXIXE   LGPYTLDRXS   LYVNGFTHXX   SXPTTSTPGT   STVXXGTSGT   PSSXPXXTXX

        18790        18800        18810        18820        18830        18840

XPLLXPFTXN   XTITNLXXXX   XMXXPGSRKF   NTTEXVLQGL   LXPXFKNXSV   GXLYSGCRLT

        18850        18860        18870        18880        18890        18900

XLRXEKXGAA   TGXDAICXHX   XXPKXPGLXX   EXLYWELSXL   TXXIXELGPY   TLDRXSLYVN

        18910        18920        18930        18940        18950        18960

GFTHRTSVPT   TSTPGTSTVH   LATSGTPSSL   PGHTAPVPLL   IPFTLNFTIT   NLQYEEDMHR

        18970        18980        18990        19000        19010        19020

PGSRKFNTTE   RVLQGLLSPI   FKNSSVGPLY   SGCRLTSLRP   EKDGAATGMD   AVCLYHPNPK

        19030        19040        19050        19060        19070        19080

RPGLDREQLY   CELSQLTHNI   TELGPYSLDR   DSLYVNGFTH   QNSVPTTSTP   GTSTVYWATT

        19090        19100        19110        19120        19130        19140

GTPSSFPGHT   XXXPLLXPFT   XNXTITNLXX   XXXMXXPGSR   KFNTTEXVLQ   GLLXPXFKNX

        19150        19160        19170        19180        19190        19200

SVGXLYSGCR   LTXLRXEKXG   AATGXDAICX   HXXXPKXPGL   XXEXLYWELS   XLTXXIXELG

        19210        19220        19230        19240        19250        19260

PYTLDRXSLY   VNGFTHWSSG   LTTSTPWTST   VDLGTSGTPS   PVPSPTTAGP   LLVPFTLNFT

        19270        19280        19290        19300        19310        19320

ITNLQYEEDM   HRPGSRKFNA   TERVLQGLLS   PIFKNTSVGP   LYSGCRLTLL   RPEKQEAATG

        19330        19340        19350        19360        19370        19380

VDTICTHRVD   PIGPGLXXEX   LYWELSXLTX   XIXELGPYTL   DRXSLYVNGF   THXXSXPTTS

        19390        19400        19410        19420        19430        19440

TPGTSTVXXG   TSGTPSSXPX   XTXXXPLLXP   FTXNXTITNL   XXXXXMXXPG   SRKFNTTEXV

        19450        19460        19470        19480        19490        19500

LQGLLXPXFK   NXSVGXLYSG   CRLTXLRXEK   XGAATGXDAI   CXHXXXPKXP   GLXXEXLYWE
```

46

```
      19510      19520      19530      19540      19550      19560

LSXLTXXIXE LGPYTLDRXS LYVNGFTHRS FGLTTSTPWT STVDLGTSGT PSPVPSPTTA

      19570      19580      19590      19600      19610      19620

GPLLVPFTLN FTITNLQYEE DMHRPGSRKF NTTERVLQGL LTPLFRNTSV SSLYSGCRLT

      19630      19640      19650      19660      19670      19680

LLRPEKDGAA TRVDAVCTHR PDPKSPGLXX EXLYWELSXL TXXIXELGPY TLDRXSLYVN

      19690      19700      19710      19720      19730      19740

GFTHXXSXPT TSTPGTSTVX XGTSGTPSSX PXXTXXXPLL XPFTXNXTIT NLXXXXXMXX

      19750      19760      19770      19780      19790      19800

PGSRKFNTTE XVLQGLLXPX FKNXSVGXLY SGCRLTXLRX EKXGAATGXD AICXHXXXPK

      19810      19820      19830      19840      19850      19860

XPGLXXEXLY WELSXLTXXI XELGPYTLDR XSLYVNGFTH WIPVPTSSTP GTSTVDLGSG

      19870      19880      19890      19900      19910      19920

TPSSLPSPTT AGPLLVPFTL NFTITNLQYG EDMGHPGSRK FNTTERVLQG LLGPIFKNTS

      19930      19940      19950      19960      19970      19980

VGPLYSGCRL TSLRSEKDGA ATGVDAICIH HLDPKSPGLX XEXLYWELSX LTXXIXELGP

      19990      20000      20010      20020      20030      20040

YTLDRXSLYV NGFTHXXSXP TTSTPGTSTV XXGTSGTPSS XPXXTXXXPL LXPFTXNXTI

      20050      20060      20070      20080      20090      20100

TNLXXXXXMX XPGSRKFNTT EXVLQGLLXP XFKNXSVGXL YSGCRLTXLR XEKXGAATGX

      20110      20120      20130      20140      20150      20160

DAICXHXXXP KXPGLXXEXL YWELSXLTXX IXELGPYTLD RXSLYVNGFT HQTFAPNTST

      20170      20180      20190      20200      20210      20220

PGTSTVDLGT SGTPSSLPSP TSAGPLLVPF TLNFTITNLQ YEEDMHHPGS RKFNTTERVL

      20230      20240      20250      20260      20270      20280

QGLLGPMFKN TSVGLLYSGC RLTLLRPEKN GAATRVDAVC THRPDPKSPG LXXEXLYWEL

      20290      20300      20310      20320      20330      20340

SXLTXXIXEL GPYTLDRXSL YVNGFTHXXS XPTTSTPGTS TVXXGTSGTP SSXPXXTAPV
```

20350     20360     20370     20380     20390     20400

PLLIPFTLNF TITNLHYEEN MQHPGSRKFN TTERVLQGLL KPLFKSTSVG PLYSGCRLTL

20410     20420     20430     20440     20450     20460

LRPEKHGAAT GVDAICTLRL DPTGPGLDRE RLYWELSQLT NSVTELGPYT LDRDSLYVNG

20470     20480     20490     20500     20510     20520

FTQRSSVPTT SIPGTSAVHL ETSGTPASLP GHTAPGPLLV PFTLNFTITN LQYEVDMRHP

20530     20540     20550     20560     20570     20580

GSRKFNTTER VLQGLLKPLF KSTSVGPLYS GCRLTLLRPE KRGAATGVDT ICTHRLDPLN

20590     20600     20610     20620     20630     20640

PGLDREQLYW ELSKLTRGII ELGPYLLDRG SLYVNGFTHR NFVPITSTPG TSTVHLGTSE

20650     20660     20670     20680     20690     20700

TPSSLPRPIV PGPLLVPFTL NFTITNLQYE EAMRHPGSRK FNTTERVLQG LLRPLFKNTS

20710     20720     20730     20740     20750     20760

IGPLYSSCRL TLLRPEKDKA ATRVDAICTH HPDPQSPGLN REQLYWELSQ LTHGITELGP

20770     20780     20790     20800     20810     20820

YTLDRDSLYV DGFTHWSPIP TTSTPGTSIV NLGTSGIPPS LPETTXXXPL LXPFTXNXTI

20830     20840     20850     20860     20870     20880

TNLXXXXXMX XPGSRKFNTT ERVLQGLLKP LFKSTSVGPL YSGCRLTLLR PEKDGVATRV

20890     20900     20910     20920     20930     20940

DAICTHRPDP KIPGLDRQQL YWELSQLTHS ITELGPYTLD RDSLYVNGFT QRSSVPTTST

20950     20960     20970     20980     20990     21000

PGTFTVQPET SETPSSLPGP TATGPVLLPF TLNFTITNLQ YEEDMHRPGS RKFNTTERVL

21010     21020     21030     21040     21050     21060

QGLLMPLFKN TSVSSLYSGC RLTLLRPEKD GAATRVDAVC THRPDPKSPG LDRERLYWKL

21070     21080     21090     21100     21110     21120

SQLTHGITEL GPYTLDRHSL YVNGFTHQSS MTTTRTPDTS TMHLATSRTP ASLSGPTTAS

21130     21140     21150     21160     21170     21180

PLLVLFTINF TITNLRYEEN MHHPGSRKFN TTERVLQGLL RPVFKNTSVG PLYSGCRLTL

```
        21190         21200         21210         21220         21230         21240

LRPKKDGAAT KVDAICTYRP DPKSPGLDRE QLYWELSQLT HSITELGPYT LDRDSLYVNG

        21250         21260         21270         21280         21290         21300

FTQRSSVPTT SIPGTPTVDL GTSGTPVSKP GPSAASPLLV LFTLNFTITN LRYEENMQHP

        21310         21320         21330         21340         21350         21360

GSRKFNTTER VLQGLLRSLF KSTSVGPLYS GCRLTLLRPE KDGTATGVDA ICTHHPDPKS

        21370         21380         21390         21400         21410         21420

PRLDREQLYW ELSQLTHNIT ELGHYALDND SLFVNGFTHR SSVSTTSTPG TPTVYLGASK

        21430         21440         21450         21460         21470         21480

TPASIFGPSA ASHLLILFTL NFTITNLRYE ENMWPGSRKF NTTERVLQGL LRPLFKNTSV

        21490         21500         21510         21520         21530         21540

GPLYSGSRLT LLRPEKDGEA TGVDAICTHR PDPTGPGLDR EQLYLELSQL THSITELGPY

        21550         21560         21570         21580         21590         21600

TLDRDSLYVN GFTHRSSVPT TSTGVVSEEP FTLNFTINNL RYMADMGQPG SLKFNITDNV

        21610         21620         21630         21640         21650         21660

MKHLLSPLFQ RSSLGARYTG CRVIALRSVK NGAETRVDLL CTYLQPLSGP GLPIKQVFHE

        21670         21680         21690         21700         21710         21720

LSQQTHGITR LGPYSLDKDS LYLNGYNEPG LDEPPTTPKP ATTFLPPLSE ATTAMGYHLK

        21730         21740         21750         21760         21770         21780

TLTLNFTISN LQYSPDMGKG SATFNSTEGV LQHLLRPLFQ KSSMGPFYLG CQLISLRPEK

        21790         21800         21810         21820         21830         21840

DGAATGVDTT CTYHPDPVGP GLDIQQLYWE LSQLTHGVTQ LGFYVLDRDS LFINGYAPQN

        21850         21860         21870         21880         21890         21900

LSIRGEYQIN FHIVNWNLSN PDPTSSEYIT LLRDIQDKVT TLYKGSQLHD TFRFCLVTNL

        21910         21920         21930         21940         21950         21960

TMDSVLVTVK ALFSSNLDPS LVEQVFLDKT LNASFHWLGS TYQLVDIHVT EMESSVYQPT

        21970         21980         21990         22000         22010         22020

SSSSTQHFYL NFTITNLPYS QDKAQPGTTN YQRNKRNIED ALNQLFRNSS IKSYFSDCQV
```

|           |           |           |           |           |           |
|-----------|-----------|-----------|-----------|-----------|-----------|
| 22030     | 22040     | 22050     | 22060     | 22070     | 22080     |
| STFRSVPNRH | HTGVDSLCNF | SPLARRVDRV | AIYEEFLRMT | RNGTQLQNFT | LDRSSVLVDG |
| 22090     | 22100     | 22110     | 22120     | 22130     | 22140     |
| YSPNRNEPLT | GNSDLPFWAV | ILIGLAGLLG | LITCLICGVL | VTTRRRKKEG | EYNVQQQCPG |
| 22150     |           |           |           |           |           |
| YYQSHLDLED | LQ        |           |           |           |           |

Most preferably, the Mucin-16 assay detects one or more soluble forms of Mucin-16. Mucin-16 is a single-pass type I membrane protein having a large extracellular domain, most or all of which is present in soluble forms of Mucin-16 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Mucin-16:

| Residues     | Length | Domain ID     |
|--------------|--------|---------------|
| 1-22152      | 22152  | Mucin-16      |
| 1-22096      | 22906  | extracellular |
| 22907-22117  | 21     | transmembrane |
| 22128-22152  | 35     | cytoplasmic   |

As used herein, the term "Carcinoembryonic antigen-related cell adhesion molecule 5" refers to one or polypeptides present in a biological sample that are derived from the Carcinoembryonic antigen-related cell adhesion molecule 5 precursor (Swiss-Prot P06731 (SEQ ID NO: 13)).

|           |           |           |           |           |           |
|-----------|-----------|-----------|-----------|-----------|-----------|
| 10        | 20        | 30        | 40        | 50        | 60        |
| MESPSAPPHR | WCIPWQRLLL | TASLLTFWNP | PTTAKLTIES | TPFNVAEGKE | VLLLVHNLPQ |
| 70        | 80        | 90        | 100       | 110       | 120       |
| HLFGYSWYKG | ERVDGNRQII | GYVIGTQQAT | PGPAYSGREI | IYPNASLLIQ | NIIQNDTGFY |
| 130       | 140       | 150       | 160       | 170       | 180       |
| TLHVIKSDLV | NEEATGQFRV | YPELPKPSIS | SNNSKPVEDK | DAVAFTCEPE | TQDATYLWWV |
| 190       | 200       | 210       | 220       | 230       | 240       |

```
NNQSLPVSPR  LQLSNGNRTL  TLFNVTRNDT  ASYKCETQNP  VSARRSDSVI  LNVLYGPDAP
        250         260         270         280         290         300

TISPLNTSYR  SGENLNLSCH  AASNPPAQYS  WFVNGTFQQS  TQELFIPNIT  VNNSGSYTCQ
        310         320         330         340         350         360

AHNSDTGLNR  TTVTTITVYA  EPPKPFITSN  NSNPVEDEDA  VALTCEPEIQ  NTTYLWWVNN
        370         380         390         400         410         420

QSLPVSPRLQ  LSNDNRTLTL  LSVTRNDVGP  YECGIQNELS  VDHSDPVILN  VLYGPDDPTI
        430         440         450         460         470         480

SPSYTYYRPG  VNLSLSCHAA  SNPPAQYSWL  IDGNIQQHTQ  ELFISNITEK  NSGLYTCQAN
        490         500         510         520         530         540

NSASGHSRTT  VKTITVSAEL  PKPSISSNNS  KPVEDKDAVA  FTCEPEAQNT  TYLWWVNGQS
        550         560         570         580         590         600

LPVSPRLQLS  NGNRTLTLFN  VTRNDARAYV  CGIQNSVSAN  RSDPVTLDVL  YGPDTPIISP
        610         620         630         640         650         660

PDSSYLSGAN  LNLSCHSASN  PSPQYSWRIN  GIPQQHTQVL  FIAKITPNNN  GTYACFVSNL
        670         680         690         700

ATGRNNSIVK  SITVSASGTS  PGLSAGATVG  IMIGVLVGVA  LI
```

The following domains have been identified in Carcinoembryonic antigen-related cell adhesion molecule 5:

| Residues | Length | Domain ID |
|---|---|---|
| 1-34 | 34 | Signal sequence |
| 35-685 | 5 | Carcinoembryonic antigen-related cell adhesion molecule 5 |
| 686-702 | 17 | Propeptide |

As used herein, the term "cellular tumor antigen p53" refers to one or more polypeptides present in a biological sample that are derived from the cellular tumor antigen p53 precursor (Swiss-Prot P04637 (SEQ ID NO: 14)).

```
        10          20          30          40          50          60

MEEPQSDPSV  EPPLSQETFS  DLWKLLPENN  VLSPLPSQAM  DDLMLSPDDI  EQWFTEDPGP
```

```
        70          80          90         100         110         120

DEAPRMPEAA  PRVAPAPAAP  TPAAPAPAPS  WPLSSSVPSQ  KTYQGSYGFR  LGFLHSGTAK

       130         140         150         160         170         180

SVTCTYSPAL  NKMFCQLAKT  CPVQLWVDST  PPPGTRVRAM  AIYKQSQHMT  EVVRRCPHHE

       190         200         210         220         230         240

RCSDSDGLAP  PQHLIRVEGN  LRVEYLDDRN  TFRHSVVVPY  EPPEVGSDCT  TIHYNYMCNS

       250         260         270         280         290         300

SCMGGMNRRP  ILTIITLEDS  SGNLLGRNSF  EVRVCACPGR  DRRTEEENLR  KKGEPHHELP

       310         320         330         340         350         360

PGSTKRALPN  NTSSSPQPKK  KPLDGEYFTL  QIRGRERFEM  FRELNEALEL  KDAQAGKEPG

       370         380         390

GSRAHSSHLK  SKKGQSTSRH  KKLMFKTEGP  DSD
```

Isoform 2 of cellular tumor antigen p53 has the following changes from this isoform 1 sequence:

332-341: IRGRERFEMF (SEQ ID NO: 15) → DGTSFQKENC (SEQ ID NO: 16)

342-393: Missing

As used herein, the term "relating a signal to the presence or amount" of an analyte reflects this understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

In this regard, the skilled artisan will understand that the signals obtained from an immunoassay are a direct result of complexes formed between one or more antibodies and the target biomolecule (*i.e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.)* and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody or other binding species. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and *The Immunoassay Handbook,* David Wild, ed. Stockton Press, New York, 1994, each of which is hereby incorporated by reference in its entirety, including all tables, figures and claims.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377, each of which is hereby incorporated by reference in its entirety, including all tables, figures and claims. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, *etc.)* as well as molecules that may be indirectly detected by production of a detectable reaction product (e.g., enzymes such as horseradish peroxidase, alkaline phosphatase, *etc.)* or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homo functional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ M$^{-1}$, and preferably between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{12}$ M$^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: $r/c = K(n-r)$: where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098, which is hereby incorporated in its entirety, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection theory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-lalpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61 ); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 m$^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anticoagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2) ), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 $m^2$ = 2 points, 20-40 mL/min/1.73 $m^2$ = 4 points, < 20 mL/min/1.73 $m^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria

males and females 18 years of age or older;

undergoing cardiovascular surgery;

Toronto/Ottawa Predictive Risk Index for Renal Replacement risk score of at least 2 (Wijeysundera et al., JAMA 297: 1801-9, 2007); and

able and willing to provide written informed consent for study participation and to comply with all study procedures.
Exclusion Criteria

known pregnancy;

previous renal transplantation;

acutely worsening renal function prior to enrollment (e.g., any category of RIFLE criteria);

already receiving dialysis (either acute or chronic) or in imminent need of dialysis at enrollment;

currently enrolled in another clinical study or expected to be enrolled in another clinical study within 7 days of cardiac surgery that involves drug infusion or a therapeutic intervention for AKI;

known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 900 adults expected to be in the ICU for at least 48 hours will be enrolled. To be enrolled in the study, each patient must meet all of the following inclusion criteria and none of the following exclusion criteria:

Inclusion Criteria

males and females 18 years of age or older;

Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment;

contrast media exposure within 24 hours of enrollment;

increased Intra-Abdominal Pressure with acute decompensated heart failure; and severe trauma as the primary reason for ICU admission and likely to be hospitalized in the ICU for 48 hours after enrollment;

Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment.

Exclusion Criteria

known pregnancy;
institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets only the SBP < 90 mmHg inclusion criterion set forth above, and does not have shock in the attending physician's or principal investigator's opinion.

After providing informed consent, an EDTA anti-coagulated blood sample (10 mL) and a urine sample (25-30 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 were each measured by standard immunoassay methods using commercially available assay reagents in the urine samples and the plasma component of the blood samples collected. Concentrations were reported as follows: Tumor necrosis factor receptor superfamily member 10B - ng/ml, Cadherin-16 - ng/ml, Caspase-9 - ng/ml, Bcl2 antagonist of cell death - absorbance units, Caspase-1 - pg/ml, Cadherin-1 - pg/ml, Poly [ADP-ribose] polymerase 1 - ng/ml, Cyclin-dependent kinase inhibitor 1 - pg/ml, Cadherin-5 - ng/ml, Myoglobin - ng/ml, Apolipoprotein A-II - ng/ml, Mucin-16, Carcinoembryonic antigen-related cell adhesion molecule 5, and Cellular tumor antigen p53 - U/ml, Carcinoembryonic antigen-related cell adhesion molecule 5 - ng/ml, and Cellular tumor antigen p53 - ng/ml.

Two cohorts were defined as described in the introduction to each of the following tables. In the following tables, the time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) was determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage was used.

The ability to distinguish cohort 1 from Cohort 2 was determined using ROC analysis. SE is the standard error of the AUC, n is the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test, and are reported as p<0.05 in tables 1-6 and p<0.10 in tables 7-14. An AUC <0.5 is indicative of a negative going marker for the comparison, and an AUC > 0.5 is indicative of a positive going marker for the comparison.

Various threshold (or "cutoff') concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

Table 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

**Apolipoprotein A-II**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 71.6 | 85.6 | 71.6 | 72.2 | 71.6 | 75.2 |
| Average | 199 | 234 | 199 | 394 | 199 | 136 |
| Stdev | 723 | 763 | 723 | 2590 | 723 | 224 |
| p(t-test) | | 0.70 | | 0.21 | | 0.58 |
| Min | 2.08 | 4.67 | 2.08 | 9.26 | 2.08 | 1.00E-9 |
| Max | 6400 | 6400 | 6400 | 24300 | 6400 | 1420 |
| n (Samp) | 366 | 74 | 366 | 88 | 366 | 41 |
| n (Patient) | 196 | 74 | 196 | 88 | 196 | 41 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 75.8 | 85.0 | 75.8 | 85.9 | 75.8 | 75.2 |
| Average | 201 | 358 | 201 | 131 | 201 | 120 |
| Stdev | 1050 | 1220 | 1050 | 145 | 1050 | 127 |
| p(t-test) | | 0.45 | | 0.69 | | 0.71 |
| Min | 1.00E-9 | 4.67 | 1.00E-9 | 9.44 | 1.00E-9 | 5.58 |
| Max | 24300 | 6400 | 24300 | 677 | 24300 | 488 |
| n (Samp) | 750 | 27 | 750 | 37 | 750 | 23 |
| n (Patient) | 294 | 27 | 294 | 37 | 294 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72.9 | 95.4 | 72.9 | 82.5 | 72.9 | 117 |
| Average | 219 | 249 | 219 | 483 | 219 | 174 |
| Stdev | 774 | 808 | 774 | 2820 | 774 | 260 |
| p(t-test) | | 0.78 | | 0.16 | | 0.73 |
| Min | 2.08 | 18.9 | 2.08 | 9.26 | 2.08 | 1.00E-9 |
| Max | 6400 | 6400 | 6400 | 24300 | 6400 | 1420 |
| n (Samp) | 297 | 65 | 297 | 74 | 297 | 35 |
| n (Patient) | 132 | 65 | 132 | 74 | 132 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.54 | 0.59 | 0.53 | 0.54 | 0.56 | 0.53 | 0.51 | 0.60 |
| SE | 0.038 | 0.058 | 0.040 | 0.035 | 0.050 | 0.038 | 0.048 | 0.062 | 0.053 |
| p | 0.066 | 0.54 | 0.019 | 0.47 | 0.45 | 0.11 | 0.57 | 0.82 | 0.068 |
| nCohort 1 | 366 | 750 | 297 | 366 | 750 | 297 | 366 | 750 | 297 |
| nCohort 2 | 74 | 27 | 65 | 88 | 37 | 74 | 41 | 23 | 35 |
| Cutoff 1 | 53.4 | 58.0 | 64.3 | 46.8 | 58.2 | 56.1 | 46.8 | 47.2 | 68.6 |
| Sens 1 | 70% | 70% | 71% | 70% | 70% | 70% | 71% | 74% | 71% |
| Spec 1 | 38% | 36% | 45% | 32% | 37% | 38% | 32% | 28% | 48% |
| Cutoff 2 | 36.3 | 34.3 | 48.8 | 40.5 | 33.2 | 44.5 | 28.5 | 41.3 | 37.4 |
| Sens 2 | 81% | 81% | 80% | 81% | 81% | 81% | 80% | 83% | 80% |
| Spec 2 | 20% | 16% | 31% | 24% | 15% | 25% | 12% | 23% | 19% |
| Cutoff 3 | 33.5 | 26.3 | 36.2 | 28.5 | 26.3 | 33.2 | 21.0 | 27.1 | 23.1 |
| Sens 3 | 91% | 93% | 91% | 91% | 92% | 91% | 90% | 91% | 91% |
| Spec 3 | 17% | 9% | 16% | 12% | 9% | 14% | 7% | 10% | 8% |
| Cutoff 4 | 107 | 114 | 113 | 107 | 114 | 113 | 107 | 114 | 113 |
| Sens 4 | 39% | 41% | 42% | 34% | 41% | 41% | 39% | 35% | 54% |
| Spec 4 | 70% | 70% | 70% | 70% | 70% | 70% | 70% | 70% | 70% |
| Cutoff 5 | 135 | 148 | 144 | 135 | 148 | 144 | 135 | 148 | 144 |
| Sens 5 | 32% | 26% | 34% | 26% | 30% | 31% | 32% | 17% | 46% |
| Spec 5 | 80% | 80% | 80% | 80% | 80% | 80% | 80% | 80% | 80% |
| Cutoff 6 | 226 | 227 | 292 | 226 | 227 | 292 | 226 | 227 | 292 |
| Sens 6 | 18% | 15% | 14% | 10% | 11% | 9% | 12% | 13% | 9% |
| Spec 6 | 90% | 90% | 90% | 90% | 90% | 90% | 90% | 90% | 90% |
| OR Quart 2 | 0.67 | 0.42 | 1.2 | 1.4 | 0.58 | 1.1 | 0.46 | 1.4 | 0.60 |
| p Value | 0.32 | 0.21 | 0.68 | 0.33 | 0.31 | 0.87 | 0.14 | 0.56 | 0.39 |
| 95% CI of OR Quart2 | 0.30 1.5 | 0.11 1.6 | 0.51 2.8 | 0.72 2.7 | 0.21 1.6 | 0.50 2.3 | 0.17 1.3 | 0.44 4.5 | 0.19 1.9 |
| OR Quart 3 | 1.2 | 1.1 | 1.7 | 0.88 | 0.79 | 0.99 | 0.72 | 0.80 | 0.60 |
| p Value | 0.59 | 0.79 | 0.22 | 0.72 | 0.62 | 0.97 | 0.48 | 0.74 | 0.39 |
| 95% CI of OR Quart3 | 0.60 2.5 | 0.41 3.2 | 0.73 3.8 | 0.43 1.8 | 0.30 2.0 | 0.46 2.1 | 0.29 1.8 | 0.21 3.0 | 0.19 1.9 |
| OR Quart 4 | 1.6 | 1.3 | 2.6 | 1.5 | 1.3 | 1.7 | 1.2 | 1.4 | 2.4 |
| p Value | 0.17 | 0.62 | 0.018 | 0.20 | 0.53 | 0.12 | 0.69 | 0.57 | 0.056 |
| 95% CI of OR Quart4 | 0.81 3.2 | 0.47 3.5 | 1.2 5.6 | 0.80 3.0 | 0.56 3.1 | 0.86 3.5 | 0.52 2.7 | 0.44 4.5 | 0.98 6.0 |

## Bcl2 antagonist of cell death

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00173 | 0.00173 | nd | nd | nd | nd |
| Average | 0.0412 | 0.00246 | nd | nd | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 0.150 | 0.00254 | nd | nd | nd | nd |
| p(t-test) | | 0.38 | nd | nd | nd | nd |
| Min | 0.00173 | 0.00173 | nd | nd | nd | nd |
| Max | 0.833 | 0.0105 | nd | nd | nd | nd |
| n (Samp) | 54 | 12 | nd | nd | nd | nd |
| n (Patient) | 36 | 12 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00173 | 0.00173 | nd | nd | nd | nd |
| Average | 0.0279 | 0.00319 | nd | nd | nd | nd |
| Stdev | 0.122 | 0.00359 | nd | nd | nd | nd |
| p(t-test) | | 0.62 | nd | nd | nd | nd |
| Min | 0.00173 | 0.00173 | nd | nd | nd | nd |
| Max | 0.833 | 0.0105 | nd | nd | nd | nd |
| n (Samp) | 84 | 6 | nd | nd | nd | nd |
| n (Patient) | 59 | 6 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00173 | 0.00173 | nd | nd | nd | nd |
| Average | 0.0457 | 0.00173 | nd | nd | nd | nd |
| Stdev | 0.172 | 0 | nd | nd | nd | nd |
| p(t-test) | | 0.38 | nd | nd | nd | nd |
| Min | 0.00173 | 0.00173 | nd | nd | nd | nd |
| Max | 0.833 | 0.00173 | nd | nd | nd | nd |
| n (Samp) | 40 | 12 | nd | nd | nd | nd |
| n (Patient) | 26 | 12 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.38 | 0.43 | 0.39 | nd | nd | nd | nd | nd | nd |
| SE | 0.094 | 0.13 | 0.097 | nd | nd | nd | nd | nd | nd |
| p | 0.20 | 0.60 | 0.24 | nd | nd | nd | nd | nd | nd |
| nCohort 1 | 54 | 84 | 40 | nd | nd | nd | nd | nd | nd |
| nCohort 2 | 12 | 6 | 12 | nd | nd | nd | nd | nd | nd |
| Cutoff 1 | 0 | 0 | 0 | nd | nd | nd | nd | nd | nd |
| Sens 1 | 100% | 100% | 100% | nd | nd | nd | nd | nd | nd |
| Spec 1 | 0% | 0% | 0% | nd | nd | nd | nd | nd | nd |
| Cutoff 2 | 0 | 0 | 0 | nd | nd | nd | nd | nd | nd |
| Sens 2 | 100% | 100% | 100% | nd | nd | nd | nd | nd | nd |
| Spec 2 | 0% | 0% | 0% | nd | nd | nd | nd | nd | nd |
| Cutoff 3 | 0 | 0 | 0 | nd | nd | nd | nd | nd | nd |
| Sens 3 | 100% | 100% | 100% | nd | nd | nd | nd | nd | nd |
| Spec 3 | 0% | 0% | 0% | nd | nd | nd | nd | nd | nd |
| Cutoff 4 | 0.00509 | 0.00173 | 0.00173 | nd | nd | nd | nd | nd | nd |
| Sens 4 | 8% | 17% | 0% | nd | nd | nd | nd | nd | nd |
| Spec 4 | 70% | 71% | 78% | nd | nd | nd | nd | nd | nd |
| Cutoff 5 | 0.0148 | 0.00943 | 0.00509 | nd | nd | nd | nd | nd | nd |
| Sens 5 | 0% | 17% | 0% | nd | nd | nd | nd | nd | nd |
| Spec 5 | 83% | 82% | 80% | nd | nd | nd | nd | nd | nd |
| Cutoff 6 | 0.0354 | 0.0235 | 0.0148 | nd | nd | nd | nd | nd | nd |
| Sens 6 | 0% | 0% | 0% | nd | nd | nd | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 6 | 91% | 92% | 90% | nd | nd | nd | nd | nd | nd |
| OR Quart 2 | 9.6 | 4.9 | >0 | nd | nd | nd | nd | nd | nd |
| p Value | 0.050 | 0.17 | <na | nd | nd | nd | nd | nd | nd |
| 95% CI of OR Quart2 | 1.0 92 | 0.50 48 | >na na | nd | nd | nd | nd | nd | nd |
| OR Quart 3 | 3.4 | 1.0 | >160 | nd | nd | nd | nd | nd | nd |
| p Value | 0.31 | 1.0 | <5.9E-4 | nd | nd | nd | nd | nd | nd |
| 95% CI of OR Quart3 | 0.32 37 | 0.059 17 | >8.8 na | nd | nd | nd | nd | nd | nd |
| OR Quart 4 | 2.3 | 0 | >0 | nd | nd | nd | nd | nd | nd |
| p Value | 0.52 | na | <na | nd | nd | nd | nd | nd | nd |
| 95% CI of OR Quart4 | 0.19 28 | na na | >na na | nd | nd | nd | nd | nd | nd |

## Caspase-9

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.469 | 0.955 | nd | nd | nd | nd |
| Average | 1.11 | 0.937 | nd | nd | nd | nd |
| Stdev | 2.89 | 1.01 | nd | nd | nd | nd |
| p(t-test) |  | 0.84 | nd | nd | nd | nd |
| Min | 0.0360 | 0.0360 | nd | nd | nd | nd |
| Max | 19.5 | 3.69 | nd | nd | nd | nd |
| n (Samp) | 54 | 12 | nd | nd | nd | nd |
| n (Patient) | 36 | 12 | nd | nd | nd | nd |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.469 | 1.05 | nd | nd | nd | nd |
| Average | 1.08 | 1.44 | nd | nd | nd | nd |
| Stdev | 2.44 | 1.42 | nd | nd | nd | nd |
| p(t-test) |  | 0.73 | nd | nd | nd | nd |
| Min | 0.0360 | 0.0360 | nd | nd | nd | nd |
| Max | 19.5 | 3.92 | nd | nd | nd | nd |
| n (Samp) | 86 | 6 | nd | nd | nd | nd |
| n (Patient) | 60 | 6 | nd | nd | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.586 | 0.790 | nd | nd | nd | nd |
| Average | 1.35 | 0.929 | nd | nd | nd | nd |
| Stdev | 3.31 | 0.998 | nd | nd | nd | nd |
| p(t-test) |  | 0.67 | nd | nd | nd | nd |
| Min | 0.0360 | 0.0360 | nd | nd | nd | nd |
| Max | 19.5 | 3.69 | nd | nd | nd | nd |
| n (Samp) | 40 | 12 | nd | nd | nd | nd |
| n (Patient) | 26 | 12 | nd | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.59 | 0.66 | 0.56 | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.094 | 0.13 | 0.097 | nd | nd | nd | nd | nd | nd |
| p | 0.32 | 0.20 | 0.52 | nd | nd | nd | nd | nd | nd |
| nCohort 1 | 54 | 86 | 40 | nd | nd | nd | nd | nd | nd |
| nCohort 2 | 12 | 6 | 12 | nd | nd | nd | nd | nd | nd |
| Cutoff 1 | 0.208 | 0.330 | 0.208 | nd | nd | nd | nd | nd | nd |
| Sens 1 | 75% | 83% | 83% | nd | nd | nd | nd | nd | nd |
| Spec 1 | 37% | 38% | 28% | nd | nd | nd | nd | nd | nd |
| Cutoff 2 | 0 | 0.330 | 0.208 | nd | nd | nd | nd | nd | nd |
| Sens 2 | 100% | 83% | 83% | nd | nd | nd | nd | nd | nd |
| Spec 2 | 0% | 38% | 28% | nd | nd | nd | nd | nd | nd |
| Cutoff 3 | 0 | 0 | 0 | nd | nd | nd | nd | nd | nd |
| Sens 3 | 100% | 100% | 100% | nd | nd | nd | nd | nd | nd |
| Spec 3 | 0% | 0% | 0% | nd | nd | nd | nd | nd | nd |
| Cutoff 4 | 0.703 | 0.811 | 0.781 | nd | nd | nd | nd | nd | nd |
| Sens 4 | 58% | 67% | 50% | nd | nd | nd | nd | nd | nd |
| Spec 4 | 70% | 71% | 72% | nd | nd | nd | nd | nd | nd |
| Cutoff 5 | 1.04 | 1.04 | 0.868 | nd | nd | nd | nd | nd | nd |
| Sens 5 | 33% | 50% | 50% | nd | nd | nd | nd | nd | nd |
| Spec 5 | 81% | 80% | 80% | nd | nd | nd | nd | nd | nd |
| Cutoff 6 | 1.76 | 2.17 | 2.17 | nd | nd | nd | nd | nd | nd |
| Sens 6 | 8% | 17% | 8% | nd | nd | nd | nd | nd | nd |
| Spec 6 | 91% | 91% | 90% | nd | nd | nd | nd | nd | nd |
| OR Quart 2 | 0.58 | 1.0 | 1.6 | nd | nd | nd | nd | nd | nd |
| p Value | 0.58 | 1.0 | 0.62 | nd | nd | nd | nd | nd | nd |
| 95% CI of OR Quart2 | 0.083 4.0 | 0.059 17 | 0.23 12 | nd | nd | nd | nd | nd | nd |
| OR Quart 3 | 0.29 | 1.0 | 1.0 | nd | nd | nd | nd | nd | nd |
| p Value | 0.31 | 1.0 | 1.0 | nd | nd | nd | nd | nd | nd |
| 95% CI of OR Quart3 | 0.027 3.1 | 0.059 17 | 0.12 8.4 | nd | nd | nd | nd | nd | nd |
| OR Quart 4 | 2.4 | 3.3 | 3.4 | nd | nd | nd | nd | nd | nd |
| p Value | 0.29 | 0.32 | 0.20 | nd | nd | nd | nd | nd | nd |
| 95% CI of OR Quart4 | 0.48 12 | 0.32 34 | 0.53 22 | nd | nd | nd | nd | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 48800 | 32700 | 48800 | 111000 | nd | nd |
| Average | 84900 | 79300 | 84900 | 143000 | nd | nd |
| Stdev | 121000 | 100000 | 121000 | 147000 | nd | nd |
| p(t-test) | | 0.86 | | 0.091 | nd | nd |
| Min | 160 | 1620 | 160 | 1660 | nd | nd |
| Max | 744000 | 363000 | 744000 | 543000 | nd | nd |
| n (Samp) | 52 | 20 | 52 | 20 | nd | nd |
| n (Patient) | 41 | 20 | 41 | 20 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 37500 | 50200 | 37500 | 111000 | nd | nd |
| Average | 75900 | 91900 | 75900 | 146000 | nd | nd |
| Stdev | 124000 | 107000 | 124000 | 140000 | nd | nd |
| p(t-test) | | 0.65 | | 0.046 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 160 | 1620 | 160 | 2220 | nd | nd |
| Max | 744000 | 363000 | 744000 | 543000 | nd | nd |
| n (Samp) | 42 | 16 | 42 | 21 | nd | nd |
| n (Patient) | 33 | 16 | 33 | 21 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | nd | 0.55 | 0.64 | nd | 0.71 | nd | nd | nd |
| SE | 0.077 | nd | 0.086 | 0.076 | nd | 0.073 | nd | nd | nd |
| p | 0.55 | nd | 0.58 | 0.064 | nd | 0.0041 | nd | nd | nd |
| nCohort 1 | 52 | nd | 42 | 52 | nd | 42 | nd | nd | nd |
| nCohort 2 | 20 | nd | 16 | 20 | nd | 21 | nd | nd | nd |
| Cutoff 1 | 10500 | nd | 21400 | 51100 | nd | 54200 | nd | nd | nd |
| Sens 1 | 70% | nd | 75% | 70% | nd | 71% | nd | nd | nd |
| Spec 1 | 15% | nd | 36% | 54% | nd | 60% | nd | nd | nd |
| Cutoff 2 | 9800 | nd | 10400 | 28500 | nd | 36500 | nd | nd | nd |
| Sens 2 | 80% | nd | 81% | 80% | nd | 81% | nd | nd | nd |
| Spec 2 | 13% | nd | 24% | 38% | nd | 50% | nd | nd | nd |
| Cutoff 3 | 4310 | nd | 3350 | 8380 | nd | 11700 | nd | nd | nd |
| Sens 3 | 90% | nd | 94% | 90% | nd | 90% | nd | nd | nd |
| Spec 3 | 8% | nd | 10% | 12% | nd | 29% | nd | nd | nd |
| Cutoff 4 | 94000 | nd | 80700 | 94000 | nd | 80700 | nd | nd | nd |
| Sens 4 | 25% | nd | 38% | 55% | nd | 62% | nd | nd | nd |
| Spec 4 | 71% | nd | 71% | 71% | nd | 71% | nd | nd | nd |
| Cutoff 5 | 134000 | nd | 122000 | 134000 | nd | 122000 | nd | nd | nd |
| Sens 5 | 20% | nd | 31% | 40% | nd | 48% | nd | nd | nd |
| Spec 5 | 81% | nd | 81% | 81% | nd | 81% | nd | nd | nd |
| Cutoff 6 | 152000 | nd | 144000 | 152000 | nd | 144000 | nd | nd | nd |
| Sens 6 | 15% | nd | 19% | 35% | nd | 33% | nd | nd | nd |
| Spec 6 | 90% | nd | 90% | 90% | nd | 90% | nd | nd | nd |
| OR Quart 2 | 0.74 | nd | 0.62 | 1.4 | nd | 0.92 | nd | nd | nd |
| p Value | 0.70 | nd | 0.59 | 0.67 | nd | 0.93 | nd | nd | nd |
| 95% CI of OR Quart2 | 0.16 3.4 | nd | 0.11 3.5 | 0.27 7.5 | nd | 0.16 5.5 | nd | nd | nd |
| OR Quart 3 | 0.74 | nd | 0.68 | 1.9 | nd | 1.8 | nd | nd | nd |
| p Value | 0.70 | nd | 0.66 | 0.43 | nd | 0.48 | nd | nd | nd |
| 95% CI of OR Quart3 | 0.16 3.4 | nd | 0.12 3.8 | 0.38 9.6 | nd | 0.35 9.5 | nd | nd | nd |
| OR Quart 4 | 1.7 | nd | 1.7 | 4.0 | nd | 6.7 | nd | nd | nd |
| p Value | 0.48 | nd | 0.52 | 0.080 | nd | 0.022 | nd | nd | nd |
| 95% CI of OR Quart4 | 0.41 6.7 | nd | 0.35 7.9 | 0.85 19 | nd | 1.3 34 | nd | nd | nd |

## Cadherin-5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00224 | 0.00224 | nd | nd | nd | nd |
| Average | 0.0957 | 0.0142 | nd | nd | nd | nd |
| Stdev | 0.210 | 0.0183 | nd | nd | nd | nd |
| p(t-test) | | 0.28 | nd | nd | nd | nd |
| Min | 0.00224 | 0.00224 | nd | nd | nd | nd |
| Max | 1.10 | 0.0502 | nd | nd | nd | nd |
| n (Samp) | 47 | 8 | nd | nd | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 30 | 8 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00224 | 0.00224 | nd | nd | nd | nd |
| Average | 0.0806 | 0.00753 | nd | nd | nd | nd |
| Stdev | 0.218 | 0.0108 | nd | nd | nd | nd |
| p(t-test) | | 0.32 | nd | nd | nd | nd |
| Min | 0.00224 | 0.00224 | nd | nd | nd | nd |
| Max | 1.10 | 0.0309 | nd | nd | nd | nd |
| n (Samp) | 34 | 9 | nd | nd | nd | nd |
| n (Patient) | 20 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | 0.44 | nd | nd | nd | nd | nd | nd |
| SE | 0.11 | nd | 0.11 | nd | nd | nd | nd | nd | nd |
| p | 0.72 | nd | 0.62 | nd | nd | nd | nd | nd | nd |
| nCohort 1 | 47 | nd | 34 | nd | nd | nd | nd | nd | nd |
| nCohort 2 | 8 | nd | 9 | nd | nd | nd | nd | nd | nd |
| Cutoff 1 | 0 | nd | 0 | nd | nd | nd | nd | nd | nd |
| Sens 1 | 100% | nd | 100% | nd | nd | nd | nd | nd | nd |
| Spec 1 | 0% | nd | 0% | nd | nd | nd | nd | nd | nd |
| Cutoff 2 | 0 | nd | 0 | nd | nd | nd | nd | nd | nd |
| Sens 2 | 100% | nd | 100% | nd | nd | nd | nd | nd | nd |
| Spec 2 | 0% | nd | 0% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |
| Spec 3 | 0% | nd | 0% | nd | nd | nd | nd | nd | nd |
| Cutoff 4 | 0.0405 | nd | 0.00224 | nd | nd | nd | nd | nd | nd |
| Sens 4 | 12% | nd | 22% | nd | nd | nd | nd | nd | nd |
| Spec 4 | 72% | nd | 71% | nd | nd | nd | nd | nd | nd |
| Cutoff 5 | 0.137 | nd | 0.127 | nd | nd | nd | nd | nd | nd |
| Sens 5 | 0% | nd | 0% | nd | nd | nd | nd | nd | nd |
| Spec 5 | 81% | nd | 82% | nd | nd | nd | nd | nd | nd |
| Cutoff 6 | 0.329 | nd | 0.239 | nd | nd | nd | nd | nd | nd |
| Sens 6 | 0% | nd | 0% | nd | nd | nd | nd | nd | nd |
| Spec 6 | 91% | nd | 91% | nd | nd | nd | nd | nd | nd |
| OR Quart 2 | 2.2 | nd | 0 | nd | nd | nd | nd | nd | nd |
| p Value | 0.55 | nd | na | nd | nd | nd | nd | nd | nd |
| 95% CI of OR Quart2 | 0.17 | nd | na | nd | nd | nd | nd | nd | nd |
| | 27 | nd | na | nd | nd | nd | nd | nd | nd |
| OR Quart 3 | 3.5 | nd | 5.4 | nd | nd | nd | nd | nd | nd |
| p Value | 0.30 | nd | 0.088 | nd | nd | nd | nd | nd | nd |
| 95% CI of OR Quart3 | 0.32 | nd | 0.78 | nd | nd | nd | nd | nd | nd |
| | 39 | nd | 38 | nd | nd | nd | nd | nd | nd |
| OR Quart 4 | 2.4 | nd | 0.50 | nd | nd | nd | nd | nd | nd |
| p Value | 0.51 | nd | 0.60 | nd | nd | nd | nd | nd | nd |
| 95% CI of OR Quart4 | 0.19 | nd | 0.038 | nd | nd | nd | nd | nd | nd |
| | 30 | nd | 6.5 | nd | nd | nd | nd | nd | nd |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.713 | 0.116 | 0.713 | 1.98 | 0.713 | 1.09 |
| Average | 103 | 8.88 | 103 | 19.5 | 103 | 5.00 |
| Stdev | 670 | 19.5 | 670 | 49.0 | 670 | 9.16 |
| p(t-test) | | 0.33 | | 0.35 | | 0.47 |
| Min | 1.14E-14 | 1.70E-14 | 1.14E-14 | 1.70E-14 | 1.14E-14 | 1.70E-14 |
| Max | 6950 | 112 | 6950 | 327 | 6950 | 42.0 |
| n (Samp) | 165 | 48 | 165 | 56 | 165 | 25 |
| n (Patient) | 102 | 48 | 102 | 56 | 102 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.09 | 5.28 | 1.09 | 10.7 | 1.09 | 2.45 |
| Average | 62.4 | 15.7 | 62.4 | 19.5 | 62.4 | 6.76 |
| Stdev | 482 | 28.6 | 482 | 21.2 | 482 | 12.3 |
| p(t-test) | | 0.70 | | 0.69 | | 0.68 |
| Min | 1.14E-14 | 0.116 | 1.14E-14 | 1.70E-14 | 1.14E-14 | 1.70E-14 |
| Max | 6950 | 112 | 6950 | 71.0 | 6950 | 44.1 |
| n (Samp) | 325 | 16 | 325 | 20 | 325 | 13 |
| n (Patient) | 168 | 16 | 168 | 20 | 168 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.850 | 0.116 | 0.850 | 2.45 | 0.850 | 1.09 |
| Average | 119 | 24.9 | 119 | 23.7 | 119 | 10.5 |
| Stdev | 729 | 97.5 | 729 | 60.2 | 729 | 24.6 |
| p(t-test) | | 0.39 | | 0.35 | | 0.48 |
| Min | 1.14E-14 | 1.70E-14 | 1.14E-14 | 0.116 | 1.14E-14 | 0.116 |
| Max | 6950 | 630 | 6950 | 327 | 6950 | 112 |
| n (Samp) | 139 | 46 | 139 | 51 | 139 | 23 |
| n (Patient) | 85 | 46 | 85 | 51 | 85 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | 0.61 | 0.45 | 0.53 | 0.62 | 0.54 | 0.46 | 0.46 | 0.49 |
| SE | 0.048 | 0.077 | 0.050 | 0.045 | 0.069 | 0.048 | 0.063 | 0.084 | 0.065 |
| p | 0.35 | 0.16 | 0.28 | 0.51 | 0.088 | 0.44 | 0.58 | 0.60 | 0.89 |
| nCohort 1 | 165 | 325 | 139 | 165 | 325 | 139 | 165 | 325 | 139 |
| nCohort 2 | 48 | 16 | 46 | 56 | 20 | 51 | 25 | 13 | 23 |
| Cutoff 1 | 1.70E-14 | 0.850 | 1.70E-14 | 1.70E-14 | 0.850 | 1.14E-14 | 1.70E-14 | 1.70E-14 | 1.14E-14 |
| Sens 1 | 98% | 75% | 98% | 96% | 75% | 100% | 96% | 92% | 100% |
| Spec 1 | 2% | 50% | 2% | 2% | 50% | 2% | 2% | 2% | 2% |
| Cutoff 2 | 1.70E-14 | 0.116 | 1.70E-14 | 1.70E-14 | 1.70E-14 | 1.14E-14 | 1.70E-14 | 1.70E-14 | 1.14E-14 |
| Sens 2 | 98% | 81% | 98% | 96% | 90% | 100% | 96% | 92% | 100% |
| Spec 2 | 2% | 43% | 2% | 2% | 2% | 2% | 2% | 2% | 2% |
| Cutoff 3 | 1.70E-14 | 1.14E-14 | 1.70E-14 | 1.70E-14 | 1.70E-14 | 1.14E-14 | 1.70E-14 | 1.70E-14 | 1.14E-14 |
| Sens 3 | 98% | 100% | 98% | 96% | 90% | 100% | 96% | 92% | 100% |
| Spec 3 | 2% | 2% | 2% | 2% | 2% | 2% | 2% | 2% | 2% |
| Cutoff 4 | 7.84 | 9.28 | 9.28 | 7.84 | 9.28 | 9.28 | 7.84 | 9.28 | 9.28 |
| Sens 4 | 23% | 25% | 26% | 38% | 50% | 35% | 24% | 15% | 22% |
| Spec 4 | 70% | 71% | 71% | 70% | 71% | 71% | 70% | 71% | 71% |
| Cutoff 5 | 18.3 | 15.5 | 22.7 | 18.3 | 15.5 | 22.7 | 18.3 | 15.5 | 22.7 |
| Sens 5 | 17% | 25% | 15% | 25% | 45% | 20% | 4% | 15% | 13% |
| Spec 5 | 80% | 80% | 81% | 80% | 80% | 81% | 80% | 80% | 81% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 | 60.3 | 44.8 | 73.1 | 60.3 | 44.8 | 73.1 | 60.3 | 44.8 | 73.1 |
| Sens 6 | 2% | 6% | 4% | 7% | 10% | 6% | 0% | 0% | 4% |
| Spec 6 | 90% | 90% | 91% | 90% | 90% | 91% | 90% | 90% | 91% |
| OR Quart 2 | 1.3 | 0.33 | 1.0 | 0.25 | 0.19 | 0.60 | 4.6 | 2.6 | 2.7 |
| p Value | 0.60 | 0.34 | 0.96 | 0.0079 | 0.13 | 0.31 | 0.027 | 0.26 | 0.13 |
| 95% CI of | 0.50 | 0.033 | 0.38 | 0.091 | 0.022 | 0.23 | 1.2 | 0.50 | 0.75 |
| OR Quart2 | 3.3 | 3.2 | 2.8 | 0.70 | 1.7 | 1.6 | 18 | 14 | 9.6 |
| OR Quart 3 | 0.78 | 2.8 | 1.2 | 0.77 | 0.79 | 1.1 | 0 | 0 | 0.23 |
| p Value | 0.64 | 0.13 | 0.76 | 0.53 | 0.73 | 0.82 | na | na | 0.20 |
| 95% CI of | 0.28 | 0.73 | 0.44 | 0.34 | 0.20 | 0.45 | na | na | 0.025 |
| OR Quart3 | 2.2 | 11 | 3.1 | 1.7 | 3.0 | 2.7 | na | na | 2.2 |
| OR Quart 4 | 2.3 | 1.3 | 1.8 | 0.90 | 2.1 | 1.2 | 4.6 | 3.2 | 2.7 |
| p Value | 0.073 | 0.71 | 0.22 | 0.79 | 0.19 | 0.70 | 0.027 | 0.16 | 0.13 |
| 95% CI of | 0.93 | 0.29 | 0.71 | 0.40 | 0.69 | 0.49 | 1.2 | 0.63 | 0.75 |
| OR Quart4 | 5.5 | 6.1 | 4.5 | 2.0 | 6.4 | 2.9 | 18 | 16 | 9.6 |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.646 | 1.40 | 0.646 | 1.30 | 0.646 | 1.38 |
| Average | 2.04 | 4.66 | 2.04 | 4.98 | 2.04 | 4.81 |
| Stdev | 4.06 | 7.77 | 4.06 | 10.7 | 4.06 | 9.92 |
| p(t-test) | | 6.7E-4 | | 7.2E-4 | | 0.0064 |
| Min | 0.00336 | 0.00336 | 0.00336 | 0.0411 | 0.00336 | 0.0844 |
| Max | 43.4 | 29.4 | 43.4 | 54.4 | 43.4 | 47.4 |
| n (Samp) | 253 | 48 | 253 | 57 | 253 | 26 |
| n (Patient) | 102 | 48 | 102 | 57 | 102 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.947 | 1.48 | 0.947 | 1.66 | 0.947 | 2.29 |
| Average | 12.1 | 3.83 | 12.1 | 7.05 | 12.1 | 3.90 |
| Stdev | 192 | 5.63 | 192 | 12.5 | 192 | 4.53 |
| p(t-test) | | 0.86 | | 0.90 | | 0.88 |
| Min | 0.00336 | 0.00336 | 0.00336 | 0.0411 | 0.00336 | 0.173 |
| Max | 4070 | 21.1 | 4070 | 51.3 | 4070 | 15.3 |
| n (Samp) | 447 | 16 | 447 | 21 | 447 | 13 |
| n (Patient) | 170 | 16 | 170 | 21 | 170 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.625 | 1.44 | 0.625 | 1.25 | 0.625 | 1.35 |
| Average | 2.35 | 4.89 | 2.35 | 4.76 | 2.35 | 4.02 |
| Stdev | 4.80 | 7.57 | 4.80 | 10.6 | 4.80 | 9.89 |
| p(t-test) | | 0.0038 | | 0.015 | | 0.16 |
| Min | 0.00336 | 0.00336 | 0.00336 | 0.0946 | 0.00336 | 0.00336 |
| Max | 43.4 | 29.4 | 43.4 | 54.4 | 43.4 | 47.4 |
| n (Samp) | 212 | 47 | 212 | 52 | 212 | 25 |
| n (Patient) | 85 | 47 | 85 | 52 | 85 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.56 | 0.64 | 0.61 | 0.62 | 0.61 | 0.60 | 0.65 | 0.55 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.046 | 0.076 | 0.047 | 0.043 | 0.067 | 0.045 | 0.061 | 0.084 | 0.062 |
| p | 0.012 | 0.44 | 0.0036 | 0.0089 | 0.081 | 0.014 | 0.11 | 0.068 | 0.41 |
| nCohort 1 | 253 | 447 | 212 | 253 | 447 | 212 | 253 | 447 | 212 |
| nCohort 2 | 48 | 16 | 47 | 57 | 21 | 52 | 26 | 13 | 25 |
| Cutoff 1 | 0.540 | 0.414 | 0.550 | 0.607 | 1.10 | 0.607 | 0.456 | 1.06 | 0.456 |
| Sens 1 | 71% | 75% | 70% | 70% | 71% | 71% | 73% | 77% | 72% |
| Spec 1 | 45% | 32% | 46% | 48% | 53% | 49% | 42% | 53% | 43% |
| Cutoff 2 | 0.383 | 0.271 | 0.485 | 0.394 | 0.394 | 0.444 | 0.303 | 0.849 | 0.303 |
| Sens 2 | 81% | 81% | 81% | 81% | 81% | 81% | 81% | 85% | 80% |
| Spec 2 | 37% | 23% | 43% | 38% | 31% | 42% | 31% | 47% | 32% |
| Cutoff 3 | 0.146 | 0.102 | 0.148 | 0.173 | 0.0990 | 0.211 | 0.146 | 0.211 | 0.0990 |
| Sens 3 | 92% | 94% | 91% | 91% | 90% | 90% | 92% | 92% | 92% |
| Spec 3 | 13% | 7% | 12% | 16% | 7% | 20% | 13% | 17% | 8% |
| Cutoff 4 | 1.54 | 1.91 | 1.54 | 1.54 | 1.91 | 1.54 | 1.54 | 1.91 | 1.54 |
| Sens 4 | 38% | 44% | 43% | 44% | 48% | 42% | 42% | 54% | 32% |
| Spec 4 | 70% | 70% | 70% | 70% | 70% | 70% | 70% | 70% | 70% |
| Cutoff 5 | 2.53 | 3.14 | 2.77 | 2.53 | 3.14 | 2.77 | 2.53 | 3.14 | 2.77 |
| Sens 5 | 35% | 44% | 34% | 28% | 33% | 25% | 27% | 31% | 16% |
| Spec 5 | 80% | 80% | 80% | 80% | 80% | 80% | 80% | 80% | 80% |
| Cutoff 6 | 5.50 | 8.51 | 6.40 | 5.50 | 8.51 | 6.40 | 5.50 | 8.51 | 6.40 |
| Sens 6 | 21% | 12% | 23% | 18% | 19% | 17% | 19% | 15% | 12% |
| Spec 6 | 90% | 90% | 90% | 90% | 90% | 90% | 90% | 90% | 90% |
| OR Quart 2 | 2.2 | 0.74 | 1.8 | 1.6 | 0.66 | 1.3 | 0.57 | 0.50 | 0.58 |
| p Value | 0.14 | 0.69 | 0.30 | 0.36 | 0.65 | 0.61 | 0.46 | 0.57 | 0.47 |
| 95% CI of OR Quart2 | 0.78 6.2 | 0.16 3.4 | 0.60 5.2 | 0.60 4.1 | 0.11 4.0 | 0.48 3.5 | 0.13 2.5 | 0.044 5.5 | 0.13 2.5 |
| OR Quart 3 | 2.4 | 0.49 | 2.2 | 3.0 | 2.8 | 3.2 | 2.1 | 2.6 | 2.8 |
| p Value | 0.093 | 0.41 | 0.14 | 0.015 | 0.14 | 0.013 | 0.19 | 0.27 | 0.074 |
| 95% CI of OR Quart3 | 0.86 6.7 | 0.087 2.7 | 0.77 6.2 | 1.2 7.4 | 0.72 11 | 1.3 7.8 | 0.69 6.6 | 0.49 14 | 0.90 8.4 |
| OR Quart 4 | 3.3 | 1.8 | 4.0 | 2.4 | 2.8 | 2.0 | 1.7 | 2.6 | 0.98 |
| p Value | 0.018 | 0.37 | 0.0064 | 0.058 | 0.14 | 0.17 | 0.40 | 0.27 | 0.98 |
| 95% CI of OR Quart4 | 1.2 8.9 | 0.51 6.3 | 1.5 11 | 0.97 6.0 | 0.72 11 | 0.76 5.0 | 0.51 5.3 | 0.49 14 | 0.27 3.6 |

## Myoglobin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.21 | 0.965 | 1.21 | 0.546 | 1.21 | 0.330 |
| Average | 26.0 | 38.3 | 26.0 | 70.8 | 26.0 | 47.2 |
| Stdev | 82.5 | 97.7 | 82.5 | 151 | 82.5 | 131 |
| p(t-test) | | 0.36 | | 0.0021 | | 0.24 |
| Min | 0.000105 | 0.000105 | 0.000105 | 0.0276 | 0.000105 | 0.0254 |
| Max | 618 | 469 | 618 | 618 | 618 | 469 |
| n (Samp) | 253 | 48 | 253 | 57 | 253 | 26 |
| n (Patient) | 102 | 48 | 102 | 57 | 102 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.608 | 0.186 | 0.608 | 0.375 | 0.608 | 0.349 |
| Average | 32.4 | 66.7 | 32.4 | 58.7 | 32.4 | 62.5 |
| Stdev | 99.0 | 156 | 99.0 | 131 | 99.0 | 173 |
| p(t-test) | | 0.18 | | 0.24 | | 0.29 |
| Min | 0.000105 | 0.000105 | 0.000105 | 0.0375 | 0.000105 | 0.0567 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 618 | 469 | 618 | 442 | 618 | 618 |
| n (Samp) | 447 | 16 | 447 | 21 | 447 | 13 |
| n (Patient) | 170 | 16 | 170 | 21 | 170 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.23 | 1.00 | 1.23 | 0.855 | 1.23 | 0.366 |
| Average | 29.6 | 29.5 | 29.6 | 76.5 | 29.6 | 68.8 |
| Stdev | 90.0 | 76.4 | 90.0 | 160 | 90.0 | 155 |
| p(t-test) | | 0.99 | | 0.0051 | | 0.062 |
| Min | 0.00616 | 0.0266 | 0.00616 | 0.000105 | 0.00616 | 0.0254 |
| Max | 618 | 469 | 618 | 618 | 618 | 469 |
| n (Samp) | 212 | 47 | 212 | 52 | 212 | 25 |
| n (Patient) | 85 | 47 | 85 | 52 | 85 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.48 | 0.51 | 0.50 | 0.53 | 0.51 | 0.45 | 0.52 | 0.50 |
| SE | 0.046 | 0.074 | 0.047 | 0.042 | 0.066 | 0.045 | 0.061 | 0.082 | 0.061 |
| p | 0.85 | 0.84 | 0.87 | 0.94 | 0.66 | 0.84 | 0.44 | 0.76 | 0.97 |
| nCohort 1 | 253 | 447 | 212 | 253 | 447 | 212 | 253 | 447 | 212 |
| nCohort 2 | 48 | 16 | 47 | 57 | 21 | 52 | 26 | 13 | 25 |
| Cutoff 1 | 0.0819 | 0.0422 | 0.0832 | 0.0855 | 0.0849 | 0.0855 | 0.0877 | 0.132 | 0.118 |
| Sens 1 | 71% | 75% | 70% | 70% | 71% | 71% | 73% | 77% | 72% |
| Spec 1 | 19% | 16% | 19% | 19% | 26% | 19% | 19% | 33% | 24% |
| Cutoff 2 | 0.0394 | 0.0333 | 0.0485 | 0.0746 | 0.0556 | 0.0767 | 0.0667 | 0.0667 | 0.0832 |
| Sens 2 | 81% | 81% | 81% | 81% | 81% | 81% | 81% | 85% | 80% |
| Spec 2 | 11% | 12% | 11% | 18% | 21% | 18% | 17% | 23% | 19% |
| Cutoff 3 | 0.0329 | 0 | 0.0333 | 0.0337 | 0.0399 | 0.0333 | 0.0296 | 0.0584 | 0.0296 |
| Sens 3 | 92% | 100% | 91% | 91% | 90% | 90% | 92% | 92% | 92% |
| Spec 3 | 8% | 0% | 7% | 8% | 15% | 7% | 6% | 22% | 5% |
| Cutoff 4 | 6.79 | 5.77 | 7.09 | 6.79 | 5.77 | 7.09 | 6.79 | 5.77 | 7.09 |
| Sens 4 | 44% | 44% | 43% | 35% | 38% | 37% | 23% | 31% | 32% |
| Spec 4 | 70% | 70% | 70% | 70% | 70% | 70% | 70% | 70% | 70% |
| Cutoff 5 | 16.7 | 16.8 | 18.6 | 16.7 | 16.8 | 18.6 | 16.7 | 16.8 | 18.6 |
| Sens 5 | 31% | 38% | 26% | 32% | 29% | 31% | 19% | 15% | 28% |
| Spec 5 | 80% | 80% | 80% | 80% | 80% | 80% | 80% | 80% | 80% |
| Cutoff 6 | 45.5 | 63.4 | 54.1 | 45.5 | 63.4 | 54.1 | 45.5 | 63.4 | 54.1 |
| Sens 6 | 19% | 12% | 13% | 21% | 19% | 23% | 12% | 15% | 16% |
| Spec 6 | 90% | 90% | 90% | 90% | 90% | 90% | 90% | 90% | 90% |
| OR Quart 2 | 0.33 | 0 | 0.33 | 0.47 | 1.0 | 0.42 | 0.65 | 1.7 | 0.47 |
| p Value | 0.020 | na | 0.025 | 0.068 | 1.0 | 0.056 | 0.51 | 0.48 | 0.24 |
| 95% CI of OR Quart2 | 0.13 | na | 0.13 | 0.21 | 0.31 | 0.17 | 0.17 | 0.40 | 0.13 |
| | 0.84 | na | 0.87 | 1.1 | 3.2 | 1.0 | 2.4 | 7.3 | 1.7 |
| OR Quart 3 | 0.33 | 0.41 | 0.39 | 0.33 | 0.32 | 0.37 | 1.4 | 0.66 | 0.60 |
| p Value | 0.020 | 0.21 | 0.045 | 0.015 | 0.17 | 0.032 | 0.57 | 0.65 | 0.40 |
| 95% CI of OR Quart3 | 0.13 | 0.10 | 0.15 | 0.14 | 0.064 | 0.15 | 0.45 | 0.11 | 0.18 |
| | 0.84 | 1.6 | 0.98 | 0.81 | 1.6 | 0.92 | 4.2 | 4.0 | 2.0 |
| OR Quart 4 | 0.84 | 0.86 | 0.83 | 0.86 | 1.2 | 0.93 | 1.4 | 1.0 | 1.0 |
| p Value | 0.66 | 0.79 | 0.65 | 0.68 | 0.78 | 0.84 | 0.56 | 1.0 | 0.97 |
| 95% CI of OR Quart4 | 0.39 | 0.28 | 0.37 | 0.41 | 0.38 | 0.43 | 0.46 | 0.20 | 0.36 |
| | 1.8 | 2.6 | 1.8 | 1.8 | 3.6 | 2.0 | 4.3 | 5.1 | 2.9 |

## Mucin-16

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.619 | 0.753 | 0.619 | 0.547 | nd | nd |
| Average | 1.06 | 1.12 | 1.06 | 23.1 | nd | nd |
| Stdev | 1.28 | 0.983 | 1.28 | 96.4 | nd | nd |
| p(t-test) | | 0.85 | | 0.10 | nd | nd |
| Min | 0.141 | 1.00E-9 | 0.141 | 0.223 | nd | nd |
| Max | 6.37 | 3.40 | 6.37 | 433 | nd | nd |
| n (Samp) | 52 | 20 | 52 | 20 | nd | nd |
| n (Patient) | 41 | 20 | 41 | 20 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.547 | 0.654 | 0.547 | 0.684 | nd | nd |
| Average | 0.951 | 1.00 | 0.951 | 22.0 | nd | nd |
| Stdev | 1.18 | 0.898 | 1.18 | 94.1 | nd | nd |
| p(t-test) | | 0.87 | | 0.15 | nd | nd |
| Min | 0.0565 | 1.00E-9 | 0.0565 | 0.223 | nd | nd |
| Max | 6.37 | 2.82 | 6.37 | 433 | nd | nd |
| n (Samp) | 42 | 16 | 42 | 21 | nd | nd |
| n (Patient) | 33 | 16 | 33 | 21 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | nd | 0.54 | 0.49 | nd | 0.56 | nd | nd | nd |
| SE | 0.077 | nd | 0.086 | 0.077 | nd | 0.078 | nd | nd | nd |
| p | 0.61 | nd | 0.67 | 0.87 | nd | 0.48 | nd | nd | nd |
| nCohort 1 | 52 | nd | 42 | 52 | nd | 42 | nd | nd | nd |
| nCohort 2 | 20 | nd | 16 | 20 | nd | 21 | nd | nd | nd |
| Cutoff 1 | 0.479 | nd | 0.412 | 0.344 | nd | 0.384 | nd | nd | nd |
| Sens 1 | 70% | nd | 75% | 70% | nd | 71% | nd | nd | nd |
| Spec 1 | 37% | nd | 36% | 17% | nd | 29% | nd | nd | nd |
| Cutoff 2 | 0.223 | nd | 0.223 | 0.278 | nd | 0.278 | nd | nd | nd |
| Sens 2 | 85% | nd | 81% | 90% | nd | 90% | nd | nd | nd |
| Spec 2 | 4% | nd | 7% | 10% | nd | 12% | nd | nd | nd |
| Cutoff 3 | 0.146 | nd | 0.0565 | 0.278 | nd | 0.278 | nd | nd | nd |
| Sens 3 | 90% | nd | 94% | 90% | nd | 90% | nd | nd | nd |
| Spec 3 | 4% | nd | 2% | 10% | nd | 12% | nd | nd | nd |
| Cutoff 4 | 0.937 | nd | 0.859 | 0.937 | nd | 0.859 | nd | nd | nd |
| Sens 4 | 45% | nd | 38% | 30% | nd | 43% | nd | nd | nd |
| Spec 4 | 71% | nd | 71% | 71% | nd | 71% | nd | nd | nd |
| Cutoff 5 | 1.24 | nd | 1.09 | 1.24 | nd | 1.09 | nd | nd | nd |
| Sens 5 | 40% | nd | 38% | 30% | nd | 33% | nd | nd | nd |
| Spec 5 | 81% | nd | 81% | 81% | nd | 81% | nd | nd | nd |
| Cutoff 6 | 1.87 | nd | 1.68 | 1.87 | nd | 1.68 | nd | nd | nd |
| Sens 6 | 20% | nd | 19% | 25% | nd | 24% | nd | nd | nd |
| Spec 6 | 90% | nd | 90% | 90% | nd | 90% | nd | nd | nd |
| OR Quart 2 | 0.74 | nd | 0.62 | 0.40 | nd | 0.50 | nd | nd | nd |
| p Value | 0.70 | nd | 0.59 | 0.26 | nd | 0.38 | nd | nd | nd |
| 95% CI of OR Quart2 | 0.16 | nd | 0.11 | 0.082 | nd | 0.11 | nd | nd | nd |
| | 3.4 | nd | 3.5 | 1.9 | nd | 2.3 | nd | nd | nd |
| OR Quart 3 | 0.52 | nd | 0.68 | 0.77 | nd | 0.50 | nd | nd | nd |
| p Value | 0.43 | nd | 0.66 | 0.72 | nd | 0.38 | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart3 | 0.10 2.6 | nd nd | 0.12 3.8 | 0.19 3.2 | nd nd | 0.11 2.3 | nd nd | nd nd | nd nd |
| OR Quart 4 | 2.1 | nd | 1.7 | 1.0 | nd | 1.2 | nd | nd | nd |
| p Value | 0.30 | nd | 0.52 | 1.0 | nd | 0.83 | nd | nd | nd |
| 95% CI of OR Quart4 | 0.52 8.3 | nd nd | 0.35 7.9 | 0.25 4.0 | nd nd | 0.28 4.9 | nd nd | nd nd | nd nd |

## Poly [ADP-ribose] polymerase 1 (cleaved)

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 1.00E-9 | 1.00E-9 | 1.00E-9 | 1.00E-9 | 0.00439 |
| Average | 0.00474 | 0.00294 | 0.00474 | 0.00432 | 0.00474 | 0.00271 |
| Stdev | 0.00688 | 0.00538 | 0.00688 | 0.00586 | 0.00688 | 0.00261 |
| p(t-test) |  | 0.11 |  | 0.70 |  | 0.16 |
| Min | 1.00E-9 | 1.00E-9 | 1.00E-9 | 1.00E-9 | 1.00E-9 | 1.00E-9 |
| Max | 0.0357 | 0.0144 | 0.0357 | 0.0183 | 0.0357 | 0.00723 |
| n (Samp) | 118 | 47 | 118 | 54 | 118 | 24 |
| n (Patient) | 97 | 47 | 97 | 54 | 97 | 24 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 1.00E-9 | 1.00E-9 | 1.00E-9 | 1.00E-9 | 1.00E-9 |
| Average | 0.00375 | 0.00338 | 0.00375 | 0.00301 | 0.00375 | 0.00350 |
| Stdev | 0.00582 | 0.00600 | 0.00582 | 0.00648 | 0.00582 | 0.00540 |
| p(t-test) |  | 0.82 |  | 0.60 |  | 0.89 |
| Min | 1.00E-9 | 1.00E-9 | 1.00E-9 | 1.00E-9 | 1.00E-9 | 1.00E-9 |
| Max | 0.0357 | 0.0144 | 0.0357 | 0.0253 | 0.0357 | 0.0144 |
| n (Samp) | 263 | 14 | 263 | 19 | 263 | 12 |
| n (Patient) | 159 | 14 | 159 | 19 | 159 | 12 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 1.00E-9 | 1.00E-9 | 0.00340 | 1.00E-9 | 0.00471 |
| Average | 0.00356 | 0.00258 | 0.00356 | 0.00527 | 0.00356 | 0.00448 |
| Stdev | 0.00628 | 0.00494 | 0.00628 | 0.00622 | 0.00628 | 0.00455 |
| p(t-test) |  | 0.36 |  | 0.12 |  | 0.51 |
| Min | 1.00E-9 | 1.00E-9 | 1.00E-9 | 1.00E-9 | 1.00E-9 | 1.00E-9 |
| Max | 0.0357 | 0.0144 | 0.0357 | 0.0183 | 0.0357 | 0.0144 |
| n (Samp) | 105 | 45 | 105 | 49 | 105 | 23 |
| n (Patient) | 84 | 45 | 84 | 49 | 84 | 23 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.43 | 0.46 | 0.47 | 0.51 | 0.45 | 0.59 | 0.50 | 0.50 | 0.62 |
| SE | 0.050 | 0.081 | 0.052 | 0.048 | 0.070 | 0.050 | 0.065 | 0.086 | 0.068 |
| p | 0.18 | 0.63 | 0.51 | 0.85 | 0.52 | 0.060 | 0.97 | 0.97 | 0.071 |
| nCohort 1 | 118 | 263 | 105 | 118 | 263 | 105 | 118 | 263 | 105 |
| nCohort 2 | 47 | 14 | 45 | 54 | 19 | 49 | 24 | 12 | 23 |
| Cutoff 1 | 0 | 0 | 0 | 0 | 0 | 0 | 0 | 0 | 0 |
| Sens 1 | 100% | 100% | 100% | 100% | 100% | 100% | 100% | 100% | 100% |
| Spec 1 | 0% | 0% | 0% | 0% | 0% | 0% | 0% | 0% | 0% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 0.00598 | 0.00471 | 0.00406 | 0.00598 | 0.00471 | 0.00406 | 0.00598 | 0.00471 | 0.00406 |
| Sens 4 | 17% | 21% | 24% | 24% | 16% | 47% | 4% | 17% | 61% |
| Spec 4 | 70% | 74% | 70% | 70% | 74% | 70% | 70% | 74% | 70% |
| Cutoff 5 | 0.0141 | 0.00723 | 0.00681 | 0.0141 | 0.00723 | 0.00681 | 0.0141 | 0.00723 | 0.00681 |
| Sens 5 | 11% | 21% | 13% | 19% | 11% | 31% | 0% | 17% | 17% |
| Spec 5 | 81% | 81% | 80% | 81% | 81% | 80% | 81% | 81% | 80% |
| Cutoff 6 | 0.0144 | 0.0144 | 0.0144 | 0.0144 | 0.0144 | 0.0144 | 0.0144 | 0.0144 | 0.0144 |
| Sens 6 | 0% | 0% | 0% | 2% | 5% | 2% | 0% | 0% | 0% |
| Spec 6 | 95% | 97% | 99% | 95% | 97% | 99% | 95% | 97% | 99% |
| OR Quart 2 | 0.59 | 0.33 | 1.3 | 2.3 | 1.0 | 1.3 | 18 | 1.5 | 1.0 |
| p Value | 0.39 | 0.34 | 0.56 | 0.073 | 0.99 | 0.63 | 0.0069 | 0.65 | 1.0 |
| 95% CI of OR Quart2 | 0.18 2.0 | 0.033 3.2 | 0.50 3.6 | 0.93 5.7 | 0.20 5.2 | 0.45 3.7 | 2.2 150 | 0.25 9.4 | 0.23 4.4 |
| OR Quart 3 | 7.4 | 3.4 | 1.0 | 1.3 | 4.6 | 2.2 | 5.6 | 3.8 | 2.3 |
| p Value | 8.9E-5 | 0.080 | 1.0 | 0.63 | 0.022 | 0.13 | 0.12 | 0.11 | 0.21 |
| 95% CI of OR Quart3 | 2.7 20 | 0.87 13 | 0.36 2.8 | 0.49 3.2 | 1.2 17 | 0.79 6.1 | 0.62 51 | 0.76 19 | 0.62 8.7 |
| OR Quart 4 | 1.0 | 0.33 | 1.5 | 1.0 | 0.33 | 2.9 | 7.2 | 0 | 2.0 |
| p Value | 0.96 | 0.34 | 0.41 | 1.0 | 0.34 | 0.038 | 0.074 | na | 0.33 |
| 95% CI of OR Quart4 | 0.35 3.1 | 0.033 3.2 | 0.56 4.1 | 0.38 2.6 | 0.033 3.2 | 1.1 7.9 | 0.82 64 | na na | 0.51 7.5 |

## KSP-Cadherin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.03 | 1.24 | 1.03 | 1.15 | nd | nd |
| Average | 1.47 | 1.58 | 1.47 | 1.90 | nd | nd |
| Stdev | 1.59 | 1.15 | 1.59 | 1.74 | nd | nd |
| p(t-test) | | 0.71 | | 0.25 | nd | nd |
| Min | 0.00263 | 0.0646 | 0.00263 | 0.291 | nd | nd |
| Max | 11.9 | 4.63 | 11.9 | 7.51 | nd | nd |
| n (Samp) | 85 | 32 | 85 | 25 | nd | nd |
| n (Patient) | 68 | 32 | 68 | 25 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.10 | 0.683 | 1.10 | 1.04 | nd | nd |
| Average | 1.58 | 1.36 | 1.58 | 1.60 | nd | nd |
| Stdev | 1.55 | 1.28 | 1.55 | 1.45 | nd | nd |
| p(t-test) | | 0.66 | | 0.98 | nd | nd |
| Min | 0.00263 | 0.0646 | 0.00263 | 0.291 | nd | nd |
| Max | 11.9 | 3.49 | 11.9 | 4.08 | nd | nd |
| n (Samp) | 152 | 10 | 152 | 6 | nd | nd |
| n (Patient) | 114 | 10 | 114 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.02 | 1.40 | 1.02 | 1.61 | nd | nd |
| Average | 1.55 | 1.73 | 1.55 | 2.07 | nd | nd |
| Stdev | 1.72 | 1.11 | 1.72 | 1.77 | nd | nd |
| p(t-test) | | 0.61 | | 0.19 | nd | nd |
| Min | 0.00263 | 0.557 | 0.00263 | 0.371 | nd | nd |
| Max | 11.9 | 4.63 | 11.9 | 7.51 | nd | nd |
| n (Samp) | 73 | 27 | 73 | 25 | nd | nd |
| n (Patient) | 59 | 27 | 59 | 25 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.44 | 0.62 | 0.58 | 0.50 | 0.60 | nd | nd | nd |
| SE | 0.061 | 0.097 | 0.065 | 0.067 | 0.12 | 0.068 | nd | nd | nd |
| p | 0.25 | 0.53 | 0.068 | 0.26 | 1.00 | 0.12 | nd | nd | nd |
| nCohort 1 | 85 | 152 | 73 | 85 | 152 | 73 | nd | nd | nd |
| nCohort 2 | 32 | 10 | 27 | 25 | 6 | 25 | nd | nd | nd |
| Cutoff 1 | 0.946 | 0.622 | 0.996 | 0.845 | 0.569 | 0.845 | nd | nd | nd |
| Sens 1 | 72% | 70% | 70% | 72% | 83% | 72% | nd | nd | nd |
| Spec 1 | 46% | 25% | 48% | 40% | 22% | 41% | nd | nd | nd |
| Cutoff 2 | 0.734 | 0.380 | 0.935 | 0.569 | 0.569 | 0.723 | nd | nd | nd |
| Sens 2 | 81% | 80% | 81% | 80% | 83% | 80% | nd | nd | nd |
| Spec 2 | 35% | 14% | 47% | 22% | 22% | 34% | nd | nd | nd |
| Cutoff 3 | 0.555 | 0.0646 | 0.739 | 0.398 | 0.252 | 0.426 | nd | nd | nd |
| Sens 3 | 91% | 90% | 93% | 92% | 100% | 92% | nd | nd | nd |
| Spec 3 | 21% | 3% | 36% | 15% | 8% | 16% | nd | nd | nd |
| Cutoff 4 | 1.66 | 1.69 | 1.71 | 1.66 | 1.69 | 1.71 | nd | nd | nd |
| Sens 4 | 28% | 40% | 30% | 40% | 33% | 40% | nd | nd | nd |
| Spec 4 | 71% | 70% | 71% | 71% | 70% | 71% | nd | nd | nd |
| Cutoff 5 | 2.10 | 2.45 | 2.19 | 2.10 | 2.45 | 2.19 | nd | nd | nd |
| Sens 5 | 25% | 30% | 26% | 36% | 33% | 36% | nd | nd | nd |
| Spec 5 | 80% | 80% | 81% | 80% | 80% | 81% | nd | nd | nd |
| Cutoff 6 | 2.74 | 3.39 | 3.17 | 2.74 | 3.39 | 3.17 | nd | nd | nd |
| Sens 6 | 16% | 10% | 11% | 16% | 17% | 20% | nd | nd | nd |
| Spec 6 | 91% | 90% | 90% | 91% | 90% | 90% | nd | nd | nd |
| OR Quart 2 | 2.2 | 0.32 | 16 | 0.76 | 0.47 | 0.95 | nd | nd | nd |
| p Value | 0.22 | 0.34 | 0.012 | 0.69 | 0.55 | 0.94 | nd | nd | nd |
| 95% CI of OR Quart2 | 0.62 | 0.032 | 1.9 | 0.20 | 0.041 | 0.24 | nd | nd | nd |
| | 7.5 | 3.3 | 140 | 2.9 | 5.5 | 3.8 | nd | nd | nd |
| OR Quart 3 | 2.5 | 1.0 | 14 | 0.80 | 0.49 | 1.3 | nd | nd | nd |
| p Value | 0.14 | 1.0 | 0.018 | 0.74 | 0.56 | 0.73 | nd | nd | nd |
| 95% CI of OR Quart3 | 0.74 | 0.19 | 1.6 | 0.21 | 0.042 | 0.33 | nd | nd | nd |
| | 8.6 | 5.3 | 120 | 3.0 | 5.6 | 4.9 | nd | nd | nd |
| OR Quart 4 | 1.7 | 1.0 | 9.3 | 1.7 | 0.97 | 2.1 | nd | nd | nd |
| p Value | 0.39 | 0.97 | 0.045 | 0.41 | 0.98 | 0.24 | nd | nd | nd |
| 95% CI of OR Quart4 | 0.50 | 0.19 | 1.1 | 0.50 | 0.13 | 0.59 | nd | nd | nd |
| | 6.1 | 5.4 | 83 | 5.5 | 7.3 | 7.7 | nd | nd | nd |

## Tumor necrosis factor receptor superfamily member 10B

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.124 | 0.573 | 0.124 | 0.395 | nd | nd |
| Average | 0.891 | 1.21 | 0.891 | 1.09 | nd | nd |
| Stdev | 1.49 | 1.55 | 1.49 | 1.82 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.43 | | 0.64 | nd | nd |
| Min | 0.00360 | 0.0182 | 0.00360 | 0.00464 | nd | nd |
| Max | 6.71 | 4.65 | 6.71 | 6.48 | nd | nd |
| n (Samp) | 52 | 20 | 52 | 20 | nd | nd |
| n (Patient) | 41 | 20 | 41 | 20 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.106 | 0.709 | 0.106 | 0.464 | nd | nd |
| Average | 0.595 | 1.40 | 0.595 | 1.25 | nd | nd |
| Stdev | 1.03 | 1.68 | 1.03 | 1.96 | nd | nd |
| p(t-test) | | 0.031 | | 0.087 | nd | nd |
| Min | 0.00360 | 0.0573 | 0.00360 | 0.0172 | nd | nd |
| Max | 4.38 | 4.65 | 4.38 | 6.48 | nd | nd |
| n (Samp) | 42 | 16 | 42 | 21 | nd | nd |
| n (Patient) | 33 | 16 | 33 | 21 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | nd | 0.71 | 0.62 | nd | 0.68 | nd | nd | nd |
| SE | 0.076 | nd | 0.081 | 0.076 | nd | 0.074 | nd | nd | nd |
| p | 0.064 | nd | 0.0099 | 0.12 | nd | 0.014 | nd | nd | nd |
| nCohort 1 | 52 | nd | 42 | 52 | nd | 42 | nd | nd | nd |
| nCohort 2 | 20 | nd | 16 | 20 | nd | 21 | nd | nd | nd |
| Cutoff 1 | 0.182 | nd | 0.107 | 0.154 | nd | 0.154 | nd | nd | nd |
| Sens 1 | 70% | nd | 75% | 70% | nd | 71% | nd | nd | nd |
| Spec 1 | 54% | nd | 52% | 52% | nd | 55% | nd | nd | nd |
| Cutoff 2 | 0.0761 | nd | 0.0761 | 0.141 | nd | 0.141 | nd | nd | nd |
| Sens 2 | 80% | nd | 81% | 80% | nd | 81% | nd | nd | nd |
| Spec 2 | 40% | nd | 40% | 52% | nd | 55% | nd | nd | nd |
| Cutoff 3 | 0.0591 | nd | 0.0591 | 0.0796 | nd | 0.101 | nd | nd | nd |
| Sens 3 | 90% | nd | 94% | 90% | nd | 90% | nd | nd | nd |
| Spec 3 | 38% | nd | 38% | 44% | nd | 48% | nd | nd | nd |
| Cutoff 4 | 0.786 | nd | 0.398 | 0.786 | nd | 0.398 | nd | nd | nd |
| Sens 4 | 45% | nd | 56% | 30% | nd | 52% | nd | nd | nd |
| Spec 4 | 71% | nd | 71% | 71% | nd | 71% | nd | nd | nd |
| Cutoff 5 | 1.56 | nd | 0.819 | 1.56 | nd | 0.819 | nd | nd | nd |
| Sens 5 | 25% | nd | 50% | 15% | nd | 33% | nd | nd | nd |
| Spec 5 | 81% | nd | 81% | 81% | nd | 81% | nd | nd | nd |
| Cutoff 6 | 3.11 | nd | 2.19 | 3.11 | nd | 2.19 | nd | nd | nd |
| Sens 6 | 15% | nd | 25% | 10% | nd | 14% | nd | nd | nd |
| Spec 6 | 90% | nd | 90% | 90% | nd | 90% | nd | nd | nd |
| OR Quart 2 | 8.5 | nd | >7.0 | 11 | nd | 11 | nd | nd | nd |
| p Value | 0.061 | nd | <0.097 | 0.036 | nd | 0.038 | nd | nd | nd |
| 95% CI of OR Quart2 | 0.90 80 | nd | >0.71 na | 1.2 100 | nd | 1.1 100 | nd | nd | nd |
| OR Quart 3 | 11 | nd | >3.8 | 11 | nd | 8.4 | nd | nd | nd |
| p Value | 0.036 | nd | <0.27 | 0.036 | nd | 0.066 | nd | nd | nd |
| 95% CI of OR Quart3 | 1.2 100 | nd | >0.35 na | 1.2 100 | nd | 0.87 81 | nd | nd | nd |
| OR Quart 4 | 8.5 | nd | >16 | 6.5 | nd | 11 | nd | nd | nd |
| p Value | 0.061 | nd | <0.017 | 0.10 | nd | 0.038 | nd | nd | nd |
| 95% CI of OR Quart4 | 0.90 80 | nd | >1.7 na | 0.68 63 | nd | 1.1 100 | nd | nd | nd |

Table 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

**Apolipoprotein A-II**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 73.2 | 102 | 73.2 | 116 | 73.2 | 81.7 |
| Average | 185 | 272 | 185 | 697 | 185 | 87.4 |
| Stdev | 645 | 1020 | 645 | 3530 | 645 | 69.4 |
| p(t-test) | | 0.44 | | 0.0018 | | 0.44 |
| Min | 2.08 | 8.69 | 2.08 | 10.8 | 2.08 | 1.00E-9 |
| Max | 6400 | 6400 | 6400 | 24300 | 6400 | 301 |
| n (Samp) | 685 | 38 | 685 | 47 | 685 | 26 |
| n (Patient) | 283 | 38 | 283 | 47 | 283 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 76.6 | 106 | 76.6 | 127 | 76.6 | 98.8 |
| Average | 198 | 891 | 198 | 176 | 198 | 108 |
| Stdev | 990 | 2230 | 990 | 158 | 990 | 84.1 |
| p(t-test) | | 0.053 | | 0.93 | | 0.74 |
| Min | 1.00E-9 | 34.5 | 1.00E-9 | 24.0 | 1.00E-9 | 6.18 |
| Max | 24300 | 6400 | 24300 | 613 | 24300 | 288 |
| n (Samp) | 891 | 8 | 891 | 13 | 891 | 13 |
| n (Patient) | 334 | 8 | 334 | 13 | 334 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 76.6 | 107 | 76.6 | 131 | 76.6 | 98.7 |
| Average | 195 | 297 | 195 | 789 | 195 | 105 |
| Stdev | 649 | 1060 | 649 | 3780 | 649 | 74.2 |
| p(t-test) | | 0.39 | | 0.0017 | | 0.52 |
| Min | 2.08 | 8.69 | 2.08 | 10.8 | 2.08 | 1.00E-9 |
| Max | 6400 | 6400 | 6400 | 24300 | 6400 | 301 |
| n (Samp) | 553 | 35 | 553 | 41 | 553 | 22 |
| n (Patient) | 202 | 35 | 202 | 41 | 202 | 22 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.60 | 0.60 | 0.66 | 0.66 | 0.67 | 0.47 | 0.51 | 0.53 |
| SE | 0.050 | 0.11 | 0.052 | 0.045 | 0.083 | 0.048 | 0.059 | 0.081 | 0.064 |
| p | 0.069 | 0.36 | 0.053 | 3.3E-4 | 0.055 | 2.3E-4 | 0.60 | 0.87 | 0.61 |
| nCohort 1 | 685 | 891 | 553 | 685 | 891 | 553 | 685 | 891 | 553 |
| nCohort 2 | 38 | 8 | 35 | 47 | 13 | 41 | 26 | 13 | 22 |
| Cutoff 1 | 70.0 | 59.9 | 76.9 | 85.8 | 85.8 | 99.4 | 39.3 | 39.3 | 56.5 |
| Sens 1 | 71% | 75% | 71% | 70% | 77% | 71% | 73% | 77% | 73% |
| Spec 1 | 48% | 37% | 50% | 59% | 56% | 63% | 22% | 21% | 35% |
| Cutoff 2 | 56.5 | 49.4 | 69.8 | 66.5 | 66.5 | 72.6 | 31.3 | 31.3 | 43.2 |
| Sens 2 | 82% | 88% | 80% | 81% | 85% | 80% | 81% | 85% | 82% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 38% | 30% | 45% | 46% | 43% | 47% | 14% | 13% | 22% |
| Cutoff 3 | 33.5 | 34.3 | 33.5 | 43.2 | 43.2 | 44.8 | 11.0 | 16.2 | 27.1 |
| Sens 3 | 92% | 100% | 91% | 91% | 92% | 90% | 92% | 92% | 91% |
| Spec 3 | 16% | 16% | 14% | 26% | 24% | 24% | 2% | 3% | 9% |
| Cutoff 4 | 110 | 122 | 118 | 110 | 122 | 118 | 110 | 122 | 118 |
| Sens 4 | 42% | 50% | 46% | 53% | 62% | 54% | 31% | 38% | 36% |
| Spec 4 | 70% | 70% | 70% | 70% | 70% | 70% | 70% | 70% | 70% |
| Cutoff 5 | 148 | 154 | 154 | 148 | 154 | 154 | 148 | 154 | 154 |
| Sens 5 | 18% | 38% | 20% | 38% | 31% | 41% | 19% | 23% | 14% |
| Spec 5 | 80% | 80% | 80% | 80% | 80% | 80% | 80% | 80% | 80% |
| Cutoff 6 | 226 | 229 | 248 | 226 | 229 | 248 | 226 | 229 | 248 |
| Sens 6 | 11% | 12% | 11% | 21% | 31% | 24% | 4% | 8% | 5% |
| Spec 6 | 90% | 90% | 90% | 90% | 90% | 90% | 90% | 90% | 90% |
| OR Quart 2 | 1.2 | 2.0 | 1.2 | 1.4 | 0.50 | 0.49 | 1.3 | 0 | 0.79 |
| p Value | 0.79 | 0.57 | 0.76 | 0.56 | 0.57 | 0.31 | 0.59 | na | 0.73 |
| 95% CI of OR Quart2 | 0.38 3.5 | 0.18 22 | 0.36 4.1 | 0.44 4.5 | 0.045 5.5 | 0.12 2.0 | 0.46 4.0 | na na | 0.21 3.0 |
| OR Quart 3 | 2.2 | 2.0 | 2.5 | 3.2 | 2.5 | 2.3 | 0.49 | 0.59 | 1.2 |
| p Value | 0.11 | 0.57 | 0.090 | 0.028 | 0.27 | 0.10 | 0.32 | 0.48 | 0.77 |
| 95% CI of OR Quart3 | 0.83 6.0 | 0.18 22 | 0.87 7.4 | 1.1 8.9 | 0.49 13 | 0.84 6.2 | 0.12 2.0 | 0.14 2.5 | 0.36 4.0 |
| OR Quart 4 | 2.1 | 3.0 | 2.5 | 4.4 | 2.5 | 3.5 | 1.5 | 1.0 | 1.4 |
| p Value | 0.16 | 0.34 | 0.090 | 0.0040 | 0.27 | 0.010 | 0.43 | 1.0 | 0.57 |
| 95% CI of OR Quart4 | 0.76 5.6 | 0.31 29 | 0.87 7.4 | 1.6 12 | 0.49 13 | 1.3 8.9 | 0.53 4.4 | 0.29 3.5 | 0.44 4.6 |

## Caspase-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.02 | 0.826 | nd | nd | nd | nd |
| Average | 1.20 | 0.896 | nd | nd | nd | nd |
| Stdev | 1.22 | 0.687 | nd | nd | nd | nd |
| p(t-test) | | 0.49 | nd | nd | nd | nd |
| Min | 0.0223 | 0.0223 | nd | nd | nd | nd |
| Max | 4.68 | 2.33 | nd | nd | nd | nd |
| n (Samp) | 55 | 8 | nd | nd | nd | nd |
| n (Patient) | 35 | 8 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.09 | 0.826 | nd | nd | nd | nd |
| Average | 1.20 | 0.896 | nd | nd | nd | nd |
| Stdev | 1.19 | 0.687 | nd | nd | nd | nd |
| p(t-test) | | 0.50 | nd | nd | nd | nd |
| Min | 0.0223 | 0.0223 | nd | nd | nd | nd |
| Max | 4.46 | 2.33 | nd | nd | nd | nd |
| n (Samp) | 41 | 8 | nd | nd | nd | nd |
| n (Patient) | 24 | 8 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | 0.46 | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.11 | nd | 0.11 | nd | nd | nd | nd | nd | nd |
| p | 0.74 | nd | 0.76 | nd | nd | nd | nd | nd | nd |
| nCohort 1 | 55 | nd | 41 | nd | nd | nd | nd | nd | nd |
| nCohort 2 | 8 | nd | 8 | nd | nd | nd | nd | nd | nd |
| Cutoff 1 | 0.496 | nd | 0.496 | nd | nd | nd | nd | nd | nd |
| Sens 1 | 75% | nd | 75% | nd | nd | nd | nd | nd | nd |
| Spec 1 | 40% | nd | 41% | nd | nd | nd | nd | nd | nd |
| Cutoff 2 | 0.298 | nd | 0.298 | nd | nd | nd | nd | nd | nd |
| Sens 2 | 88% | nd | 88% | nd | nd | nd | nd | nd | nd |
| Spec 2 | 36% | nd | 37% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |
| Spec 3 | 0% | nd | 0% | nd | nd | nd | nd | nd | nd |
| Cutoff 4 | 1.54 | nd | 1.54 | nd | nd | nd | nd | nd | nd |
| Sens 4 | 12% | nd | 12% | nd | nd | nd | nd | nd | nd |
| Spec 4 | 71% | nd | 71% | nd | nd | nd | nd | nd | nd |
| Cutoff 5 | 2.07 | nd | 2.07 | nd | nd | nd | nd | nd | nd |
| Sens 5 | 12% | nd | 12% | nd | nd | nd | nd | nd | nd |
| Spec 5 | 82% | nd | 85% | nd | nd | nd | nd | nd | nd |
| Cutoff 6 | 2.59 | nd | 2.59 | nd | nd | nd | nd | nd | nd |
| Sens 6 | 0% | nd | 0% | nd | nd | nd | nd | nd | nd |
| Spec 6 | 91% | nd | 90% | nd | nd | nd | nd | nd | nd |
| OR Quart 2 | 1.0 | nd | 2.4 | nd | nd | nd | nd | nd | nd |
| p Value | 1.0 | nd | 0.50 | nd | nd | nd | nd | nd | nd |
| 95% CI of OR Quart2 | 0.057 18 | nd | 0.19 31 | nd | nd | nd | nd | nd | nd |
| OR Quart 3 | 6.8 | nd | 6.0 | nd | nd | nd | nd | nd | nd |
| p Value | 0.099 | nd | 0.14 | nd | nd | nd | nd | nd | nd |
| 95% CI of OR Quart3 | 0.69 67 | nd | 0.56 64 | nd | nd | nd | nd | nd | nd |
| OR Quart 4 | 1.1 | nd | 1.1 | nd | nd | nd | nd | nd | nd |
| p Value | 0.96 | nd | 0.95 | nd | nd | nd | nd | nd | nd |
| 95% CI of OR Quart4 | 0.061 19 | nd | 0.061 20 | nd | nd | nd | nd | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.469 | 0.547 | nd | nd | nd | nd |
| Average | 0.965 | 0.998 | nd | nd | nd | nd |
| Stdev | 2.44 | 1.52 | nd | nd | nd | nd |
| p(t-test) | | 0.97 | nd | nd | nd | nd |
| Min | 0.0360 | 0.0360 | nd | nd | nd | nd |
| Max | 19.5 | 4.64 | nd | nd | nd | nd |
| n (Samp) | 78 | 8 | nd | nd | nd | nd |
| n (Patient) | 56 | 8 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.469 | 0.547 | nd | nd | nd | nd |
| Average | 1.12 | 0.998 | nd | nd | nd | nd |
| Stdev | 2.73 | 1.52 | nd | nd | nd | nd |
| p(t-test) | | 0.90 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.0360 | 0.0360 | nd | nd | nd | nd |
| Max | 19.5 | 4.64 | nd | nd | nd | nd |
| n (Samp) | 61 | 8 | nd | nd | nd | nd |
| n (Patient) | 43 | 8 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | nd | 0.50 | nd | nd | nd | nd | nd | nd |
| SE | 0.11 | nd | 0.11 | nd | nd | nd | nd | nd | nd |
| p | 0.73 | nd | 0.98 | nd | nd | nd | nd | nd | nd |
| nCohort 1 | 78 | nd | 61 | nd | nd | nd | nd | nd | nd |
| nCohort 2 | 8 | nd | 8 | nd | nd | nd | nd | nd | nd |
| Cutoff 1 | 0.208 | nd | 0.208 | nd | nd | nd | nd | nd | nd |
| Sens 1 | 75% | nd | 75% | nd | nd | nd | nd | nd | nd |
| Spec 1 | 36% | nd | 28% | nd | nd | nd | nd | nd | nd |
| Cutoff 2 | 0 | nd | 0 | nd | nd | nd | nd | nd | nd |
| Sens 2 | 100% | nd | 100% | nd | nd | nd | nd | nd | nd |
| Spec 2 | 0% | nd | 0% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |
| Spec 3 | 0% | nd | 0% | nd | nd | nd | nd | nd | nd |
| Cutoff 4 | 0.781 | nd | 0.781 | nd | nd | nd | nd | nd | nd |
| Sens 4 | 38% | nd | 38% | nd | nd | nd | nd | nd | nd |
| Spec 4 | 72% | nd | 70% | nd | nd | nd | nd | nd | nd |
| Cutoff 5 | 0.955 | nd | 0.955 | nd | nd | nd | nd | nd | nd |
| Sens 5 | 25% | nd | 25% | nd | nd | nd | nd | nd | nd |
| Spec 5 | 81% | nd | 80% | nd | nd | nd | nd | nd | nd |
| Cutoff 6 | 1.66 | nd | 1.49 | nd | nd | nd | nd | nd | nd |
| Sens 6 | 12% | nd | 12% | nd | nd | nd | nd | nd | nd |
| Spec 6 | 91% | nd | 90% | nd | nd | nd | nd | nd | nd |
| OR Quart 2 | 0.95 | nd | 0.47 | nd | nd | nd | nd | nd | nd |
| p Value | 0.96 | nd | 0.55 | nd | nd | nd | nd | nd | nd |
| 95% CI of | 0.12 | nd | 0.038 | nd | nd | nd | nd | nd | nd |
| OR Quart2 | 7.4 | nd | 5.7 | nd | nd | nd | nd | nd | nd |
| OR Quart 3 | 1.0 | nd | 1.0 | nd | nd | nd | nd | nd | nd |
| p Value | 1.0 | nd | 1.0 | nd | nd | nd | nd | nd | nd |
| 95% CI of | 0.13 | nd | 0.12 | nd | nd | nd | nd | nd | nd |
| OR Quart3 | 7.9 | nd | 8.1 | nd | nd | nd | nd | nd | nd |
| OR Quart 4 | 0.95 | nd | 1.5 | nd | nd | nd | nd | nd | nd |
| p Value | 0.96 | nd | 0.68 | nd | nd | nd | nd | nd | nd |
| 95% CI of | 0.12 | nd | 0.22 | nd | nd | nd | nd | nd | nd |
| OR Quart4 | 7.4 | nd | 10 | nd | nd | nd | nd | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 57300 | 62400 | nd | nd |
| Average | nd | nd | 102000 | 78500 | nd | nd |
| Stdev | nd | nd | 128000 | 81700 | nd | nd |
| p(t-test) | nd | nd | | 0.47 | nd | nd |
| Min | nd | nd | 160 | 1620 | nd | nd |
| Max | nd | nd | 744000 | 260000 | nd | nd |
| n (Samp) | nd | nd | 96 | 16 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | nd | nd | 73 | 16 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 58600 | 59500 | nd | nd |
| Average | nd | nd | 104000 | 82000 | nd | nd |
| Stdev | nd | nd | 134000 | 82800 | nd | nd |
| p(t-test) | nd | nd | | 0.54 | nd | nd |
| Min | nd | nd | 160 | 1620 | nd | nd |
| Max | nd | nd | 744000 | 260000 | nd | nd |
| n (Samp) | nd | nd | 81 | 15 | nd | nd |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.44 | nd | 0.47 | nd | nd | nd |
| SE | nd | nd | nd | 0.080 | nd | 0.082 | nd | nd | nd |
| p | nd | nd | nd | 0.49 | nd | 0.72 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 12500 | nd | 12900 | nd | nd | nd |
| Sens 1 | nd | nd | nd | 75% | nd | 73% | nd | nd | nd |
| Spec 1 | nd | nd | nd | 19% | nd | 22% | nd | nd | nd |
| Cutoff 2 | nd | nd | nd | 9460 | nd | 11700 | nd | nd | nd |
| Sens 2 | nd | nd | nd | 81% | nd | 80% | nd | nd | nd |
| Spec 2 | nd | nd | nd | 11% | nd | 22% | nd | nd | nd |
| Cutoff 3 | nd | nd | nd | 1660 | nd | 1660 | nd | nd | nd |
| Sens 3 | nd | nd | nd | 94% | nd | 93% | nd | nd | nd |
| Spec 3 | nd | nd | nd | 2% | nd | 2% | nd | nd | nd |
| Cutoff 4 | nd | nd | nd | 128000 | nd | 125000 | nd | nd | nd |
| Sens 4 | nd | nd | nd | 25% | nd | 27% | nd | nd | nd |
| Spec 4 | nd | nd | nd | 71% | nd | 70% | nd | nd | nd |
| Cutoff 5 | nd | nd | nd | 141000 | nd | 144000 | nd | nd | nd |
| Sens 5 | nd | nd | nd | 25% | nd | 27% | nd | nd | nd |
| Spec 5 | nd | nd | nd | 80% | nd | 80% | nd | nd | nd |
| Cutoff 6 | nd | nd | nd | 219000 | nd | 219000 | nd | nd | nd |
| Sens 6 | nd | nd | nd | 6% | nd | 7% | nd | nd | nd |
| Spec 6 | nd | nd | nd | 91% | nd | 90% | nd | nd | nd |
| OR Quart 2 | nd | nd | nd | 1.3 | nd | 1.0 | nd | nd | nd |
| p Value | nd | nd | nd | 0.72 | nd | 1.0 | nd | nd | nd |
| 95% CI of OR Quart2 | nd | nd | nd | 0.31 | nd | 0.22 | nd | nd | nd |
| | nd | nd | nd | 5.5 | nd | 4.6 | nd | nd | nd |
| OR Quart 3 | nd | nd | nd | 0 | nd | 0.45 | nd | nd | nd |
| p Value | nd | nd | nd | na | nd | 0.39 | nd | nd | nd |
| 95% CI of OR Quart3 | nd | nd | nd | na | nd | 0.075 | nd | nd | nd |
| | nd | nd | nd | na | nd | 2.8 | nd | nd | nd |
| OR Quart 4 | nd | nd | nd | 2.0 | nd | 1.3 | nd | nd | nd |
| p Value | nd | nd | nd | 0.32 | nd | 0.71 | nd | nd | nd |
| 95% CI of OR Quart4 | nd | nd | nd | 0.51 | nd | 0.31 | nd | nd | nd |
| | nd | nd | nd | 7.8 | nd | 5.6 | nd | nd | nd |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.09 | 3.35 | 1.09 | 4.76 | 1.09 | 2.45 |
| Average | 62.1 | 42.8 | 62.1 | 57.1 | 62.1 | 13.0 |
| Stdev | 497 | 133 | 497 | 173 | 497 | 18.2 |
| p(t-test) | | 0.85 | | 0.95 | | 0.68 |
| Min | 1.14E-14 | 1.70E-14 | 1.14E-14 | 0.116 | 1.14E-14 | 0.116 |
| Max | 6950 | 630 | 6950 | 907 | 6950 | 52.9 |
| n (Samp) | 302 | 24 | 302 | 35 | 302 | 17 |
| n (Patient) | 164 | 24 | 164 | 35 | 164 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.23 | 7.84 | 1.23 | 9.80 |
| Average | nd | nd | 58.3 | 15.2 | 58.3 | 15.4 |
| Stdev | nd | nd | 447 | 17.6 | 447 | 16.5 |
| p(t-test) | nd | nd | | 0.80 | | 0.80 |
| Min | nd | nd | 1.14E-14 | 0.116 | 1.14E-14 | 0.116 |
| Max | nd | nd | 6950 | 42.1 | 6950 | 39.2 |
| n (Samp) | nd | nd | 380 | 7 | 380 | 7 |
| n (Patient) | nd | nd | 196 | 7 | 196 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.850 | 3.35 | 0.850 | 2.87 | 0.850 | 2.66 |
| Average | 70.1 | 42.8 | 70.1 | 62.4 | 70.1 | 12.3 |
| Stdev | 538 | 133 | 538 | 183 | 538 | 17.6 |
| p(t-test) | | 0.80 | | 0.94 | | 0.67 |
| Min | 1.14E-14 | 1.70E-14 | 1.14E-14 | 0.116 | 1.14E-14 | 0.116 |
| Max | 6950 | 630 | 6950 | 907 | 6950 | 52.9 |
| n (Samp) | 257 | 24 | 257 | 31 | 257 | 16 |
| n (Patient) | 134 | 24 | 134 | 31 | 134 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | nd | 0.54 | 0.59 | 0.64 | 0.58 | 0.55 | 0.60 | 0.59 |
| SE | 0.063 | nd | 0.063 | 0.053 | 0.11 | 0.056 | 0.074 | 0.11 | 0.077 |
| p | 0.46 | nd | 0.50 | 0.080 | 0.21 | 0.16 | 0.49 | 0.36 | 0.24 |
| nCohort 1 | 302 | nd | 257 | 302 | 380 | 257 | 302 | 380 | 257 |
| nCohort 2 | 24 | nd | 24 | 35 | 7 | 31 | 17 | 7 | 16 |
| Cutoff 1 | 1.70E-14 | nd | 1.70E-14 | 0.713 | 6.55 | 1.70E-14 | 1.70E-14 | 2.02 | 0.116 |
| Sens 1 | 96% | nd | 96% | 71% | 71% | 100% | 100% | 71% | 75% |
| Spec 1 | 2% | nd | 2% | 47% | 65% | 2% | 2% | 52% | 45% |
| Cutoff 2 | 1.70E-14 | nd | 1.70E-14 | 1.70E-14 | 0.850 | 1.70E-14 | 1.70E-14 | 1.70E-14 | 1.70E-14 |
| Sens 2 | 96% | nd | 96% | 100% | 86% | 100% | 100% | 100% | 100% |
| Spec 2 | 2% | nd | 2% | 2% | 47% | 2% | 2% | 2% | 2% |
| Cutoff 3 | 1.70E-14 | nd | 1.70E-14 | 1.70E-14 | 1.70E-14 | 1.70E-14 | 1.70E-14 | 1.70E-14 | 1.70E-14 |
| Sens 3 | 96% | nd | 96% | 100% | 100% | 100% | 100% | 100% | 100% |
| Spec 3 | 2% | nd | 2% | 2% | 2% | 2% | 2% | 2% | 2% |
| Cutoff 4 | 7.84 | nd | 7.29 | 7.84 | 9.35 | 7.29 | 7.84 | 9.35 | 7.29 |
| Sens 4 | 38% | nd | 38% | 43% | 29% | 42% | 41% | 57% | 44% |
| Spec 4 | 70% | nd | 70% | 70% | 71% | 70% | 70% | 71% | 70% |
| Cutoff 5 | 16.1 | nd | 13.9 | 16.1 | 18.3 | 13.9 | 16.1 | 18.3 | 13.9 |
| Sens 5 | 21% | nd | 21% | 29% | 29% | 32% | 24% | 43% | 31% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | nd | 80% | 80% | 80% | 80% | 80% | 80% | 80% |
| Cutoff 6 | 43.4 | nd | 44.8 | 43.4 | 48.9 | 44.8 | 43.4 | 48.9 | 44.8 |
| Sens 6 | 12% | nd | 12% | 11% | 0% | 13% | 12% | 0% | 12% |
| Spec 6 | 90% | nd | 90% | 90% | 90% | 90% | 90% | 90% | 90% |
| OR Quart 2 | 0.23 | nd | 0 | 1.4 | 0.99 | 2.9 | >7.6 | 0 | 0.24 |
| p Value | 0.067 | nd | na | 0.55 | 0.99 | 0.13 | <0.061 | na | 0.21 |
| 95% CI of OR Quart2 | 0.047 | nd | na | 0.44 | 0.061 | 0.73 | >0.91 | na | 0.026 |
| | 1.1 | nd | na | 4.7 | 16 | 11 | na | na | 2.2 |
| OR Quart 3 | 0.86 | nd | 0.87 | 2.6 | 3.0 | 3.3 | >3.1 | 0.99 | 1.3 |
| p Value | 0.79 | nd | 0.80 | 0.082 | 0.34 | 0.084 | <0.33 | 0.99 | 0.73 |
| 95% CI of OR Quart3 | 0.30 | nd | 0.32 | 0.88 | 0.31 | 0.85 | >0.31 | 0.14 | 0.33 |
| | 2.5 | nd | 2.4 | 7.8 | 30 | 13 | na | 7.2 | 4.9 |
| OR Quart 4 | 0.85 | nd | 0.74 | 2.3 | 2.0 | 4.1 | >7.6 | 1.5 | 1.5 |
| p Value | 0.77 | nd | 0.58 | 0.13 | 0.57 | 0.035 | <0.061 | 0.66 | 0.53 |
| 95% CI of OR Quart4 | 0.29 | nd | 0.26 | 0.78 | 0.18 | 1.1 | >0.91 | 0.25 | 0.41 |
| | 2.5 | nd | 2.1 | 7.1 | 22 | 16 | na | 9.2 | 5.7 |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.852 | 1.70 | 0.852 | 2.56 | 0.852 | 1.46 |
| Average | 12.6 | 4.03 | 12.6 | 5.68 | 12.6 | 4.91 |
| Stdev | 199 | 4.99 | 199 | 9.51 | 199 | 11.3 |
| p(t-test) | | 0.83 | | 0.84 | | 0.87 |
| Min | 0.00336 | 0.00336 | 0.00336 | 0.0950 | 0.00336 | 0.148 |
| Max | 4070 | 19.5 | 4070 | 50.6 | 4070 | 47.4 |
| n (Samp) | 419 | 26 | 419 | 34 | 419 | 17 |
| n (Patient) | 164 | 26 | 164 | 34 | 164 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.978 | 3.62 | 0.978 | 5.87 |
| Average | nd | nd | 11.1 | 5.25 | 11.1 | 6.39 |
| Stdev | nd | nd | 180 | 7.06 | 180 | 4.92 |
| p(t-test) | nd | nd | | 0.93 | | 0.94 |
| Min | nd | nd | 0.00336 | 0.102 | 0.00336 | 0.173 |
| Max | nd | nd | 4070 | 22.0 | 4070 | 14.6 |
| n (Samp) | nd | nd | 511 | 8 | 511 | 7 |
| n (Patient) | nd | nd | 198 | 8 | 198 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.852 | 1.48 | 0.852 | 2.42 | 0.852 | 1.12 |
| Average | 14.6 | 3.87 | 14.6 | 5.34 | 14.6 | 4.41 |
| Stdev | 216 | 5.02 | 216 | 9.63 | 216 | 11.3 |
| p(t-test) | | 0.80 | | 0.81 | | 0.85 |
| Min | 0.00336 | 0.0573 | 0.00336 | 0.00336 | 0.00336 | 0.148 |
| Max | 4070 | 19.5 | 4070 | 50.6 | 4070 | 47.4 |
| n (Samp) | 355 | 26 | 355 | 30 | 355 | 17 |
| n (Patient) | 134 | 26 | 134 | 30 | 134 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | nd | 0.61 | 0.67 | 0.65 | 0.66 | 0.57 | 0.76 | 0.54 |
| SE | 0.060 | nd | 0.061 | 0.052 | 0.11 | 0.056 | 0.074 | 0.11 | 0.073 |
| p | 0.029 | nd | 0.059 | 0.0014 | 0.16 | 0.0056 | 0.34 | 0.016 | 0.58 |
| nCohort 1 | 419 | nd | 355 | 419 | 511 | 355 | 419 | 511 | 355 |
| nCohort 2 | 26 | nd | 26 | 34 | 8 | 30 | 17 | 7 | 17 |
| Cutoff 1 | 0.969 | nd | 0.816 | 0.942 | 2.26 | 0.942 | 0.607 | 3.47 | 0.491 |
| Sens 1 | 73% | nd | 73% | 71% | 75% | 70% | 71% | 71% | 71% |
| Spec 1 | 53% | nd | 50% | 52% | 73% | 51% | 43% | 80% | 39% |
| Cutoff 2 | 0.590 | nd | 0.590 | 0.546 | 0.214 | 0.684 | 0.279 | 2.55 | 0.279 |
| Sens 2 | 81% | nd | 81% | 82% | 88% | 80% | 82% | 86% | 82% |
| Spec 2 | 42% | nd | 43% | 41% | 17% | 47% | 25% | 74% | 25% |
| Cutoff 3 | 0.150 | nd | 0.150 | 0.214 | 0.0990 | 0.444 | 0.150 | 0.172 | 0.150 |
| Sens 3 | 92% | nd | 92% | 91% | 100% | 90% | 94% | 100% | 94% |
| Spec 3 | 12% | nd | 12% | 18% | 7% | 37% | 12% | 14% | 12% |
| Cutoff 4 | 1.76 | nd | 1.84 | 1.76 | 1.97 | 1.84 | 1.76 | 1.97 | 1.84 |
| Sens 4 | 50% | nd | 46% | 59% | 75% | 57% | 41% | 86% | 29% |
| Spec 4 | 70% | nd | 70% | 70% | 70% | 70% | 70% | 70% | 70% |
| Cutoff 5 | 3.16 | nd | 3.25 | 3.16 | 3.51 | 3.25 | 3.16 | 3.51 | 3.25 |
| Sens 5 | 38% | nd | 35% | 44% | 50% | 40% | 24% | 57% | 18% |
| Spec 5 | 80% | nd | 80% | 80% | 80% | 80% | 80% | 80% | 80% |
| Cutoff 6 | 8.38 | nd | 8.86 | 8.38 | 8.51 | 8.86 | 8.38 | 8.51 | 8.86 |
| Sens 6 | 15% | nd | 12% | 15% | 12% | 13% | 12% | 29% | 6% |
| Spec 6 | 90% | nd | 90% | 90% | 90% | 90% | 90% | 90% | 90% |
| OR Quart 2 | 1.3 | nd | 1.7 | 1.5 | 0 | 2.4 | 0.74 | 0 | 1.7 |
| p Value | 0.70 | nd | 0.47 | 0.52 | na | 0.21 | 0.70 | na | 0.47 |
| 95% CI of | 0.29 | nd | 0.40 | 0.42 | na | 0.61 | 0.16 | na | 0.40 |
| OR Quart2 | 6.2 | nd | 7.3 | 5.6 | na | 9.7 | 3.4 | na | 7.3 |
| OR Quart 3 | 3.2 | nd | 3.2 | 2.1 | 0.99 | 2.4 | 1.3 | 1.0 | 1.7 |
| p Value | 0.090 | nd | 0.088 | 0.24 | 0.99 | 0.21 | 0.73 | 1.0 | 0.47 |
| 95% CI of | 0.84 | nd | 0.84 | 0.61 | 0.14 | 0.61 | 0.33 | 0.062 | 0.40 |
| OR Quart3 | 12 | nd | 12 | 7.1 | 7.2 | 9.7 | 4.8 | 16 | 7.3 |
| OR Quart 4 | 3.5 | nd | 3.2 | 4.4 | 2.0 | 4.8 | 1.3 | 5.1 | 1.3 |
| p Value | 0.061 | nd | 0.091 | 0.0096 | 0.42 | 0.017 | 0.73 | 0.14 | 0.70 |
| 95% CI of | 0.94 | nd | 0.83 | 1.4 | 0.36 | 1.3 | 0.33 | 0.59 | 0.29 |
| OR Quart4 | 13 | nd | 12 | 14 | 11 | 17 | 4.8 | 44 | 6.2 |

## Myoglobin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.860 | 0.477 | 0.860 | 0.126 | 0.860 | 0.176 |
| Average | 35.5 | 18.1 | 35.5 | 44.2 | 35.5 | 6.85 |
| Stdev | 103 | 38.1 | 103 | 130 | 103 | 15.5 |
| p(t-test) | | 0.39 | | 0.64 | | 0.25 |
| Min | 0.000105 | 0.0151 | 0.000105 | 0.000105 | 0.000105 | 0.0254 |
| Max | 618 | 163 | 618 | 469 | 618 | 49.1 |
| n (Samp) | 419 | 26 | 419 | 34 | 419 | 17 |
| n (Patient) | 164 | 26 | 164 | 34 | 164 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.599 | 4.97 | 0.599 | 0.349 |
| Average | nd | nd | 37.0 | 95.0 | 37.0 | 19.7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | nd | nd | 109 | 171 | 109 | 28.5 |
| p(t-test) | nd | nd | | 0.14 | | 0.68 |
| Min | nd | nd | 0.000105 | 0.0561 | 0.000105 | 0.0683 |
| Max | nd | nd | 618 | 420 | 618 | 71.6 |
| n (Samp) | nd | nd | 511 | 8 | 511 | 7 |
| n (Patient) | nd | nd | 198 | 8 | 198 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.885 | 0.354 | 0.885 | 0.108 | 0.885 | 0.176 |
| Average | 40.5 | 17.6 | 40.5 | 36.1 | 40.5 | 5.29 |
| Stdev | 112 | 38.2 | 112 | 119 | 112 | 12.1 |
| p(t-test) | | 0.30 | | 0.84 | | 0.20 |
| Min | 0.000105 | 0.0151 | 0.000105 | 0.000105 | 0.000105 | 0.0254 |
| Max | 618 | 163 | 618 | 469 | 618 | 49.1 |
| n (Samp) | 355 | 26 | 355 | 30 | 355 | 17 |
| n (Patient) | 134 | 26 | 134 | 30 | 134 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | nd | 0.45 | 0.39 | 0.57 | 0.38 | 0.40 | 0.55 | 0.39 |
| SE | 0.059 | nd | 0.060 | 0.053 | 0.11 | 0.057 | 0.074 | 0.11 | 0.074 |
| p | 0.72 | nd | 0.36 | 0.045 | 0.53 | 0.031 | 0.16 | 0.67 | 0.15 |
| nCohort 1 | 419 | nd | 355 | 419 | 511 | 355 | 419 | 511 | 355 |
| nCohort 2 | 26 | nd | 26 | 34 | 8 | 30 | 17 | 7 | 17 |
| Cutoff 1 | 0.0772 | nd | 0.0667 | 0.0584 | 0.118 | 0.0562 | 0.0849 | 0.132 | 0.0834 |
| Sens 1 | 73% | nd | 73% | 71% | 75% | 70% | 71% | 71% | 71% |
| Spec 1 | 22% | nd | 20% | 19% | 32% | 18% | 23% | 32% | 22% |
| Cutoff 2 | 0.0511 | nd | 0.0422 | 0.0375 | 0.0589 | 0.0366 | 0.0667 | 0.0849 | 0.0630 |
| Sens 2 | 81% | nd | 81% | 82% | 88% | 80% | 82% | 86% | 82% |
| Spec 2 | 18% | nd | 13% | 12% | 22% | 11% | 21% | 26% | 19% |
| Cutoff 3 | 0.0375 | nd | 0.0366 | 0.0331 | 0.0556 | 0.0331 | 0.0337 | 0.0667 | 0.0337 |
| Sens 3 | 92% | nd | 92% | 91% | 100% | 90% | 94% | 100% | 94% |
| Spec 3 | 12% | nd | 11% | 10% | 21% | 9% | 11% | 23% | 10% |
| Cutoff 4 | 7.09 | nd | 7.24 | 7.09 | 6.05 | 7.24 | 7.09 | 6.05 | 7.24 |
| Sens 4 | 31% | nd | 27% | 21% | 50% | 20% | 18% | 43% | 24% |
| Spec 4 | 70% | nd | 70% | 70% | 70% | 70% | 70% | 70% | 70% |
| Cutoff 5 | 19.0 | nd | 23.8 | 19.0 | 17.9 | 23.8 | 19.0 | 17.9 | 23.8 |
| Sens 5 | 19% | nd | 19% | 15% | 25% | 13% | 12% | 43% | 6% |
| Spec 5 | 80% | nd | 80% | 80% | 80% | 80% | 80% | 80% | 80% |
| Cutoff 6 | 66.4 | nd | 79.4 | 66.4 | 70.5 | 79.4 | 66.4 | 70.5 | 79.4 |
| Sens 6 | 12% | nd | 8% | 12% | 25% | 7% | 0% | 14% | 0% |
| Spec 6 | 90% | nd | 90% | 90% | 90% | 90% | 90% | 90% | 90% |
| OR Quart 2 | 0.83 | nd | 0.66 | 0.83 | 0.99 | 0.66 | 0.66 | 3.0 | 2.0 |
| p Value | 0.77 | nd | 0.53 | 0.77 | 0.99 | 0.53 | 0.65 | 0.34 | 0.42 |
| 95% CI of OR Quart2 | 0.25 | nd | 0.18 | 0.25 | 0.14 | 0.18 | 0.11 | 0.31 | 0.37 |
| | 2.8 | nd | 2.4 | 2.8 | 7.2 | 2.4 | 4.0 | 29 | 11 |
| OR Quart 3 | 1.0 | nd | 1.0 | 1.2 | 0.49 | 0.83 | 2.1 | 0 | 2.0 |
| p Value | 0.99 | nd | 0.99 | 0.76 | 0.56 | 0.77 | 0.32 | na | 0.42 |
| 95% CI of OR Quart3 | 0.32 | nd | 0.31 | 0.39 | 0.044 | 0.25 | 0.50 | na | 0.37 |
| | 3.2 | nd | 3.3 | 3.7 | 5.5 | 2.8 | 8.4 | na | 11 |
| OR Quart 4 | 1.6 | nd | 1.8 | 3.0 | 1.5 | 2.8 | 2.1 | 3.0 | 3.7 |
| p Value | 0.42 | nd | 0.29 | 0.029 | 0.66 | 0.042 | 0.32 | 0.34 | 0.11 |
| 95% CI of | 0.54 | nd | 0.61 | 1.1 | 0.25 | 1.0 | 0.50 | 0.31 | 0.75 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart4 | 4.5 | nd | 5.1 | 7.9 | 9.1 | 7.6 | 8.4 | 29 | 18 |

## Mucin-16

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.684 | 0.822 | nd | nd |
| Average | nd | nd | 1.24 | 28.1 | nd | nd |
| Stdev | nd | nd | 1.59 | 108 | nd | nd |
| p(t-test) | nd | nd | | 0.014 | nd | nd |
| Min | nd | nd | 1.00E-9 | 0.304 | nd | nd |
| Max | nd | nd | 10.1 | 433 | nd | nd |
| n (Samp) | nd | nd | 96 | 16 | nd | nd |
| n (Patient) | nd | nd | 73 | 16 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.623 | 0.822 | nd | nd |
| Average | nd | nd | 1.23 | 29.9 | nd | nd |
| Stdev | nd | nd | 1.64 | 111 | nd | nd |
| p(t-test) | nd | nd | | 0.020 | nd | nd |
| Min | nd | nd | 1.00E-9 | 0.304 | nd | nd |
| Max | nd | nd | 10.1 | 433 | nd | nd |
| n (Samp) | nd | nd | 81 | 15 | nd | nd |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.55 | nd | 0.56 | nd | nd | nd |
| SE | nd | nd | nd | 0.080 | nd | 0.083 | nd | nd | nd |
| p | nd | nd | nd | 0.49 | nd | 0.45 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 0.412 | nd | 0.412 | nd | nd | nd |
| Sens 1 | nd | nd | nd | 75% | nd | 73% | nd | nd | nd |
| Spec 1 | nd | nd | nd | 32% | nd | 36% | nd | nd | nd |
| Cutoff 2 | nd | nd | nd | 0.344 | nd | 0.344 | nd | nd | nd |
| Sens 2 | nd | nd | nd | 88% | nd | 87% | nd | nd | nd |
| Spec 2 | nd | nd | nd | 21% | nd | 26% | nd | nd | nd |
| Cutoff 3 | nd | nd | nd | 0.278 | nd | 0.278 | nd | nd | nd |
| Sens 3 | nd | nd | nd | 100% | nd | 100% | nd | nd | nd |
| Spec 3 | nd | nd | nd | 11% | nd | 15% | nd | nd | nd |
| Cutoff 4 | nd | nd | nd | 1.17 | nd | 1.09 | nd | nd | nd |
| Sens 4 | nd | nd | nd | 25% | nd | 33% | nd | nd | nd |
| Spec 4 | nd | nd | nd | 71% | nd | 70% | nd | nd | nd |
| Cutoff 5 | nd | nd | nd | 1.41 | nd | 1.44 | nd | nd | nd |
| Sens 5 | nd | nd | nd | 19% | nd | 20% | nd | nd | nd |
| Spec 5 | nd | nd | nd | 80% | nd | 80% | nd | nd | nd |
| Cutoff 6 | nd | nd | nd | 3.40 | nd | 3.26 | nd | nd | nd |
| Sens 6 | nd | nd | nd | 12% | nd | 13% | nd | nd | nd |
| Spec 6 | nd | nd | nd | 91% | nd | 90% | nd | nd | nd |
| OR Quart 2 | nd | nd | nd | 0.72 | nd | 1.0 | nd | nd | nd |
| p Value | nd | nd | nd | 0.69 | nd | 1.0 | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart2 | nd | nd | nd | 0.15 | nd | 0.18 | nd | nd | nd |
| | nd | nd | nd | 3.6 | nd | 5.5 | nd | nd | nd |
| OR Quart 3 | nd | nd | nd | 1.3 | nd | 1.8 | nd | nd | nd |
| p Value | nd | nd | nd | 0.72 | nd | 0.44 | nd | nd | nd |
| 95% CI of OR Quart3 | nd | nd | nd | 0.31 | nd | 0.39 | nd | nd | nd |
| | nd | nd | nd | 5.5 | nd | 8.8 | nd | nd | nd |
| OR Quart 4 | nd | nd | nd | 1.0 | nd | 1.4 | nd | nd | nd |
| p Value | nd | nd | nd | 1.0 | nd | 0.68 | nd | nd | nd |
| 95% CI of OR Quart4 | nd | nd | nd | 0.22 | nd | 0.28 | nd | nd | nd |
| | nd | nd | nd | 4.5 | nd | 7.1 | nd | nd | nd |

**Tumor necrosis factor receptor superfamily member 10B**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.344 | 0.306 | nd | nd |
| Average | nd | nd | 0.956 | 1.45 | nd | nd |
| Stdev | nd | nd | 1.45 | 1.97 | nd | nd |
| p(t-test) | nd | nd | | 0.24 | nd | nd |
| Min | nd | nd | 0.00360 | 0.0172 | nd | nd |
| Max | nd | nd | 6.71 | 5.86 | nd | nd |
| n (Samp) | nd | nd | 96 | 16 | nd | nd |
| n (Patient) | nd | nd | 73 | 16 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.343 | 0.287 | nd | nd |
| Average | nd | nd | 0.805 | 1.42 | nd | nd |
| Stdev | nd | nd | 1.26 | 2.00 | nd | nd |
| p(t-test) | nd | nd | | 0.12 | nd | nd |
| Min | nd | nd | 0.00360 | 0.0172 | nd | nd |
| Max | nd | nd | 6.48 | 5.86 | nd | nd |
| n (Samp) | nd | nd | 81 | 15 | nd | nd |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.56 | nd | 0.55 | nd | nd | nd |
| SE | nd | nd | nd | 0.080 | nd | 0.083 | nd | nd | nd |
| p | nd | nd | nd | 0.46 | nd | 0.53 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 0.109 | nd | 0.109 | nd | nd | nd |
| Sens 1 | nd | nd | nd | 75% | nd | 73% | nd | nd | nd |
| Spec 1 | nd | nd | nd | 35% | nd | 36% | nd | nd | nd |
| Cutoff 2 | nd | nd | nd | 0.101 | nd | 0.101 | nd | nd | nd |
| Sens 2 | nd | nd | nd | 81% | nd | 80% | nd | nd | nd |
| Spec 2 | nd | nd | nd | 31% | nd | 32% | nd | nd | nd |
| Cutoff 3 | nd | nd | nd | 0.0198 | nd | 0.0182 | nd | nd | nd |
| Sens 3 | nd | nd | nd | 94% | nd | 93% | nd | nd | nd |
| Spec 3 | nd | nd | nd | 11% | nd | 11% | nd | nd | nd |
| Cutoff 4 | nd | nd | nd | 0.855 | nd | 0.819 | nd | nd | nd |
| Sens 4 | nd | nd | nd | 38% | nd | 33% | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 4 | nd | nd | nd | 71% | nd | 70% | nd | nd | nd |
| Cutoff 5 | nd | nd | nd | 1.56 | nd | 0.979 | nd | nd | nd |
| Sens 5 | nd | nd | nd | 31% | nd | 33% | nd | nd | nd |
| Spec 5 | nd | nd | nd | 80% | nd | 80% | nd | nd | nd |
| Cutoff 6 | nd | nd | nd | 3.11 | nd | 2.25 | nd | nd | nd |
| Sens 6 | nd | nd | nd | 19% | nd | 27% | nd | nd | nd |
| Spec 6 | nd | nd | nd | 91% | nd | 90% | nd | nd | nd |
| OR Quart 2 | nd | nd | nd | 4.3 | nd | 3.7 | nd | nd | nd |
| p Value | nd | nd | nd | 0.086 | nd | 0.14 | nd | nd | nd |
| 95% CI of OR Quart2 | nd | nd | nd | 0.81 | nd | 0.66 | nd | nd | nd |
| | nd | nd | nd | 23 | nd | 20 | nd | nd | nd |
| OR Quart 3 | nd | nd | nd | 0.48 | nd | 1.0 | nd | nd | nd |
| p Value | nd | nd | nd | 0.56 | nd | 1.0 | nd | nd | nd |
| 95% CI of OR Quart3 | nd | nd | nd | 0.041 | nd | 0.13 | nd | nd | nd |
| | nd | nd | nd | 5.6 | nd | 7.7 | nd | nd | nd |
| OR Quart 4 | nd | nd | nd | 3.5 | nd | 2.9 | nd | nd | nd |
| p Value | nd | nd | nd | 0.14 | nd | 0.23 | nd | nd | nd |
| 95% CI of OR Quart4 | nd | nd | nd | 0.65 | nd | 0.50 | nd | nd | nd |
| | nd | nd | nd | 19 | nd | 17 | nd | nd | nd |

## Cellular tumor antigen p53

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.00E-9 | 0.000165 | nd | nd |
| Average | nd | nd | 0.00163 | 0.00180 | nd | nd |
| Stdev | nd | nd | 0.00319 | 0.00245 | nd | nd |
| p(t-test) | nd | nd | | 0.84 | nd | nd |
| Min | nd | nd | 1.00E-9 | 1.00E-9 | nd | nd |
| Max | nd | nd | 0.0202 | 0.00839 | nd | nd |
| n (Samp) | nd | nd | 96 | 16 | nd | nd |
| n (Patient) | nd | nd | 73 | 16 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.00E-9 | 3.53E-5 | nd | nd |
| Average | nd | nd | 0.00186 | 0.00145 | nd | nd |
| Stdev | nd | nd | 0.00355 | 0.00190 | nd | nd |
| p(t-test) | nd | nd | | 0.66 | nd | nd |
| Min | nd | nd | 1.00E-9 | 1.00E-9 | nd | nd |
| Max | nd | nd | 0.0202 | 0.00470 | nd | nd |
| n (Samp) | nd | nd | 81 | 15 | nd | nd |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.58 | nd | 0.55 | nd | nd | nd |
| SE | nd | nd | nd | 0.080 | nd | 0.083 | nd | nd | nd |
| p | nd | nd | nd | 0.30 | nd | 0.56 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 0 | nd | 0 | nd | nd | nd |
| Sens 1 | nd | nd | nd | 100% | nd | 100% | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 1 | nd | nd | nd | 0% | nd | 0% | nd | nd | nd |
| Cutoff 2 | nd | nd | nd | 0 | nd | 0 | nd | nd | nd |
| Sens 2 | nd | nd | nd | 100% | nd | 100% | nd | nd | nd |
| Spec 2 | nd | nd | nd | 0% | nd | 0% | nd | nd | nd |
| Cutoff 3 | nd | nd | nd | 0 | nd | 0 | nd | nd | nd |
| Sens 3 | nd | nd | nd | 100% | nd | 100% | nd | nd | nd |
| Spec 3 | nd | nd | nd | 0% | nd | 0% | nd | nd | nd |
| Cutoff 4 | nd | nd | nd | 0.00160 | nd | 0.00160 | nd | nd | nd |
| Sens 4 | nd | nd | nd | 38% | nd | 33% | nd | nd | nd |
| Spec 4 | nd | nd | nd | 73% | nd | 72% | nd | nd | nd |
| Cutoff 5 | nd | nd | nd | 0.00313 | nd | 0.00343 | nd | nd | nd |
| Sens 5 | nd | nd | nd | 38% | nd | 20% | nd | nd | nd |
| Spec 5 | nd | nd | nd | 81% | nd | 80% | nd | nd | nd |
| Cutoff 6 | nd | nd | nd | 0.00470 | nd | 0.00599 | nd | nd | nd |
| Sens 6 | nd | nd | nd | 6% | nd | 0% | nd | nd | nd |
| Spec 6 | nd | nd | nd | 92% | nd | 91% | nd | nd | nd |
| OR Quart 2 | nd | nd | nd | 0.46 | nd | 0.17 | nd | nd | nd |
| p Value | nd | nd | nd | 0.40 | nd | 0.11 | nd | nd | nd |
| 95% CI of | nd | nd | nd | 0.077 | nd | 0.018 | nd | nd | nd |
| OR Quart2 | nd | nd | nd | 2.8 | nd | 1.5 | nd | nd | nd |
| OR Quart 3 | nd | nd | nd | 1.0 | nd | 0.76 | nd | nd | nd |
| p Value | nd | nd | nd | 1.0 | nd | 0.71 | nd | nd | nd |
| 95% CI of | nd | nd | nd | 0.22 | nd | 0.18 | nd | nd | nd |
| OR Quart3 | nd | nd | nd | 4.5 | nd | 3.3 | nd | nd | nd |
| OR Quart 4 | nd | nd | nd | 1.6 | nd | 1.0 | nd | nd | nd |
| p Value | nd | nd | nd | 0.49 | nd | 1.0 | nd | nd | nd |
| 95% CI of | nd | nd | nd | 0.41 | nd | 0.25 | nd | nd | nd |
| OR Quart4 | nd | nd | nd | 6.6 | nd | 4.0 | nd | nd | nd |

Table 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

**Apolipoprotein A-II**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 78.2 | 125 | 77.5 | 247 | 85.8 | 106 |
| Average | 156 | 187 | 167 | 307 | 149 | 132 |
| Stdev | 303 | 193 | 368 | 295 | 238 | 77.5 |
| p(t-test) |  | 0.58 |  | 0.30 |  | 0.74 |
| Min | 4.67 | 18.9 | 4.67 | 23.0 | 21.4 | 18.9 |
| Max | 2000 | 845 | 2000 | 845 | 1790 | 323 |
| n (Samp) | 77 | 33 | 29 | 9 | 61 | 24 |
| n (Patient) | 77 | 33 | 29 | 9 | 61 | 24 |

|  | At Enrollment | | |
|---|---|---|---|
|  | sCr or UO | sCr only | UO only |
| AUC | 0.64 | 0.76 | 0.60 |
| SE | 0.059 | 0.10 | 0.070 |
| p | 0.016 | 0.011 | 0.16 |
| nCohort 1 | 77 | 29 | 61 |
| nCohort 2 | 33 | 9 | 24 |

|  | At Enrollment | | |
|---|---|---|---|
|  | sCr or UO | sCr only | UO only |
| Cutoff 1 | 82.3 | 125 | 85.8 |
| Sens 1 | 73% | 78% | 71% |
| Spec 1 | 53% | 72% | 51% |
| Cutoff 2 | 67.3 | 78.5 | 65.3 |
| Sens 2 | 82% | 89% | 83% |
| Spec 2 | 47% | 55% | 43% |
| Cutoff 3 | 58.1 | 21.6 | 62.3 |
| Sens 3 | 91% | 100% | 92% |
| Spec 3 | 42% | 10% | 41% |
| Cutoff 4 | 126 | 125 | 148 |
| Sens 4 | 48% | 78% | 33% |
| Spec 4 | 70% | 72% | 70% |
| Cutoff 5 | 166 | 166 | 180 |
| Sens 5 | 33% | 56% | 21% |
| Spec 5 | 81% | 83% | 80% |
| Cutoff 6 | 314 | 195 | 314 |
| Sens 6 | 12% | 56% | 4% |
| Spec 6 | 91% | 93% | 90% |
| OR Quart 2 | 3.2 | 0.89 | 12 |
| p Value | 0.12 | 0.94 | 0.025 |
| 95% CI of | 0.75 | 0.047 | 1.4 |
| OR Quart2 | 14 | 17 | 110 |
| OR Quart 3 | 5.5 | 2.3 | 15 |
| p Value | 0.019 | 0.53 | 0.015 |
| 95% CI of | 1.3 | 0.17 | 1.7 |
| OR Quart3 | 23 | 31 | 130 |
| OR Quart 4 | 5.2 | 8.0 | 7.5 |
| p Value | 0.023 | 0.092 | 0.075 |
| 95% CI of | 1.3 | 0.71 | 0.82 |
| OR Quart4 | 21 | 90 | 69 |

## Myoglobin

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.515 | 0.0847 | nd | nd | 0.758 | 0.0561 |
| Average | 42.9 | 26.9 | nd | nd | 49.3 | 3.71 |
| Stdev | 122 | 94.2 | nd | nd | 131 | 12.0 |
| p(t-test) |  | 0.57 | nd | nd |  | 0.16 |
| Min | 0.000105 | 0.000105 | nd | nd | 0.0176 | 0.0145 |
| Max | 618 | 460 | nd | nd | 618 | 49.1 |
| n (Samp) | 52 | 24 | nd | nd | 44 | 17 |
| n (Patient) | 52 | 24 | nd | nd | 44 | 17 |

|  | At Enrollment | | |
|---|---|---|---|
|  | sCr or UO | sCr only | UO only |
| AUC | 0.40 | nd | 0.30 |
| SE | 0.072 | nd | 0.079 |
| p | 0.17 | nd | 0.011 |
| nCohort 1 | 52 | nd | 44 |
| nCohort 2 | 24 | nd | 17 |
| Cutoff 1 | 0.0365 | nd | 0.0347 |
| Sens 1 | 71% | nd | 71% |
| Spec 1 | 15% | nd | 14% |
| Cutoff 2 | 0.0324 | nd | 0.0324 |

|  | At Enrollment |  |  |
| --- | --- | --- | --- |
|  | sCr or UO | sCr only | UO only |
| Sens 2 | 83% | nd | 82% |
| Spec 2 | 12% | nd | 11% |
| Cutoff 3 | 0.0176 | nd | 0.0176 |
| Sens 3 | 92% | nd | 94% |
| Spec 3 | 4% | nd | 2% |
| Cutoff 4 | 13.5 | nd | 13.9 |
| Sens 4 | 21% | nd | 6% |
| Spec 4 | 71% | nd | 70% |
| Cutoff 5 | 33.1 | nd | 34.7 |
| Sens 5 | 12% | nd | 6% |
| Spec 5 | 81% | nd | 82% |
| Cutoff 6 | 63.7 | nd | 85.7 |
| Sens 6 | 8% | nd | 0% |
| Spec 6 | 90% | nd | 91% |
| OR Quart 2 | 0.75 | nd | 1.8 |
| p Value | 0.70 | nd | 0.57 |
| 95% CI of | 0.17 | nd | 0.25 |
| OR Quart2 | 3.4 | nd | 12 |
| OR Quart 3 | 1.6 | nd | 3.5 |
| p Value | 0.49 | nd | 0.18 |
| 95% CI of | 0.41 | nd | 0.56 |
| OR Quart3 | 6.5 | nd | 22 |
| OR Quart 4 | 2.0 | nd | 6.1 |
| p Value | 0.31 | nd | 0.048 |
| 95% CI of | 0.52 | nd | 1.0 |
| OR Quart4 | 8.0 | nd | 37 |

## KSP-Cadherin

|  | sCr or UO | | sCr only | | UO only | |
| --- | --- | --- | --- | --- | --- | --- |
|  | Cohort 1 | Cohort 2 | Cohort 1 | Cohort 2 | Cohort 1 | Cohort 2 |
| Median | 1.48 | 0.809 | nd | nd | 1.46 | 0.996 |
| Average | 1.74 | 1.25 | nd | nd | 1.73 | 1.18 |
| Stdev | 1.18 | 1.36 | nd | nd | 1.10 | 1.26 |
| p(t-test) |  | 0.18 | nd | nd |  | 0.21 |
| Min | 0.131 | 0.00263 | nd | nd | 0.196 | 0.00263 |
| Max | 4.63 | 4.23 | nd | nd | 4.63 | 3.88 |
| n (Samp) | 40 | 16 | nd | nd | 31 | 9 |
| n (Patient) | 40 | 16 | nd | nd | 31 | 9 |

|  | At Enrollment |  |  |
| --- | --- | --- | --- |
|  | sCr or UO | sCr only | UO only |
| AUC | 0.33 | nd | 0.31 |
| SE | 0.084 | nd | 0.11 |
| p | 0.037 | nd | 0.081 |
| nCohort 1 | 40 | nd | 31 |
| nCohort 2 | 16 | nd | 9 |
| Cutoff 1 | 0.258 | nd | 0.196 |
| Sens 1 | 75% | nd | 78% |
| Spec 1 | 5% | nd | 3% |
| Cutoff 2 | 0.196 | nd | 0.00263 |
| Sens 2 | 81% | nd | 89% |
| Spec 2 | 5% | nd | 0% |
| Cutoff 3 | 0.00263 | nd | 0 |
| Sens 3 | 94% | nd | 100% |

|  | At Enrollment | | |
|  | sCr or UO | sCr only | UO only |
| Spec 3 | 0% | nd | 0% |
| Cutoff 4 | 1.84 | nd | 1.84 |
| Sens 4 | 25% | nd | 22% |
| Spec 4 | 70% | nd | 71% |
| Cutoff 5 | 2.57 | nd | 2.54 |
| Sens 5 | 19% | nd | 11% |
| Spec 5 | 80% | nd | 81% |
| Cutoff 6 | 3.39 | nd | 3.11 |
| Sens 6 | 12% | nd | 11% |
| Spec 6 | 90% | nd | 90% |
| OR Quart 2 | 0 | nd | 0 |
| p Value | na | nd | na |
| 95% CI of | na | nd | na |
| OR Quart2 | na | nd | na |
| OR Quart 3 | 1.0 | nd | 1.7 |
| p Value | 1.0 | nd | 0.61 |
| 95% CI of | 0.19 | nd | 0.22 |
| OR Quart3 | 5.2 | nd | 13 |
| OR Quart 4 | 3.3 | nd | 2.7 |
| p Value | 0.13 | nd | 0.34 |
| 95% CI of | 0.69 | nd | 0.36 |
| OR Quart4 | 16 | nd | 20 |

Table 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

**Apolipoprotein A-II**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 76.0 | 265 | 76.0 | 217 | 76.0 | 157 |
| Average | 241 | 1760 | 241 | 1460 | 241 | 225 |
| Stdev | 841 | 5360 | 841 | 5260 | 841 | 193 |
| p(t-test) | | 3.5E-4 | | 0.0035 | | 0.95 |
| Min | 5.33 | 27.6 | 5.33 | 9.51 | 5.33 | 55.6 |
| Max | 6400 | 24300 | 6400 | 24300 | 6400 | 764 |
| n (Samp) | 196 | 21 | 196 | 21 | 196 | 11 |
| n (Patient) | 196 | 21 | 196 | 21 | 196 | 11 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 97.8 | 268 | 97.8 | 265 | 97.8 | 216 |
| Average | 342 | 954 | 342 | 393 | 342 | 213 |
| Stdev | 1610 | 1880 | 1610 | 510 | 1610 | 68.6 |
| p(t-test) | | 0.22 | | 0.92 | | 0.84 |
| Min | 5.33 | 27.6 | 5.33 | 9.51 | 5.33 | 131 |
| Max | 24300 | 6400 | 24300 | 1860 | 24300 | 288 |
| n (Samp) | 294 | 11 | 294 | 11 | 294 | 6 |
| n (Patient) | 294 | 11 | 294 | 11 | 294 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 87.2 | 174 | 87.2 | 174 | 87.2 | 149 |
| Average | 294 | 2240 | 294 | 1850 | 294 | 227 |
| Stdev | 972 | 6320 | 972 | 6230 | 972 | 214 |
| p(t-test) | | 0.0012 | | 0.0086 | | 0.84 |
| Min | 5.33 | 55.6 | 5.33 | 45.8 | 5.33 | 55.6 |
| Max | 6400 | 24300 | 6400 | 24300 | 6400 | 764 |
| n (Samp) | 132 | 15 | 132 | 15 | 132 | 9 |
| n (Patient) | 132 | 15 | 132 | 15 | 132 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.78 | 0.79 | 0.77 | 0.76 | 0.76 | 0.78 | 0.78 | 0.73 |
| SE | 0.059 | 0.084 | 0.072 | 0.062 | 0.086 | 0.075 | 0.084 | 0.11 | 0.098 |
| p | 2.8E-7 | 9.4E-4 | 4.7E-5 | 8.7E-6 | 0.0029 | 5.7E-4 | 0.0010 | 0.014 | 0.019 |
| nCohort 1 | 196 | 294 | 132 | 196 | 294 | 132 | 196 | 294 | 132 |
| nCohort 2 | 21 | 11 | 15 | 21 | 11 | 15 | 11 | 6 | 9 |
| Cutoff 1 | 148 | 165 | 146 | 148 | 165 | 146 | 146 | 157 | 111 |
| Sens 1 | 71% | 73% | 73% | 71% | 73% | 73% | 73% | 83% | 78% |
| Spec 1 | 80% | 74% | 77% | 80% | 74% | 77% | 79% | 71% | 63% |
| Cutoff 2 | 129 | 157 | 129 | 129 | 157 | 129 | 111 | 157 | 100 |
| Sens 2 | 81% | 82% | 80% | 81% | 82% | 80% | 82% | 83% | 89% |
| Spec 2 | 74% | 71% | 73% | 74% | 71% | 73% | 67% | 71% | 58% |
| Cutoff 3 | 100 | 135 | 100 | 54.2 | 129 | 54.3 | 100 | 129 | 54.3 |
| Sens 3 | 90% | 91% | 93% | 90% | 91% | 93% | 91% | 100% | 100% |
| Spec 3 | 62% | 65% | 58% | 37% | 64% | 32% | 62% | 64% | 32% |
| Cutoff 4 | 124 | 155 | 127 | 124 | 155 | 127 | 124 | 155 | 127 |
| Sens 4 | 81% | 82% | 80% | 81% | 82% | 80% | 73% | 83% | 67% |
| Spec 4 | 70% | 70% | 70% | 70% | 70% | 70% | 70% | 70% | 70% |
| Cutoff 5 | 153 | 200 | 164 | 153 | 200 | 164 | 153 | 200 | 164 |
| Sens 5 | 67% | 64% | 53% | 67% | 64% | 53% | 55% | 50% | 33% |
| Spec 5 | 80% | 80% | 80% | 80% | 80% | 80% | 80% | 80% | 80% |
| Cutoff 6 | 296 | 335 | 312 | 296 | 335 | 312 | 296 | 335 | 312 |
| Sens 6 | 29% | 27% | 20% | 24% | 18% | 20% | 9% | 0% | 11% |
| Spec 6 | 90% | 90% | 90% | 90% | 90% | 90% | 90% | 90% | 90% |
| OR Quart 2 | 1.0 | 0 | >1.0 | 2.0 | 0 | 0.97 | >1.0 | >0 | >1.0 |
| p Value | 1.0 | na | <1.0 | 0.57 | na | 0.98 | <1.0 | <na | <0.98 |
| 95% CI of OR Quart2 | 0.061 | na | >0.060 | 0.18 | na | 0.058 | >0.061 | >na | >0.062 |
| | 16 | na | na | 23 | na | 16 | na | na | na |
| OR Quart 3 | 4.2 | 3.1 | >5.6 | 3.1 | 3.1 | 4.2 | >2.0 | >3.1 | >2.1 |
| p Value | 0.20 | 0.33 | <0.12 | 0.33 | 0.33 | 0.21 | <0.57 | <0.33 | <0.55 |
| 95% CI of OR Quart3 | 0.46 | 0.31 | >0.62 | 0.31 | 0.31 | 0.45 | >0.18 | >0.32 | >0.18 |
| | 39 | 30 | na | 31 | 30 | 40 | na | na | na |
| OR Quart 4 | 20 | 7.5 | >12 | 20 | 7.5 | 11 | >9.3 | >3.1 | >7.0 |
| p Value | 0.0046 | 0.063 | <0.024 | 0.0046 | 0.063 | 0.026 | <0.039 | <0.33 | <0.079 |
| 95% CI of OR Quart4 | 2.5 | 0.90 | >1.4 | 2.5 | 0.90 | 1.3 | >1.1 | >0.32 | >0.80 |
| | 160 | 63 | na | 160 | 63 | 94 | na | na | na |

## Caspase-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.28 | 4.81 | 1.28 | 2.33 | 1.28 | 0.988 |
| Average | 1.56 | 10.5 | 1.56 | 4.46 | 1.56 | 1.77 |
| Stdev | 1.38 | 15.7 | 1.38 | 6.02 | 1.38 | 2.36 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.0057 | | 0.027 | | 0.78 |
| Min | 0.0223 | 0.0223 | 0.0223 | 0.0223 | 0.0223 | 0.0223 |
| Max | 4.68 | 47.1 | 4.68 | 17.3 | 4.68 | 6.25 |
| n (Samp) | 26 | 8 | 26 | 7 | 26 | 6 |
| n (Patient) | 26 | 8 | 26 | 7 | 26 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | nd | nd | 0.64 | nd | nd | 0.45 | nd | nd |
| SE | 0.11 | nd | nd | 0.12 | nd | nd | 0.13 | nd | nd |
| p | 0.021 | nd | nd | 0.25 | nd | nd | 0.72 | nd | nd |
| nCohort 1 | 26 | nd | nd | 26 | nd | nd | 26 | nd | nd |
| nCohort 2 | 8 | nd | nd | 7 | nd | nd | 6 | nd | nd |
| Cutoff 1 | 2.08 | nd | nd | 1.02 | nd | nd | 0 | nd | nd |
| Sens 1 | 75% | nd | nd | 71% | nd | nd | 100% | nd | nd |
| Spec 1 | 69% | nd | nd | 46% | nd | nd | 0% | nd | nd |
| Cutoff 2 | 0.496 | nd | nd | 0.496 | nd | nd | 0 | nd | nd |
| Sens 2 | 88% | nd | nd | 86% | nd | nd | 100% | nd | nd |
| Spec 2 | 31% | nd | nd | 31% | nd | nd | 0% | nd | nd |
| Cutoff 3 | 0 | nd | nd | 0 | nd | nd | 0 | nd | nd |
| Sens 3 | 100% | nd | nd | 100% | nd | nd | 100% | nd | nd |
| Spec 3 | 0% | nd | nd | 0% | nd | nd | 0% | nd | nd |
| Cutoff 4 | 2.33 | nd | nd | 2.33 | nd | nd | 2.33 | nd | nd |
| Sens 4 | 62% | nd | nd | 43% | nd | nd | 17% | nd | nd |
| Spec 4 | 73% | nd | nd | 73% | nd | nd | 73% | nd | nd |
| Cutoff 5 | 2.59 | nd | nd | 2.59 | nd | nd | 2.59 | nd | nd |
| Sens 5 | 62% | nd | nd | 43% | nd | nd | 17% | nd | nd |
| Spec 5 | 85% | nd | nd | 85% | nd | nd | 85% | nd | nd |
| Cutoff 6 | 3.90 | nd | nd | 3.90 | nd | nd | 3.90 | nd | nd |
| Sens 6 | 50% | nd | nd | 29% | nd | nd | 17% | nd | nd |
| Spec 6 | 92% | nd | nd | 92% | nd | nd | 92% | nd | nd |
| OR Quart 2 | 0.88 | nd | nd | 1.0 | nd | nd | 1.0 | nd | nd |
| p Value | 0.93 | nd | nd | 1.0 | nd | nd | 1.0 | nd | nd |
| 95% CI of OR Quart2 | 0.046 17 | nd | nd | 0.052 19 | nd | nd | 0.052 19 | nd | nd |
| OR Quart 3 | 1.0 | nd | nd | 2.3 | nd | nd | 2.3 | nd | nd |
| p Value | 1.0 | nd | nd | 0.53 | nd | nd | 0.53 | nd | nd |
| 95% CI of OR Quart3 | 0.052 19 | nd | nd | 0.17 33 | nd | nd | 0.17 33 | nd | nd |
| OR Quart 4 | 8.8 | nd | nd | 3.5 | nd | nd | 2.3 | nd | nd |
| p Value | 0.086 | nd | nd | 0.33 | nd | nd | 0.53 | nd | nd |
| 95% CI of OR Quart4 | 0.74 100 | nd | nd | 0.28 43 | nd | nd | 0.17 33 | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.549 | 3.00 | 0.549 | 2.16 | 0.549 | 1.09 |
| Average | 1.25 | 2.65 | 1.25 | 1.79 | 1.25 | 1.35 |
| Stdev | 3.21 | 1.71 | 3.21 | 1.60 | 3.21 | 1.25 |
| p(t-test) | | 0.24 | | 0.67 | | 0.94 |
| Min | 0.0360 | 0.146 | 0.0360 | 0.146 | 0.0360 | 0.146 |
| Max | 19.5 | 4.64 | 19.5 | 4.64 | 19.5 | 3.33 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 36 | 8 | 36 | 7 | 36 | 6 |
| n (Patient) | 36 | 8 | 36 | 7 | 36 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.79 | nd | nd | 0.69 | nd | nd | 0.64 | nd | nd |
| SE | 0.10 | nd | nd | 0.12 | nd | nd | 0.13 | nd | nd |
| p | 0.0034 | nd | nd | 0.11 | nd | nd | 0.27 | nd | nd |
| nCohort 1 | 36 | nd | nd | 36 | nd | nd | 36 | nd | nd |
| nCohort 2 | 8 | nd | nd | 7 | nd | nd | 6 | nd | nd |
| Cutoff 1 | 1.76 | nd | nd | 0.473 | nd | nd | 0.208 | nd | nd |
| Sens 1 | 75% | nd | nd | 71% | nd | nd | 83% | nd | nd |
| Spec 1 | 89% | nd | nd | 50% | nd | nd | 33% | nd | nd |
| Cutoff 2 | 0.208 | nd | nd | 0.208 | nd | nd | 0.208 | nd | nd |
| Sens 2 | 88% | nd | nd | 86% | nd | nd | 83% | nd | nd |
| Spec 2 | 33% | nd | nd | 33% | nd | nd | 33% | nd | nd |
| Cutoff 3 | 0.135 | nd | nd | 0.135 | nd | nd | 0.135 | nd | nd |
| Sens 3 | 100% | nd | nd | 100% | nd | nd | 100% | nd | nd |
| Spec 3 | 28% | nd | nd | 28% | nd | nd | 28% | nd | nd |
| Cutoff 4 | 0.868 | nd | nd | 0.868 | nd | nd | 0.868 | nd | nd |
| Sens 4 | 75% | nd | nd | 57% | nd | nd | 50% | nd | nd |
| Spec 4 | 72% | nd | nd | 72% | nd | nd | 72% | nd | nd |
| Cutoff 5 | 1.22 | nd | nd | 1.22 | nd | nd | 1.22 | nd | nd |
| Sens 5 | 75% | nd | nd | 57% | nd | nd | 50% | nd | nd |
| Spec 5 | 81% | nd | nd | 81% | nd | nd | 81% | nd | nd |
| Cutoff 6 | 2.17 | nd | nd | 2.17 | nd | nd | 2.17 | nd | nd |
| Sens 6 | 62% | nd | nd | 29% | nd | nd | 17% | nd | nd |
| Spec 6 | 92% | nd | nd | 92% | nd | nd | 92% | nd | nd |
| OR Quart 2 | >2.4 | nd | nd | >2.2 | nd | nd | >2.2 | nd | nd |
| p Value | <0.49 | nd | nd | <0.54 | nd | nd | <0.54 | nd | nd |
| 95% CI of OR Quart2 | >0.19 na | nd | nd | >0.17 na | nd | nd | >0.17 na | nd | nd |
| OR Quart 3 | >0 | nd | nd | >1.0 | nd | nd | >1.1 | nd | nd |
| p Value | <na | nd | nd | <1.0 | nd | nd | <0.94 | nd | nd |
| 95% CI of OR Quart3 | >na na | nd | nd | >0.055 na | nd | nd | >0.060 na | nd | nd |
| OR Quart 4 | >13 | nd | nd | >5.7 | nd | nd | >3.8 | nd | nd |
| p Value | <0.033 | nd | nd | <0.15 | nd | nd | <0.29 | nd | nd |
| 95% CI of OR Quart4 | >1.2 na | nd | nd | >0.52 na | nd | nd | >0.32 na | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 66800 | 66800 | 66800 | 66800 | nd | nd |
| Average | 99700 | 95000 | 99700 | 95000 | nd | nd |
| Stdev | 131000 | 95400 | 131000 | 95400 | nd | nd |
| p(t-test) | | 0.92 | | 0.92 | nd | nd |
| Min | 3290 | 2220 | 3290 | 2220 | nd | nd |
| Max | 744000 | 260000 | 744000 | 260000 | nd | nd |
| n (Samp) | 41 | 8 | 41 | 8 | nd | nd |
| n (Patient) | 41 | 8 | 41 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | nd | 0.49 | nd | nd | nd | nd | nd |
| SE | 0.11 | nd | nd | 0.11 | nd | nd | nd | nd | nd |
| p | 0.91 | nd | nd | 0.91 | nd | nd | nd | nd | nd |
| nCohort 1 | 41 | nd | nd | 41 | nd | nd | nd | nd | nd |
| nCohort 2 | 8 | nd | nd | 8 | nd | nd | nd | nd | nd |
| Cutoff 1 | 16300 | nd | nd | 16300 | nd | nd | nd | nd | nd |
| Sens 1 | 75% | nd | nd | 75% | nd | nd | nd | nd | nd |
| Spec 1 | 17% | nd | nd | 17% | nd | nd | nd | nd | nd |
| Cutoff 2 | 8380 | nd | nd | 8380 | nd | nd | nd | nd | nd |
| Sens 2 | 88% | nd | nd | 88% | nd | nd | nd | nd | nd |
| Spec 2 | 10% | nd | nd | 10% | nd | nd | nd | nd | nd |
| Cutoff 3 | 0 | nd | nd | 0 | nd | nd | nd | nd | nd |
| Sens 3 | 100% | nd | nd | 100% | nd | nd | nd | nd | nd |
| Spec 3 | 0% | nd | nd | 0% | nd | nd | nd | nd | nd |
| Cutoff 4 | 123000 | nd | nd | 123000 | nd | nd | nd | nd | nd |
| Sens 4 | 38% | nd | nd | 38% | nd | nd | nd | nd | nd |
| Spec 4 | 71% | nd | nd | 71% | nd | nd | nd | nd | nd |
| Cutoff 5 | 138000 | nd | nd | 138000 | nd | nd | nd | nd | nd |
| Sens 5 | 25% | nd | nd | 25% | nd | nd | nd | nd | nd |
| Spec 5 | 80% | nd | nd | 80% | nd | nd | nd | nd | nd |
| Cutoff 6 | 155000 | nd | nd | 155000 | nd | nd | nd | nd | nd |
| Sens 6 | 25% | nd | nd | 25% | nd | nd | nd | nd | nd |
| Spec 6 | 90% | nd | nd | 90% | nd | nd | nd | nd | nd |
| OR Quart 2 | 0.30 | nd | nd | 0.30 | nd | nd | nd | nd | nd |
| p Value | 0.33 | nd | nd | 0.33 | nd | nd | nd | nd | nd |
| 95% CI of | 0.027 | nd | nd | 0.027 | nd | nd | nd | nd | nd |
| OR Quart2 | 3.4 | nd | nd | 3.4 | nd | nd | nd | nd | nd |
| OR Quart 3 | 0.30 | nd | nd | 0.30 | nd | nd | nd | nd | nd |
| p Value | 0.33 | nd | nd | 0.33 | nd | nd | nd | nd | nd |
| 95% CI of | 0.027 | nd | nd | 0.027 | nd | nd | nd | nd | nd |
| OR Quart3 | 3.4 | nd | nd | 3.4 | nd | nd | nd | nd | nd |
| OR Quart 4 | 1.1 | nd | nd | 1.1 | nd | nd | nd | nd | nd |
| p Value | 0.91 | nd | nd | 0.91 | nd | nd | nd | nd | nd |
| 95% CI of | 0.18 | nd | nd | 0.18 | nd | nd | nd | nd | nd |
| OR Quart4 | 7.0 | nd | nd | 7.0 | nd | nd | nd | nd | nd |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.09 | 14.9 | 3.09 | 9.35 | 3.09 | 4.76 |
| Average | 88.7 | 37.1 | 88.7 | 36.6 | 88.7 | 15.2 |
| Stdev | 688 | 81.6 | 688 | 81.8 | 688 | 16.5 |
| p(t-test) | | 0.77 | | 0.77 | | 0.75 |
| Min | 0.116 | 0.116 | 0.116 | 0.116 | 0.116 | 1.64 |
| Max | 6950 | 327 | 6950 | 327 | 6950 | 44.1 |
| n (Samp) | 102 | 15 | 102 | 15 | 102 | 9 |
| n (Patient) | 102 | 15 | 102 | 15 | 102 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.90 | 18.8 | 4.90 | 15.7 | nd | nd |
| Average | 69.2 | 21.5 | 69.2 | 20.7 | nd | nd |
| Stdev | 541 | 18.2 | 541 | 18.6 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.80 | | 0.80 | nd | nd |
| Min | 1.14E-14 | 0.116 | 1.14E-14 | 0.116 | nd | nd |
| Max | 6950 | 44.1 | 6950 | 44.1 | nd | nd |
| n (Samp) | 168 | 8 | 168 | 8 | nd | nd |
| n (Patient) | 168 | 8 | 168 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.29 | 14.9 | 3.29 | 14.9 | 3.29 | 4.76 |
| Average | 101 | 50.0 | 101 | 50.0 | 101 | 14.4 |
| Stdev | 753 | 105 | 753 | 105 | 753 | 16.4 |
| p(t-test) | | 0.84 | | 0.84 | | 0.76 |
| Min | 0.116 | 2.45 | 0.116 | 2.45 | 0.116 | 2.45 |
| Max | 6950 | 327 | 6950 | 327 | 6950 | 44.1 |
| n (Samp) | 85 | 9 | 85 | 9 | 85 | 7 |
| n (Patient) | 85 | 9 | 85 | 9 | 85 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.66 | 0.69 | 0.65 | 0.65 | 0.68 | 0.63 | nd | 0.63 |
| SE | 0.081 | 0.11 | 0.10 | 0.081 | 0.11 | 0.10 | 0.10 | nd | 0.12 |
| p | 0.050 | 0.15 | 0.067 | 0.062 | 0.18 | 0.078 | 0.22 | nd | 0.28 |
| nCohort 1 | 102 | 168 | 85 | 102 | 168 | 85 | 102 | nd | 85 |
| nCohort 2 | 15 | 8 | 9 | 15 | 8 | 9 | 9 | nd | 7 |
| Cutoff 1 | 2.45 | 7.35 | 2.45 | 2.45 | 7.35 | 2.45 | 2.02 | nd | 2.45 |
| Sens 1 | 73% | 75% | 78% | 73% | 75% | 78% | 89% | nd | 71% |
| Spec 1 | 46% | 59% | 47% | 46% | 59% | 47% | 46% | nd | 47% |
| Cutoff 2 | 2.02 | 1.36 | 2.02 | 2.02 | 1.36 | 2.02 | 2.02 | nd | 2.02 |
| Sens 2 | 87% | 88% | 100% | 87% | 88% | 100% | 89% | nd | 100% |
| Spec 2 | 46% | 40% | 47% | 46% | 40% | 47% | 46% | nd | 47% |
| Cutoff 3 | 1.09 | 1.14E-14 | 2.02 | 1.09 | 1.14E-14 | 2.02 | 1.09 | nd | 2.02 |
| Sens 3 | 93% | 100% | 100% | 93% | 100% | 100% | 100% | nd | 100% |
| Spec 3 | 44% | 1% | 47% | 44% | 1% | 47% | 44% | nd | 47% |
| Cutoff 4 | 12.3 | 13.6 | 12.3 | 12.3 | 13.6 | 12.3 | 12.3 | nd | 12.3 |
| Sens 4 | 53% | 62% | 56% | 47% | 50% | 56% | 44% | nd | 43% |
| Spec 4 | 71% | 70% | 71% | 71% | 70% | 71% | 71% | nd | 71% |
| Cutoff 5 | 25.7 | 26.7 | 25.7 | 25.7 | 26.7 | 25.7 | 25.7 | nd | 25.7 |
| Sens 5 | 33% | 38% | 33% | 33% | 38% | 33% | 33% | nd | 29% |
| Spec 5 | 80% | 80% | 80% | 80% | 80% | 80% | 80% | nd | 80% |
| Cutoff 6 | 55.7 | 56.1 | 44.8 | 55.7 | 56.1 | 44.8 | 55.7 | nd | 44.8 |
| Sens 6 | 7% | 0% | 11% | 7% | 0% | 11% | 0% | nd | 0% |
| Spec 6 | 90% | 90% | 91% | 90% | 90% | 91% | 90% | nd | 91% |
| OR Quart 2 | 4.5 | >2.1 | >3.3 | 4.5 | >2.1 | >3.3 | >4.5 | nd | >3.4 |
| p Value | 0.19 | <0.55 | <0.32 | 0.19 | <0.55 | <0.32 | <0.19 | nd | <0.30 |
| 95% CI of OR Quart2 | 0.47 43 | >0.18 na | >0.32 na | 0.47 43 | >0.18 na | >0.32 na | >0.47 na | nd | >0.33 na |
| OR Quart 3 | 4.5 | >3.2 | >3.4 | 4.5 | >3.2 | >3.4 | >2.1 | nd | >2.2 |
| p Value | 0.19 | <0.32 | <0.30 | 0.19 | <0.32 | <0.30 | <0.56 | nd | <0.53 |
| 95% CI of OR Quart3 | 0.47 43 | >0.32 na | >0.33 na | 0.47 43 | >0.32 na | >0.33 na | >0.18 na | nd | >0.18 na |
| OR Quart 4 | 7.0 | >3.2 | >3.3 | 7.0 | >3.2 | >3.3 | >3.2 | nd | >2.2 |
| p Value | 0.081 | <0.32 | <0.32 | 0.081 | <0.32 | <0.32 | <0.32 | nd | <0.53 |
| 95% CI of OR Quart4 | 0.79 62 | >0.32 na | >0.32 na | 0.79 62 | >0.32 na | >0.32 na | >0.32 na | nd | >0.18 na |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.14 | 5.45 | 1.14 | 2.86 | 1.14 | 2.98 |
| Average | 3.19 | 7.89 | 3.19 | 7.48 | 3.19 | 5.77 |
| Stdev | 5.64 | 11.7 | 5.64 | 12.1 | 5.64 | 4.90 |
| p(t-test) | | 0.0094 | | 0.021 | | 0.19 |
| Min | 0.00336 | 0.282 | 0.00336 | 0.219 | 0.00336 | 0.852 |
| Max | 43.4 | 50.1 | 43.4 | 50.1 | 43.4 | 14.6 |
| n (Samp) | 102 | 17 | 102 | 16 | 102 | 9 |
| n (Patient) | 102 | 17 | 102 | 16 | 102 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.57 | 4.46 | 1.57 | 3.11 | nd | nd |
| Average | 28.3 | 4.33 | 28.3 | 3.91 | nd | nd |
| Stdev | 312 | 3.44 | 312 | 3.51 | nd | nd |
| p(t-test) | | 0.83 | | 0.83 | nd | nd |
| Min | 0.00336 | 0.282 | 0.00336 | 0.219 | nd | nd |
| Max | 4070 | 10.9 | 4070 | 10.9 | nd | nd |
| n (Samp) | 170 | 8 | 170 | 8 | nd | nd |
| n (Patient) | 170 | 8 | 170 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.19 | 6.79 | 1.19 | 4.88 | 1.19 | 2.98 |
| Average | 3.63 | 10.4 | 3.63 | 10.3 | 3.63 | 6.19 |
| Stdev | 6.41 | 14.1 | 6.41 | 14.7 | 6.41 | 5.50 |
| p(t-test) | | 0.0070 | | 0.011 | | 0.31 |
| Min | 0.00336 | 0.356 | 0.00336 | 0.852 | 0.00336 | 0.852 |
| Max | 43.4 | 50.1 | 43.4 | 50.1 | 43.4 | 14.6 |
| n (Samp) | 85 | 11 | 85 | 10 | 85 | 7 |
| n (Patient) | 85 | 11 | 85 | 10 | 85 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.62 | 0.75 | 0.69 | 0.57 | 0.77 | 0.76 | nd | 0.74 |
| SE | 0.075 | 0.11 | 0.089 | 0.078 | 0.11 | 0.091 | 0.096 | nd | 0.11 |
| p | 0.0072 | 0.28 | 0.0055 | 0.015 | 0.50 | 0.0030 | 0.0080 | nd | 0.034 |
| nCohort 1 | 102 | 170 | 85 | 102 | 170 | 85 | 102 | nd | 85 |
| nCohort 2 | 17 | 8 | 11 | 16 | 8 | 10 | 9 | nd | 7 |
| Cutoff 1 | 2.56 | 2.59 | 2.88 | 2.26 | 2.26 | 2.55 | 2.53 | nd | 2.53 |
| Sens 1 | 71% | 75% | 73% | 75% | 75% | 70% | 78% | nd | 71% |
| Spec 1 | 71% | 65% | 71% | 67% | 61% | 68% | 70% | nd | 68% |
| Cutoff 2 | 0.816 | 0.450 | 1.40 | 1.44 | 0.238 | 2.53 | 1.44 | nd | 1.40 |
| Sens 2 | 82% | 88% | 82% | 81% | 88% | 80% | 89% | nd | 86% |
| Spec 2 | 42% | 20% | 56% | 58% | 8% | 68% | 58% | nd | 56% |
| Cutoff 3 | 0.339 | 0.281 | 0.816 | 0.238 | 0.214 | 1.40 | 0.816 | nd | 0.816 |
| Sens 3 | 94% | 100% | 91% | 94% | 100% | 90% | 100% | nd | 100% |
| Spec 3 | 19% | 10% | 39% | 9% | 5% | 56% | 42% | nd | 39% |
| Cutoff 4 | 2.56 | 3.33 | 2.88 | 2.56 | 3.33 | 2.88 | 2.56 | nd | 2.88 |
| Sens 4 | 71% | 62% | 73% | 56% | 50% | 60% | 67% | nd | 57% |
| Spec 4 | 71% | 70% | 71% | 71% | 70% | 71% | 71% | nd | 71% |
| Cutoff 5 | 4.03 | 5.77 | 5.67 | 4.03 | 5.77 | 5.67 | 4.03 | nd | 5.67 |
| Sens 5 | 53% | 25% | 55% | 44% | 25% | 50% | 44% | nd | 43% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 5 | 80% | 80% | 80% | 80% | 80% | 80% | 80% | nd | 80% |
| Cutoff 6 | 8.86 | 11.0 | 9.55 | 8.86 | 11.0 | 9.55 | 8.86 | nd | 9.55 |
| Sens 6 | 29% | 0% | 45% | 25% | 0% | 40% | 33% | nd | 43% |
| Spec 6 | 90% | 90% | 91% | 90% | 90% | 91% | 90% | nd | 91% |
| OR Quart 2 | 0.30 | 0 | 1.0 | 0.47 | 0 | >1.0 | >1.0 | nd | >1.0 |
| p Value | 0.31 | na | 1.0 | 0.54 | na | <1.0 | <1.0 | nd | <0.98 |
| 95% CI of | 0.029 | na | 0.059 | 0.040 | na | >0.059 | >0.059 | nd | >0.062 |
| OR Quart2 | 3.1 | na | 17 | 5.4 | na | na | na | nd | na |
| OR Quart 3 | 1.3 | 1.0 | 3.3 | 3.5 | 1.5 | >4.6 | >4.5 | nd | >3.4 |
| p Value | 0.72 | 1.0 | 0.32 | 0.15 | 0.65 | <0.19 | <0.19 | nd | <0.30 |
| 95% CI of | 0.27 | 0.13 | 0.32 | 0.65 | 0.24 | >0.47 | >0.47 | nd | >0.33 |
| OR Quart3 | 6.6 | 7.4 | 34 | 19 | 9.7 | na | na | nd | na |
| OR Quart 4 | 3.7 | 2.0 | 7.7 | 4.1 | 1.5 | >6.1 | >4.5 | nd | >3.4 |
| p Value | 0.072 | 0.42 | 0.070 | 0.097 | 0.67 | <0.11 | <0.19 | nd | <0.30 |
| 95% CI of | 0.89 | 0.36 | 0.85 | 0.78 | 0.24 | >0.65 | >0.47 | nd | >0.33 |
| OR Quart4 | 15 | 12 | 70 | 22 | 9.4 | na | na | nd | na |

## Myoglobin

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 5.42 | 18.9 | 5.42 | 11.9 | 5.42 | 44.9 |
| Average | 51.4 | 149 | 51.4 | 155 | 51.4 | 174 |
| Stdev | 121 | 203 | 121 | 208 | 121 | 213 |
| p(t-test) |  | 0.0066 |  | 0.0052 |  | 0.0076 |
| Min | 0.00616 | 0.0439 | 0.00616 | 0.0439 | 0.00616 | 0.0772 |
| Max | 618 | 469 | 618 | 469 | 618 | 469 |
| n (Samp) | 102 | 17 | 102 | 16 | 102 | 9 |
| n (Patient) | 102 | 17 | 102 | 16 | 102 | 9 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.65 | 31.9 | 3.65 | 29.4 | nd | nd |
| Average | 63.1 | 142 | 63.1 | 141 | nd | nd |
| Stdev | 142 | 195 | 142 | 196 | nd | nd |
| p(t-test) |  | 0.13 |  | 0.14 | nd | nd |
| Min | 0.00616 | 0.0439 | 0.00616 | 0.0439 | nd | nd |
| Max | 618 | 469 | 618 | 469 | nd | nd |
| n (Samp) | 170 | 8 | 170 | 8 | nd | nd |
| n (Patient) | 170 | 8 | 170 | 8 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.83 | 0.377 | 3.83 | 2.87 | 3.83 | 5.36 |
| Average | 56.6 | 143 | 56.6 | 152 | 56.6 | 150 |
| Stdev | 128 | 203 | 128 | 212 | 128 | 207 |
| p(t-test) |  | 0.055 |  | 0.042 |  | 0.081 |
| Min | 0.00616 | 0.0696 | 0.00616 | 0.0772 | 0.00616 | 0.0772 |
| Max | 618 | 469 | 618 | 469 | 618 | 469 |
| n (Samp) | 85 | 11 | 85 | 10 | 85 | 7 |
| n (Patient) | 85 | 11 | 85 | 10 | 85 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.64 | 0.49 | 0.57 | 0.63 | 0.51 | 0.62 | nd | 0.54 |
| SE | 0.078 | 0.11 | 0.093 | 0.080 | 0.11 | 0.097 | 0.10 | nd | 0.12 |
| p | 0.36 | 0.21 | 0.95 | 0.36 | 0.24 | 0.93 | 0.25 | nd | 0.73 |
| nCohort 1 | 102 | 170 | 85 | 102 | 170 | 85 | 102 | nd | 85 |
| nCohort 2 | 17 | 8 | 11 | 16 | 8 | 10 | 9 | nd | 7 |
| Cutoff 1 | 0.288 | 10.2 | 0.117 | 0.288 | 9.39 | 0.288 | 0.288 | nd | 0.288 |
| Sens 1 | 71% | 75% | 73% | 75% | 75% | 70% | 78% | nd | 71% |
| Spec 1 | 21% | 61% | 14% | 21% | 61% | 20% | 21% | nd | 20% |
| Cutoff 2 | 0.0772 | 0.0780 | 0.0772 | 0.117 | 0.0780 | 0.117 | 0.117 | nd | 0.117 |
| Sens 2 | 82% | 88% | 82% | 81% | 88% | 80% | 89% | nd | 86% |
| Spec 2 | 13% | 14% | 11% | 16% | 14% | 14% | 16% | nd | 14% |
| Cutoff 3 | 0.0606 | 0.0411 | 0.0696 | 0.0606 | 0.0411 | 0.0772 | 0.0606 | nd | 0.0661 |
| Sens 3 | 94% | 100% | 91% | 94% | 100% | 90% | 100% | nd | 100% |
| Spec 3 | 13% | 9% | 11% | 13% | 9% | 11% | 13% | nd | 11% |
| Cutoff 4 | 17.2 | 18.6 | 17.9 | 17.2 | 18.6 | 17.9 | 17.2 | nd | 17.9 |
| Sens 4 | 53% | 62% | 45% | 44% | 50% | 40% | 56% | nd | 43% |
| Spec 4 | 71% | 70% | 71% | 71% | 70% | 71% | 71% | nd | 71% |
| Cutoff 5 | 38.9 | 45.3 | 55.8 | 38.9 | 45.3 | 55.8 | 38.9 | nd | 55.8 |
| Sens 5 | 47% | 38% | 36% | 44% | 38% | 40% | 56% | nd | 43% |
| Spec 5 | 80% | 80% | 80% | 80% | 80% | 80% | 80% | nd | 80% |
| Cutoff 6 | 222 | 234 | 232 | 222 | 234 | 232 | 222 | nd | 232 |
| Sens 6 | 29% | 25% | 27% | 31% | 25% | 30% | 33% | nd | 29% |
| Spec 6 | 90% | 90% | 91% | 90% | 90% | 91% | 90% | nd | 91% |
| OR Quart 2 | 0.13 | 0 | 0 | 0.13 | 0 | 0.21 | 0.30 | nd | 0 |
| p Value | 0.070 | na | na | 0.070 | na | 0.17 | 0.31 | nd | na |
| 95% CI of OR Quart2 | 0.015 | na | na | 0.015 | na | 0.021 | 0.029 | nd | na |
| | 1.2 | na | na | 1.2 | na | 2.0 | 3.0 | nd | na |
| OR Quart 3 | 0.27 | 1.0 | 0.17 | 0.28 | 1.0 | 0.21 | 0 | nd | 0.30 |
| p Value | 0.13 | 1.0 | 0.11 | 0.15 | 1.0 | 0.17 | na | nd | 0.32 |
| 95% CI of OR Quart3 | 0.050 | 0.13 | 0.018 | 0.052 | 0.13 | 0.021 | na | nd | 0.029 |
| | 1.5 | 7.4 | 1.5 | 1.5 | 7.4 | 2.0 | na | nd | 3.2 |
| OR Quart 4 | 1.4 | 2.0 | 1.0 | 1.2 | 2.0 | 0.95 | 1.7 | nd | 1.0 |
| p Value | 0.59 | 0.42 | 1.0 | 0.81 | 0.42 | 0.95 | 0.48 | nd | 1.0 |
| 95% CI of OR Quart4 | 0.42 | 0.36 | 0.25 | 0.34 | 0.36 | 0.21 | 0.37 | nd | 0.18 |
| | 4.7 | 12 | 4.0 | 4.0 | 12 | 4.4 | 8.1 | nd | 5.6 |

## Mucin-16

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.684 | 1.03 | 0.684 | 1.03 | nd | nd |
| Average | 1.18 | 54.9 | 1.18 | 54.9 | nd | nd |
| Stdev | 1.41 | 153 | 1.41 | 153 | nd | nd |
| p(t-test) | | 0.023 | | 0.023 | nd | nd |
| Min | 0.141 | 0.304 | 0.141 | 0.304 | nd | nd |
| Max | 6.37 | 433 | 6.37 | 433 | nd | nd |
| n (Samp) | 41 | 8 | 41 | 8 | nd | nd |
| n (Patient) | 41 | 8 | 41 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | nd | nd | 0.65 | nd | nd | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| SE | 0.11 | nd | nd | 0.11 | nd | nd | nd | nd | nd |
| p | 0.20 | nd | nd | 0.20 | nd | nd | nd | nd | nd |
| nCohort 1 | 41 | nd | nd | 41 | nd | nd | nd | nd | nd |
| nCohort 2 | 8 | nd | nd | 8 | nd | nd | nd | nd | nd |
| Cutoff 1 | 0.781 | nd | nd | 0.781 | nd | nd | nd | nd | nd |
| Sens 1 | 75% | nd | nd | 75% | nd | nd | nd | nd | nd |
| Spec 1 | 61% | nd | nd | 61% | nd | nd | nd | nd | nd |
| Cutoff 2 | 0.464 | nd | nd | 0.464 | nd | nd | nd | nd | nd |
| Sens 2 | 88% | nd | nd | 88% | nd | nd | nd | nd | nd |
| Spec 2 | 32% | nd | nd | 32% | nd | nd | nd | nd | nd |
| Cutoff 3 | 0.278 | nd | nd | 0.278 | nd | nd | nd | nd | nd |
| Sens 3 | 100% | nd | nd | 100% | nd | nd | nd | nd | nd |
| Spec 3 | 10% | nd | nd | 10% | nd | nd | nd | nd | nd |
| Cutoff 4 | 0.961 | nd | nd | 0.961 | nd | nd | nd | nd | nd |
| Sens 4 | 50% | nd | nd | 50% | nd | nd | nd | nd | nd |
| Spec 4 | 71% | nd | nd | 71% | nd | nd | nd | nd | nd |
| Cutoff 5 | 1.31 | nd | nd | 1.31 | nd | nd | nd | nd | nd |
| Sens 5 | 25% | nd | nd | 25% | nd | nd | nd | nd | nd |
| Spec 5 | 80% | nd | nd | 80% | nd | nd | nd | nd | nd |
| Cutoff 6 | 3.42 | nd | nd | 3.42 | nd | nd | nd | nd | nd |
| Sens 6 | 12% | nd | nd | 12% | nd | nd | nd | nd | nd |
| Spec 6 | 90% | nd | nd | 90% | nd | nd | nd | nd | nd |
| OR Quart 2 | 1.0 | nd | nd | 1.0 | nd | nd | nd | nd | nd |
| p Value | 1.0 | nd | nd | 1.0 | nd | nd | nd | nd | nd |
| 95% CI of OR Quart2 | 0.055 18 | nd | nd | 0.055 18 | nd | nd | nd | nd | nd |
| OR Quart 3 | 3.7 | nd | nd | 3.7 | nd | nd | nd | nd | nd |
| p Value | 0.29 | nd | nd | 0.29 | nd | nd | nd | nd | nd |
| 95% CI of OR Quart3 | 0.32 42 | nd | nd | 0.32 42 | nd | nd | nd | nd | nd |
| OR Quart 4 | 3.3 | nd | nd | 3.3 | nd | nd | nd | nd | nd |
| p Value | 0.33 | nd | nd | 0.33 | nd | nd | nd | nd | nd |
| 95% CI of OR Quart4 | 0.29 37 | nd | nd | 0.29 37 | nd | nd | nd | nd | nd |

## Poly [ADP-ribose] polymerase 1 (cleaved)

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 0.0140 | 1.00E-9 | 0.0140 | 1.00E-9 | 1.00E-9 |
| Average | 0.00479 | 0.0101 | 0.00479 | 0.00956 | 0.00479 | 0.00605 |
| Stdev | 0.00701 | 0.00857 | 0.00701 | 0.00900 | 0.00701 | 0.00754 |
| p(t-test) |  | 0.018 |  | 0.034 |  | 0.65 |
| Min | 1.00E-9 | 1.00E-9 | 1.00E-9 | 1.00E-9 | 1.00E-9 | 1.00E-9 |
| Max | 0.0357 | 0.0253 | 0.0357 | 0.0253 | 0.0357 | 0.0144 |
| n (Samp) | 97 | 12 | 97 | 12 | 97 | 7 |
| n (Patient) | 97 | 12 | 97 | 12 | 97 | 7 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 0.00990 | 1.00E-9 | 0.00691 | nd | nd |
| Average | 0.00475 | 0.00992 | 0.00475 | 0.00892 | nd | nd |
| Stdev | 0.00651 | 0.00984 | 0.00651 | 0.0106 | nd | nd |
| p(t-test) |  | 0.063 |  | 0.13 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 1.00E-9 | 1.00E-9 | 1.00E-9 | 1.00E-9 | nd | nd |
| Max | 0.0357 | 0.0253 | 0.0357 | 0.0253 | nd | nd |
| n (Samp) | 159 | 6 | 159 | 6 | nd | nd |
| n (Patient) | 159 | 6 | 159 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 0.0140 | 1.00E-9 | 0.0140 | 1.00E-9 | 0.00691 |
| Average | 0.00380 | 0.00938 | 0.00380 | 0.00938 | 0.00380 | 0.00706 |
| Stdev | 0.00658 | 0.00789 | 0.00658 | 0.00789 | 0.00658 | 0.00773 |
| p(t-test) | | 0.027 | | 0.027 | | 0.25 |
| Min | 1.00E-9 | 1.00E-9 | 1.00E-9 | 1.00E-9 | 1.00E-9 | 1.00E-9 |
| Max | 0.0357 | 0.0183 | 0.0357 | 0.0183 | 0.0357 | 0.0144 |
| n (Samp) | 84 | 8 | 84 | 8 | 84 | 6 |
| n (Patient) | 84 | 8 | 84 | 8 | 84 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | 0.65 | 0.69 | 0.64 | 0.58 | 0.69 | 0.52 | nd | 0.60 |
| SE | 0.090 | 0.12 | 0.11 | 0.091 | 0.12 | 0.11 | 0.11 | nd | 0.13 |
| p | 0.059 | 0.23 | 0.081 | 0.13 | 0.51 | 0.081 | 0.87 | nd | 0.43 |
| nCohort 1 | 97 | 159 | 84 | 97 | 159 | 84 | 97 | nd | 84 |
| nCohort 2 | 12 | 6 | 8 | 12 | 6 | 8 | 7 | nd | 6 |
| Cutoff 1 | 0 | 0 | 0 | 0 | 0 | 0 | 0 | nd | 0 |
| Sens 1 | 100% | 100% | 100% | 100% | 100% | 100% | 100% | nd | 100% |
| Spec 1 | 0% | 0% | 0% | 0% | 0% | 0% | 0% | nd | 0% |
| Cutoff 2 | 0 | 0 | 0 | 0 | 0 | 0 | 0 | nd | 0 |
| Sens 2 | 100% | 100% | 100% | 100% | 100% | 100% | 100% | nd | 100% |
| Spec 2 | 0% | 0% | 0% | 0% | 0% | 0% | 0% | nd | 0% |
| Cutoff 3 | 0 | 0 | 0 | 0 | 0 | 0 | 0 | nd | 0 |
| Sens 3 | 100% | 100% | 100% | 100% | 100% | 100% | 100% | nd | 100% |
| Spec 3 | 0% | 0% | 0% | 0% | 0% | 0% | 0% | nd | 0% |
| Cutoff 4 | 0.00598 | 0.00598 | 0.00471 | 0.00598 | 0.00598 | 0.00471 | 0.00598 | nd | 0.00471 |
| Sens 4 | 58% | 50% | 62% | 58% | 50% | 62% | 43% | nd | 50% |
| Spec 4 | 70% | 71% | 74% | 70% | 71% | 74% | 70% | nd | 74% |
| Cutoff 5 | 0.0144 | 0.0144 | 0.0120 | 0.0144 | 0.0144 | 0.0120 | 0.0144 | nd | 0.0120 |
| Sens 5 | 17% | 17% | 62% | 17% | 17% | 62% | 0% | nd | 50% |
| Spec 5 | 94% | 96% | 81% | 94% | 96% | 81% | 94% | nd | 81% |
| Cutoff 6 | 0.0144 | 0.0144 | 0.0144 | 0.0144 | 0.0144 | 0.0144 | 0.0144 | nd | 0.0144 |
| Sens 6 | 17% | 17% | 12% | 17% | 17% | 12% | 0% | nd | 0% |
| Spec 6 | 94% | 96% | 99% | 94% | 96% | 99% | 94% | nd | 99% |
| OR Quart 2 | 1.0 | 1.0 | 2.1 | 1.6 | 2.1 | 2.1 | 3.3 | nd | 2.0 |
| p Value | 1.0 | 1.0 | 0.56 | 0.64 | 0.56 | 0.56 | 0.32 | nd | 0.58 |
| 95% CI of OR Quart2 | 0.13 | 0.060 | 0.18 | 0.24 | 0.18 | 0.18 | 0.32 | nd | 0.17 |
| | 7.7 | 17 | 25 | 10 | 24 | 25 | 34 | nd | 24 |
| OR Quart 3 | 1.0 | 1.0 | 0 | 0 | 0 | 0 | 1.0 | nd | 0 |
| p Value | 1.0 | 1.0 | na | na | na | na | 1.0 | nd | na |
| 95% CI of OR Quart3 | 0.13 | 0.060 | na | na | na | na | 0.059 | nd | na |
| | 7.7 | 17 | na | na | na | na | 17 | nd | na |
| OR Quart 4 | 3.4 | 3.1 | 6.1 | 4.2 | 3.1 | 6.1 | 2.1 | nd | 3.1 |
| p Value | 0.16 | 0.34 | 0.11 | 0.095 | 0.34 | 0.11 | 0.56 | nd | 0.34 |
| 95% CI of OR Quart4 | 0.62 | 0.31 | 0.65 | 0.78 | 0.31 | 0.65 | 0.18 | nd | 0.30 |
| | 19 | 31 | 57 | 22 | 31 | 57 | 25 | nd | 33 |

## KSP-Cadherin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.23 | 2.26 | 1.23 | 2.26 | nd | nd |
| Average | 1.66 | 2.48 | 1.66 | 2.48 | nd | nd |
| Stdev | 1.71 | 1.19 | 1.71 | 1.19 | nd | nd |
| p(t-test) | | 0.17 | | 0.17 | nd | nd |
| Min | 0.00263 | 0.562 | 0.00263 | 0.562 | nd | nd |
| Max | 11.9 | 4.23 | 11.9 | 4.23 | nd | nd |
| n (Samp) | 68 | 9 | 68 | 9 | nd | nd |
| n (Patient) | 68 | 9 | 68 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.17 | 1.87 | 1.17 | 1.87 | nd | nd |
| Average | 1.76 | 2.07 | 1.76 | 2.07 | nd | nd |
| Stdev | 1.84 | 1.22 | 1.84 | 1.22 | nd | nd |
| p(t-test) | | 0.69 | | 0.69 | nd | nd |
| Min | 0.00263 | 0.562 | 0.00263 | 0.562 | nd | nd |
| Max | 11.9 | 4.23 | 11.9 | 4.23 | nd | nd |
| n (Samp) | 59 | 6 | 59 | 6 | nd | nd |
| n (Patient) | 59 | 6 | 59 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.74 | nd | 0.64 | 0.74 | nd | 0.64 | nd | nd | nd |
| SE | 0.099 | nd | 0.13 | 0.099 | nd | 0.13 | nd | nd | nd |
| p | 0.017 | nd | 0.29 | 0.017 | nd | 0.29 | nd | nd | nd |
| nCohort 1 | 68 | nd | 59 | 68 | nd | 59 | nd | nd | nd |
| nCohort 2 | 9 | nd | 6 | 9 | nd | 6 | nd | nd | nd |
| Cutoff 1 | 1.61 | nd | 1.51 | 1.61 | nd | 1.51 | nd | nd | nd |
| Sens 1 | 78% | nd | 83% | 78% | nd | 83% | nd | nd | nd |
| Spec 1 | 60% | nd | 59% | 60% | nd | 59% | nd | nd | nd |
| Cutoff 2 | 1.56 | nd | 1.51 | 1.56 | nd | 1.51 | nd | nd | nd |
| Sens 2 | 89% | nd | 83% | 89% | nd | 83% | nd | nd | nd |
| Spec 2 | 60% | nd | 59% | 60% | nd | 59% | nd | nd | nd |
| Cutoff 3 | 0.466 | nd | 0.494 | 0.466 | nd | 0.494 | nd | nd | nd |
| Sens 3 | 100% | nd | 100% | 100% | nd | 100% | nd | nd | nd |
| Spec 3 | 19% | nd | 19% | 19% | nd | 19% | nd | nd | nd |
| Cutoff 4 | 1.99 | nd | 2.08 | 1.99 | nd | 2.08 | nd | nd | nd |
| Sens 4 | 67% | nd | 50% | 67% | nd | 50% | nd | nd | nd |
| Spec 4 | 71% | nd | 71% | 71% | nd | 71% | nd | nd | nd |
| Cutoff 5 | 2.24 | nd | 2.54 | 2.24 | nd | 2.54 | nd | nd | nd |
| Sens 5 | 56% | nd | 17% | 56% | nd | 17% | nd | nd | nd |
| Spec 5 | 81% | nd | 81% | 81% | nd | 81% | nd | nd | nd |
| Cutoff 6 | 3.17 | nd | 3.77 | 3.17 | nd | 3.77 | nd | nd | nd |
| Sens 6 | 22% | nd | 17% | 22% | nd | 17% | nd | nd | nd |
| Spec 6 | 91% | nd | 92% | 91% | nd | 92% | nd | nd | nd |
| OR Quart 2 | 0 | nd | 0 | 0 | nd | 0 | nd | nd | nd |
| p Value | na | nd | na | na | nd | na | nd | nd | nd |
| 95% CI of OR Quart2 | na | nd | na | na | nd | na | nd | nd | nd |
| OR Quart 3 | 3.4 | nd | 3.5 | 3.4 | nd | 3.5 | nd | nd | nd |
| p Value | 0.31 | nd | 0.31 | 0.31 | nd | 0.31 | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.32 | nd | 0.32 | 0.32 | nd | 0.32 | nd | nd | nd |
| OR Quart3 | 36 | nd | 37 | 36 | nd | 37 | nd | nd | nd |
| OR Quart 4 | 6.0 | nd | 2.0 | 6.0 | nd | 2.0 | nd | nd | nd |
| p Value | 0.12 | nd | 0.59 | 0.12 | nd | 0.59 | nd | nd | nd |
| 95% CI of | 0.63 | nd | 0.16 | 0.63 | nd | 0.16 | nd | nd | nd |
| OR Quart4 | 57 | nd | 24 | 57 | nd | 24 | nd | nd | nd |

## Tumor necrosis factor receptor superfamily member 10B

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.141 | 0.904 | 0.141 | 0.904 | nd | nd |
| Average | 0.957 | 1.95 | 0.957 | 1.95 | nd | nd |
| Stdev | 1.61 | 2.64 | 1.61 | 2.64 | nd | nd |
| p(t-test) | | 0.16 | | 0.16 | nd | nd |
| Min | 0.00360 | 0.104 | 0.00360 | 0.104 | nd | nd |
| Max | 6.71 | 7.54 | 6.71 | 7.54 | nd | nd |
| n (Samp) | 41 | 8 | 41 | 8 | nd | nd |
| n (Patient) | 41 | 8 | 41 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | nd | nd | 0.69 | nd | nd | nd | nd | nd |
| SE | 0.11 | nd | nd | 0.11 | nd | nd | nd | nd | nd |
| p | 0.092 | nd | nd | 0.092 | nd | nd | nd | nd | nd |
| nCohort 1 | 41 | nd | nd | 41 | nd | nd | nd | nd | nd |
| nCohort 2 | 8 | nd | nd | 8 | nd | nd | nd | nd | nd |
| Cutoff 1 | 0.141 | nd | nd | 0.141 | nd | nd | nd | nd | nd |
| Sens 1 | 75% | nd | nd | 75% | nd | nd | nd | nd | nd |
| Spec 1 | 51% | nd | nd | 51% | nd | nd | nd | nd | nd |
| Cutoff 2 | 0.107 | nd | nd | 0.107 | nd | nd | nd | nd | nd |
| Sens 2 | 88% | nd | nd | 88% | nd | nd | nd | nd | nd |
| Spec 2 | 49% | nd | nd | 49% | nd | nd | nd | nd | nd |
| Cutoff 3 | 0.0796 | nd | nd | 0.0796 | nd | nd | nd | nd | nd |
| Sens 3 | 100% | nd | nd | 100% | nd | nd | nd | nd | nd |
| Spec 3 | 41% | nd | nd | 41% | nd | nd | nd | nd | nd |
| Cutoff 4 | 0.693 | nd | nd | 0.693 | nd | nd | nd | nd | nd |
| Sens 4 | 50% | nd | nd | 50% | nd | nd | nd | nd | nd |
| Spec 4 | 71% | nd | nd | 71% | nd | nd | nd | nd | nd |
| Cutoff 5 | 1.56 | nd | nd | 1.56 | nd | nd | nd | nd | nd |
| Sens 5 | 38% | nd | nd | 38% | nd | nd | nd | nd | nd |
| Spec 5 | 80% | nd | nd | 80% | nd | nd | nd | nd | nd |
| Cutoff 6 | 3.31 | nd | nd | 3.31 | nd | nd | nd | nd | nd |
| Sens 6 | 25% | nd | nd | 25% | nd | nd | nd | nd | nd |
| Spec 6 | 90% | nd | nd | 90% | nd | nd | nd | nd | nd |
| OR Quart 2 | >4.0 | nd | nd | >4.0 | nd | nd | nd | nd | nd |
| p Value | <0.26 | nd | nd | <0.26 | nd | nd | nd | nd | nd |
| 95% CI of | >0.35 | nd | nd | >0.35 | nd | nd | nd | nd | nd |
| OR Quart2 | na | nd | nd | na | nd | nd | nd | nd | nd |
| OR Quart 3 | >1.1 | nd | nd | >1.1 | nd | nd | nd | nd | nd |
| p Value | <0.95 | nd | nd | <0.95 | nd | nd | nd | nd | nd |
| 95% CI of | >0.061 | nd | nd | >0.061 | nd | nd | nd | nd | nd |
| OR Quart3 | na | nd | nd | na | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 4 | >5.3 | nd | nd | >5.3 | nd | nd | nd | nd | nd |
| p Value | <0.16 | nd | nd | <0.16 | nd | nd | nd | nd | nd |
| 95% CI of | >0.51 | nd | nd | >0.51 | nd | nd | nd | nd | nd |
| OR Quart4 | na | nd | nd | na | nd | nd | nd | nd | nd |

Table 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

**Apolipoprotein A-II**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 50600 | 49600 | 50600 | 44300 | 50600 | 54300 |
| Average | 51700 | 52000 | 51700 | 50700 | 51700 | 57500 |
| Stdev | 22200 | 21100 | 22200 | 36000 | 22200 | 32700 |
| p(t-test) | | 0.94 | | 0.83 | | 0.29 |
| Min | 5450 | 7970 | 5450 | 7680 | 5450 | 8560 |
| Max | 105000 | 97000 | 105000 | 253000 | 105000 | 152000 |
| n (Samp) | 105 | 45 | 105 | 50 | 105 | 24 |
| n (Patient) | 97 | 45 | 97 | 50 | 97 | 24 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 49500 | 59900 | 49500 | 50300 | 49500 | 57100 |
| Average | 51700 | 60100 | 51700 | 64100 | 51700 | 67500 |
| Stdev | 26400 | 18100 | 26400 | 55400 | 26400 | 44100 |
| p(t-test) | | 0.26 | | 0.097 | | 0.062 |
| Min | 1810 | 34500 | 1810 | 12100 | 1810 | 10900 |
| Max | 253000 | 95300 | 253000 | 251000 | 253000 | 176000 |
| n (Samp) | 246 | 13 | 246 | 16 | 246 | 11 |
| n (Patient) | 160 | 13 | 160 | 16 | 160 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 50200 | 49600 | 50200 | 43800 | 50200 | 51400 |
| Average | 52500 | 51300 | 52500 | 51600 | 52500 | 58800 |
| Stdev | 20200 | 21600 | 20200 | 38000 | 20200 | 32900 |
| p(t-test) | | 0.76 | | 0.86 | | 0.25 |
| Min | 8680 | 7970 | 8680 | 7680 | 8680 | 8560 |
| Max | 123000 | 97000 | 123000 | 253000 | 123000 | 152000 |
| n (Samp) | 96 | 40 | 96 | 44 | 96 | 21 |
| n (Patient) | 84 | 40 | 84 | 44 | 84 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.63 | 0.48 | 0.45 | 0.55 | 0.44 | 0.55 | 0.60 | 0.55 |
| SE | 0.052 | 0.085 | 0.055 | 0.050 | 0.076 | 0.053 | 0.066 | 0.092 | 0.071 |
| p | 0.89 | 0.12 | 0.75 | 0.35 | 0.55 | 0.24 | 0.50 | 0.27 | 0.49 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 105 | 246 | 96 | 105 | 246 | 96 | 105 | 246 | 96 |
| nCohort 2 | 45 | 13 | 40 | 50 | 16 | 44 | 24 | 11 | 21 |
| Cutoff 1 | 39500 | 43000 | 39500 | 35500 | 37700 | 35000 | 40700 | 46100 | 40900 |
| Sens 1 | 71% | 77% | 70% | 70% | 75% | 70% | 71% | 73% | 71% |
| Spec 1 | 33% | 40% | 28% | 24% | 31% | 19% | 35% | 46% | 31% |
| Cutoff 2 | 33000 | 42600 | 32500 | 29600 | 35500 | 25400 | 29600 | 37000 | 35000 |
| Sens 2 | 80% | 85% | 80% | 80% | 81% | 82% | 83% | 82% | 81% |
| Spec 2 | 22% | 39% | 16% | 19% | 24% | 6% | 19% | 29% | 19% |
| Cutoff 3 | 23400 | 37000 | 23400 | 20800 | 14000 | 20800 | 14000 | 36200 | 21600 |
| Sens 3 | 91% | 92% | 90% | 90% | 94% | 91% | 92% | 91% | 90% |
| Spec 3 | 10% | 29% | 5% | 6% | 5% | 3% | 4% | 27% | 4% |
| Cutoff 4 | 61300 | 61500 | 61500 | 61300 | 61500 | 61500 | 61300 | 61500 | 61500 |
| Sens 4 | 33% | 46% | 30% | 32% | 44% | 34% | 46% | 36% | 48% |
| Spec 4 | 70% | 70% | 71% | 70% | 70% | 71% | 70% | 70% | 71% |
| Cutoff 5 | 69000 | 69400 | 67400 | 69000 | 69400 | 67400 | 69000 | 69400 | 67400 |
| Sens 5 | 24% | 38% | 22% | 18% | 38% | 16% | 33% | 36% | 43% |
| Spec 5 | 80% | 80% | 80% | 80% | 80% | 80% | 80% | 80% | 80% |
| Cutoff 6 | 82000 | 82800 | 77900 | 82000 | 82800 | 77900 | 82000 | 82800 | 77900 |
| Sens 6 | 9% | 8% | 12% | 8% | 12% | 11% | 21% | 36% | 19% |
| Spec 6 | 90% | 90% | 91% | 90% | 90% | 91% | 90% | 90% | 91% |
| OR Quart 2 | 1.6 | 4.1 | 0.74 | 0.60 | 0.98 | 0.76 | 0.80 | 4.2 | 0.61 |
| p Value | 0.36 | 0.21 | 0.58 | 0.32 | 0.98 | 0.60 | 0.74 | 0.21 | 0.49 |
| 95% CI of | 0.59 | 0.45 | 0.25 | 0.22 | 0.24 | 0.27 | 0.22 | 0.46 | 0.15 |
| OR Quart2 | 4.2 | 38 | 2.2 | 1.6 | 4.1 | 2.1 | 3.0 | 39 | 2.5 |
| OR Quart 3 | 0.87 | 3.0 | 1.1 | 1.1 | 0.48 | 0.65 | 0.62 | 2.0 | 0.28 |
| p Value | 0.79 | 0.34 | 0.79 | 0.81 | 0.41 | 0.42 | 0.49 | 0.57 | 0.15 |
| 95% CI of | 0.31 | 0.31 | 0.41 | 0.44 | 0.086 | 0.22 | 0.16 | 0.18 | 0.052 |
| OR Quart3 | 2.5 | 30 | 3.2 | 2.8 | 2.7 | 1.9 | 2.4 | 23 | 1.5 |
| OR Quart 4 | 1.2 | 5.2 | 1.1 | 1.2 | 1.5 | 1.8 | 1.6 | 4.1 | 1.6 |
| p Value | 0.67 | 0.14 | 0.79 | 0.75 | 0.53 | 0.22 | 0.42 | 0.21 | 0.41 |
| 95% CI of | 0.46 | 0.60 | 0.41 | 0.46 | 0.41 | 0.69 | 0.50 | 0.45 | 0.50 |
| OR Quart4 | 3.4 | 46 | 3.2 | 3.0 | 5.7 | 4.9 | 5.2 | 38 | 5.4 |

## Bcl2 antagonist of cell death

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.432 | 0.221 | nd | nd | nd | nd |
| Average | 0.590 | 0.255 | nd | nd | nd | nd |
| Stdev | 0.478 | 0.204 | nd | nd | nd | nd |
| p(t-test) | | 0.10 | nd | nd | nd | nd |
| Min | 0.0873 | 0.0786 | nd | nd | nd | nd |
| Max | 2.49 | 0.651 | nd | nd | nd | nd |
| n (Samp) | 32 | 6 | nd | nd | nd | nd |
| n (Patient) | 20 | 6 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.596 | 0.213 | nd | nd | nd | nd |
| Average | 0.643 | 0.243 | nd | nd | nd | nd |
| Stdev | 0.503 | 0.174 | nd | nd | nd | nd |
| p(t-test) | | 0.035 | nd | nd | nd | nd |
| Min | 0.0873 | 0.0786 | nd | nd | nd | nd |
| Max | 2.49 | 0.651 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 27 | 8 | nd | nd | nd | nd |
| n (Patient) | 17 | 8 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.21 | nd | 0.19 | nd | nd | nd | nd | nd | nd |
| SE | 0.12 | nd | 0.098 | nd | nd | nd | nd | nd | nd |
| p | 0.012 | nd | 0.0013 | nd | nd | nd | nd | nd | nd |
| nCohort 1 | 32 | nd | 27 | nd | nd | nd | nd | nd | nd |
| nCohort 2 | 6 | nd | 8 | nd | nd | nd | nd | nd | nd |
| Cutoff 1 | 0.114 | nd | 0.181 | nd | nd | nd | nd | nd | nd |
| Sens 1 | 83% | nd | 75% | nd | nd | nd | nd | nd | nd |
| Spec 1 | 6% | nd | 15% | nd | nd | nd | nd | nd | nd |
| Cutoff 2 | 0.114 | nd | 0.114 | nd | nd | nd | nd | nd | nd |
| Sens 2 | 83% | nd | 88% | nd | nd | nd | nd | nd | nd |
| Spec 2 | 6% | nd | 7% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |
| Spec 3 | 0% | nd | 0% | nd | nd | nd | nd | nd | nd |
| Cutoff 4 | 0.703 | nd | 0.771 | nd | nd | nd | nd | nd | nd |
| Sens 4 | 0% | nd | 0% | nd | nd | nd | nd | nd | nd |
| Spec 4 | 72% | nd | 70% | nd | nd | nd | nd | nd | nd |
| Cutoff 5 | 0.991 | nd | 1.01 | nd | nd | nd | nd | nd | nd |
| Sens 5 | 0% | nd | 0% | nd | nd | nd | nd | nd | nd |
| Spec 5 | 81% | nd | 81% | nd | nd | nd | nd | nd | nd |
| Cutoff 6 | 1.05 | nd | 1.13 | nd | nd | nd | nd | nd | nd |
| Sens 6 | 0% | nd | 0% | nd | nd | nd | nd | nd | nd |
| Spec 6 | 91% | nd | 93% | nd | nd | nd | nd | nd | nd |
| OR Quart 2 | >1.2 | nd | >1.1 | nd | nd | nd | nd | nd | nd |
| p Value | <0.88 | nd | <0.94 | nd | nd | nd | nd | nd | nd |
| 95% CI of OR Quart2 | >0.067 na | nd | >0.060 na | nd | nd | nd | nd | nd | nd |
| OR Quart 3 | >2.5 | nd | >4.5 | nd | nd | nd | nd | nd | nd |
| p Value | <0.49 | nd | <0.24 | nd | nd | nd | nd | nd | nd |
| 95% CI of OR Quart3 | >0.19 na | nd | >0.37 na | nd | nd | nd | nd | nd | nd |
| OR Quart 4 | >5.0 | nd | >9.0 | nd | nd | nd | nd | nd | nd |
| p Value | <0.20 | nd | <0.083 | nd | nd | nd | nd | nd | nd |
| 95% CI of OR Quart4 | >0.42 na | nd | >0.75 na | nd | nd | nd | nd | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 25.5 | 14.8 | nd | nd | nd | nd |
| Average | 44.5 | 24.6 | nd | nd | nd | nd |
| Stdev | 60.6 | 24.9 | nd | nd | nd | nd |
| p(t-test) | | 0.27 | nd | nd | nd | nd |
| Min | 0.400 | 4.59 | nd | nd | nd | nd |
| Max | 366 | 78.6 | nd | nd | nd | nd |
| n (Samp) | 57 | 12 | nd | nd | nd | nd |
| n (Patient) | 37 | 12 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.8 | 13.2 | nd | nd | nd | nd |
| Average | 42.5 | 12.5 | nd | nd | nd | nd |
| Stdev | 58.2 | 7.24 | nd | nd | nd | nd |
| p(t-test) | | 0.21 | nd | nd | nd | nd |
| Min | 0.400 | 4.32 | nd | nd | nd | nd |
| Max | 366 | 21.7 | nd | nd | nd | nd |
| n (Samp) | 89 | 6 | nd | nd | nd | nd |
| n (Patient) | 61 | 6 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 30.1 | 9.06 | nd | nd | nd | nd |
| Average | 50.3 | 20.6 | nd | nd | nd | nd |
| Stdev | 67.6 | 24.4 | nd | nd | nd | nd |
| p(t-test) | | 0.12 | nd | nd | nd | nd |
| Min | 6.05 | 3.79 | nd | nd | nd | nd |
| Max | 366 | 78.6 | nd | nd | nd | nd |
| n (Samp) | 42 | 14 | nd | nd | nd | nd |
| n (Patient) | 27 | 14 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.39 | 0.28 | 0.26 | nd | nd | nd | nd | nd | nd |
| SE | 0.094 | 0.12 | 0.083 | nd | nd | nd | nd | nd | nd |
| p | 0.25 | 0.077 | 0.0038 | nd | nd | nd | nd | nd | nd |
| nCohort 1 | 57 | 89 | 42 | nd | nd | nd | nd | nd | nd |
| nCohort 2 | 12 | 6 | 14 | nd | nd | nd | nd | nd | nd |
| Cutoff 1 | 8.91 | 3.65 | 6.05 | nd | nd | nd | nd | nd | nd |
| Sens 1 | 75% | 100% | 71% | nd | nd | nd | nd | nd | nd |
| Spec 1 | 21% | 7% | 2% | nd | nd | nd | nd | nd | nd |
| Cutoff 2 | 8.22 | 3.65 | 4.59 | nd | nd | nd | nd | nd | nd |
| Sens 2 | 83% | 100% | 86% | nd | nd | nd | nd | nd | nd |
| Spec 2 | 19% | 7% | 0% | nd | nd | nd | nd | nd | nd |
| Cutoff 3 | 4.59 | 3.65 | 3.79 | nd | nd | nd | nd | nd | nd |
| Sens 3 | 92% | 100% | 93% | nd | nd | nd | nd | nd | nd |
| Spec 3 | 9% | 7% | 0% | nd | nd | nd | nd | nd | nd |
| Cutoff 4 | 46.1 | 45.3 | 49.3 | nd | nd | nd | nd | nd | nd |
| Sens 4 | 17% | 0% | 14% | nd | nd | nd | nd | nd | nd |
| Spec 4 | 70% | 71% | 71% | nd | nd | nd | nd | nd | nd |
| Cutoff 5 | 54.5 | 57.5 | 54.5 | nd | nd | nd | nd | nd | nd |
| Sens 5 | 17% | 0% | 14% | nd | nd | nd | nd | nd | nd |
| Spec 5 | 81% | 81% | 81% | nd | nd | nd | nd | nd | nd |
| Cutoff 6 | 90.6 | 94.1 | 106 | nd | nd | nd | nd | nd | nd |
| Sens 6 | 0% | 0% | 0% | nd | nd | nd | nd | nd | nd |
| Spec 6 | 91% | 91% | 90% | nd | nd | nd | nd | nd | nd |
| OR Quart 2 | 1.1 | >1.0 | 1.0 | nd | nd | nd | nd | nd | nd |
| p Value | 0.95 | <0.98 | 1.0 | nd | nd | nd | nd | nd | nd |
| 95% CI of OR Quart2 | 0.13 8.6 | >0.062 na | 0.12 8.3 | nd | nd | nd | nd | nd | nd |
| OR Quart 3 | 1.7 | >3.4 | 1.0 | nd | nd | nd | nd | nd | nd |
| p Value | 0.58 | <0.30 | 1.0 | nd | nd | nd | nd | nd | nd |
| 95% CI of OR Quart3 | 0.25 12 | >0.33 na | 0.12 8.3 | nd | nd | nd | nd | nd | nd |
| OR Quart 4 | 3.3 | >2.3 | 8.0 | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.19 | <0.51 | 0.026 | nd | nd | nd | nd | nd | nd |
| 95% CI of OR Quart4 | 0.55 | >0.19 | 1.3 | nd | nd | nd | nd | nd | nd |
| | 20 | na | 50 | nd | nd | nd | nd | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72700 | 88200 | 72700 | 92800 | 72700 | 98500 |
| Average | 102000 | 129000 | 102000 | 127000 | 102000 | 134000 |
| Stdev | 106000 | 88400 | 106000 | 90200 | 106000 | 91700 |
| p(t-test) | | 0.26 | | 0.28 | | 0.28 |
| Min | 14500 | 34900 | 14500 | 3320 | 14500 | 25800 |
| Max | 621000 | 334000 | 621000 | 340000 | 621000 | 283000 |
| n (Samp) | 52 | 28 | 52 | 31 | 52 | 16 |
| n (Patient) | 50 | 28 | 50 | 31 | 50 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 86800 | 130000 | 86800 | 73000 | 86800 | 109000 |
| Average | 116000 | 145000 | 116000 | 120000 | 116000 | 116000 |
| Stdev | 94600 | 90700 | 94600 | 94900 | 94600 | 73700 |
| p(t-test) | | 0.35 | | 0.87 | | 0.99 |
| Min | 3320 | 34900 | 3320 | 30700 | 3320 | 56100 |
| Max | 621000 | 334000 | 621000 | 340000 | 621000 | 283000 |
| n (Samp) | 123 | 10 | 123 | 16 | 123 | 8 |
| n (Patient) | 96 | 10 | 96 | 16 | 96 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 81200 | 88200 | 81200 | 103000 | 81200 | 89400 |
| Average | 119000 | 122000 | 119000 | 122000 | 119000 | 139000 |
| Stdev | 114000 | 85000 | 114000 | 78600 | 114000 | 94300 |
| p(t-test) | | 0.89 | | 0.89 | | 0.59 |
| Min | 39700 | 38100 | 39700 | 3320 | 39700 | 25800 |
| Max | 621000 | 314000 | 621000 | 300000 | 621000 | 277000 |
| n (Samp) | 51 | 22 | 51 | 27 | 51 | 11 |
| n (Patient) | 44 | 22 | 44 | 27 | 44 | 11 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.63 | 0.54 | 0.59 | 0.47 | 0.57 | 0.62 | 0.56 | 0.59 |
| SE | 0.067 | 0.098 | 0.075 | 0.065 | 0.078 | 0.069 | 0.083 | 0.11 | 0.098 |
| p | 0.066 | 0.18 | 0.57 | 0.15 | 0.72 | 0.32 | 0.13 | 0.57 | 0.38 |
| nCohort 1 | 52 | 123 | 51 | 52 | 123 | 51 | 52 | 123 | 51 |
| nCohort 2 | 28 | 10 | 22 | 31 | 16 | 27 | 16 | 8 | 11 |
| Cutoff 1 | 78500 | 79100 | 75400 | 62800 | 40700 | 67700 | 61700 | 61700 | 79100 |
| Sens 1 | 71% | 70% | 73% | 71% | 75% | 70% | 75% | 75% | 73% |
| Spec 1 | 56% | 46% | 41% | 42% | 9% | 37% | 40% | 31% | 49% |
| Cutoff 2 | 52700 | 78500 | 52700 | 47600 | 39700 | 54300 | 57000 | 57000 | 70100 |
| Sens 2 | 82% | 80% | 82% | 81% | 81% | 81% | 81% | 88% | 82% |
| Spec 2 | 21% | 46% | 12% | 17% | 8% | 14% | 29% | 26% | 39% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 3 | 40700 | 72100 | 47600 | 39700 | 31800 | 39700 | 48100 | 54700 | 47600 |
| Sens 3 | 93% | 90% | 91% | 90% | 94% | 93% | 94% | 100% | 91% |
| Spec 3 | 10% | 41% | 10% | 8% | 6% | 2% | 19% | 23% | 10% |
| Cutoff 4 | 97000 | 131000 | 100000 | 97000 | 131000 | 100000 | 97000 | 131000 | 100000 |
| Sens 4 | 46% | 50% | 32% | 48% | 38% | 52% | 50% | 25% | 45% |
| Spec 4 | 71% | 71% | 71% | 71% | 71% | 71% | 71% | 71% | 71% |
| Cutoff 5 | 114000 | 165000 | 138000 | 114000 | 165000 | 138000 | 114000 | 165000 | 138000 |
| Sens 5 | 36% | 40% | 27% | 42% | 38% | 33% | 38% | 12% | 36% |
| Spec 5 | 81% | 80% | 80% | 81% | 80% | 80% | 81% | 80% | 80% |
| Cutoff 6 | 153000 | 243000 | 242000 | 153000 | 243000 | 242000 | 153000 | 243000 | 242000 |
| Sens 6 | 32% | 20% | 18% | 39% | 6% | 11% | 31% | 12% | 27% |
| Spec 6 | 90% | 90% | 90% | 90% | 90% | 90% | 90% | 90% | 90% |
| OR Quart 2 | 0.78 | 3.2 | 0.74 | 0.47 | 0.14 | 0.54 | 1.0 | 2.0 | 0.93 |
| p Value | 0.72 | 0.33 | 0.70 | 0.27 | 0.079 | 0.41 | 1.0 | 0.58 | 0.94 |
| 95% CI of OR Quart2 | 0.19 3.1 | 0.32 32 | 0.16 3.4 | 0.12 1.8 | 0.016 1.3 | 0.13 2.3 | 0.17 5.8 | 0.17 23 | 0.11 7.6 |
| OR Quart 3 | 1.3 | 2.1 | 1.7 | 0.60 | 0.62 | 1.6 | 1.4 | 4.3 | 1.6 |
| p Value | 0.74 | 0.56 | 0.48 | 0.44 | 0.50 | 0.50 | 0.67 | 0.21 | 0.63 |
| 95% CI of OR Quart3 | 0.33 4.7 | 0.18 24 | 0.41 6.7 | 0.16 2.2 | 0.16 2.4 | 0.42 5.9 | 0.27 7.7 | 0.45 41 | 0.23 11 |
| OR Quart 4 | 2.3 | 4.3 | 1.2 | 2.0 | 0.83 | 1.8 | 2.5 | 0.97 | 2.2 |
| p Value | 0.20 | 0.21 | 0.80 | 0.27 | 0.78 | 0.39 | 0.25 | 0.98 | 0.42 |
| 95% CI of OR Quart4 | 0.64 8.5 | 0.45 40 | 0.29 4.9 | 0.58 6.9 | 0.23 3.0 | 0.48 6.6 | 0.52 13 | 0.058 16 | 0.33 14 |

## Cadherin-5

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 20.4 | 21.2 | nd | nd | nd | nd |
| Average | 20.5 | 23.9 | nd | nd | nd | nd |
| Stdev | 5.01 | 5.05 | nd | nd | nd | nd |
| p(t-test) |  | 0.065 | nd | nd | nd | nd |
| Min | 12.4 | 18.0 | nd | nd | nd | nd |
| Max | 35.9 | 32.1 | nd | nd | nd | nd |
| n (Samp) | 48 | 9 | nd | nd | nd | nd |
| n (Patient) | 30 | 9 | nd | nd | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 20.5 | 21.2 | nd | nd | nd | nd |
| Average | 20.7 | 23.3 | nd | nd | nd | nd |
| Stdev | 4.09 | 5.02 | nd | nd | nd | nd |
| p(t-test) |  | 0.080 | nd | nd | nd | nd |
| Min | 12.4 | 17.0 | nd | nd | nd | nd |
| Max | 29.3 | 32.1 | nd | nd | nd | nd |
| n (Samp) | 34 | 11 | nd | nd | nd | nd |
| n (Patient) | 20 | 11 | nd | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.69 | nd | 0.64 | nd | nd | nd | nd | nd | nd |
| SE | 0.10 | nd | 0.10 | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.077 | nd | 0.16 | nd | nd | nd | nd | nd | nd |
| nCohort 1 | 48 | nd | 34 | nd | nd | nd | nd | nd | nd |
| nCohort 2 | 9 | nd | 11 | nd | nd | nd | nd | nd | nd |
| Cutoff 1 | 20.6 | nd | 19.5 | nd | nd | nd | nd | nd | nd |
| Sens 1 | 78% | nd | 73% | nd | nd | nd | nd | nd | nd |
| Spec 1 | 54% | nd | 44% | nd | nd | nd | nd | nd | nd |
| Cutoff 2 | 18.9 | nd | 18.9 | nd | nd | nd | nd | nd | nd |
| Sens 2 | 89% | nd | 82% | nd | nd | nd | nd | nd | nd |
| Spec 2 | 42% | nd | 38% | nd | nd | nd | nd | nd | nd |
| Cutoff 3 | 17.1 | nd | 17.1 | nd | nd | nd | nd | nd | nd |
| Sens 3 | 100% | nd | 91% | nd | nd | nd | nd | nd | nd |
| Spec 3 | 33% | nd | 26% | nd | nd | nd | nd | nd | nd |
| Cutoff 4 | 22.6 | nd | 22.7 | nd | nd | nd | nd | nd | nd |
| Sens 4 | 44% | nd | 45% | nd | nd | nd | nd | nd | nd |
| Spec 4 | 71% | nd | 71% | nd | nd | nd | nd | nd | nd |
| Cutoff 5 | 24.0 | nd | 24.0 | nd | nd | nd | nd | nd | nd |
| Sens 5 | 44% | nd | 45% | nd | nd | nd | nd | nd | nd |
| Spec 5 | 81% | nd | 82% | nd | nd | nd | nd | nd | nd |
| Cutoff 6 | 26.5 | nd | 26.2 | nd | nd | nd | nd | nd | nd |
| Sens 6 | 33% | nd | 27% | nd | nd | nd | nd | nd | nd |
| Spec 6 | 92% | nd | 91% | nd | nd | nd | nd | nd | nd |
| OR Quart 2 | >2.3 | nd | 3.8 | nd | nd | nd | nd | nd | nd |
| p Value | <0.51 | nd | 0.29 | nd | nd | nd | nd | nd | nd |
| 95% CI of | >0.19 | nd | 0.32 | nd | nd | nd | nd | nd | nd |
| OR Quart2 | na | nd | 43 | nd | nd | nd | nd | nd | nd |
| OR Quart 3 | >3.8 | nd | 2.2 | nd | nd | nd | nd | nd | nd |
| p Value | <0.27 | nd | 0.54 | nd | nd | nd | nd | nd | nd |
| 95% CI of | >0.35 | nd | 0.17 | nd | nd | nd | nd | nd | nd |
| OR Quart3 | na | nd | 29 | nd | nd | nd | nd | nd | nd |
| OR Quart 4 | >5.1 | nd | 7.1 | nd | nd | nd | nd | nd | nd |
| p Value | <0.17 | nd | 0.10 | nd | nd | nd | nd | nd | nd |
| 95% CI of | >0.50 | nd | 0.68 | nd | nd | nd | nd | nd | nd |
| OR Quart4 | na | nd | 75 | nd | nd | nd | nd | nd | nd |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 208 | 138 | nd | nd | nd | nd |
| Average | 1230 | 1120 | nd | nd | nd | nd |
| Stdev | 2090 | 1790 | nd | nd | nd | nd |
| p(t-test) | | 0.87 | nd | nd | nd | nd |
| Min | 0.116 | 28.5 | nd | nd | nd | nd |
| Max | 6840 | 5430 | nd | nd | nd | nd |
| n (Samp) | 56 | 12 | nd | nd | nd | nd |
| n (Patient) | 37 | 12 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 197 | 171 | nd | nd | nd | nd |
| Average | 1140 | 463 | nd | nd | nd | nd |
| Stdev | 1940 | 716 | nd | nd | nd | nd |
| p(t-test) | | 0.40 | nd | nd | nd | nd |
| Min | 0.116 | 73.9 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 6840 | 1910 | nd | nd | nd | nd |
| n (Samp) | 88 | 6 | nd | nd | nd | nd |
| n (Patient) | 61 | 6 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 381 | 151 | nd | nd | nd | nd |
| Average | 1460 | 1310 | nd | nd | nd | nd |
| Stdev | 2310 | 2210 | nd | nd | nd | nd |
| p(t-test) | | 0.84 | nd | nd | nd | nd |
| Min | 0.116 | 28.5 | nd | nd | nd | nd |
| Max | 6840 | 6400 | nd | nd | nd | nd |
| n (Samp) | 41 | 14 | nd | nd | nd | nd |
| n (Patient) | 27 | 14 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | 0.46 | 0.44 | nd | nd | nd | nd | nd | nd |
| SE | 0.093 | 0.12 | 0.091 | nd | nd | nd | nd | nd | nd |
| p | 0.91 | 0.78 | 0.52 | nd | nd | nd | nd | nd | nd |
| nCohort 1 | 56 | 88 | 41 | nd | nd | nd | nd | nd | nd |
| nCohort 2 | 12 | 6 | 14 | nd | nd | nd | nd | nd | nd |
| Cutoff 1 | 116 | 123 | 108 | nd | nd | nd | nd | nd | nd |
| Sens 1 | 75% | 83% | 71% | nd | nd | nd | nd | nd | nd |
| Spec 1 | 32% | 33% | 27% | nd | nd | nd | nd | nd | nd |
| Cutoff 2 | 96.8 | 123 | 73.1 | nd | nd | nd | nd | nd | nd |
| Sens 2 | 83% | 83% | 86% | nd | nd | nd | nd | nd | nd |
| Spec 2 | 30% | 33% | 17% | nd | nd | nd | nd | nd | nd |
| Cutoff 3 | 47.1 | 73.1 | 42.3 | nd | nd | nd | nd | nd | nd |
| Sens 3 | 92% | 100% | 93% | nd | nd | nd | nd | nd | nd |
| Spec 3 | 12% | 17% | 12% | nd | nd | nd | nd | nd | nd |
| Cutoff 4 | 715 | 715 | 902 | nd | nd | nd | nd | nd | nd |
| Sens 4 | 33% | 17% | 21% | nd | nd | nd | nd | nd | nd |
| Spec 4 | 71% | 70% | 71% | nd | nd | nd | nd | nd | nd |
| Cutoff 5 | 1430 | 1430 | 1760 | nd | nd | nd | nd | nd | nd |
| Sens 5 | 25% | 17% | 21% | nd | nd | nd | nd | nd | nd |
| Spec 5 | 80% | 82% | 80% | nd | nd | nd | nd | nd | nd |
| Cutoff 6 | 5560 | 5160 | 6560 | nd | nd | nd | nd | nd | nd |
| Sens 6 | 0% | 0% | 0% | nd | nd | nd | nd | nd | nd |
| Spec 6 | 91% | 91% | 90% | nd | nd | nd | nd | nd | nd |
| OR Quart 2 | 0.62 | 2.2 | 1.0 | nd | nd | nd | nd | nd | nd |
| p Value | 0.63 | 0.53 | 1.0 | nd | nd | nd | nd | nd | nd |
| 95% CI of OR Quart2 | 0.090 4.3 | 0.18 26 | 0.16 6.1 | nd | nd | nd | nd | nd | nd |
| OR Quart 3 | 1.9 | 2.1 | 2.0 | nd | nd | nd | nd | nd | nd |
| p Value | 0.42 | 0.56 | 0.41 | nd | nd | nd | nd | nd | nd |
| 95% CI of OR Quart3 | 0.38 9.9 | 0.18 25 | 0.38 11 | nd | nd | nd | nd | nd | nd |
| OR Quart 4 | 0.62 | 1.0 | 1.1 | nd | nd | nd | nd | nd | nd |
| p Value | 0.63 | 0.98 | 0.92 | nd | nd | nd | nd | nd | nd |
| 95% CI of OR Quart4 | 0.090 4.3 | 0.062 18 | 0.18 6.8 | nd | nd | nd | nd | nd | nd |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.66 | 2.13 | 1.66 | 2.36 | 1.66 | 2.26 |
| Average | 2.32 | 3.66 | 2.32 | 3.88 | 2.32 | 3.00 |
| Stdev | 2.32 | 4.90 | 2.32 | 5.35 | 2.32 | 2.72 |
| p(t-test) | | 0.0028 | | 6.3E-4 | | 0.17 |
| Min | 0.183 | 0.453 | 0.183 | 0.562 | 0.183 | 0.363 |
| Max | 20.8 | 30.1 | 20.8 | 33.6 | 20.8 | 12.5 |
| n (Samp) | 260 | 51 | 260 | 56 | 260 | 25 |
| n (Patient) | 110 | 51 | 110 | 56 | 110 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.77 | 1.68 | 1.77 | 2.00 | 1.77 | 1.91 |
| Average | 2.76 | 3.12 | 2.76 | 3.22 | 2.76 | 1.68 |
| Stdev | 3.35 | 4.09 | 3.35 | 3.30 | 3.35 | 0.833 |
| p(t-test) | | 0.66 | | 0.54 | | 0.25 |
| Min | 0.183 | 0.245 | 0.183 | 0.355 | 0.183 | 0.363 |
| Max | 33.6 | 17.1 | 33.6 | 14.5 | 33.6 | 2.69 |
| n (Samp) | 466 | 18 | 466 | 21 | 466 | 13 |
| n (Patient) | 180 | 18 | 180 | 21 | 180 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.62 | 2.22 | 1.62 | 2.36 | 1.62 | 2.26 |
| Average | 2.47 | 3.81 | 2.47 | 3.82 | 2.47 | 3.39 |
| Stdev | 2.58 | 4.85 | 2.58 | 5.27 | 2.58 | 2.98 |
| p(t-test) | | 0.0067 | | 0.0083 | | 0.11 |
| Min | 0.183 | 0.622 | 0.183 | 0.562 | 0.183 | 0.591 |
| Max | 20.8 | 30.1 | 20.8 | 33.6 | 20.8 | 12.5 |
| n (Samp) | 213 | 51 | 213 | 53 | 213 | 23 |
| n (Patient) | 89 | 51 | 89 | 53 | 89 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.48 | 0.63 | 0.63 | 0.54 | 0.62 | 0.60 | 0.43 | 0.62 |
| SE | 0.045 | 0.070 | 0.046 | 0.043 | 0.066 | 0.045 | 0.062 | 0.084 | 0.065 |
| p | 0.026 | 0.73 | 0.0052 | 0.0029 | 0.51 | 0.0056 | 0.12 | 0.44 | 0.073 |
| nCohort 1 | 260 | 466 | 213 | 260 | 466 | 213 | 260 | 466 | 213 |
| nCohort 2 | 51 | 18 | 51 | 56 | 21 | 53 | 25 | 13 | 23 |
| Cutoff 1 | 1.35 | 1.17 | 1.52 | 1.70 | 1.22 | 1.77 | 1.43 | 1.05 | 1.57 |
| Sens 1 | 71% | 72% | 71% | 71% | 71% | 72% | 72% | 77% | 74% |
| Spec 1 | 40% | 31% | 47% | 53% | 32% | 58% | 43% | 26% | 49% |
| Cutoff 2 | 1.10 | 0.591 | 1.11 | 1.02 | 1.01 | 1.02 | 0.855 | 0.971 | 0.855 |
| Sens 2 | 80% | 83% | 80% | 80% | 81% | 81% | 80% | 85% | 83% |
| Spec 2 | 32% | 8% | 32% | 30% | 25% | 30% | 23% | 23% | 22% |
| Cutoff 3 | 0.893 | 0.448 | 0.919 | 0.787 | 0.731 | 0.787 | 0.679 | 0.417 | 0.699 |
| Sens 3 | 90% | 94% | 90% | 91% | 90% | 91% | 92% | 92% | 91% |
| Spec 3 | 25% | 4% | 23% | 20% | 13% | 18% | 16% | 3% | 15% |
| Cutoff 4 | 2.42 | 2.71 | 2.52 | 2.42 | 2.71 | 2.52 | 2.42 | 2.71 | 2.52 |
| Sens 4 | 39% | 28% | 45% | 45% | 43% | 42% | 48% | 0% | 48% |
| Spec 4 | 70% | 70% | 71% | 70% | 70% | 71% | 70% | 70% | 71% |
| Cutoff 5 | 3.34 | 3.88 | 3.74 | 3.34 | 3.88 | 3.74 | 3.34 | 3.88 | 3.74 |
| Sens 5 | 31% | 22% | 31% | 32% | 29% | 28% | 32% | 0% | 30% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | 80% | 80% | 80% | 80% | 80% | 80% | 80% | 80% |
| Cutoff 6 | 4.93 | 5.28 | 5.88 | 4.93 | 5.28 | 5.88 | 4.93 | 5.28 | 5.88 |
| Sens 6 | 20% | 17% | 12% | 18% | 14% | 11% | 12% | 0% | 17% |
| Spec 6 | 90% | 90% | 90% | 90% | 90% | 90% | 90% | 90% | 90% |
| OR Quart 2 | 2.0 | 0.59 | 1.1 | 0.67 | 0.99 | 0.45 | 0.48 | >7.4 | 0.79 |
| p Value | 0.17 | 0.48 | 0.80 | 0.44 | 0.99 | 0.17 | 0.31 | <0.063 | 0.73 |
| 95% CI of | 0.75 | 0.14 | 0.41 | 0.24 | 0.28 | 0.15 | 0.11 | >0.90 | 0.20 |
| OR Quart2 | 5.3 | 2.5 | 3.2 | 1.9 | 3.5 | 1.4 | 2.0 | na | 3.1 |
| OR Quart 3 | 2.2 | 1.0 | 2.5 | 2.3 | 0.58 | 2.4 | 1.2 | >3.1 | 1.0 |
| p Value | 0.11 | 1.0 | 0.050 | 0.046 | 0.47 | 0.041 | 0.77 | <0.33 | 1.0 |
| 95% CI of | 0.83 | 0.28 | 1.0 | 1.0 | 0.14 | 1.0 | 0.38 | >0.32 | 0.27 |
| OR Quart3 | 5.8 | 3.5 | 6.3 | 5.4 | 2.5 | 5.7 | 3.7 | na | 3.7 |
| OR Quart 4 | 2.8 | 1.0 | 2.5 | 2.2 | 1.6 | 2.1 | 1.5 | >3.1 | 1.9 |
| p Value | 0.033 | 1.0 | 0.050 | 0.068 | 0.40 | 0.10 | 0.43 | <0.33 | 0.26 |
| 95% CI of | 1.1 | 0.28 | 1.0 | 0.94 | 0.52 | 0.87 | 0.52 | >0.32 | 0.61 |
| OR Quart4 | 7.2 | 3.5 | 6.3 | 5.1 | 5.1 | 4.9 | 4.6 | na | 6.2 |

## Myoglobin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 26.8 | 36.2 | 26.8 | 41.7 | 26.8 | 54.3 |
| Average | 66.0 | 106 | 66.0 | 168 | 66.0 | 113 |
| Stdev | 145 | 166 | 145 | 301 | 145 | 194 |
| p(t-test) | | 0.078 | | 1.8E-4 | | 0.13 |
| Min | 4.60 | 6.86 | 4.60 | 4.40 | 4.60 | 7.57 |
| Max | 1720 | 737 | 1720 | 1470 | 1720 | 988 |
| n (Samp) | 260 | 51 | 260 | 56 | 260 | 25 |
| n (Patient) | 110 | 51 | 110 | 56 | 110 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 33.0 | 62.6 | 33.0 | 64.7 | 33.0 | 69.7 |
| Average | 83.7 | 266 | 83.7 | 272 | 83.7 | 94.9 |
| Stdev | 191 | 465 | 191 | 433 | 191 | 93.0 |
| p(t-test) | | 2.8E-4 | | 5.2E-5 | | 0.83 |
| Min | 3.68 | 8.01 | 3.68 | 4.40 | 3.68 | 7.57 |
| Max | 2130 | 1880 | 2130 | 1470 | 2130 | 308 |
| n (Samp) | 466 | 18 | 466 | 21 | 466 | 13 |
| n (Patient) | 180 | 18 | 180 | 21 | 180 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 27.4 | 37.3 | 27.4 | 41.0 | 27.4 | 62.1 |
| Average | 72.4 | 102 | 72.4 | 147 | 72.4 | 165 |
| Stdev | 153 | 158 | 153 | 260 | 153 | 320 |
| p(t-test) | | 0.22 | | 0.0071 | | 0.017 |
| Min | 4.60 | 6.86 | 4.60 | 8.81 | 4.60 | 8.40 |
| Max | 1720 | 737 | 1720 | 1410 | 1720 | 1310 |
| n (Samp) | 213 | 51 | 213 | 53 | 213 | 23 |
| n (Patient) | 89 | 51 | 89 | 53 | 89 | 23 |

| | 0hr prior to AKI stage | 24hr prior to AKI stage | 48hr prior to AKI stage |
|---|---|---|---|

| | sCr or UO | sCr only | UO only | sCr or UO | sCr only | UO only | sCr or UO | sCr only | UO only |
|---|---|---|---|---|---|---|---|---|---|
| AUC | 0.60 | 0.65 | 0.59 | 0.65 | 0.61 | 0.64 | 0.63 | 0.62 | 0.63 |
| SE | 0.045 | 0.072 | 0.046 | 0.043 | 0.067 | 0.045 | 0.062 | 0.084 | 0.065 |
| p | 0.034 | 0.041 | 0.039 | 7.0E-4 | 0.086 | 0.0019 | 0.036 | 0.15 | 0.051 |
| nCohort 1 | 260 | 466 | 213 | 260 | 466 | 213 | 260 | 466 | 213 |
| nCohort 2 | 51 | 18 | 51 | 56 | 21 | 53 | 25 | 13 | 23 |
| Cutoff 1 | 21.3 | 26.6 | 20.9 | 27.5 | 24.5 | 27.5 | 20.6 | 25.6 | 20.4 |
| Sens 1 | 71% | 72% | 71% | 71% | 71% | 72% | 72% | 77% | 74% |
| Spec 1 | 40% | 43% | 40% | 52% | 38% | 50% | 39% | 41% | 40% |
| Cutoff 2 | 18.6 | 25.4 | 18.6 | 18.6 | 19.2 | 18.9 | 17.4 | 17.4 | 16.3 |
| Sens 2 | 80% | 83% | 80% | 80% | 81% | 81% | 80% | 85% | 83% |
| Spec 2 | 33% | 41% | 34% | 33% | 31% | 35% | 30% | 25% | 29% |
| Cutoff 3 | 12.5 | 8.56 | 14.8 | 12.3 | 12.9 | 13.2 | 9.64 | 8.39 | 10.6 |
| Sens 3 | 90% | 94% | 90% | 91% | 90% | 91% | 92% | 92% | 91% |
| Spec 3 | 18% | 8% | 26% | 18% | 17% | 21% | 13% | 8% | 15% |
| Cutoff 4 | 50.2 | 60.4 | 56.0 | 50.2 | 60.4 | 56.0 | 50.2 | 60.4 | 56.0 |
| Sens 4 | 43% | 50% | 41% | 46% | 52% | 45% | 52% | 54% | 52% |
| Spec 4 | 70% | 70% | 70% | 70% | 70% | 70% | 70% | 70% | 70% |
| Cutoff 5 | 74.3 | 93.4 | 84.8 | 74.3 | 93.4 | 84.8 | 74.3 | 93.4 | 84.8 |
| Sens 5 | 35% | 39% | 35% | 43% | 38% | 42% | 44% | 31% | 43% |
| Spec 5 | 80% | 80% | 80% | 80% | 80% | 80% | 80% | 80% | 80% |
| Cutoff 6 | 138 | 171 | 174 | 138 | 171 | 174 | 138 | 171 | 174 |
| Sens 6 | 22% | 33% | 16% | 29% | 29% | 26% | 20% | 15% | 13% |
| Spec 6 | 90% | 90% | 90% | 90% | 90% | 90% | 90% | 90% | 90% |
| OR Quart 2 | 1.6 | 2.6 | 2.9 | 0.88 | 0.99 | 2.0 | 1.0 | 0.99 | 0.74 |
| p Value | 0.36 | 0.27 | 0.038 | 0.80 | 0.99 | 0.16 | 1.0 | 0.99 | 0.70 |
| 95% CI of OR Quart2 | 0.60 4.1 | 0.49 13 | 1.1 8.1 | 0.32 2.4 | 0.24 4.1 | 0.75 5.5 | 0.24 4.2 | 0.14 7.2 | 0.16 3.4 |
| OR Quart 3 | 1.7 | 1.5 | 2.0 | 1.8 | 0.99 | 1.7 | 1.5 | 1.5 | 1.3 |
| p Value | 0.26 | 0.65 | 0.20 | 0.19 | 0.99 | 0.31 | 0.51 | 0.66 | 0.73 |
| 95% CI of OR Quart3 | 0.67 4.4 | 0.25 9.2 | 0.69 5.8 | 0.75 4.5 | 0.24 4.1 | 0.61 4.7 | 0.42 5.7 | 0.25 9.1 | 0.32 5.0 |
| OR Quart 4 | 2.6 | 4.2 | 4.0 | 3.4 | 2.3 | 4.1 | 3.0 | 3.1 | 3.2 |
| p Value | 0.039 | 0.073 | 0.0059 | 0.0045 | 0.17 | 0.0030 | 0.070 | 0.17 | 0.063 |
| 95% CI of OR Quart4 | 1.1 6.4 | 0.88 20 | 1.5 11 | 1.5 7.9 | 0.70 7.8 | 1.6 10 | 0.91 10.0 | 0.61 16 | 0.94 11 |

## Mucin-16

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.11 | 1.51 | 1.11 | 2.02 | 1.11 | 1.59 |
| Average | 3.53 | 10.4 | 3.53 | 13.9 | 3.53 | 3.72 |
| Stdev | 14.4 | 41.9 | 14.4 | 46.3 | 14.4 | 6.27 |
| p(t-test) | | 0.19 | | 0.063 | | 0.95 |
| Min | 0.117 | 0.204 | 0.117 | 0.122 | 0.117 | 0.122 |
| Max | 131 | 253 | 131 | 248 | 131 | 31.2 |
| n (Samp) | 82 | 36 | 82 | 45 | 82 | 26 |
| n (Patient) | 75 | 36 | 75 | 45 | 75 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.41 | 1.27 | 1.41 | 1.83 | 1.41 | 1.24 |
| Average | 8.87 | 1.81 | 8.87 | 5.69 | 8.87 | 1.52 |
| Stdev | 35.3 | 1.57 | 35.3 | 9.55 | 35.3 | 1.06 |
| p(t-test) | | 0.49 | | 0.74 | | 0.53 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.117 | 0.481 | 0.117 | 0.631 | 0.117 | 0.631 |
| Max | 263 | 5.56 | 263 | 37.0 | 263 | 4.16 |
| n (Samp) | 197 | 12 | 197 | 14 | 197 | 9 |
| n (Patient) | 131 | 12 | 131 | 14 | 131 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.24 | 1.60 | 1.24 | 2.52 | 1.24 | 2.44 |
| Average | 3.82 | 12.7 | 3.82 | 14.8 | 3.82 | 4.48 |
| Stdev | 15.1 | 46.6 | 15.1 | 47.3 | 15.1 | 6.81 |
| p(t-test) | | 0.14 | | 0.067 | | 0.84 |
| Min | 0.122 | 0.204 | 0.122 | 0.122 | 0.122 | 0.122 |
| Max | 131 | 253 | 131 | 248 | 131 | 31.2 |
| n (Samp) | 75 | 29 | 75 | 43 | 75 | 21 |
| n (Patient) | 62 | 29 | 62 | 43 | 62 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.47 | 0.61 | 0.65 | 0.61 | 0.64 | 0.62 | 0.45 | 0.66 |
| SE | 0.058 | 0.087 | 0.064 | 0.052 | 0.082 | 0.054 | 0.066 | 0.10 | 0.071 |
| p | 0.036 | 0.72 | 0.084 | 0.0055 | 0.18 | 0.0073 | 0.068 | 0.64 | 0.024 |
| nCohort 1 | 82 | 197 | 75 | 82 | 197 | 75 | 82 | 197 | 75 |
| nCohort 2 | 36 | 12 | 29 | 45 | 14 | 43 | 26 | 9 | 21 |
| Cutoff 1 | 1.06 | 1.09 | 0.999 | 1.06 | 1.33 | 1.12 | 1.06 | 1.01 | 1.25 |
| Sens 1 | 72% | 75% | 72% | 71% | 71% | 72% | 73% | 78% | 71% |
| Spec 1 | 50% | 41% | 44% | 50% | 47% | 47% | 50% | 38% | 52% |
| Cutoff 2 | 0.803 | 0.821 | 0.761 | 0.821 | 1.02 | 0.803 | 0.999 | 0.754 | 1.06 |
| Sens 2 | 81% | 83% | 83% | 80% | 86% | 81% | 85% | 89% | 81% |
| Spec 2 | 46% | 36% | 33% | 46% | 38% | 43% | 48% | 29% | 47% |
| Cutoff 3 | 0.464 | 0.481 | 0.379 | 0.334 | 1.01 | 0.334 | 0.630 | 0.630 | 0.999 |
| Sens 3 | 92% | 92% | 93% | 91% | 93% | 91% | 92% | 100% | 90% |
| Spec 3 | 16% | 16% | 11% | 10% | 38% | 8% | 24% | 22% | 44% |
| Cutoff 4 | 2.09 | 3.28 | 1.96 | 2.09 | 3.28 | 1.96 | 2.09 | 3.28 | 1.96 |
| Sens 4 | 39% | 17% | 45% | 49% | 43% | 56% | 38% | 11% | 52% |
| Spec 4 | 72% | 70% | 71% | 72% | 70% | 71% | 72% | 70% | 71% |
| Cutoff 5 | 2.78 | 5.21 | 2.98 | 2.78 | 5.21 | 2.98 | 2.78 | 5.21 | 2.98 |
| Sens 5 | 36% | 8% | 41% | 44% | 29% | 47% | 27% | 0% | 38% |
| Spec 5 | 80% | 80% | 80% | 80% | 80% | 80% | 80% | 80% | 80% |
| Cutoff 6 | 4.88 | 8.80 | 5.21 | 4.88 | 8.80 | 5.21 | 4.88 | 8.80 | 5.21 |
| Sens 6 | 28% | 0% | 31% | 29% | 14% | 28% | 12% | 0% | 14% |
| Spec 6 | 90% | 90% | 91% | 90% | 90% | 91% | 90% | 90% | 91% |
| OR Quart 2 | 2.4 | 1.0 | 1.5 | 2.9 | 5.3 | 2.2 | 3.4 | 1.0 | 2.9 |
| p Value | 0.16 | 0.98 | 0.51 | 0.071 | 0.13 | 0.18 | 0.10 | 0.99 | 0.23 |
| 95% CI of OR Quart2 | 0.70 8.2 | 0.14 7.5 | 0.42 5.7 | 0.91 8.9 | 0.60 47 | 0.69 7.1 | 0.78 14 | 0.062 17 | 0.50 17 |
| OR Quart 3 | 1.8 | 3.3 | 1.0 | 1.2 | 3.1 | 1.7 | 2.3 | 6.7 | 3.7 |
| p Value | 0.35 | 0.15 | 1.0 | 0.80 | 0.34 | 0.37 | 0.28 | 0.085 | 0.14 |
| 95% CI of OR Quart3 | 0.52 6.5 | 0.64 17 | 0.25 4.0 | 0.34 4.0 | 0.31 30 | 0.52 5.7 | 0.51 10 | 0.77 57 | 0.66 20 |
| OR Quart 4 | 3.7 | 1.0 | 3.6 | 6.1 | 5.3 | 5.0 | 4.0 | 1.0 | 5.5 |
| p Value | 0.034 | 0.98 | 0.043 | 0.0018 | 0.13 | 0.0061 | 0.060 | 0.99 | 0.046 |
| 95% CI of OR Quart4 | 1.1 12 | 0.14 7.5 | 1.0 12 | 2.0 19 | 0.60 47 | 1.6 16 | 0.95 17 | 0.062 17 | 1.0 29 |

**Tumor necrosis factor receptor superfamily member 10B**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 1.00E-9 | 1.00E-9 | 0.00240 | 1.00E-9 | 1.00E-9 |
| Average | 0.00715 | 0.0102 | 0.00715 | 0.0133 | 0.00715 | 0.0150 |
| Stdev | 0.0203 | 0.0434 | 0.0203 | 0.0446 | 0.0203 | 0.0622 |
| p(t-test) | | 0.60 | | 0.30 | | 0.34 |
| Min | 1.00E-9 | 1.00E-9 | 1.00E-9 | 1.00E-9 | 1.00E-9 | 1.00E-9 |
| Max | 0.114 | 0.262 | 0.114 | 0.286 | 0.114 | 0.300 |
| n (Samp) | 79 | 36 | 79 | 41 | 79 | 23 |
| n (Patient) | 72 | 36 | 72 | 41 | 72 | 23 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 1.00E-9 | 1.00E-9 | 0.00507 | 1.00E-9 | 1.00E-9 |
| Average | 0.00582 | 0.0263 | 0.00582 | 0.0316 | 0.00582 | 0.0334 |
| Stdev | 0.0146 | 0.0783 | 0.0146 | 0.0849 | 0.0146 | 0.0938 |
| p(t-test) | | 0.0040 | | 6.0E-4 | | 7.1E-4 |
| Min | 1.00E-9 | 1.00E-9 | 1.00E-9 | 1.00E-9 | 1.00E-9 | 1.00E-9 |
| Max | 0.114 | 0.262 | 0.114 | 0.286 | 0.114 | 0.300 |
| n (Samp) | 187 | 11 | 187 | 11 | 187 | 10 |
| n (Patient) | 127 | 11 | 127 | 11 | 127 | 10 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00131 | 1.00E-9 | 0.00131 | 0.00202 | 0.00131 | 1.00E-9 |
| Average | 0.00817 | 0.00387 | 0.00817 | 0.0104 | 0.00817 | 0.00218 |
| Stdev | 0.0213 | 0.00693 | 0.0213 | 0.0213 | 0.0213 | 0.00328 |
| p(t-test) | | 0.28 | | 0.60 | | 0.25 |
| Min | 1.00E-9 | 1.00E-9 | 1.00E-9 | 1.00E-9 | 1.00E-9 | 1.00E-9 |
| Max | 0.114 | 0.0300 | 0.114 | 0.108 | 0.114 | 0.00903 |
| n (Samp) | 70 | 30 | 70 | 40 | 70 | 17 |
| n (Patient) | 56 | 30 | 56 | 40 | 56 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.44 | 0.45 | 0.61 | 0.62 | 0.57 | 0.47 | 0.51 | 0.43 |
| SE | 0.059 | 0.092 | 0.064 | 0.055 | 0.093 | 0.058 | 0.069 | 0.094 | 0.080 |
| p | 0.65 | 0.52 | 0.43 | 0.038 | 0.21 | 0.22 | 0.72 | 0.91 | 0.35 |
| nCohort 1 | 79 | 187 | 70 | 79 | 187 | 70 | 79 | 187 | 70 |
| nCohort 2 | 36 | 11 | 30 | 41 | 11 | 40 | 23 | 10 | 17 |
| Cutoff 1 | 0 | 0 | 0 | 0 | 0 | 0 | 0 | 0 | 0 |
| Sens 1 | 100% | 100% | 100% | 100% | 100% | 100% | 100% | 100% | 100% |
| Spec 1 | 0% | 0% | 0% | 0% | 0% | 0% | 0% | 0% | 0% |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 0.00274 | 0.00377 | 0.00377 | 0.00274 | 0.00377 | 0.00377 | 0.00274 | 0.00377 | 0.00377 |
| Sens 4 | 33% | 27% | 33% | 49% | 64% | 42% | 30% | 40% | 24% |
| Spec 4 | 71% | 71% | 71% | 71% | 71% | 71% | 71% | 71% | 71% |
| Cutoff 5 | 0.00507 | 0.00819 | 0.00813 | 0.00507 | 0.00819 | 0.00813 | 0.00507 | 0.00819 | 0.00813 |
| Sens 5 | 28% | 27% | 17% | 37% | 27% | 35% | 26% | 30% | 12% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 81% | 82% | 80% | 81% | 82% | 80% | 81% | 82% | 80% |
| Cutoff 6 | 0.0166 | 0.0136 | 0.0165 | 0.0166 | 0.0136 | 0.0165 | 0.0166 | 0.0136 | 0.0165 |
| Sens 6 | 6% | 18% | 7% | 17% | 18% | 18% | 4% | 20% | 0% |
| Spec 6 | 91% | 90% | 90% | 91% | 90% | 90% | 91% | 90% | 90% |
| OR Quart 2 | 0.12 | 0 | 0.24 | 6.0 | 0 | 1.1 | 1.1 | 0.48 | 0.71 |
| p Value | 0.011 | na | 0.057 | 0.012 | na | 0.84 | 0.94 | 0.41 | 0.68 |
| 95% CI of OR Quart2 | 0.024 0.61 | na na | 0.056 1.0 | 1.5 24 | na na | 0.36 3.5 | 0.29 3.8 | 0.084 2.7 | 0.14 3.6 |
| OR Quart 3 | 2.0 | 1.4 | 1.2 | 5.2 | 1.0 | 1.2 | 2.4 | 0 | 1.7 |
| p Value | 0.19 | 0.70 | 0.77 | 0.021 | 1.0 | 0.77 | 0.14 | na | 0.47 |
| 95% CI of OR Quart3 | 0.71 5.7 | 0.29 6.4 | 0.38 3.7 | 1.3 21 | 0.24 4.2 | 0.38 3.8 | 0.74 8.1 | na na | 0.40 7.1 |
| OR Quart 4 | 0.55 | 1.4 | 0.84 | 9.0 | 0.72 | 2.4 | 0 | 0.98 | 1.1 |
| p Value | 0.30 | 0.68 | 0.77 | 0.0020 | 0.68 | 0.13 | na | 0.98 | 0.94 |
| 95% CI of OR Quart4 | 0.17 1.7 | 0.30 6.6 | 0.26 2.7 | 2.2 36 | 0.15 3.4 | 0.78 7.2 | na na | 0.23 4.2 | 0.23 4.9 |

Table 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

## Apolipoprotein A-II

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 54100 | 49700 | 54100 | 45500 | 54100 | 43300 |
| Average | 55400 | 59300 | 55400 | 51900 | 55400 | 46600 |
| Stdev | 29200 | 19900 | 29200 | 32000 | 29200 | 33800 |
| p(t-test) | | 0.57 | | 0.57 | | 0.26 |
| Min | 5450 | 32800 | 5450 | 10700 | 5450 | 1810 |
| Max | 253000 | 97000 | 253000 | 176000 | 253000 | 152000 |
| n (Samp) | 230 | 19 | 230 | 26 | 230 | 15 |
| n (Patient) | 158 | 19 | 158 | 26 | 158 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 52600 | 51800 | 52600 | 45500 | 52600 | 43200 |
| Average | 54900 | 58300 | 54900 | 51000 | 54900 | 47000 |
| Stdev | 29800 | 21200 | 29800 | 31800 | 29800 | 35000 |
| p(t-test) | | 0.62 | | 0.54 | | 0.35 |
| Min | 7680 | 23300 | 7680 | 10700 | 7680 | 1810 |
| Max | 253000 | 97000 | 253000 | 176000 | 253000 | 152000 |
| n (Samp) | 201 | 19 | 201 | 26 | 201 | 14 |
| n (Patient) | 133 | 19 | 133 | 26 | 133 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | nd | 0.56 | 0.44 | nd | 0.43 | 0.36 | nd | 0.37 |
| SE | 0.071 | nd | 0.071 | 0.061 | nd | 0.062 | 0.079 | nd | 0.082 |
| p | 0.38 | nd | 0.40 | 0.30 | nd | 0.24 | 0.069 | nd | 0.11 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 230 | nd | 201 | 230 | nd | 201 | 230 | nd | 201 |
| nCohort 2 | 19 | nd | 19 | 26 | nd | 26 | 15 | nd | 14 |
| Cutoff 1 | 43600 | nd | 43600 | 35400 | nd | 35000 | 36000 | nd | 36000 |
| Sens 1 | 74% | nd | 74% | 73% | nd | 73% | 73% | nd | 71% |
| Spec 1 | 35% | nd | 36% | 21% | nd | 21% | 21% | nd | 21% |
| Cutoff 2 | 43000 | nd | 40500 | 30600 | nd | 30600 | 35900 | nd | 31700 |
| Sens 2 | 84% | nd | 84% | 81% | nd | 81% | 80% | nd | 86% |
| Spec 2 | 34% | nd | 30% | 17% | nd | 18% | 21% | nd | 19% |
| Cutoff 3 | 40200 | nd | 32500 | 28300 | nd | 26600 | 7970 | nd | 7970 |
| Sens 3 | 95% | nd | 95% | 92% | nd | 92% | 93% | nd | 93% |
| Spec 3 | 30% | nd | 20% | 16% | nd | 14% | 1% | nd | 1% |
| Cutoff 4 | 64400 | nd | 64800 | 64400 | nd | 64800 | 64400 | nd | 64800 |
| Sens 4 | 42% | nd | 42% | 19% | nd | 15% | 13% | nd | 14% |
| Spec 4 | 70% | nd | 70% | 70% | nd | 70% | 70% | nd | 70% |
| Cutoff 5 | 72600 | nd | 72300 | 72600 | nd | 72300 | 72600 | nd | 72300 |
| Sens 5 | 26% | nd | 26% | 12% | nd | 12% | 13% | nd | 14% |
| Spec 5 | 80% | nd | 80% | 80% | nd | 80% | 80% | nd | 80% |
| Cutoff 6 | 87100 | nd | 83600 | 87100 | nd | 83600 | 87100 | nd | 83600 |
| Sens 6 | 16% | nd | 16% | 8% | nd | 8% | 7% | nd | 7% |
| Spec 6 | 90% | nd | 90% | 90% | nd | 90% | 90% | nd | 90% |
| OR Quart 2 | 12 | nd | 5.2 | 1.2 | nd | 1.9 | 0 | nd | 0 |
| p Value | 0.021 | nd | 0.041 | 0.75 | nd | 0.35 | na | nd | na |
| 95% CI of | 1.5 | nd | 1.1 | 0.35 | nd | 0.51 | na | nd | na |
| OR Quart2 | 95 | nd | 25 | 4.2 | nd | 6.7 | na | nd | na |
| OR Quart 3 | 1.0 | nd | 0.49 | 1.2 | nd | 1.6 | 3.9 | nd | 3.9 |
| p Value | 1.0 | nd | 0.57 | 0.75 | nd | 0.51 | 0.099 | nd | 0.10 |
| 95% CI of | 0.061 | nd | 0.043 | 0.35 | nd | 0.42 | 0.77 | nd | 0.77 |
| OR Quart3 | 16 | nd | 5.6 | 4.2 | nd | 5.8 | 20 | nd | 20 |
| OR Quart 4 | 7.6 | nd | 3.9 | 1.9 | nd | 2.5 | 3.3 | nd | 2.7 |
| p Value | 0.061 | nd | 0.10 | 0.26 | nd | 0.14 | 0.16 | nd | 0.25 |
| 95% CI of | 0.91 | nd | 0.77 | 0.61 | nd | 0.73 | 0.63 | nd | 0.50 |
| OR Quart4 | 64 | nd | 20 | 6.1 | nd | 8.8 | 17 | nd | 15 |

## Caspase-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 70.5 | 58.2 | nd | nd | nd | nd |
| Average | 90.5 | 69.4 | nd | nd | nd | nd |
| Stdev | 59.1 | 45.7 | nd | nd | nd | nd |
| p(t-test) | | 0.31 | nd | nd | nd | nd |
| Min | 20.6 | 36.3 | nd | nd | nd | nd |
| Max | 326 | 188 | nd | nd | nd | nd |
| n (Samp) | 60 | 9 | nd | nd | nd | nd |
| n (Patient) | 37 | 9 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72.3 | 58.2 | nd | nd | nd | nd |
| Average | 91.4 | 69.4 | nd | nd | nd | nd |
| Stdev | 54.9 | 45.7 | nd | nd | nd | nd |
| p(t-test) | | 0.27 | nd | nd | nd | nd |
| Min | 33.8 | 36.3 | nd | nd | nd | nd |
| Max | 271 | 188 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 45 | 9 | nd | nd | nd | nd |
| n (Patient) | 26 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.32 | nd | 0.31 | nd | nd | nd | nd | nd | nd |
| SE | 0.10 | nd | 0.10 | nd | nd | nd | nd | nd | nd |
| p | 0.089 | nd | 0.066 | nd | nd | nd | nd | nd | nd |
| nCohort 1 | 60 | nd | 45 | nd | nd | nd | nd | nd | nd |
| nCohort 2 | 9 | nd | 9 | nd | nd | nd | nd | nd | nd |
| Cutoff 1 | 45.8 | nd | 45.8 | nd | nd | nd | nd | nd | nd |
| Sens 1 | 78% | nd | 78% | nd | nd | nd | nd | nd | nd |
| Spec 1 | 15% | nd | 11% | nd | nd | nd | nd | nd | nd |
| Cutoff 2 | 42.1 | nd | 42.0 | nd | nd | nd | nd | nd | nd |
| Sens 2 | 89% | nd | 89% | nd | nd | nd | nd | nd | nd |
| Spec 2 | 13% | nd | 9% | nd | nd | nd | nd | nd | nd |
| Cutoff 3 | 35.1 | nd | 33.8 | nd | nd | nd | nd | nd | nd |
| Sens 3 | 100% | nd | 100% | nd | nd | nd | nd | nd | nd |
| Spec 3 | 5% | nd | 2% | nd | nd | nd | nd | nd | nd |
| Cutoff 4 | 95.6 | nd | 95.9 | nd | nd | nd | nd | nd | nd |
| Sens 4 | 11% | nd | 11% | nd | nd | nd | nd | nd | nd |
| Spec 4 | 70% | nd | 73% | nd | nd | nd | nd | nd | nd |
| Cutoff 5 | 110 | nd | 103 | nd | nd | nd | nd | nd | nd |
| Sens 5 | 11% | nd | 11% | nd | nd | nd | nd | nd | nd |
| Spec 5 | 80% | nd | 80% | nd | nd | nd | nd | nd | nd |
| Cutoff 6 | 147 | nd | 186 | nd | nd | nd | nd | nd | nd |
| Sens 6 | 11% | nd | 11% | nd | nd | nd | nd | nd | nd |
| Spec 6 | 90% | nd | 91% | nd | nd | nd | nd | nd | nd |
| OR Quart 2 | 1.1 | nd | 1.1 | nd | nd | nd | nd | nd | nd |
| p Value | 0.97 | nd | 0.96 | nd | nd | nd | nd | nd | nd |
| 95% CI of OR Quart2 | 0.061 18 | nd | 0.061 19 | nd | nd | nd | nd | nd | nd |
| OR Quart 3 | 5.2 | nd | 5.2 | nd | nd | nd | nd | nd | nd |
| p Value | 0.16 | nd | 0.17 | nd | nd | nd | nd | nd | nd |
| 95% CI of OR Quart3 | 0.52 53 | nd | 0.50 54 | nd | nd | nd | nd | nd | nd |
| OR Quart 4 | 3.6 | nd | 3.9 | nd | nd | nd | nd | nd | nd |
| p Value | 0.29 | nd | 0.27 | nd | nd | nd | nd | nd | nd |
| 95% CI of OR Quart4 | 0.34 39 | nd | 0.35 43 | nd | nd | nd | nd | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.3 | 7.75 | nd | nd | nd | nd |
| Average | 42.0 | 23.4 | nd | nd | nd | nd |
| Stdev | 59.4 | 35.2 | nd | nd | nd | nd |
| p(t-test) | | 0.36 | nd | nd | nd | nd |
| Min | 0.400 | 3.79 | nd | nd | nd | nd |
| Max | 366 | 114 | nd | nd | nd | nd |
| n (Samp) | 82 | 9 | nd | nd | nd | nd |
| n (Patient) | 59 | 9 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.9 | 7.75 | nd | nd | nd | nd |
| Average | 46.6 | 23.4 | nd | nd | nd | nd |
| Stdev | 65.1 | 35.2 | nd | nd | nd | nd |
| p(t-test) | | 0.30 | nd | nd | nd | nd |
| Min | 0.400 | 3.79 | nd | nd | nd | nd |
| Max | 366 | 114 | nd | nd | nd | nd |
| n (Samp) | 64 | 9 | nd | nd | nd | nd |
| n (Patient) | 46 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.32 | nd | 0.28 | nd | nd | nd | nd | nd | nd |
| SE | 0.10 | nd | 0.10 | nd | nd | nd | nd | nd | nd |
| p | 0.085 | nd | 0.032 | nd | nd | nd | nd | nd | nd |
| nCohort 1 | 82 | nd | 64 | nd | nd | nd | nd | nd | nd |
| nCohort 2 | 9 | nd | 9 | nd | nd | nd | nd | nd | nd |
| Cutoff 1 | 4.69 | nd | 4.69 | nd | nd | nd | nd | nd | nd |
| Sens 1 | 78% | nd | 78% | nd | nd | nd | nd | nd | nd |
| Spec 1 | 9% | nd | 3% | nd | nd | nd | nd | nd | nd |
| Cutoff 2 | 4.59 | nd | 4.59 | nd | nd | nd | nd | nd | nd |
| Sens 2 | 89% | nd | 89% | nd | nd | nd | nd | nd | nd |
| Spec 2 | 9% | nd | 3% | nd | nd | nd | nd | nd | nd |
| Cutoff 3 | 3.65 | nd | 0.400 | nd | nd | nd | nd | nd | nd |
| Sens 3 | 100% | nd | 100% | nd | nd | nd | nd | nd | nd |
| Spec 3 | 6% | nd | 2% | nd | nd | nd | nd | nd | nd |
| Cutoff 4 | 43.6 | nd | 45.3 | nd | nd | nd | nd | nd | nd |
| Sens 4 | 11% | nd | 11% | nd | nd | nd | nd | nd | nd |
| Spec 4 | 71% | nd | 70% | nd | nd | nd | nd | nd | nd |
| Cutoff 5 | 52.2 | nd | 54.5 | nd | nd | nd | nd | nd | nd |
| Sens 5 | 11% | nd | 11% | nd | nd | nd | nd | nd | nd |
| Spec 5 | 80% | nd | 81% | nd | nd | nd | nd | nd | nd |
| Cutoff 6 | 87.8 | nd | 106 | nd | nd | nd | nd | nd | nd |
| Sens 6 | 11% | nd | 11% | nd | nd | nd | nd | nd | nd |
| Spec 6 | 90% | nd | 91% | nd | nd | nd | nd | nd | nd |
| OR Quart 2 | 2.1 | nd | 2.2 | nd | nd | nd | nd | nd | nd |
| p Value | 0.56 | nd | 0.52 | nd | nd | nd | nd | nd | nd |
| 95% CI of OR Quart2 | 0.18 25 | nd | 0.19 27 | nd | nd | nd | nd | nd | nd |
| OR Quart 3 | 1.0 | nd | 1.1 | nd | nd | nd | nd | nd | nd |
| p Value | 1.0 | nd | 0.97 | nd | nd | nd | nd | nd | nd |
| 95% CI of OR Quart3 | 0.059 17 | nd | 0.061 18 | nd | nd | nd | nd | nd | nd |
| OR Quart 4 | 6.5 | nd | 6.9 | nd | nd | nd | nd | nd | nd |
| p Value | 0.10 | nd | 0.094 | nd | nd | nd | nd | nd | nd |
| 95% CI of OR Quart4 | 0.69 61 | nd | 0.72 67 | nd | nd | nd | nd | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 77700 | 123000 | 77700 | 103000 | 77700 | 117000 |
| Average | 113000 | 130000 | 113000 | 122000 | 113000 | 146000 |
| Stdev | 97300 | 57100 | 97300 | 77800 | 97300 | 81700 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.64 | | 0.70 | | 0.26 |
| Min | 14500 | 51600 | 14500 | 3320 | 14500 | 50500 |
| Max | 621000 | 231000 | 621000 | 340000 | 621000 | 285000 |
| n (Samp) | 123 | 7 | 123 | 20 | 123 | 12 |
| n (Patient) | 97 | 7 | 97 | 20 | 97 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 86600 | 95800 | 86600 | 105000 |
| Average | nd | nd | 123000 | 105000 | 123000 | 128000 |
| Stdev | nd | nd | 101000 | 52100 | 101000 | 80100 |
| p(t-test) | nd | nd | | 0.44 | | 0.88 |
| Min | nd | nd | 25800 | 3320 | 25800 | 50500 |
| Max | nd | nd | 621000 | 209000 | 621000 | 277000 |
| n (Samp) | nd | nd | 104 | 20 | 104 | 10 |
| n (Patient) | nd | nd | 81 | 20 | 81 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | nd | nd | 0.60 | nd | 0.54 | 0.69 | nd | 0.57 |
| SE | 0.12 | nd | nd | 0.071 | nd | 0.072 | 0.088 | nd | 0.098 |
| p | 0.14 | nd | nd | 0.16 | nd | 0.54 | 0.031 | nd | 0.47 |
| nCohort 1 | 123 | nd | nd | 123 | nd | 104 | 123 | nd | 104 |
| nCohort 2 | 7 | nd | nd | 20 | nd | 20 | 12 | nd | 10 |
| Cutoff 1 | 108000 | nd | nd | 81200 | nd | 85700 | 100000 | nd | 79100 |
| Sens 1 | 71% | nd | nd | 70% | nd | 70% | 75% | nd | 70% |
| Spec 1 | 70% | nd | nd | 53% | nd | 50% | 67% | nd | 45% |
| Cutoff 2 | 91400 | nd | nd | 69300 | nd | 70100 | 79100 | nd | 75400 |
| Sens 2 | 86% | nd | nd | 80% | nd | 80% | 83% | nd | 80% |
| Spec 2 | 63% | nd | nd | 45% | nd | 38% | 52% | nd | 40% |
| Cutoff 3 | 51000 | nd | nd | 54100 | nd | 63600 | 75400 | nd | 54100 |
| Sens 3 | 100% | nd | nd | 90% | nd | 90% | 92% | nd | 90% |
| Spec 3 | 20% | nd | nd | 24% | nd | 33% | 48% | nd | 18% |
| Cutoff 4 | 110000 | nd | nd | 110000 | nd | 114000 | 110000 | nd | 114000 |
| Sens 4 | 57% | nd | nd | 40% | nd | 30% | 50% | nd | 40% |
| Spec 4 | 71% | nd | nd | 71% | nd | 70% | 71% | nd | 70% |
| Cutoff 5 | 165000 | nd | nd | 165000 | nd | 184000 | 165000 | nd | 184000 |
| Sens 5 | 29% | nd | nd | 25% | nd | 10% | 25% | nd | 20% |
| Spec 5 | 80% | nd | nd | 80% | nd | 81% | 80% | nd | 81% |
| Cutoff 6 | 248000 | nd | nd | 248000 | nd | 257000 | 248000 | nd | 257000 |
| Sens 6 | 0% | nd | nd | 5% | nd | 0% | 25% | nd | 20% |
| Spec 6 | 90% | nd | nd | 90% | nd | 90% | 90% | nd | 90% |
| OR Quart 2 | 0 | nd | nd | 0.97 | nd | 3.5 | 2.0 | nd | 0.96 |
| p Value | na | nd | nd | 0.97 | nd | 0.15 | 0.58 | nd | 0.97 |
| 95% CI of | na | nd | nd | 0.18 | nd | 0.64 | 0.17 | nd | 0.13 |
| OR Quart2 | na | nd | nd | 5.2 | nd | 19 | 23 | nd | 7.4 |
| OR Quart 3 | 4.4 | nd | nd | 3.0 | nd | 5.0 | 6.9 | nd | 2.2 |
| p Value | 0.19 | nd | nd | 0.12 | nd | 0.054 | 0.083 | nd | 0.40 |
| 95% CI of | 0.47 | nd | nd | 0.74 | nd | 0.98 | 0.78 | nd | 0.36 |
| OR Quart3 | 42 | nd | nd | 13 | nd | 26 | 60 | nd | 13 |
| OR Quart 4 | 2.0 | nd | nd | 2.1 | nd | 2.1 | 3.1 | nd | 0.96 |
| p Value | 0.58 | nd | nd | 0.31 | nd | 0.40 | 0.34 | nd | 0.97 |
| 95% CI of | 0.17 | nd | nd | 0.49 | nd | 0.36 | 0.31 | nd | 0.13 |
| OR Quart4 | 23 | nd | nd | 9.3 | nd | 13 | 31 | nd | 7.4 |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 178 | 415 | nd | nd | nd | nd |
| Average | 989 | 2040 | nd | nd | nd | nd |
| Stdev | 1850 | 2760 | nd | nd | nd | nd |
| p(t-test) | | 0.13 | nd | nd | nd | nd |
| Min | 0.116 | 55.2 | nd | nd | nd | nd |
| Max | 6840 | 6400 | nd | nd | nd | nd |
| n (Samp) | 80 | 9 | nd | nd | nd | nd |
| n (Patient) | 58 | 9 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 182 | 415 | nd | nd | nd | nd |
| Average | 1090 | 2040 | nd | nd | nd | nd |
| Stdev | 2000 | 2760 | nd | nd | nd | nd |
| p(t-test) | | 0.21 | nd | nd | nd | nd |
| Min | 0.116 | 55.2 | nd | nd | nd | nd |
| Max | 6840 | 6400 | nd | nd | nd | nd |
| n (Samp) | 62 | 9 | nd | nd | nd | nd |
| n (Patient) | 45 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | nd | 0.56 | nd | nd | nd | nd | nd | nd |
| SE | 0.10 | nd | 0.11 | nd | nd | nd | nd | nd | nd |
| p | 0.44 | nd | 0.55 | nd | nd | nd | nd | nd | nd |
| nCohort 1 | 80 | nd | 62 | nd | nd | nd | nd | nd | nd |
| nCohort 2 | 9 | nd | 9 | nd | nd | nd | nd | nd | nd |
| Cutoff 1 | 82.7 | nd | 82.7 | nd | nd | nd | nd | nd | nd |
| Sens 1 | 78% | nd | 78% | nd | nd | nd | nd | nd | nd |
| Spec 1 | 20% | nd | 19% | nd | nd | nd | nd | nd | nd |
| Cutoff 2 | 61.7 | nd | 55.2 | nd | nd | nd | nd | nd | nd |
| Sens 2 | 89% | nd | 89% | nd | nd | nd | nd | nd | nd |
| Spec 2 | 18% | nd | 16% | nd | nd | nd | nd | nd | nd |
| Cutoff 3 | 53.6 | nd | 52.0 | nd | nd | nd | nd | nd | nd |
| Sens 3 | 100% | nd | 100% | nd | nd | nd | nd | nd | nd |
| Spec 3 | 15% | nd | 15% | nd | nd | nd | nd | nd | nd |
| Cutoff 4 | 429 | nd | 470 | nd | nd | nd | nd | nd | nd |
| Sens 4 | 44% | nd | 44% | nd | nd | nd | nd | nd | nd |
| Spec 4 | 70% | nd | 71% | nd | nd | nd | nd | nd | nd |
| Cutoff 5 | 1300 | nd | 1410 | nd | nd | nd | nd | nd | nd |
| Sens 5 | 33% | nd | 33% | nd | nd | nd | nd | nd | nd |
| Spec 5 | 80% | nd | 81% | nd | nd | nd | nd | nd | nd |
| Cutoff 6 | 3790 | nd | 5160 | nd | nd | nd | nd | nd | nd |
| Sens 6 | 33% | nd | 22% | nd | nd | nd | nd | nd | nd |
| Spec 6 | 90% | nd | 90% | nd | nd | nd | nd | nd | nd |
| OR Quart 2 | 0.30 | nd | 0.27 | nd | nd | nd | nd | nd | nd |
| p Value | 0.32 | nd | 0.29 | nd | nd | nd | nd | nd | nd |
| 95% CI of OR Quart2 | 0.029 3.2 | nd | 0.026 2.9 | nd | nd | nd | nd | nd | nd |
| OR Quart 3 | 0.30 | nd | 0.58 | nd | nd | nd | nd | nd | nd |
| p Value | 0.32 | nd | 0.58 | nd | nd | nd | nd | nd | nd |
| 95% CI of | 0.029 | nd | 0.085 | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart3 | 3.2 | nd | 4.0 | nd | nd | nd | nd | nd | nd |
| OR Quart 4 | 1.3 | nd | 0.93 | nd | nd | nd | nd | nd | nd |
| p Value | 0.73 | nd | 0.94 | nd | nd | nd | nd | nd | nd |
| 95% CI of | 0.26 | nd | 0.16 | nd | nd | nd | nd | nd | nd |
| OR Quart4 | 6.8 | nd | 5.4 | nd | nd | nd | nd | nd | nd |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.71 | 2.13 | 1.71 | 2.32 | 1.71 | 2.36 |
| Average | 2.73 | 2.72 | 2.73 | 2.84 | 2.73 | 2.41 |
| Stdev | 3.35 | 2.76 | 3.35 | 2.87 | 3.35 | 1.73 |
| p(t-test) | | 0.99 | | 0.86 | | 0.69 |
| Min | 0.183 | 0.324 | 0.183 | 0.245 | 0.183 | 0.355 |
| Max | 33.6 | 14.1 | 33.6 | 16.6 | 33.6 | 7.86 |
| n (Samp) | 434 | 27 | 434 | 34 | 434 | 17 |
| n (Patient) | 173 | 27 | 173 | 34 | 173 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.74 | 2.90 | 1.74 | 3.41 | 1.74 | 2.55 |
| Average | 2.76 | 4.92 | 2.76 | 2.99 | 2.76 | 3.08 |
| Stdev | 3.28 | 6.27 | 3.28 | 1.97 | 3.28 | 1.69 |
| p(t-test) | | 0.11 | | 0.84 | | 0.80 |
| Min | 0.183 | 0.324 | 0.183 | 0.245 | 0.183 | 0.355 |
| Max | 33.6 | 17.1 | 33.6 | 5.37 | 33.6 | 5.44 |
| n (Samp) | 535 | 6 | 535 | 9 | 535 | 7 |
| n (Patient) | 207 | 6 | 207 | 9 | 207 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.77 | 2.36 | 1.77 | 2.32 | 1.77 | 1.93 |
| Average | 2.89 | 2.85 | 2.89 | 2.92 | 2.89 | 2.26 |
| Stdev | 3.55 | 2.71 | 3.55 | 2.91 | 3.55 | 1.79 |
| p(t-test) | | 0.95 | | 0.97 | | 0.47 |
| Min | 0.183 | 0.491 | 0.183 | 0.710 | 0.183 | 0.324 |
| Max | 33.6 | 14.1 | 33.6 | 16.6 | 33.6 | 7.86 |
| n (Samp) | 359 | 27 | 359 | 32 | 359 | 17 |
| n (Patient) | 139 | 27 | 139 | 32 | 139 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.57 | 0.55 | 0.55 | 0.58 | 0.55 | 0.55 | 0.65 | 0.48 |
| SE | 0.058 | 0.12 | 0.059 | 0.053 | 0.10 | 0.054 | 0.073 | 0.11 | 0.072 |
| p | 0.56 | 0.57 | 0.43 | 0.35 | 0.42 | 0.37 | 0.54 | 0.20 | 0.81 |
| nCohort 1 | 434 | 535 | 359 | 434 | 535 | 359 | 434 | 535 | 359 |
| nCohort 2 | 27 | 6 | 27 | 34 | 9 | 32 | 17 | 7 | 17 |
| Cutoff 1 | 1.26 | 0.825 | 1.49 | 1.23 | 1.22 | 1.27 | 1.68 | 2.44 | 1.62 |
| Sens 1 | 70% | 83% | 70% | 71% | 78% | 72% | 71% | 71% | 71% |
| Spec 1 | 35% | 16% | 41% | 34% | 32% | 34% | 48% | 66% | 45% |
| Cutoff 2 | 1.04 | 0.825 | 1.09 | 0.971 | 0.562 | 1.06 | 1.04 | 2.28 | 0.679 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 2 | 81% | 83% | 81% | 82% | 89% | 81% | 82% | 86% | 82% |
| Spec 2 | 28% | 16% | 28% | 25% | 7% | 27% | 28% | 63% | 10% |
| Cutoff 3 | 0.768 | 0.314 | 0.883 | 0.795 | 0.241 | 0.872 | 0.611 | 0.314 | 0.562 |
| Sens 3 | 93% | 100% | 93% | 91% | 100% | 91% | 94% | 100% | 94% |
| Spec 3 | 15% | 2% | 18% | 17% | 1% | 18% | 10% | 2% | 6% |
| Cutoff 4 | 2.71 | 2.71 | 3.17 | 2.71 | 2.71 | 3.17 | 2.71 | 2.71 | 3.17 |
| Sens 4 | 26% | 50% | 22% | 38% | 56% | 34% | 29% | 43% | 12% |
| Spec 4 | 70% | 70% | 70% | 70% | 70% | 70% | 70% | 70% | 70% |
| Cutoff 5 | 3.99 | 3.88 | 4.22 | 3.99 | 3.88 | 4.22 | 3.99 | 3.88 | 4.22 |
| Sens 5 | 11% | 33% | 11% | 18% | 33% | 16% | 12% | 29% | 12% |
| Spec 5 | 80% | 80% | 80% | 80% | 80% | 80% | 80% | 80% | 80% |
| Cutoff 6 | 5.44 | 5.46 | 5.97 | 5.44 | 5.46 | 5.97 | 5.44 | 5.46 | 5.97 |
| Sens 6 | 7% | 17% | 7% | 12% | 0% | 3% | 6% | 0% | 6% |
| Spec 6 | 90% | 90% | 90% | 90% | 90% | 90% | 90% | 90% | 90% |
| OR Quart 2 | 1.2 | 0 | 1.5 | 0.85 | 0.50 | 0.99 | 0.99 | 0 | 4.3 |
| p Value | 0.76 | na | 0.53 | 0.78 | 0.57 | 0.99 | 0.99 | na | 0.071 |
| 95% CI of OR Quart2 | 0.36 | na | 0.41 | 0.28 | 0.044 | 0.31 | 0.20 | na | 0.88 |
| | 4.1 | na | 5.6 | 2.6 | 5.5 | 3.2 | 5.0 | na | 21 |
| OR Quart 3 | 2.1 | 0.50 | 3.0 | 1.8 | 0.50 | 2.3 | 3.1 | 3.0 | 1.5 |
| p Value | 0.19 | 0.57 | 0.070 | 0.24 | 0.57 | 0.10 | 0.092 | 0.34 | 0.65 |
| 95% CI of OR Quart3 | 0.69 | 0.044 | 0.91 | 0.68 | 0.044 | 0.84 | 0.83 | 0.31 | 0.25 |
| | 6.3 | 5.5 | 9.7 | 4.7 | 5.5 | 6.4 | 12 | 30 | 9.3 |
| OR Quart 4 | 1.2 | 1.5 | 1.5 | 1.3 | 2.6 | 1.2 | 0.65 | 3.0 | 2.0 |
| p Value | 0.77 | 0.66 | 0.53 | 0.61 | 0.27 | 0.79 | 0.65 | 0.34 | 0.42 |
| 95% CI of OR Quart4 | 0.36 | 0.25 | 0.41 | 0.47 | 0.49 | 0.38 | 0.11 | 0.31 | 0.37 |
| | 4.0 | 9.1 | 5.6 | 3.6 | 13 | 3.6 | 4.0 | 29 | 11 |

## Myoglobin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 28.5 | 65.8 | 28.5 | 80.9 | 28.5 | 82.0 |
| Average | 82.5 | 171 | 82.5 | 252 | 82.5 | 221 |
| Stdev | 182 | 372 | 182 | 471 | 182 | 396 |
| p(t-test) | | 0.024 | | 1.2E-5 | | 0.0040 |
| Min | 3.55 | 4.22 | 3.55 | 3.96 | 3.55 | 5.12 |
| Max | 2130 | 1880 | 2130 | 1880 | 2130 | 1310 |
| n (Samp) | 434 | 27 | 434 | 34 | 434 | 17 |
| n (Patient) | 173 | 27 | 173 | 34 | 173 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 32.7 | 142 | 32.7 | 125 | 32.7 | 151 |
| Average | 87.4 | 640 | 87.4 | 653 | 87.4 | 396 |
| Stdev | 195 | 849 | 195 | 793 | 195 | 429 |
| p(t-test) | | 3.2E-10 | | 3.3E-14 | | 5.1E-5 |
| Min | 3.55 | 35.2 | 3.55 | 13.0 | 3.55 | 17.5 |
| Max | 2130 | 1880 | 2130 | 1880 | 2130 | 1180 |
| n (Samp) | 535 | 6 | 535 | 9 | 535 | 7 |
| n (Patient) | 207 | 6 | 207 | 9 | 207 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 30.3 | 56.9 | 30.3 | 86.9 | 30.3 | 79.1 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 83.3 | 103 | 83.3 | 171 | 83.3 | 261 |
| Stdev | 185 | 149 | 185 | 306 | 185 | 518 |
| p(t-test) | | 0.59 | | 0.016 | | 7.1E-4 |
| Min | 4.60 | 4.22 | 4.60 | 3.96 | 4.60 | 5.12 |
| Max | 2130 | 646 | 2130 | 1410 | 2130 | 1880 |
| n (Samp) | 359 | 27 | 359 | 32 | 359 | 17 |
| n (Patient) | 139 | 27 | 139 | 32 | 139 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.83 | 0.64 | 0.66 | 0.70 | 0.67 | 0.64 | 0.81 | 0.65 |
| SE | 0.059 | 0.10 | 0.059 | 0.053 | 0.098 | 0.054 | 0.074 | 0.099 | 0.074 |
| p | 0.0056 | 0.0015 | 0.015 | 0.0030 | 0.041 | 0.0022 | 0.050 | 0.0018 | 0.040 |
| nCohort 1 | 434 | 535 | 359 | 434 | 535 | 359 | 434 | 535 | 359 |
| nCohort 2 | 27 | 6 | 27 | 34 | 9 | 32 | 17 | 7 | 17 |
| Cutoff 1 | 42.7 | 66.9 | 42.7 | 34.8 | 31.4 | 38.6 | 34.8 | 101 | 34.8 |
| Sens 1 | 70% | 83% | 70% | 71% | 78% | 72% | 71% | 71% | 71% |
| Spec 1 | 64% | 73% | 63% | 57% | 50% | 59% | 57% | 82% | 55% |
| Cutoff 2 | 22.9 | 66.9 | 22.9 | 16.2 | 19.2 | 18.9 | 17.5 | 87.6 | 18.7 |
| Sens 2 | 81% | 83% | 81% | 82% | 89% | 81% | 82% | 86% | 82% |
| Spec 2 | 40% | 73% | 39% | 25% | 30% | 31% | 27% | 78% | 30% |
| Cutoff 3 | 18.3 | 34.8 | 18.3 | 12.9 | 12.9 | 12.9 | 11.1 | 17.4 | 11.1 |
| Sens 3 | 93% | 100% | 93% | 91% | 100% | 91% | 94% | 100% | 94% |
| Spec 3 | 30% | 53% | 30% | 18% | 17% | 18% | 13% | 25% | 12% |
| Cutoff 4 | 56.0 | 60.1 | 57.8 | 56.0 | 60.1 | 57.8 | 56.0 | 60.1 | 57.8 |
| Sens 4 | 52% | 83% | 48% | 62% | 56% | 69% | 65% | 86% | 65% |
| Spec 4 | 70% | 70% | 70% | 70% | 70% | 70% | 70% | 70% | 70% |
| Cutoff 5 | 93.4 | 95.1 | 95.1 | 93.4 | 95.1 | 95.1 | 93.4 | 95.1 | 95.1 |
| Sens 5 | 33% | 67% | 22% | 44% | 56% | 47% | 35% | 71% | 35% |
| Spec 5 | 80% | 80% | 80% | 80% | 80% | 80% | 80% | 80% | 80% |
| Cutoff 6 | 182 | 180 | 188 | 182 | 180 | 188 | 182 | 180 | 188 |
| Sens 6 | 15% | 33% | 7% | 24% | 44% | 19% | 18% | 43% | 18% |
| Spec 6 | 90% | 90% | 90% | 90% | 90% | 90% | 90% | 90% | 90% |
| OR Quart 2 | 2.6 | >0 | 2.6 | 0.27 | 2.0 | 0.32 | 1.5 | 0 | 1.0 |
| p Value | 0.27 | <na | 0.27 | 0.11 | 0.57 | 0.16 | 0.66 | na | 1.0 |
| 95% CI of OR Quart2 | 0.49 14 | >na na | 0.48 13 | 0.056 1.3 | 0.18 22 | 0.062 1.6 | 0.25 9.2 | na na | 0.14 7.3 |
| OR Quart 3 | 4.8 | >2.0 | 4.9 | 1.0 | 1.0 | 1.2 | 0.99 | 0 | 2.0 |
| p Value | 0.048 | <0.57 | 0.047 | 1.0 | 1.0 | 0.79 | 0.99 | na | 0.42 |
| 95% CI of OR Quart3 | 1.0 23 | >0.18 na | 1.0 23 | 0.34 2.9 | 0.062 16 | 0.38 3.6 | 0.14 7.2 | na na | 0.37 11 |
| OR Quart 4 | 5.9 | >4.1 | 6.0 | 2.9 | 5.2 | 3.2 | 5.3 | 6.2 | 4.9 |
| p Value | 0.023 | <0.21 | 0.022 | 0.024 | 0.14 | 0.020 | 0.033 | 0.094 | 0.047 |
| 95% CI of OR Quart4 | 1.3 27 | >0.45 na | 1.3 28 | 1.1 7.1 | 0.59 45 | 1.2 8.5 | 1.1 25 | 0.73 52 | 1.0 23 |

## Mucin-16

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.34 | 1.72 | 1.34 | 1.78 | 1.34 | 2.02 |
| Average | 4.05 | 40.6 | 4.05 | 23.6 | 4.05 | 19.4 |
| Stdev | 10.9 | 98.2 | 10.9 | 66.0 | 10.9 | 61.0 |
| p(t-test) | | 4.2E-6 | | 2.0E-4 | | 0.0027 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.117 | 1.12 | 0.117 | 0.191 | 0.117 | 0.549 |
| Max | 131 | 263 | 131 | 253 | 131 | 248 |
| n (Samp) | 196 | 7 | 196 | 22 | 196 | 16 |
| n (Patient) | 130 | 7 | 130 | 22 | 130 | 16 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.46 | 2.02 | 1.46 | 3.28 |
| Average | nd | nd | 4.51 | 27.2 | 4.51 | 23.4 |
| Stdev | nd | nd | 11.7 | 70.6 | 11.7 | 67.5 |
| p(t-test) | nd | nd | | 1.8E-4 | | 0.0018 |
| Min | nd | nd | 0.122 | 0.191 | 0.122 | 0.549 |
| Max | nd | nd | 131 | 253 | 131 | 248 |
| n (Samp) | nd | nd | 170 | 19 | 170 | 13 |
| n (Patient) | nd | nd | 108 | 19 | 108 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | nd | nd | 0.60 | nd | 0.59 | 0.64 | nd | 0.66 |
| SE | 0.11 | nd | nd | 0.067 | nd | 0.072 | 0.077 | nd | 0.085 |
| p | 0.16 | nd | nd | 0.12 | nd | 0.23 | 0.078 | nd | 0.059 |
| nCohort 1 | 196 | nd | nd | 196 | nd | 170 | 196 | nd | 170 |
| nCohort 2 | 7 | nd | nd | 22 | nd | 19 | 16 | nd | 13 |
| Cutoff 1 | 1.33 | nd | nd | 1.09 | nd | 1.12 | 1.23 | nd | 1.28 |
| Sens 1 | 71% | nd | nd | 73% | nd | 74% | 75% | nd | 77% |
| Spec 1 | 50% | nd | nd | 41% | nd | 39% | 46% | nd | 44% |
| Cutoff 2 | 1.23 | nd | nd | 1.01 | nd | 0.633 | 1.01 | nd | 1.01 |
| Sens 2 | 86% | nd | nd | 82% | nd | 84% | 81% | nd | 85% |
| Spec 2 | 46% | nd | nd | 38% | nd | 22% | 38% | nd | 35% |
| Cutoff 3 | 1.09 | nd | nd | 0.464 | nd | 0.424 | 0.761 | nd | 0.821 |
| Sens 3 | 100% | nd | nd | 91% | nd | 95% | 94% | nd | 92% |
| Spec 3 | 41% | nd | nd | 14% | nd | 12% | 29% | nd | 32% |
| Cutoff 4 | 2.77 | nd | nd | 2.77 | nd | 3.55 | 2.77 | nd | 3.55 |
| Sens 4 | 43% | nd | nd | 41% | nd | 37% | 44% | nd | 46% |
| Spec 4 | 70% | nd | nd | 70% | nd | 70% | 70% | nd | 70% |
| Cutoff 5 | 4.41 | nd | nd | 4.41 | nd | 4.88 | 4.41 | nd | 4.88 |
| Sens 5 | 29% | nd | nd | 32% | nd | 32% | 38% | nd | 38% |
| Spec 5 | 80% | nd | nd | 80% | nd | 80% | 80% | nd | 80% |
| Cutoff 6 | 6.95 | nd | nd | 6.95 | nd | 7.65 | 6.95 | nd | 7.65 |
| Sens 6 | 29% | nd | nd | 27% | nd | 32% | 31% | nd | 38% |
| Spec 6 | 90% | nd | nd | 90% | nd | 90% | 90% | nd | 90% |
| OR Quart 2 | >2.0 | nd | nd | 0.98 | nd | 1.0 | 6.6 | nd | 3.1 |
| p Value | <0.57 | nd | nd | 0.98 | nd | 1.0 | 0.085 | nd | 0.34 |
| 95% CI of | >0.18 | nd | nd | 0.23 | nd | 0.23 | 0.77 | nd | 0.31 |
| OR Quart2 | na | nd | nd | 4.1 | nd | 4.3 | 57 | nd | 31 |
| OR Quart 3 | >3.1 | nd | nd | 1.6 | nd | 1.0 | 3.1 | nd | 3.1 |
| p Value | <0.33 | nd | nd | 0.51 | nd | 1.0 | 0.33 | nd | 0.34 |
| 95% CI of | >0.31 | nd | nd | 0.42 | nd | 0.23 | 0.31 | nd | 0.31 |
| OR Quart3 | na | nd | nd | 5.9 | nd | 4.3 | 31 | nd | 31 |
| OR Quart 4 | >2.0 | nd | nd | 2.1 | nd | 1.8 | 6.6 | nd | 6.6 |
| p Value | <0.57 | nd | nd | 0.24 | nd | 0.36 | 0.085 | nd | 0.087 |
| 95% CI of | >0.18 | nd | nd | 0.60 | nd | 0.50 | 0.77 | nd | 0.76 |
| OR Quart4 | na | nd | nd | 7.5 | nd | 6.7 | 57 | nd | 57 |

## Tumor necrosis factor receptor superfamily member 10B

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 0.00692 | 1.00E-9 | 0.00181 | 1.00E-9 | 0.00572 |
| Average | 0.00543 | 0.0417 | 0.00543 | 0.0242 | 0.00543 | 0.0256 |
| Stdev | 0.0150 | 0.0972 | 0.0150 | 0.0668 | 0.0150 | 0.0736 |
| p(t-test) | | 4.5E-5 | | 0.0018 | | 0.0020 |
| Min | 1.00E-9 | 1.00E-9 | 1.00E-9 | 1.00E-9 | 1.00E-9 | 1.00E-9 |
| Max | 0.114 | 0.262 | 0.114 | 0.286 | 0.114 | 0.300 |
| n (Samp) | 189 | 7 | 189 | 18 | 189 | 16 |
| n (Patient) | 125 | 7 | 125 | 18 | 125 | 16 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.000420 | 0.00142 | 0.000420 | 0.00220 |
| Average | nd | nd | 0.00637 | 0.0151 | 0.00637 | 0.00604 |
| Stdev | nd | nd | 0.0161 | 0.0277 | 0.0161 | 0.00762 |
| p(t-test) | nd | nd | | 0.053 | | 0.94 |
| Min | nd | nd | 1.00E-9 | 1.00E-9 | 1.00E-9 | 1.00E-9 |
| Max | nd | nd | 0.114 | 0.108 | 0.114 | 0.0222 |
| n (Samp) | nd | nd | 162 | 17 | 162 | 13 |
| n (Patient) | nd | nd | 101 | 17 | 101 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | nd | nd | 0.61 | nd | 0.59 | 0.65 | nd | 0.57 |
| SE | 0.12 | nd | nd | 0.073 | nd | 0.076 | 0.077 | nd | 0.086 |
| p | 0.25 | nd | nd | 0.12 | nd | 0.25 | 0.060 | nd | 0.38 |
| nCohort 1 | 189 | nd | nd | 189 | nd | 162 | 189 | nd | 162 |
| nCohort 2 | 7 | nd | nd | 18 | nd | 17 | 16 | nd | 13 |
| Cutoff 1 | 0 | nd | nd | 0 | nd | 0 | 0 | nd | 0 |
| Sens 1 | 100% | nd | nd | 100% | nd | 100% | 100% | nd | 100% |
| Spec 1 | 0% | nd | nd | 0% | nd | 0% | 0% | nd | 0% |
| Cutoff 2 | 0 | nd | nd | 0 | nd | 0 | 0 | nd | 0 |
| Sens 2 | 100% | nd | nd | 100% | nd | 100% | 100% | nd | 100% |
| Spec 2 | 0% | nd | nd | 0% | nd | 0% | 0% | nd | 0% |
| Cutoff 3 | 0 | nd | nd | 0 | nd | 0 | 0 | nd | 0 |
| Sens 3 | 100% | nd | nd | 100% | nd | 100% | 100% | nd | 100% |
| Spec 3 | 0% | nd | nd | 0% | nd | 0% | 0% | nd | 0% |
| Cutoff 4 | 0.00367 | nd | nd | 0.00367 | nd | 0.00444 | 0.00367 | nd | 0.00444 |
| Sens 4 | 57% | nd | nd | 44% | nd | 47% | 50% | nd | 38% |
| Spec 4 | 70% | nd | nd | 70% | nd | 70% | 70% | nd | 70% |
| Cutoff 5 | 0.00692 | nd | nd | 0.00692 | nd | 0.00819 | 0.00692 | nd | 0.00819 |
| Sens 5 | 43% | nd | nd | 39% | nd | 35% | 50% | nd | 31% |
| Spec 5 | 81% | nd | nd | 81% | nd | 82% | 81% | nd | 82% |
| Cutoff 6 | 0.0124 | nd | nd | 0.0124 | nd | 0.0136 | 0.0124 | nd | 0.0136 |
| Sens 6 | 29% | nd | nd | 28% | nd | 29% | 31% | nd | 23% |
| Spec 6 | 91% | nd | nd | 91% | nd | 90% | 91% | nd | 90% |
| OR Quart 2 | >3.2 | nd | nd | >7.9 | nd | 1.3 | >5.5 | nd | 4.2 |
| p Value | <0.32 | nd | nd | <0.057 | nd | 0.72 | <0.12 | nd | 0.21 |
| 95% CI of OR Quart2 | >0.32 | nd | nd | >0.94 | nd | 0.28 | >0.62 | nd | 0.45 |
| | na | nd | nd | na | nd | 6.3 | na | nd | 39 |
| OR Quart 3 | >0 | nd | nd | >4.2 | nd | 0.98 | >3.2 | nd | 3.1 |
| p Value | <na | nd | nd | <0.20 | nd | 0.98 | <0.32 | nd | 0.34 |
| 95% CI of OR Quart3 | >na | nd | nd | >0.46 | nd | 0.19 | >0.32 | nd | 0.31 |
| | na | nd | nd | na | nd | 5.1 | na | nd | 31 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 4 | >4.4 | nd | nd | >7.9 | nd | 2.5 | >9.3 | nd | 5.4 |
| p Value | <0.20 | nd | nd | <0.057 | nd | 0.20 | <0.039 | nd | 0.13 |
| 95% CI of | >0.47 | nd | nd | >0.94 | nd | 0.61 | >1.1 | nd | 0.60 |
| OR Quart4 | na | nd | nd | na | nd | 10 | na | nd | 48 |

## Cellular tumor antigen p53

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 1.00E-9 | 1.00E-9 | 1.00E-9 | 1.00E-9 | 1.00E-9 |
| Average | 0.0690 | 0.289 | 0.0690 | 0.143 | 0.0690 | 0.172 |
| Stdev | 0.419 | 0.698 | 0.419 | 0.551 | 0.419 | 0.595 |
| p(t-test) | | 0.19 | | 0.49 | | 0.36 |
| Min | 1.00E-9 | 1.00E-9 | 1.00E-9 | 1.00E-9 | 1.00E-9 | 1.00E-9 |
| Max | 3.70 | 1.87 | 3.70 | 2.35 | 3.70 | 2.39 |
| n (Samp) | 189 | 7 | 189 | 18 | 189 | 16 |
| n (Patient) | 125 | 7 | 125 | 18 | 125 | 16 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.00E-9 | 1.00E-9 | 1.00E-9 | 1.00E-9 |
| Average | nd | nd | 0.0804 | 0.0131 | 0.0804 | 0.0283 |
| Stdev | nd | nd | 0.452 | 0.0316 | 0.452 | 0.0790 |
| p(t-test) | nd | nd | | 0.54 | | 0.68 |
| Min | nd | nd | 1.00E-9 | 1.00E-9 | 1.00E-9 | 1.00E-9 |
| Max | nd | nd | 3.70 | 0.117 | 3.70 | 0.285 |
| n (Samp) | nd | nd | 162 | 17 | 162 | 13 |
| n (Patient) | nd | nd | 101 | 17 | 101 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | nd | nd | 0.62 | nd | 0.59 | 0.54 | nd | 0.52 |
| SE | 0.11 | nd | nd | 0.073 | nd | 0.076 | 0.077 | nd | 0.084 |
| p | 0.21 | nd | nd | 0.091 | nd | 0.23 | 0.57 | nd | 0.80 |
| nCohort 1 | 189 | nd | nd | 189 | nd | 162 | 189 | nd | 162 |
| nCohort 2 | 7 | nd | nd | 18 | nd | 17 | 16 | nd | 13 |
| Cutoff 1 | 0 | nd | nd | 0 | nd | 0 | 0 | nd | 0 |
| Sens 1 | 100% | nd | nd | 100% | nd | 100% | 100% | nd | 100% |
| Spec 1 | 0% | nd | nd | 0% | nd | 0% | 0% | nd | 0% |
| Cutoff 2 | 0 | nd | nd | 0 | nd | 0 | 0 | nd | 0 |
| Sens 2 | 100% | nd | nd | 100% | nd | 100% | 100% | nd | 100% |
| Spec 2 | 0% | nd | nd | 0% | nd | 0% | 0% | nd | 0% |
| Cutoff 3 | 0 | nd | nd | 0 | nd | 0 | 0 | nd | 0 |
| Sens 3 | 100% | nd | nd | 100% | nd | 100% | 100% | nd | 100% |
| Spec 3 | 0% | nd | nd | 0% | nd | 0% | 0% | nd | 0% |
| Cutoff 4 | 1.00E-9 | nd | nd | 1.00E-9 | nd | 1.00E-9 | 1.00E-9 | nd | 1.00E-9 |
| Sens 4 | 43% | nd | nd | 44% | nd | 41% | 25% | nd | 23% |
| Spec 4 | 81% | nd | nd | 81% | nd | 80% | 81% | nd | 80% |
| Cutoff 5 | 1.00E-9 | nd | nd | 1.00E-9 | nd | 0.000790 | 1.00E-9 | nd | 0.000790 |
| Sens 5 | 43% | nd | nd | 44% | nd | 35% | 25% | nd | 23% |
| Spec 5 | 81% | nd | nd | 81% | nd | 81% | 81% | nd | 81% |
| Cutoff 6 | 0.0225 | nd | nd | 0.0225 | nd | 0.0267 | 0.0225 | nd | 0.0267 |
| Sens 6 | 43% | nd | nd | 17% | nd | 12% | 25% | nd | 15% |
| Spec 6 | 90% | nd | nd | 90% | nd | 90% | 90% | nd | 90% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 2 | 3.1 | nd | nd | 10 | nd | >13 | >16 | nd | >13 |
| p Value | 0.33 | nd | nd | 0.029 | nd | <0.018 | <0.0096 | nd | <0.018 |
| 95% CI of | 0.31 | nd | nd | 1.3 | nd | >1.5 | >2.0 | nd | >1.5 |
| OR Quart2 | 31 | nd | nd | 86 | nd | na | na | nd | na |
| OR Quart 3 | 0 | nd | nd | 0 | nd | >0 | >0 | nd | >0 |
| p Value | na | nd | nd | na | nd | <na | <na | nd | <na |
| 95% CI of | na | nd | nd | na | nd | >na | >na | nd | >na |
| OR Quart3 | na | nd | nd | na | nd | na | na | nd | na |
| OR Quart 4 | 3.1 | nd | nd | 9.1 | nd | >8.1 | >4.2 | nd | >3.1 |
| p Value | 0.33 | nd | nd | 0.041 | nd | <0.055 | <0.20 | nd | <0.33 |
| 95% CI of | 0.31 | nd | nd | 1.1 | nd | >0.95 | >0.46 | nd | >0.31 |
| OR Quart4 | 31 | nd | nd | 76 | nd | na | na | nd | na |

Table 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

**Apolipoprotein A-II**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 57100 | 48800 | nd | nd | 57100 | 45400 |
| Average | 57200 | 51600 | nd | nd | 57000 | 46300 |
| Stdev | 36400 | 24300 | nd | nd | 39000 | 21400 |
| p(t-test) |  | 0.53 | nd | nd |  | 0.34 |
| Min | 7970 | 1810 | nd | nd | 7970 | 1810 |
| Max | 251000 | 100000 | nd | nd | 251000 | 75500 |
| n (Samp) | 50 | 19 | nd | nd | 41 | 14 |
| n (Patient) | 50 | 19 | nd | nd | 41 | 14 |

|  | At Enrollment | | |
|---|---|---|---|
|  | sCr or UO | sCr only | UO only |
| AUC | 0.47 | nd | 0.44 |
| SE | 0.079 | nd | 0.091 |
| p | 0.69 | nd | 0.49 |
| nCohort 1 | 50 | nd | 41 |
| nCohort 2 | 19 | nd | 14 |
| Cutoff 1 | 37400 | nd | 32500 |
| Sens 1 | 74% | nd | 71% |
| Spec 1 | 26% | nd | 24% |
| Cutoff 2 | 28800 | nd | 27600 |
| Sens 2 | 84% | nd | 86% |
| Spec 2 | 18% | nd | 22% |
| Cutoff 3 | 23900 | nd | 23900 |
| Sens 3 | 95% | nd | 93% |
| Spec 3 | 16% | nd | 20% |
| Cutoff 4 | 64400 | nd | 66800 |
| Sens 4 | 26% | nd | 21% |
| Spec 4 | 70% | nd | 71% |
| Cutoff 5 | 72300 | nd | 72300 |
| Sens 5 | 21% | nd | 14% |
| Spec 5 | 80% | nd | 80% |
| Cutoff 6 | 83600 | nd | 85700 |

|  | At Enrollment | | |
|---|---|---|---|
|  | sCr or UO | sCr only | UO only |
| Sens 6 | 11% | nd | 0% |
| Spec 6 | 90% | nd | 90% |
| OR Quart 2 | 0.80 | nd | 1.0 |
| p Value | 0.77 | nd | 1.0 |
| 95% CI of | 0.17 | nd | 0.16 |
| OR Quart2 | 3.7 | nd | 6.1 |
| OR Quart 3 | 1.1 | nd | 1.5 |
| p Value | 0.91 | nd | 0.66 |
| 95% CI of | 0.25 | nd | 0.26 |
| OR Quart3 | 4.7 | nd | 8.2 |
| OR Quart 4 | 1.1 | nd | 1.6 |
| p Value | 0.91 | nd | 0.58 |
| 95% CI of | 0.25 | nd | 0.29 |
| OR Quart4 | 4.7 | nd | 9.3 |

## Myoglobin

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 40.1 | 64.7 | nd | nd | 35.8 | 79.1 |
| Average | 139 | 182 | nd | nd | 132 | 88.9 |
| Stdev | 314 | 344 | nd | nd | 329 | 98.2 |
| p(t-test) |  | 0.60 | nd | nd |  | 0.60 |
| Min | 7.19 | 4.16 | nd | nd | 7.19 | 4.16 |
| Max | 2130 | 1310 | nd | nd | 2130 | 397 |
| n (Samp) | 54 | 23 | nd | nd | 46 | 17 |
| n (Patient) | 54 | 23 | nd | nd | 46 | 17 |

|  | At Enrollment | | |
|---|---|---|---|
|  | sCr or UO | sCr only | UO only |
| AUC | 0.55 | nd | 0.54 |
| SE | 0.073 | nd | 0.083 |
| p | 0.48 | nd | 0.66 |
| nCohort 1 | 54 | nd | 46 |
| nCohort 2 | 23 | nd | 17 |
| Cutoff 1 | 30.3 | nd | 34.2 |
| Sens 1 | 74% | nd | 71% |
| Spec 1 | 43% | nd | 50% |
| Cutoff 2 | 17.8 | nd | 15.3 |
| Sens 2 | 83% | nd | 82% |
| Spec 2 | 19% | nd | 13% |
| Cutoff 3 | 12.9 | nd | 4.16 |
| Sens 3 | 91% | nd | 94% |
| Spec 3 | 13% | nd | 0% |
| Cutoff 4 | 91.7 | nd | 90.0 |
| Sens 4 | 35% | nd | 41% |
| Spec 4 | 70% | nd | 72% |
| Cutoff 5 | 189 | nd | 141 |
| Sens 5 | 17% | nd | 18% |
| Spec 5 | 81% | nd | 80% |
| Cutoff 6 | 328 | nd | 315 |
| Sens 6 | 13% | nd | 6% |
| Spec 6 | 91% | nd | 91% |
| OR Quart 2 | 0.41 | nd | 0.29 |
| p Value | 0.26 | nd | 0.18 |

|  | At Enrollment | | |
|---|---|---|---|
|  | sCr or UO | sCr only | UO only |
| 95% CI of | 0.085 | nd | 0.046 |
| OR Quart2 | 1.9 | nd | 1.8 |
| OR Quart 3 | 1.6 | nd | 0.91 |
| p Value | 0.50 | nd | 0.90 |
| 95% CI of | 0.42 | nd | 0.20 |
| OR Quart3 | 5.9 | nd | 4.1 |
| OR Quart 4 | 0.93 | nd | 0.91 |
| p Value | 0.91 | nd | 0.90 |
| 95% CI of | 0.24 | nd | 0.20 |
| OR Quart4 | 3.6 | nd | 4.1 |

Table 8: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

**Apolipoprotein A-II**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 52600 | 43600 | 52600 | 41100 | 52600 | 57700 |
| Average | 52700 | 57800 | 52700 | 57100 | 52700 | 72600 |
| Stdev | 22500 | 48900 | 22500 | 51100 | 22500 | 58900 |
| p(t-test) | | 0.55 | | 0.62 | | 0.068 |
| Min | 5450 | 10700 | 5450 | 10700 | 5450 | 9430 |
| Max | 105000 | 176000 | 105000 | 176000 | 105000 | 176000 |
| n (Samp) | 97 | 11 | 97 | 10 | 97 | 6 |
| n (Patient) | 97 | 11 | 97 | 10 | 97 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 53400 | 57800 | 53400 | 52600 | 53400 | 57700 |
| Average | 53900 | 66500 | 53900 | 64700 | 53900 | 72600 |
| Stdev | 20600 | 54800 | 20600 | 54700 | 20600 | 58900 |
| p(t-test) | | 0.18 | | 0.25 | | 0.073 |
| Min | 8680 | 10700 | 8680 | 10700 | 8680 | 9430 |
| Max | 123000 | 176000 | 123000 | 176000 | 123000 | 176000 |
| n (Samp) | 84 | 8 | 84 | 8 | 84 | 6 |
| n (Patient) | 84 | 8 | 84 | 8 | 84 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | 0.53 | 0.45 | nd | 0.51 | 0.57 | nd | 0.56 |
| SE | 0.094 | nd | 0.11 | 0.098 | nd | 0.11 | 0.13 | nd | 0.13 |
| p | 0.69 | nd | 0.78 | 0.61 | nd | 0.94 | 0.59 | nd | 0.66 |
| nCohort 1 | 97 | nd | 84 | 97 | nd | 84 | 97 | nd | 84 |
| nCohort 2 | 11 | nd | 8 | 10 | nd | 8 | 6 | nd | 6 |
| Cutoff 1 | 37600 | nd | 37600 | 37600 | nd | 37600 | 37600 | nd | 37600 |
| Sens 1 | 73% | nd | 75% | 70% | nd | 75% | 83% | nd | 83% |
| Spec 1 | 27% | nd | 19% | 27% | nd | 19% | 27% | nd | 19% |
| Cutoff 2 | 14000 | nd | 10700 | 14000 | nd | 10700 | 37600 | nd | 37600 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 2 | 82% | nd | 88% | 80% | nd | 88% | 83% | nd | 83% |
| Spec 2 | 4% | nd | 1% | 4% | nd | 1% | 27% | nd | 19% |
| Cutoff 3 | 10700 | nd | 8680 | 10700 | nd | 8680 | 8680 | nd | 8680 |
| Sens 3 | 91% | nd | 100% | 90% | nd | 100% | 100% | nd | 100% |
| Spec 3 | 2% | nd | 1% | 2% | nd | 1% | 2% | nd | 1% |
| Cutoff 4 | 61600 | nd | 61700 | 61600 | nd | 61700 | 61600 | nd | 61700 |
| Sens 4 | 36% | nd | 50% | 40% | nd | 50% | 50% | nd | 50% |
| Spec 4 | 70% | nd | 70% | 70% | nd | 70% | 70% | nd | 70% |
| Cutoff 5 | 70000 | nd | 69100 | 70000 | nd | 69100 | 70000 | nd | 69100 |
| Sens 5 | 36% | nd | 50% | 30% | nd | 38% | 50% | nd | 50% |
| Spec 5 | 80% | nd | 81% | 80% | nd | 81% | 80% | nd | 81% |
| Cutoff 6 | 82900 | nd | 77900 | 82900 | nd | 77900 | 82900 | nd | 77900 |
| Sens 6 | 18% | nd | 25% | 20% | nd | 25% | 33% | nd | 33% |
| Spec 6 | 91% | nd | 90% | 91% | nd | 90% | 91% | nd | 90% |
| OR Quart 2 | 0 | nd | 0.30 | 0.31 | nd | 0.30 | 2.0 | nd | 0.45 |
| p Value | na | nd | 0.32 | 0.32 | nd | 0.32 | 0.58 | nd | 0.53 |
| 95% CI of OR Quart2 | na | nd | 0.029 | 0.030 | nd | 0.029 | 0.17 | nd | 0.038 |
|  | na | nd | 3.2 | 3.2 | nd | 3.2 | 24 | nd | 5.4 |
| OR Quart 3 | 1.0 | nd | 0 | 1.0 | nd | 0.30 | 0 | nd | 0 |
| p Value | 1.0 | nd | na | 1.0 | nd | 0.32 | na | nd | na |
| 95% CI of OR Quart3 | 0.22 | nd | na | 0.18 | nd | 0.029 | na | nd | na |
|  | 4.5 | nd | na | 5.5 | nd | 3.2 | na | nd | na |
| OR Quart 4 | 0.72 | nd | 1.4 | 1.0 | nd | 1.0 | 3.1 | nd | 1.5 |
| p Value | 0.69 | nd | 0.68 | 0.96 | nd | 1.0 | 0.34 | nd | 0.67 |
| 95% CI of OR Quart4 | 0.14 | nd | 0.28 | 0.19 | nd | 0.18 | 0.30 | nd | 0.23 |
|  | 3.6 | nd | 7.1 | 5.7 | nd | 5.6 | 32 | nd | 10.0 |

## Caspase-1

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 91.6 | 138 | 91.6 | 90.7 | 91.6 | 83.0 |
| Average | 108 | 154 | 108 | 121 | 108 | 101 |
| Stdev | 74.7 | 96.4 | 74.7 | 71.9 | 74.7 | 53.3 |
| p(t-test) |  | 0.17 |  | 0.69 |  | 0.83 |
| Min | 33.8 | 44.7 | 33.8 | 44.7 | 33.8 | 44.7 |
| Max | 326 | 328 | 326 | 241 | 326 | 192 |
| n (Samp) | 26 | 8 | 26 | 7 | 26 | 6 |
| n (Patient) | 26 | 8 | 26 | 7 | 26 | 6 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.67 | nd | nd | 0.56 | nd | nd | 0.51 | nd | nd |
| SE | 0.12 | nd | nd | 0.13 | nd | nd | 0.13 | nd | nd |
| p | 0.15 | nd | nd | 0.62 | nd | nd | 0.94 | nd | nd |
| nCohort 1 | 26 | nd | nd | 26 | nd | nd | 26 | nd | nd |
| nCohort 2 | 8 | nd | nd | 7 | nd | nd | 6 | nd | nd |
| Cutoff 1 | 71.0 | nd | nd | 70.7 | nd | nd | 66.1 | nd | nd |
| Sens 1 | 75% | nd | nd | 71% | nd | nd | 83% | nd | nd |
| Spec 1 | 38% | nd | nd | 38% | nd | nd | 31% | nd | nd |
| Cutoff 2 | 70.7 | nd | nd | 66.1 | nd | nd | 66.1 | nd | nd |
| Sens 2 | 88% | nd | nd | 86% | nd | nd | 83% | nd | nd |
| Spec 2 | 38% | nd | nd | 31% | nd | nd | 31% | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 | 42.1 | nd | nd | 42.1 | nd | nd | 42.1 | nd | nd |
| Sens 3 | 100% | nd | nd | 100% | nd | nd | 100% | nd | nd |
| Spec 3 | 15% | nd | nd | 15% | nd | nd | 15% | nd | nd |
| Cutoff 4 | 103 | nd | nd | 103 | nd | nd | 103 | nd | nd |
| Sens 4 | 62% | nd | nd | 43% | nd | nd | 33% | nd | nd |
| Spec 4 | 73% | nd | nd | 73% | nd | nd | 73% | nd | nd |
| Cutoff 5 | 121 | nd | nd | 121 | nd | nd | 121 | nd | nd |
| Sens 5 | 62% | nd | nd | 43% | nd | nd | 33% | nd | nd |
| Spec 5 | 81% | nd | nd | 81% | nd | nd | 81% | nd | nd |
| Cutoff 6 | 254 | nd | nd | 254 | nd | nd | 254 | nd | nd |
| Sens 6 | 12% | nd | nd | 0% | nd | nd | 0% | nd | nd |
| Spec 6 | 92% | nd | nd | 92% | nd | nd | 92% | nd | nd |
| OR Quart 2 | 2.0 | nd | nd | 2.3 | nd | nd | 2.3 | nd | nd |
| p Value | 0.60 | nd | nd | 0.53 | nd | nd | 0.53 | nd | nd |
| 95% CI of OR Quart2 | 0.15 27 | nd | nd | 0.17 33 | nd | nd | 0.17 33 | nd | nd |
| OR Quart 3 | 0 | nd | nd | 1.0 | nd | nd | 1.0 | nd | nd |
| p Value | na | nd | nd | 1.0 | nd | nd | 1.0 | nd | nd |
| 95% CI of OR Quart3 | na na | nd | nd | 0.052 19 | nd | nd | 0.052 19 | nd | nd |
| OR Quart 4 | 8.8 | nd | nd | 3.5 | nd | nd | 2.3 | nd | nd |
| p Value | 0.086 | nd | nd | 0.33 | nd | nd | 0.53 | nd | nd |
| 95% CI of OR Quart4 | 0.74 100 | nd | nd | 0.28 43 | nd | nd | 0.17 33 | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 28.7 | 12.2 | 28.7 | 6.83 | 28.7 | 7.20 |
| Average | 50.0 | 16.5 | 50.0 | 13.7 | 50.0 | 14.2 |
| Stdev | 69.0 | 14.3 | 69.0 | 15.0 | 69.0 | 16.2 |
| p(t-test) | | 0.16 | | 0.18 | | 0.22 |
| Min | 2.58 | 4.32 | 2.58 | 3.79 | 2.58 | 3.79 |
| Max | 366 | 46.4 | 366 | 46.4 | 366 | 46.4 |
| n (Samp) | 37 | 9 | 37 | 7 | 37 | 6 |
| n (Patient) | 37 | 9 | 37 | 7 | 37 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.29 | nd | nd | 0.22 | nd | nd | 0.23 | nd | nd |
| SE | 0.10 | nd | nd | 0.11 | nd | nd | 0.12 | nd | nd |
| p | 0.040 | nd | nd | 0.012 | nd | nd | 0.024 | nd | nd |
| nCohort 1 | 37 | nd | nd | 37 | nd | nd | 37 | nd | nd |
| nCohort 2 | 9 | nd | nd | 7 | nd | nd | 6 | nd | nd |
| Cutoff 1 | 6.05 | nd | nd | 6.05 | nd | nd | 4.34 | nd | nd |
| Sens 1 | 78% | nd | nd | 71% | nd | nd | 83% | nd | nd |
| Spec 1 | 11% | nd | nd | 11% | nd | nd | 8% | nd | nd |
| Cutoff 2 | 4.34 | nd | nd | 4.34 | nd | nd | 4.34 | nd | nd |
| Sens 2 | 89% | nd | nd | 86% | nd | nd | 83% | nd | nd |
| Spec 2 | 8% | nd | nd | 8% | nd | nd | 8% | nd | nd |
| Cutoff 3 | 3.30 | nd | nd | 3.30 | nd | nd | 3.30 | nd | nd |
| Sens 3 | 100% | nd | nd | 100% | nd | nd | 100% | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 5% | nd | nd | 5% | nd | nd | 5% | nd | nd |
| Cutoff 4 | 50.7 | nd | nd | 50.7 | nd | nd | 50.7 | nd | nd |
| Sens 4 | 0% | nd | nd | 0% | nd | nd | 0% | nd | nd |
| Spec 4 | 70% | nd | nd | 70% | nd | nd | 70% | nd | nd |
| Cutoff 5 | 71.4 | nd | nd | 71.4 | nd | nd | 71.4 | nd | nd |
| Sens 5 | 0% | nd | nd | 0% | nd | nd | 0% | nd | nd |
| Spec 5 | 81% | nd | nd | 81% | nd | nd | 81% | nd | nd |
| Cutoff 6 | 106 | nd | nd | 106 | nd | nd | 106 | nd | nd |
| Sens 6 | 0% | nd | nd | 0% | nd | nd | 0% | nd | nd |
| Spec 6 | 92% | nd | nd | 92% | nd | nd | 92% | nd | nd |
| OR Quart 2 | >4.5 | nd | nd | >1.1 | nd | nd | >1.1 | nd | nd |
| p Value | <0.23 | nd | nd | <0.95 | nd | nd | <0.95 | nd | nd |
| 95% CI of | >0.39 | nd | nd | >0.060 | nd | nd | >0.060 | nd | nd |
| OR Quart2 | na | nd | nd | na | nd | nd | na | nd | nd |
| OR Quart 3 | >2.4 | nd | nd | >2.4 | nd | nd | >1.1 | nd | nd |
| p Value | <0.50 | nd | nd | <0.49 | nd | nd | <0.95 | nd | nd |
| 95% CI of | >0.19 | nd | nd | >0.19 | nd | nd | >0.060 | nd | nd |
| OR Quart3 | na | nd | nd | na | nd | nd | na | nd | nd |
| OR Quart 4 | >6.9 | nd | nd | >6.3 | nd | nd | >7.3 | nd | nd |
| p Value | <0.11 | nd | nd | <0.13 | nd | nd | <0.10 | nd | nd |
| 95% CI of | >0.63 | nd | nd | >0.58 | nd | nd | >0.66 | nd | nd |
| OR Quart4 | na | nd | nd | na | nd | nd | na | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72700 | 112000 | 72700 | 112000 | 72700 | 162000 |
| Average | 103000 | 157000 | 103000 | 154000 | 103000 | 174000 |
| Stdev | 108000 | 103000 | 108000 | 105000 | 108000 | 96900 |
| p(t-test) | | 0.13 | | 0.15 | | 0.13 |
| Min | 14500 | 38300 | 14500 | 38300 | 14500 | 50500 |
| Max | 621000 | 340000 | 621000 | 340000 | 621000 | 285000 |
| n (Samp) | 50 | 12 | 50 | 12 | 50 | 6 |
| n (Patient) | 50 | 12 | 50 | 12 | 50 | 6 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 77600 | 151000 | 77600 | 132000 | nd | nd |
| Average | 110000 | 170000 | 110000 | 163000 | nd | nd |
| Stdev | 96000 | 127000 | 96000 | 132000 | nd | nd |
| p(t-test) | | 0.15 | | 0.20 | nd | nd |
| Min | 14500 | 38300 | 14500 | 38300 | nd | nd |
| Max | 621000 | 340000 | 621000 | 340000 | nd | nd |
| n (Samp) | 96 | 6 | 96 | 6 | nd | nd |
| n (Patient) | 96 | 6 | 96 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 80200 | 162000 | 80200 | 162000 | 80200 | 162000 |
| Average | 114000 | 170000 | 114000 | 170000 | 114000 | 174000 |
| Stdev | 117000 | 86300 | 117000 | 86300 | 117000 | 96900 |
| p(t-test) | | 0.21 | | 0.21 | | 0.24 |
| Min | 39700 | 64400 | 39700 | 64400 | 39700 | 50500 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 621000 | 285000 | 621000 | 285000 | 621000 | 285000 |
| n (Samp) | 44 | 8 | 44 | 8 | 44 | 6 |
| n (Patient) | 44 | 8 | 44 | 8 | 44 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | 0.62 | 0.77 | 0.66 | 0.56 | 0.77 | 0.77 | nd | 0.74 |
| SE | 0.092 | 0.13 | 0.10 | 0.093 | 0.13 | 0.10 | 0.12 | nd | 0.12 |
| p | 0.038 | 0.35 | 0.0087 | 0.094 | 0.65 | 0.0087 | 0.021 | nd | 0.050 |
| nCohort 1 | 50 | 96 | 44 | 50 | 96 | 44 | 50 | nd | 44 |
| nCohort 2 | 12 | 6 | 8 | 12 | 6 | 8 | 6 | nd | 6 |
| Cutoff 1 | 88900 | 53300 | 105000 | 62800 | 53300 | 105000 | 105000 | nd | 105000 |
| Sens 1 | 75% | 83% | 75% | 75% | 83% | 75% | 83% | nd | 83% |
| Spec 1 | 62% | 22% | 75% | 42% | 22% | 75% | 76% | nd | 75% |
| Cutoff 2 | 62800 | 53300 | 88900 | 54100 | 53300 | 88900 | 105000 | nd | 105000 |
| Sens 2 | 83% | 83% | 88% | 83% | 83% | 88% | 83% | nd | 83% |
| Spec 2 | 42% | 22% | 59% | 24% | 22% | 59% | 76% | nd | 75% |
| Cutoff 3 | 52600 | 38100 | 62800 | 52600 | 38100 | 62800 | 48100 | nd | 47600 |
| Sens 3 | 92% | 100% | 100% | 92% | 100% | 100% | 100% | nd | 100% |
| Spec 3 | 22% | 6% | 34% | 22% | 6% | 34% | 20% | nd | 11% |
| Cutoff 4 | 97000 | 108000 | 97500 | 97000 | 108000 | 97500 | 97000 | nd | 97500 |
| Sens 4 | 58% | 50% | 75% | 58% | 50% | 75% | 83% | nd | 83% |
| Spec 4 | 70% | 71% | 70% | 70% | 71% | 70% | 70% | nd | 70% |
| Cutoff 5 | 114000 | 151000 | 131000 | 114000 | 151000 | 131000 | 114000 | nd | 131000 |
| Sens 5 | 50% | 50% | 50% | 50% | 50% | 50% | 67% | nd | 50% |
| Spec 5 | 80% | 80% | 82% | 80% | 80% | 82% | 80% | nd | 82% |
| Cutoff 6 | 153000 | 242000 | 153000 | 153000 | 242000 | 153000 | 153000 | nd | 153000 |
| Sens 6 | 42% | 33% | 50% | 42% | 33% | 50% | 50% | nd | 50% |
| Spec 6 | 90% | 91% | 91% | 90% | 91% | 91% | 90% | nd | 91% |
| OR Quart 2 | 0.43 | 0 | >1.1 | 0.27 | 0 | >1.1 | 0 | nd | 0 |
| p Value | 0.51 | na | <0.96 | 0.28 | na | <0.96 | na | nd | na |
| 95% CI of OR Quart2 | 0.035 5.3 | na na | >0.061 na | 0.025 2.9 | na na | >0.061 na | na na | nd nd | na na |
| OR Quart 3 | 1.6 | 0.48 | >3.9 | 0.62 | 0 | >3.9 | 1.0 | nd | 1.0 |
| p Value | 0.63 | 0.56 | <0.27 | 0.63 | na | <0.27 | 1.0 | nd | 1.0 |
| 95% CI of OR Quart3 | 0.23 11 | 0.041 5.7 | >0.35 na | 0.087 4.3 | na na | >0.35 na | 0.056 18 | nd nd | 0.055 18 |
| OR Quart 4 | 3.9 | 1.5 | >5.8 | 2.4 | 0.96 | >5.8 | 5.2 | nd | 4.9 |
| p Value | 0.14 | 0.67 | <0.14 | 0.29 | 0.96 | <0.14 | 0.17 | nd | 0.19 |
| 95% CI of OR Quart4 | 0.64 24 | 0.23 9.8 | >0.55 na | 0.47 12 | 0.17 5.3 | >0.55 na | 0.50 54 | nd nd | 0.46 52 |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 212 | 243 | 212 | 255 | 212 | 299 |
| Average | 1210 | 333 | 1210 | 363 | 1210 | 402 |
| Stdev | 1930 | 337 | 1930 | 383 | 1930 | 405 |
| p(t-test) | | 0.19 | | 0.26 | | 0.32 |
| Min | 42.3 | 73.9 | 42.3 | 68.7 | 42.3 | 68.7 |
| Max | 6840 | 1190 | 6840 | 1190 | 6840 | 1190 |
| n (Samp) | 37 | 9 | 37 | 7 | 37 | 6 |
| n (Patient) | 37 | 9 | 37 | 7 | 37 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | nd | 0.45 | nd | nd | 0.47 | nd | nd |
| SE | 0.11 | nd | nd | 0.12 | nd | nd | 0.13 | nd | nd |
| p | 0.71 | nd | nd | 0.68 | nd | nd | 0.81 | nd | nd |
| nCohort 1 | 37 | nd | nd | 37 | nd | nd | 37 | nd | nd |
| nCohort 2 | 9 | nd | nd | 7 | nd | nd | 6 | nd | nd |
| Cutoff 1 | 138 | nd | nd | 138 | nd | nd | 138 | nd | nd |
| Sens 1 | 78% | nd | nd | 71% | nd | nd | 83% | nd | nd |
| Spec 1 | 38% | nd | nd | 38% | nd | nd | 38% | nd | nd |
| Cutoff 2 | 125 | nd | nd | 125 | nd | nd | 138 | nd | nd |
| Sens 2 | 89% | nd | nd | 86% | nd | nd | 83% | nd | nd |
| Spec 2 | 32% | nd | nd | 32% | nd | nd | 38% | nd | nd |
| Cutoff 3 | 73.1 | nd | nd | 61.7 | nd | nd | 61.7 | nd | nd |
| Sens 3 | 100% | nd | nd | 100% | nd | nd | 100% | nd | nd |
| Spec 3 | 14% | nd | nd | 11% | nd | nd | 11% | nd | nd |
| Cutoff 4 | 866 | nd | nd | 866 | nd | nd | 866 | nd | nd |
| Sens 4 | 11% | nd | nd | 14% | nd | nd | 17% | nd | nd |
| Spec 4 | 70% | nd | nd | 70% | nd | nd | 70% | nd | nd |
| Cutoff 5 | 1760 | nd | nd | 1760 | nd | nd | 1760 | nd | nd |
| Sens 5 | 0% | nd | nd | 0% | nd | nd | 0% | nd | nd |
| Spec 5 | 81% | nd | nd | 81% | nd | nd | 81% | nd | nd |
| Cutoff 6 | 5160 | nd | nd | 5160 | nd | nd | 5160 | nd | nd |
| Sens 6 | 0% | nd | nd | 0% | nd | nd | 0% | nd | nd |
| Spec 6 | 92% | nd | nd | 92% | nd | nd | 92% | nd | nd |
| OR Quart 2 | 6.3 | nd | nd | 3.8 | nd | nd | 3.8 | nd | nd |
| p Value | 0.13 | nd | nd | 0.29 | nd | nd | 0.29 | nd | nd |
| 95% CI of OR Quart2 | 0.58 68 | nd | nd | 0.32 43 | nd | nd | 0.32 43 | nd | nd |
| OR Quart 3 | 3.7 | nd | nd | 2.2 | nd | nd | 1.0 | nd | nd |
| p Value | 0.29 | nd | nd | 0.54 | nd | nd | 1.0 | nd | nd |
| 95% CI of OR Quart3 | 0.32 42 | nd | nd | 0.17 29 | nd | nd | 0.055 18 | nd | nd |
| OR Quart 4 | 1.1 | nd | nd | 1.0 | nd | nd | 1.1 | nd | nd |
| p Value | 0.95 | nd | nd | 1.0 | nd | nd | 0.94 | nd | nd |
| 95% CI of OR Quart4 | 0.060 20 | nd | nd | 0.055 18 | nd | nd | 0.060 20 | nd | nd |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.72 | 3.47 | 1.72 | 3.47 | 1.72 | 2.26 |
| Average | 2.52 | 4.00 | 2.52 | 3.11 | 2.52 | 2.81 |
| Stdev | 2.90 | 3.87 | 2.90 | 1.92 | 2.90 | 1.94 |
| p(t-test) | | 0.065 | | 0.42 | | 0.77 |
| Min | 0.183 | 0.726 | 0.183 | 0.563 | 0.183 | 0.726 |
| Max | 20.8 | 17.1 | 20.8 | 5.94 | 20.8 | 5.94 |
| n (Samp) | 110 | 17 | 110 | 17 | 110 | 9 |
| n (Patient) | 110 | 17 | 110 | 17 | 110 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.88 | 2.35 | 1.88 | 2.35 | nd | nd |
| Average | 3.12 | 2.81 | 3.12 | 2.75 | nd | nd |
| Stdev | 4.09 | 2.17 | 4.09 | 2.23 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.83 | | 0.80 | nd | nd |
| Min | 0.183 | 0.726 | 0.183 | 0.563 | nd | nd |
| Max | 33.6 | 5.44 | 33.6 | 5.44 | nd | nd |
| n (Samp) | 180 | 8 | 180 | 8 | nd | nd |
| n (Patient) | 180 | 8 | 180 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.71 | 5.01 | 1.71 | 3.72 | 1.71 | 3.47 |
| Average | 2.73 | 5.08 | 2.73 | 3.75 | 2.73 | 3.38 |
| Stdev | 3.23 | 4.31 | 3.23 | 1.66 | 3.23 | 1.82 |
| p(t-test) | | 0.030 | | 0.30 | | 0.60 |
| Min | 0.183 | 1.30 | 0.183 | 1.30 | 0.183 | 1.30 |
| Max | 20.8 | 17.1 | 20.8 | 5.94 | 20.8 | 5.94 |
| n (Samp) | 89 | 11 | 89 | 11 | 89 | 7 |
| n (Patient) | 89 | 11 | 89 | 11 | 89 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.50 | 0.78 | 0.63 | 0.48 | 0.75 | 0.60 | nd | 0.70 |
| SE | 0.076 | 0.10 | 0.086 | 0.077 | 0.11 | 0.088 | 0.10 | nd | 0.11 |
| p | 0.033 | 1.00 | 0.0012 | 0.086 | 0.83 | 0.0047 | 0.34 | nd | 0.085 |
| nCohort 1 | 110 | 180 | 89 | 110 | 180 | 89 | 110 | nd | 89 |
| nCohort 2 | 17 | 8 | 11 | 17 | 8 | 11 | 9 | nd | 7 |
| Cutoff 1 | 1.44 | 0.826 | 2.71 | 1.44 | 0.710 | 2.52 | 1.28 | nd | 2.23 |
| Sens 1 | 71% | 75% | 73% | 71% | 75% | 73% | 78% | nd | 71% |
| Spec 1 | 45% | 16% | 73% | 45% | 12% | 72% | 40% | nd | 64% |
| Cutoff 2 | 0.886 | 0.825 | 2.52 | 0.886 | 0.649 | 2.23 | 0.886 | nd | 1.49 |
| Sens 2 | 82% | 88% | 82% | 82% | 88% | 82% | 89% | nd | 86% |
| Spec 2 | 25% | 16% | 72% | 25% | 8% | 64% | 25% | nd | 46% |
| Cutoff 3 | 0.825 | 0.710 | 1.49 | 0.649 | 0.504 | 1.49 | 0.710 | nd | 1.28 |
| Sens 3 | 94% | 100% | 91% | 94% | 100% | 91% | 100% | nd | 100% |
| Spec 3 | 23% | 12% | 46% | 14% | 6% | 46% | 17% | nd | 40% |
| Cutoff 4 | 2.42 | 2.71 | 2.52 | 2.42 | 2.71 | 2.52 | 2.42 | nd | 2.52 |
| Sens 4 | 65% | 50% | 82% | 59% | 50% | 73% | 44% | nd | 57% |
| Spec 4 | 70% | 70% | 72% | 70% | 70% | 72% | 70% | nd | 72% |
| Cutoff 5 | 3.25 | 3.88 | 3.74 | 3.25 | 3.88 | 3.74 | 3.25 | nd | 3.74 |
| Sens 5 | 53% | 38% | 55% | 53% | 38% | 45% | 44% | nd | 29% |
| Spec 5 | 80% | 80% | 81% | 80% | 80% | 81% | 80% | nd | 81% |
| Cutoff 6 | 5.56 | 6.28 | 6.77 | 5.56 | 6.28 | 6.77 | 5.56 | nd | 6.77 |
| Sens 6 | 12% | 0% | 9% | 6% | 0% | 0% | 11% | nd | 0% |
| Spec 6 | 90% | 90% | 91% | 90% | 90% | 91% | 90% | nd | 91% |
| OR Quart 2 | 0.97 | 0 | >2.2 | 0.97 | 0 | >2.2 | 3.1 | nd | >2.2 |
| p Value | 0.97 | na | <0.54 | 0.97 | na | <0.54 | 0.34 | nd | <0.54 |
| 95% CI of OR Quart2 | 0.18 | na | >0.18 | 0.18 | na | >0.18 | 0.30 | nd | >0.18 |
| | 5.2 | na | na | 5.2 | na | na | 32 | nd | na |
| OR Quart 3 | 0.62 | 0 | >2.2 | 0.62 | 0 | >2.2 | 0.97 | nd | >1.0 |
| p Value | 0.62 | na | <0.54 | 0.62 | na | <0.54 | 0.98 | nd | <0.98 |
| 95% CI of OR Quart3 | 0.097 | na | >0.18 | 0.097 | na | >0.18 | 0.058 | nd | >0.062 |
| | 4.0 | na | na | 4.0 | na | na | 16 | nd | na |
| OR Quart 4 | 3.7 | 1.0 | >9.7 | 3.7 | 1.0 | >9.7 | 4.3 | nd | >4.8 |
| p Value | 0.073 | 1.0 | <0.041 | 0.073 | 1.0 | <0.041 | 0.20 | nd | <0.18 |
| 95% CI of OR Quart4 | 0.88 | 0.23 | >1.1 | 0.88 | 0.23 | >1.1 | 0.45 | nd | >0.50 |
| | 15 | 4.3 | na | 15 | 4.3 | na | 41 | nd | na |

## Myoglobin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 48.8 | 222 | 48.8 | 145 | 48.8 | 122 |
| Average | 109 | 574 | 109 | 498 | 109 | 407 |
| Stdev | 211 | 653 | 211 | 658 | 211 | 480 |
| p(t-test) | | 4.0E-8 | | 3.3E-6 | | 4.7E-4 |
| Min | 5.55 | 16.2 | 5.55 | 16.2 | 5.55 | 16.2 |
| Max | 1720 | 1880 | 1720 | 1880 | 1720 | 1180 |
| n (Samp) | 110 | 17 | 110 | 17 | 110 | 9 |
| n (Patient) | 110 | 17 | 110 | 17 | 110 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 56.7 | 475 | 56.7 | 475 | nd | nd |
| Average | 143 | 694 | 143 | 683 | nd | nd |
| Stdev | 283 | 718 | 283 | 729 | nd | nd |
| p(t-test) | | 2.1E-6 | | 3.5E-6 | nd | nd |
| Min | 5.55 | 16.2 | 5.55 | 16.2 | nd | nd |
| Max | 2130 | 1880 | 2130 | 1880 | nd | nd |
| n (Samp) | 180 | 8 | 180 | 8 | nd | nd |
| n (Patient) | 180 | 8 | 180 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 46.2 | 222 | 46.2 | 145 | 46.2 | 122 |
| Average | 120 | 547 | 120 | 438 | 120 | 352 |
| Stdev | 223 | 581 | 223 | 574 | 223 | 423 |
| p(t-test) | | 6.9E-6 | | 5.8E-4 | | 0.016 |
| Min | 5.55 | 86.7 | 5.55 | 69.7 | 5.55 | 69.7 |
| Max | 1720 | 1660 | 1720 | 1660 | 1720 | 1160 |
| n (Samp) | 89 | 11 | 89 | 11 | 89 | 7 |
| n (Patient) | 89 | 11 | 89 | 11 | 89 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | 0.75 | 0.86 | 0.76 | 0.68 | 0.82 | 0.76 | nd | 0.79 |
| SE | 0.064 | 0.10 | 0.072 | 0.071 | 0.11 | 0.080 | 0.095 | nd | 0.10 |
| p | 5.2E-7 | 0.016 | 3.9E-7 | 3.1E-4 | 0.096 | 8.3E-5 | 0.0061 | nd | 0.0048 |
| nCohort 1 | 110 | 180 | 89 | 110 | 180 | 89 | 110 | nd | 89 |
| nCohort 2 | 17 | 8 | 11 | 17 | 8 | 11 | 9 | nd | 7 |
| Cutoff 1 | 124 | 101 | 138 | 101 | 30.6 | 105 | 79.5 | nd | 87.6 |
| Sens 1 | 71% | 75% | 73% | 71% | 75% | 73% | 78% | nd | 71% |
| Spec 1 | 82% | 69% | 80% | 77% | 32% | 75% | 71% | nd | 69% |
| Cutoff 2 | 101 | 34.3 | 124 | 66.9 | 19.2 | 101 | 66.9 | nd | 79.5 |
| Sens 2 | 82% | 88% | 82% | 82% | 88% | 82% | 89% | nd | 86% |
| Spec 2 | 77% | 35% | 78% | 62% | 19% | 74% | 62% | nd | 67% |
| Cutoff 3 | 33.6 | 15.7 | 108 | 19.2 | 15.7 | 87.6 | 15.0 | nd | 66.9 |
| Sens 3 | 94% | 100% | 91% | 94% | 100% | 91% | 100% | nd | 100% |
| Spec 3 | 38% | 12% | 76% | 21% | 12% | 69% | 14% | nd | 61% |
| Cutoff 4 | 78.9 | 105 | 97.8 | 78.9 | 105 | 97.8 | 78.9 | nd | 97.8 |
| Sens 4 | 88% | 62% | 91% | 76% | 62% | 82% | 78% | nd | 57% |
| Spec 4 | 70% | 70% | 71% | 70% | 70% | 71% | 70% | nd | 71% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 5 | 112 | 150 | 146 | 112 | 150 | 146 | 112 | nd | 146 |
| Sens 5 | 71% | 62% | 64% | 59% | 62% | 45% | 56% | nd | 43% |
| Spec 5 | 80% | 80% | 81% | 80% | 80% | 81% | 80% | nd | 81% |
| Cutoff 6 | 212 | 315 | 323 | 212 | 315 | 323 | 212 | nd | 323 |
| Sens 6 | 53% | 50% | 36% | 41% | 50% | 27% | 44% | nd | 29% |
| Spec 6 | 90% | 90% | 91% | 90% | 90% | 91% | 90% | nd | 91% |
| OR Quart 2 | 0.97 | 1.0 | >0 | 0.47 | 0.49 | >0 | 0 | nd | >0 |
| p Value | 0.98 | 1.0 | <na | 0.54 | 0.56 | <na | na | nd | <na |
| 95% CI of | 0.058 | 0.061 | >na | 0.040 | 0.043 | >na | na | nd | >na |
| OR Quart2 | 16 | 16 | na | 5.4 | 5.6 | na | na | nd | na |
| OR Quart 3 | 3.1 | 1.0 | >4.8 | 2.1 | 0 | >6.2 | 3.1 | nd | >4.8 |
| p Value | 0.34 | 1.0 | <0.18 | 0.42 | na | <0.11 | 0.34 | nd | <0.18 |
| 95% CI of | 0.30 | 0.061 | >0.49 | 0.35 | na | >0.67 | 0.30 | nd | >0.50 |
| OR Quart3 | 32 | 16 | na | 12 | na | na | 32 | nd | na |
| OR Quart 4 | 18 | 5.5 | >9.7 | 6.6 | 2.7 | >7.9 | 5.6 | nd | >3.4 |
| p Value | 0.0075 | 0.13 | <0.041 | 0.022 | 0.25 | <0.066 | 0.13 | nd | <0.30 |
| 95% CI of | 2.2 | 0.61 | >1.1 | 1.3 | 0.49 | >0.88 | 0.61 | nd | >0.33 |
| OR Quart4 | 150 | 49 | na | 33 | 15 | na | 51 | nd | na |

## Mucin-16

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.17 | 8.40 | 1.17 | 7.52 | nd | nd |
| Average | 3.77 | 31.2 | 3.77 | 30.6 | nd | nd |
| Stdev | 15.1 | 67.4 | 15.1 | 67.6 | nd | nd |
| p(t-test) |  | 0.0035 |  | 0.0043 | nd | nd |
| Min | 0.117 | 2.02 | 0.117 | 2.02 | nd | nd |
| Max | 131 | 198 | 131 | 198 | nd | nd |
| n (Samp) | 75 | 8 | 75 | 8 | nd | nd |
| n (Patient) | 75 | 8 | 75 | 8 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.36 | 7.47 | 1.36 | 7.47 | nd | nd |
| Average | 4.38 | 38.3 | 4.38 | 38.3 | nd | nd |
| Stdev | 16.6 | 78.2 | 16.6 | 78.2 | nd | nd |
| p(t-test) |  | 0.0042 |  | 0.0042 | nd | nd |
| Min | 0.122 | 2.02 | 0.122 | 2.02 | nd | nd |
| Max | 131 | 198 | 131 | 198 | nd | nd |
| n (Samp) | 62 | 6 | 62 | 6 | nd | nd |
| n (Patient) | 62 | 6 | 62 | 6 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.89 | nd | 0.85 | 0.88 | nd | 0.85 | nd | nd | nd |
| SE | 0.079 | nd | 0.100 | 0.080 | nd | 0.100 | nd | nd | nd |
| p | 9.8E-7 | nd | 3.7E-4 | 1.6E-6 | nd | 3.7E-4 | nd | nd | nd |
| nCohort 1 | 75 | nd | 62 | 75 | nd | 62 | nd | nd | nd |
| nCohort 2 | 8 | nd | 6 | 8 | nd | 6 | nd | nd | nd |
| Cutoff 1 | 4.24 | nd | 2.09 | 4.24 | nd | 2.09 | nd | nd | nd |
| Sens 1 | 75% | nd | 83% | 75% | nd | 83% | nd | nd | nd |
| Spec 1 | 88% | nd | 68% | 88% | nd | 68% | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 2.09 | nd | 2.09 | 2.09 | nd | 2.09 | nd | nd | nd |
| Sens 2 | 88% | nd | 83% | 88% | nd | 83% | nd | nd | nd |
| Spec 2 | 69% | nd | 68% | 69% | nd | 68% | nd | nd | nd |
| Cutoff 3 | 1.96 | nd | 1.96 | 1.96 | nd | 1.96 | nd | nd | nd |
| Sens 3 | 100% | nd | 100% | 100% | nd | 100% | nd | nd | nd |
| Spec 3 | 67% | nd | 66% | 67% | nd | 66% | nd | nd | nd |
| Cutoff 4 | 2.20 | nd | 2.56 | 2.20 | nd | 2.56 | nd | nd | nd |
| Sens 4 | 75% | nd | 67% | 75% | nd | 67% | nd | nd | nd |
| Spec 4 | 72% | nd | 71% | 72% | nd | 71% | nd | nd | nd |
| Cutoff 5 | 2.98 | nd | 3.41 | 2.98 | nd | 3.41 | nd | nd | nd |
| Sens 5 | 75% | nd | 67% | 75% | nd | 67% | nd | nd | nd |
| Spec 5 | 80% | nd | 81% | 80% | nd | 81% | nd | nd | nd |
| Cutoff 6 | 5.21 | nd | 6.93 | 5.21 | nd | 6.93 | nd | nd | nd |
| Sens 6 | 62% | nd | 50% | 62% | nd | 50% | nd | nd | nd |
| Spec 6 | 91% | nd | 90% | 91% | nd | 90% | nd | nd | nd |
| OR Quart 2 | >0 | nd | >0 | >0 | nd | >0 | nd | nd | nd |
| p Value | <na | nd | <na | <na | nd | <na | nd | nd | nd |
| 95% CI of OR Quart2 | >na na | nd | >na na | >na na | nd | >na na | nd | nd | nd |
| OR Quart 3 | >2.1 | nd | >2.3 | >2.1 | nd | >2.3 | nd | nd | nd |
| p Value | <0.56 | nd | <0.52 | <0.56 | nd | <0.52 | nd | nd | nd |
| 95% CI of OR Quart3 | >0.18 na | nd | >0.19 na | >0.18 na | nd | >0.19 na | nd | nd | nd |
| OR Quart 4 | >8.0 | nd | >5.2 | >8.0 | nd | >5.2 | nd | nd | nd |
| p Value | <0.066 | nd | <0.16 | <0.066 | nd | <0.16 | nd | nd | nd |
| 95% CI of OR Quart4 | >0.87 na | nd | >0.52 na | >0.87 na | nd | >0.52 na | nd | nd | nd |

## Tumor necrosis factor receptor superfamily member 10B

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 0.0138 | 1.00E-9 | 0.0129 | nd | nd |
| Average | 0.00697 | 0.0265 | 0.00697 | 0.0259 | nd | nd |
| Stdev | 0.0202 | 0.0355 | 0.0202 | 0.0359 | nd | nd |
| p(t-test) | | 0.020 | | 0.024 | nd | nd |
| Min | 1.00E-9 | 1.00E-9 | 1.00E-9 | 1.00E-9 | nd | nd |
| Max | 0.114 | 0.108 | 0.114 | 0.108 | nd | nd |
| n (Samp) | 72 | 8 | 72 | 8 | nd | nd |
| n (Patient) | 72 | 8 | 72 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.81 | nd | nd | 0.79 | nd | nd | nd | nd | nd |
| SE | 0.095 | nd | nd | 0.098 | nd | nd | nd | nd | nd |
| p | 0.0012 | nd | nd | 0.0031 | nd | nd | nd | nd | nd |
| nCohort 1 | 72 | nd | nd | 72 | nd | nd | nd | nd | nd |
| nCohort 2 | 8 | nd | nd | 8 | nd | nd | nd | nd | nd |
| Cutoff 1 | 0.00462 | nd | nd | 0.00438 | nd | nd | nd | nd | nd |
| Sens 1 | 75% | nd | nd | 75% | nd | nd | nd | nd | nd |
| Spec 1 | 79% | nd | nd | 79% | nd | nd | nd | nd | nd |
| Cutoff 2 | 0.00438 | nd | nd | 0.00207 | nd | nd | nd | nd | nd |
| Sens 2 | 88% | nd | nd | 88% | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 79% | nd | nd | 65% | nd | nd | nd | nd | nd |
| Cutoff 3 | 0 | nd | nd | 0 | nd | nd | nd | nd | nd |
| Sens 3 | 100% | nd | nd | 100% | nd | nd | nd | nd | nd |
| Spec 3 | 0% | nd | nd | 0% | nd | nd | nd | nd | nd |
| Cutoff 4 | 0.00274 | nd | nd | 0.00274 | nd | nd | nd | nd | nd |
| Sens 4 | 88% | nd | nd | 75% | nd | nd | nd | nd | nd |
| Spec 4 | 71% | nd | nd | 71% | nd | nd | nd | nd | nd |
| Cutoff 5 | 0.00507 | nd | nd | 0.00507 | nd | nd | nd | nd | nd |
| Sens 5 | 62% | nd | nd | 50% | nd | nd | nd | nd | nd |
| Spec 5 | 81% | nd | nd | 81% | nd | nd | nd | nd | nd |
| Cutoff 6 | 0.0124 | nd | nd | 0.0124 | nd | nd | nd | nd | nd |
| Sens 6 | 50% | nd | nd | 50% | nd | nd | nd | nd | nd |
| Spec 6 | 90% | nd | nd | 90% | nd | nd | nd | nd | nd |
| OR Quart 2 | >1.1 | nd | nd | >1.1 | nd | nd | nd | nd | nd |
| p Value | <0.97 | nd | nd | <0.97 | nd | nd | nd | nd | nd |
| 95% CI of | >0.061 | nd | nd | >0.061 | nd | nd | nd | nd | nd |
| OR Quart2 | na | nd | nd | na | nd | nd | nd | nd | nd |
| OR Quart 3 | >2.2 | nd | nd | >2.2 | nd | nd | nd | nd | nd |
| p Value | <0.53 | nd | nd | <0.53 | nd | nd | nd | nd | nd |
| 95% CI of | >0.19 | nd | nd | >0.19 | nd | nd | nd | nd | nd |
| OR Quart3 | na | nd | nd | na | nd | nd | nd | nd | nd |
| OR Quart 4 | >6.7 | nd | nd | >6.7 | nd | nd | nd | nd | nd |
| p Value | <0.098 | nd | nd | <0.098 | nd | nd | nd | nd | nd |
| 95% CI of | >0.70 | nd | nd | >0.70 | nd | nd | nd | nd | nd |
| OR Quart4 | na | nd | nd | na | nd | nd | nd | nd | nd |

While the invention has been described and exemplified in sufficient detail for those skilled in this art to make and use it, various alternatives, modifications, and improvements should be apparent without departing from the spirit and scope of the invention. The examples provided herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth within the following claims.

The invention is further characterized by the following aspects:

1. A method for evaluating renal status in a subject, comprising:

performing one or more assaysconfigured to detect one or more biomarkers selected from the group consisting of Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin,

Apolipoprotein A-II, Mucin-16, Carcinoembryonic antigen-related cell adhesion molecule 5, and Cellular tumor antigen p53 on a body fluid sample obtained from the subject to provide an assay result; and correlating the assay result(s) to the renal status of the subject.

Aspect 2. A method according to aspect 1, wherein said correlation step comprises correlating the assay result(s) to one or more of risk stratification, diagnosis, staging, classifying and monitoring of the renal status of the subject.

Aspect 3. A method according to aspect 1, wherein said correlating step comprises assigning a likelihood of one or more future changes in renal status to the subject based on the assay result(s).

Aspect 4. A method according to aspect 1, wherein said one or more future changes in renal status comprise one or more of a future injury to renal function, future reduced renal function, future improvement in renal function, and future acute renal failure (ARF). Aspect 5. A method according to aspect 1, wherein the subject is not in acute renal failure.

Aspect 6. A method according to aspect 1, wherein the subject has not experienced a 1.5-fold or greater increase in serum creatinine over a baseline value determined prior to the time at which the body fluid sample is obtained.

Aspect 7. A method according to aspect 1, wherein the subject has a urine output of at least 0.5 ml/kg/hr over the 12 hours preceding the time at which the body fluid sample is obtained.

Aspect 8. A method according to aspect 1, wherein the subject has not experienced an increase of 0.3 mg/dL or greater in serum creatinine over a baseline value determined prior to the time at which the body fluid sample is obtained.

Aspect 9. A method according to aspect 1, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained.

Aspect 10. A method according to aspect 1, wherein the subject is in RIFLE stage 0 or R.

Aspect 11. A method according to aspect 10, wherein the subject is in RIFLE stage 0, and said correlating step comprises assigning a likelihood that the subject will reach RIFLE stage R, I or F within 72 hours.

Aspect 12. A method according to aspect 10, wherein the subject is in RIFLE stage 0 or R, and said correlating step comprises assigning a likelihood that the subject will reach RIFLE stage I or F within 72 hours.

Aspect 13. A method according to aspect 12, wherein the subject is in RIFLE stage 0, and said correlating step comprises assigning a likelihood that the subject will reach RIFLE stage F within 72 hours.

Aspect 14. A method according to aspect 12, wherein the subject is in RIFLE stage R, and said correlating step comprises assigning a likelihood that the subject will reach RIFLE stage I or F within 72 hours.

Aspect 15. A method according to aspect 1, wherein the subject is in RIFLE stage 0, R, or I, and said correlating step comprises assigning a likelihood that the subject will reach RIFLE stage F within 72 hours.

Aspect 16. A method according to aspect 15, wherein the subject is in RIFLE stage I, and said correlating step comprises assigning a likelihood that the subject will reach RIFLE stage F within 72 hours.

Aspect 17. A method according to aspect 11, wherein said correlating step comprises assigning likelihood that the subject will reach RIFLE stage R, I or F within 48 hours.

Aspect 18. A method according to aspect 12, wherein said correlating step comprises assigning a likelihood that the subject will reach RIFLE stage I or F within 48 hours.

Aspect 19. A method according to aspect 13, wherein said correlating step comprises assigning a likelihood that the subject will reach RIFLE stage I or F within 48 hours.

Aspect 20. A method according to aspect 17, wherein said correlating step comprises assigning a likelihood that the subject will reach RIFLE stage F within 48 hours.

Aspect 21. A method according to aspect 18, wherein said correlating step comprises assigning a likelihood that the subject will reach RIFLE stage F within 48 hours.

Aspect 22. A method according to aspect 19, wherein said correlating step comprises assigning a likelihood that the subject will reach RIFLE stage F within 48 hours.

Aspect 23. A method according to aspect 17, wherein said correlating step comprises assigning likelihood that the subject will reach RIFLE stage R, I or F within 24 hours.

Aspect 24. A method according to aspect 18, wherein said correlating step comprises assigning a likelihood that the subject will reach RIFLE stage I or F within 24 hours.

Aspect 25. A method according to aspect 19, wherein said correlating step comprises assigning a likelihood that the subject will reach RIFLE stage I or F within 24 hours.

Aspect 26. A method according to aspect 20, wherein said correlating step comprises assigning a likelihood that the subject will reach RIFLE stage F within 24 hours.

Aspect 27. A method according to aspect 21, wherein said correlating step comprises assigning a likelihood that the subject will reach RIFLE stage F within 24 hours.

Aspect 28. A method according to aspect 22, wherein said correlating step comprises assigning a likelihood that the subject will reach RIFLE stage F within 24 hours.

Aspect 29. A method according to aspect 1, wherein said assay result(s) comprise one or more of:

a measured urine or plasma concentration of Tumor necrosis factor receptor superfamily member 10B,

a measured urine or plasma concentration of Cadherin-16,

a measured urine or plasma concentration of Caspase-9,

a measured urine or plasma concentration of Bcl2 antagonist of cell death,

a measured urine or plasma concentration of Caspase-1,

a measured urine or plasma concentration of Cadherin-1,

a measured urine or plasma concentration of Poly [ADP-ribose] polymerase 1,

a measured urine or plasma concentration of Cyclin-dependent kinase inhibitor 1,

a measured urine or plasma concentration of Cadherin-5,

a measured urine or plasma concentration of Myoglobin,

a measured urine or plasma concentration of Apolipoprotein A-II,

a measured urine or plasma concentration of Mucin-16,

a measured urine or plasma concentration of Carcinoembryonic antigen-related cell adhesion molecule 5, or

a measured urine or plasma concentration of Cellular tumor antigen p53

and said correlation step comprises comparing each measured concentration to a corresponding threshold concentration, and

for a positive going marker, assigning an increased likelihood of progression to a worsening RIFLE stage to the subject, relative to the subject's current RIFLE stage, when the measured concentration is above the threshold, or assigning a decreased likelihood of progressing to a worsening RIFLE stage to the subject, relative to the subject's current RIFLE stage, when the measured concentration is below the threshold, or

for a negative going marker, assigning a decreased likelihood of progression to a worsening RIFLE stage to the subject, relative to the subject's current RIFLE stage, when the measured concentration is above the threshold, or assigning an increased likelihood of progressing to a worsening RIFLE stage to the subject, relative to the subject's current RIFLE stage, when the measured concentration is below the threshold.

Aspect 30. A method according to aspect 1, wherein said assay result(s) comprise one or more of:

a measured urine or plasma concentration of Tumor necrosis factor receptor superfamily member 10B,

a measured urine or plasma concentration of Cadherin-16,

a measured urine or plasma concentration of Caspase-9,

a measured urine or plasma concentration of Bcl2 antagonist of cell death,

a measured urine or plasma concentration of Caspase-1,

a measured urine or plasma concentration of Cadherin-1,

a measured urine or plasma concentration of Poly [ADP-ribose] polymerase 1,

a measured urine or plasma concentration of Cyclin-dependent kinase inhibitor 1,

a measured urine or plasma concentration of Cadherin-5,

a measured urine or plasma concentration of Myoglobin,

a measured urine or plasma concentration of Apolipoprotein A-II,

a measured urine or plasma concentration of Mucin-16,

a measured urine or plasma concentration of Carcinoembryonic antigen-related cell adhesion molecule 5, or

a measured urine or plasma concentration of Cellular tumor antigen p53

and said correlation step comprises comparing each measured concentration to a corresponding threshold concentration, and

for a positive going marker, assigning an increased likelihood of progressing to a need for renal replacement therapy to the subject when the measured concentration is above the threshold, or assigning a decreased likelihood of progressing to a need for renal replacement therapy when the measured concentration is below the threshold, or for a negative going marker, assigning a decreased likelihood of progressing to a need for renal replacement therapy to the subject when the measured concentration is above the threshold, or assigning an increased likelihood of progressing to a need for renal replacement therapy when the measured concentration is below the threshold.

Aspect 31. A method according to aspect 5, wherein said assay result(s) comprise one or more of:

a measured urine or plasma concentration of Tumor necrosis factor receptor superfamily member 10B,

a measured urine or plasma concentration of Cadherin-16,

a measured urine or plasma concentration of Caspase-9,

a measured urine or plasma concentration of Bcl2 antagonist of cell death,

a measured urine or plasma concentration of Caspase-1,

a measured urine or plasma concentration of Cadherin-1,

a measured urine or plasma concentration of Poly [ADP-ribose] polymerase 1,

a measured urine or plasma concentration of Cyclin-dependent kinase inhibitor 1,

a measured urine or plasma concentration of Cadherin-5,

a measured urine or plasma concentration of Myoglobin,

a measured urine or plasma concentration of Apolipoprotein A-II,

a measured urine or plasma concentration of Mucin-16,

a measured urine or plasma concentration of Carcinoembryonic antigen-related cell adhesion molecule 5, or

a measured urine or plasma concentration of Cellular tumor antigen p53

and said correlation step comprises comparing each measured concentration to a corresponding threshold concentration, and

for a positive going marker, assigning an increased likelihood of progressing to acute renal failure when the measured concentration is above the threshold, or assigning a decreased likelihood of progressing to acute renal failure to the subject when the measured concentration is below the threshold, or

for a negative going marker, assigning a decreased likelihood of progressing to acute renal failure when the measured concentration is above the threshold, or assigning an increased likelihood of progressing to acute renal failure to the subject when the measured concentration is below the threshold.

Aspect 32. A method according to aspect 11, wherein said assay result(s) comprise one or more of:

a measured urine or plasma concentration of Tumor necrosis factor receptor superfamily member 10B,

a measured urine or plasma concentration of Cadherin-16,

a measured urine or plasma concentration of Caspase-9,

a measured urine or plasma concentration of Bcl2 antagonist of cell death,

a measured urine or plasma concentration of Caspase-1,

a measured urine or plasma concentration of Cadherin-1,

a measured urine or plasma concentration of Poly [ADP-ribose] polymerase 1,

a measured urine or plasma concentration of Cyclin-dependent kinase inhibitor 1,

a measured urine or plasma concentration of Cadherin-5,

a measured urine or plasma concentration of Myoglobin,

a measured urine or plasma concentration of Apolipoprotein A-II,

a measured urine or plasma concentration of Mucin-16,

a measured urine or plasma concentration of Carcinoembryonic antigen-related cell adhesion molecule 5, or

a measured urine or plasma concentration of Cellular tumor antigen p53

and said correlation step comprises comparing each measured concentration to a corresponding threshold concentration, and

for a positive going marker, assigning an increased likelihood of progressing to RIFLE stage R, I or F to the subject, when the measured concentration is above the threshold, or assigning a decreased likelihood of progressing to RIFLE stage R, I or F to the subject when the measured concentration is below the threshold, or

for a negative going marker, assigning a decreased likelihood of progressing to RIFLE stage R, I or F to the subject, when the measured concentration is above the threshold, or assigning an increased likelihood of progressing to RIFLE stage R, I or F to the subject when the measured concentration is below the threshold.

Aspect 33. A method according to aspect 12, wherein said assay result(s) comprise one or more of:

a measured urine or plasma concentration of Tumor necrosis factor receptor superfamily member 10B,

a measured urine or plasma concentration of Cadherin-16,

a measured urine or plasma concentration of Caspase-9,

a measured urine or plasma concentration of Bcl2 antagonist of cell death,

a measured urine or plasma concentration of Caspase-1,

a measured urine or plasma concentration of Cadherin-1,

a measured urine or plasma concentration of Poly [ADP-ribose] polymerase 1,

a measured urine or plasma concentration of Cyclin-dependent kinase inhibitor 1,

a measured urine or plasma concentration of Cadherin-5,

a measured urine or plasma concentration of Myoglobin,

a measured urine or plasma concentration of Apolipoprotein A-II,

a measured urine or plasma concentration of Mucin-16,

a measured urine or plasma concentration of Carcinoembryonic antigen-related cell adhesion molecule 5, or

a measured urine or plasma concentration of Cellular tumor antigen p53

and said correlation step comprises comparing each measured concentration to a corresponding threshold concentration, and

for a positive going marker, assigning an increased likelihood of progressing to RIFLE stage I or F to the subject, when the measured concentration is above the threshold, or assigning a decreased likelihood of progressing to RIFLE stage I or F to the subject when the measured concentration is below the threshold, or

for a negative going marker, assigning a decreased likelihood of progressing to RIFLE stage I or F to the subject, when the measured concentration is above the threshold, or assigning an increased likelihood of progressing to RIFLE stage I or F to the subject when the measured concentration is below the threshold.

34. A method according to aspect 13, wherein said assay result(s) comprise one or more of:

a measured urine or plasma concentration of Tumor necrosis factor receptor superfamily member 10B,

a measured urine or plasma concentration of Cadherin-16,

a measured urine or plasma concentration of Caspase-9,

a measured urine or plasma concentration of Bcl2 antagonist of cell death,

a measured urine or plasma concentration of Caspase-1,

a measured urine or plasma concentration of Cadherin-1,

a measured urine or plasma concentration of Poly [ADP-ribose] polymerase 1,

a measured urine or plasma concentration of Cyclin-dependent kinase inhibitor 1,

a measured urine or plasma concentration of Cadherin-5,

a measured urine or plasma concentration of Myoglobin,

a measured urine or plasma concentration of Apolipoprotein A-II,

a measured urine or plasma concentration of Mucin-16,

a measured urine or plasma concentration of Carcinoembryonic antigen-related cell adhesion molecule 5, or

a measured urine or plasma concentration of Cellular tumor antigen p53

and said correlation step comprises comparing each measured concentration to a corresponding threshold concentration, and

for a positive going marker, assigning an increased likelihood of progressing to RIFLE stage F to the subject, when the measured concentration is above the threshold, or assigning a decreased likelihood of progressing to RIFLE stage I or F to the subject when the measured concentration is below the threshold, or

for a negative going marker, assigning a decreased likelihood of progressing to RIFLE stage F to the subject, when the measured concentration is above the threshold, or assigning an increased likelihood of progressing to RIFLE stage I or F to the subject when the measured concentration is below the threshold.

Aspect 35. A method according to aspect 14, wherein said assay result(s) comprise one or more of:

a measured urine or plasma concentration of Tumor necrosis factor receptor superfamily member 10B,

a measured urine or plasma concentration of Cadherin-16,

a measured urine or plasma concentration of Caspase-9,

a measured urine or plasma concentration of Bcl2 antagonist of cell death,

a measured urine or plasma concentration of Caspase-1,

a measured urine or plasma concentration of Cadherin-1,

a measured urine or plasma concentration of Poly [ADP-ribose] polymerase 1,

a measured urine or plasma concentration of Cyclin-dependent kinase inhibitor 1,

a measured urine or plasma concentration of Cadherin-5,

a measured urine or plasma concentration of Myoglobin,

a measured urine or plasma concentration of Apolipoprotein A-II,

a measured urine or plasma concentration of Mucin-16,

a measured urine or plasma concentration of Carcinoembryonic antigen-related cell adhesion molecule 5, or

a measured urine or plasma concentration of Cellular tumor antigen p53

and said correlation step comprises comparing each measured concentration to a corresponding threshold concentration, and

for a positive going marker, assigning an increased likelihood of progressing to RIFLE stage F to the subject, when the measured concentration is above the threshold, or assigning a decreased likelihood of progressing to RIFLE stage I or F to the subject when the measured concentration is below the threshold, or

for a negative going marker, assigning a decreased likelihood of progressing to RIFLE stage F to the subject, when the measured concentration is above the threshold, or assigning an increased likelihood of progressing to RIFLE stage I or F to the subject when the measured concentration is below the threshold.

Aspect 36. A method according to aspect 15, wherein said assay result(s) comprise one or more of:

a measured urine or plasma concentration of Tumor necrosis factor receptor superfamily member 10B,

a measured urine or plasma concentration of Cadherin-16,

a measured urine or plasma concentration of Caspase-9,

a measured urine or plasma concentration of Bcl2 antagonist of cell death,

a measured urine or plasma concentration of Caspase-1,

a measured urine or plasma concentration of Cadherin-1,

a measured urine or plasma concentration of Poly [ADP-ribose] polymerase 1,

a measured urine or plasma concentration of Cyclin-dependent kinase inhibitor 1,

a measured urine or plasma concentration of Cadherin-5,

a measured urine or plasma concentration of Myoglobin,

a measured urine or plasma concentration of Apolipoprotein A-II,

a measured urine or plasma concentration of Mucin-16,

a measured urine or plasma concentration of Carcinoembryonic antigen-related cell adhesion molecule 5, or

a measured urine or plasma concentration of Cellular tumor antigen p53

and said correlation step comprises comparing each measured concentration to a corresponding threshold concentration, and

for a positive going marker, assigning an increased likelihood of progressing to RIFLE stage F to the subject, when the measured concentration is above the threshold, or assigning a decreased likelihood of progressing to RIFLE stage F to the subject when the measured concentration is below the threshold, or

for a negative going marker, assigning a decreased likelihood of progressing to RIFLE stage F to the subject, when the measured concentration is above the threshold, or assigning an increased likelihood of progressing to RIFLE stage F to the subject when the measured concentration is below the threshold.

Aspect 37. A method according to aspect 16, wherein said assay result(s) comprise one or more of:

a measured urine or plasma concentration of Tumor necrosis factor receptor superfamily member 10B,

a measured urine or plasma concentration of Cadherin-16,

a measured urine or plasma concentration of Caspase-9,

a measured urine or plasma concentration of Bcl2 antagonist of cell death,

a measured urine or plasma concentration of Caspase-1,

a measured urine or plasma concentration of Cadherin-1,

a measured urine or plasma concentration of Poly [ADP-ribose] polymerase 1,

a measured urine or plasma concentration of Cyclin-dependent kinase inhibitor 1,

a measured urine or plasma concentration of Cadherin-5,

a measured urine or plasma concentration of Myoglobin,

a measured urine or plasma concentration of Apolipoprotein A-II,

a measured urine or plasma concentration of Mucin-16,

a measured urine or plasma concentration of Carcinoembryonic antigen-related cell adhesion molecule 5, or

a measured urine or plasma concentration of Cellular tumor antigen p53

and said correlation step comprises comparing each measured concentration to a corresponding threshold concentration, and

for a positive going marker, assigning an increased likelihood of progressing to RIFLE stage F to the subject, when the measured concentration is above the threshold, or assigning a decreased likelihood of progressing to RIFLE stage F to the subject when the measured concentration is below the threshold, or

for a negative going marker, assigning a decreased likelihood of progressing to RIFLE stage F to the subject, when the measured concentration is above the threshold, or assigning an increased likelihood of progressing to RIFLE stage F to the subject when the measured concentration is below the threshold.

Aspect 38. A method according to aspect 1, wherein the subject is selected for evaluation of renal status based on the pre-existence in the subject of one or more known risk factors for prerenal, intrinsic renal, or postrenal ARF.

Aspect 39. A method according to aspect 1, wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

Aspect 40. Measurement of IGFBP7 for the diagnosis, risk stratification, prognosis, classifying and monitoring of renal status of a subject not receiving renal replacement therapy.

Aspect 41. Measurement of one or more biomarkers selected from the group consisting of Tumor necrosis factor receptor superfamily member 10B,Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 for the diagnosis, risk stratification, prognosis, classifying and monitoring of renal status of a subject not in acute renal failure.

Aspect 42. Measurement of one or more biomarkers selected from the group consisting of Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 for assigning an increased likelihood of progressing to a worsening RIFLE stage to a subject, relative to the subject's current RIFLE stage.

**Claims**

1. A method for evaluating renal status in a subject, comprising:

    performing one or more assays configured to detect one or more biomarkers, the one or more biomarkers comprising Carcinoembryonic antigen-related cell adhesion molecule 5, and optionally further comprising Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, and/or Cellular tumor antigen p53 on a body fluid sample obtained from the subject to provide an assay result; and correlating the assay result(s) to the renal status of the subject.

2. A method according to claim 1, wherein said correlation step comprises

    (i) correlating the assay result(s) to one or more of risk stratification, diagnosis, staging, classifying and monitoring of the renal status of the subject or
    (ii) assigning a likelihood of one or more future changes in renal status to the subject based on the assay result(s).

3. A method according to claim 1, wherein said one or more future changes in renal status comprise one or more of a future injury to renal function, future reduced renal function, future improvement in renal function, and future acute renal failure (ARF).

4. A method according to claim 1, wherein the subject is not in acute renal failure.

5. A method according to claim 1, wherein the subject has not experienced a 1.5-fold or greater increase in serum creatinine over a baseline value determined prior to the time at which the body fluid sample is obtained, or wherein the subject has a urine output of at least 0.5 ml/kg/hr over the 12 hours preceding the time at which the body fluid sample is obtained, or

313

wherein the subject has not experienced an increase of 0.3 mg/dL or greater in serum creatinine over a baseline value determined prior to the time at which the body fluid sample is obtained, or

wherein the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained.

**6.** A method according to claim 1, wherein the subject is in RIFLE stage 0 or R, wherein the subject is optionally in RIFLE stage 0, and said correlating step comprises assigning a likelihood that the subject will reach RIFLE stage R, I or F within 72 hours, within 48 hours or within 24 hours, or

wherein the subject is optionally in RIFLE stage 0 or R, and said correlating step comprises assigning a likelihood that the subject will reach RIFLE stage I or F within 72 hours, within 48 hours or within 24 hours,

wherein the subject is optionally in RIFLE stage 0, and said correlating step comprises assigning a likelihood that the subject will reach RIFLE stage F within 72 hours, within 48 hours or within 24 hours, or

wherein the subject is optionally in RIFLE stage R, and said correlating step comprises assigning a likelihood that the subject will reach RIFLE stage I or F within 72 hours, within 48 hours or within 24 hours.

**7.** A method according to claim 1, wherein the subject is in RIFLE stage 0, R, or I, and said correlating step comprises assigning a likelihood that the subject will reach RIFLE stage F within 72 hours,

wherein the subject is optionally in RIFLE stage I, and said correlating step comprises assigning a likelihood that the subject will reach RIFLE stage F within 72 hours, within 48 hours or within 24 hours.

**8.** A method according to claim 1, wherein said assay result(s) comprise a measured urine or plasma concentration of Carcinoembryonic antigen-related cell adhesion molecule 5, and optionally further one or more of:

a measured urine or plasma concentration of Tumor necrosis factor receptor superfamily member 10B,
a measured urine or plasma concentration of Cadherin-16,
a measured urine or plasma concentration of Caspase-9,
a measured urine or plasma concentration of Bcl2 antagonist of cell death,
a measured urine or plasma concentration of Caspase-1,
a measured urine or plasma concentration of Cadherin-1,
a measured urine or plasma concentration of Poly [ADP-ribose] polymerase 1,
a measured urine or plasma concentration of Cyclin-dependent kinase inhibitor 1,
a measured urine or plasma concentration of Cadherin-5,
a measured urine or plasma concentration of Myoglobin,
a measured urine or plasma concentration of Apolipoprotein A-II,
a measured urine or plasma concentration of Mucin-16, or
a measured urine or plasma concentration of Cellular tumor antigen p53 and said correlation step comprises comparing each measured concentration to a corresponding threshold concentration, and

(A) for a positive going marker, assigning an increased likelihood of progression to a worsening RIFLE stage to the subject, relative to the subject's current RIFLE stage, when the measured concentration is above the threshold, or assigning a decreased likelihood of progressing to a worsening RIFLE stage to the subject, relative to the subject's current RIFLE stage, when the measured concentration is below the threshold, or

for a negative going marker, assigning a decreased likelihood of progression to a worsening RIFLE stage to the subject, relative to the subject's current RIFLE stage, when the measured concentration is above the threshold, or assigning an increased likelihood of progressing to a worsening RIFLE stage to the subject, relative to the subject's current RIFLE stage, when the measured concentration is below the threshold,

(B) for a positive going marker, assigning an increased likelihood of progressing to a need for renal replacement therapy to the subject when the measured concentration is above the threshold, or assigning a decreased likelihood of progressing to a need for renal replacement therapy when the measured concentration is below the threshold, or

for a negative going marker, assigning a decreased likelihood of progressing to a need for renal replacement therapy to the subject when the measured concentration is above the threshold, or assigning an increased likelihood of progressing to a need for renal replacement therapy when the measured concentration is below the threshold,

(C) where the subject is not in acute renal failure,

for a positive going marker, assigning an increased likelihood of progressing to acute renal failure when the measured concentration is above the threshold, or assigning a decreased likelihood of progressing to acute renal failure to the subject when the measured concentration is below the threshold, or

for a negative going marker, assigning a decreased likelihood of progressing to acute renal failure when the measured concentration is above the threshold, or assigning an increased likelihood of progressing to acute renal failure to the subject when the measured concentration is below the threshold,

(D) where the subject is wherein the subject is in RIFLE stage 0, and the correlating step comprises assigning a likelihood that the subject will reach RIFLE stage R, I or F within 72 hours,

for a positive going marker, assigning an increased likelihood of progressing to RIFLE stage R, I or F to the subject, when the measured concentration is above the threshold, or assigning a decreased likelihood of progressing to RIFLE stage R, I or F to the subject when the measured concentration is below the threshold, or

for a negative going marker, assigning a decreased likelihood of progressing to RIFLE stage R, I or F to the subject, when the measured concentration is above the threshold, or assigning an increased likelihood of progressing to RIFLE stage R, I or F to the subject when the measured concentration is below the threshold,

(E) where the subject is in RIFLE stage 0 or R, and the correlating step comprises assigning a likelihood that the subject will reach RIFLE stage I or F within 72 hours,

for a positive going marker, assigning an increased likelihood of progressing to RIFLE stage I or F to the subject, when the measured concentration is above the threshold, or assigning a decreased likelihood of progressing to RIFLE stage I or F to the subject when the measured concentration is below the threshold, or

for a negative going marker, assigning a decreased likelihood of progressing to RIFLE stage I or F to the subject, when the measured concentration is above the threshold, or assigning an increased likelihood of progressing to RIFLE stage I or F to the subject when the measured concentration is below the threshold,

(F) where the subject is in RIFLE stage 0, and the correlating step comprises assigning a likelihood that the subject will reach RIFLE stage F within 72 hours,

for a positive going marker, assigning an increased likelihood of progressing to RIFLE stage F to the subject, when the measured concentration is above the threshold, or

assigning a decreased likelihood of progressing to RIFLE stage I or F to the subject when the measured concentration is below the threshold, or

for a negative going marker, assigning a decreased likelihood of progressing to RIFLE stage F to the subject, when the measured concentration is above the threshold, or

assigning an increased likelihood of progressing to RIFLE stage I or F to the subject when the measured concentration is below the threshold,

(G) where the subject is in RIFLE stage R, and the correlating step comprises assigning a likelihood that the subject will reach RIFLE stage I or F within 72 hours,

for a positive going marker, assigning an increased likelihood of progressing to RIFLE stage F to the subject, when the measured concentration is above the threshold, or assigning a decreased likelihood of progressing to RIFLE stage I or F to the subject when the measured concentration is below the threshold, or

for a negative going marker, assigning a decreased likelihood of progressing to RIFLE stage F to the subject, when the measured concentration is above the threshold, or

assigning an increased likelihood of progressing to RIFLE stage I or F to the subject when the measured concentration is below the threshold,

(H) where the subject is in RIFLE stage 0, R, or I, and the correlating step comprises assigning a likelihood that the subject will reach RIFLE stage F within 72 hours,

for a positive going marker, assigning an increased likelihood of progressing to RIFLE stage F to the subject, when the measured concentration is above the threshold, or

assigning a decreased likelihood of progressing to RIFLE stage F to the subject when the measured concentration is below the threshold, or

for a negative going marker, assigning a decreased likelihood of progressing to RIFLE stage F to the subject, when the measured concentration is above the threshold, or assigning an increased likelihood of progressing to RIFLE stage F to the subject when the measured concentration is below the threshold, or

(I) where the subject is in RIFLE stage I, and the correlating step comprises assigning a likelihood that the subject will reach RIFLE stage F within 72 hours,

for a positive going marker, assigning an increased likelihood of progressing to RIFLE stage F to the subject, when the measured concentration is above the threshold, or

assigning a decreased likelihood of progressing to RIFLE stage F to the subject when the measured concentration is below the threshold, or

for a negative going marker, assigning a decreased likelihood of progressing to RIFLE stage F to the subject, when the measured concentration is above the threshold, or

assigning an increased likelihood of progressing to RIFLE stage F to the subject when the measured concentration is below the threshold.

9. A method according to claim 1, wherein the subject is selected for evaluation of renal status (i) based on the pre-existence in the subject of one or more known risk factors for prerenal, intrinsic renal, or postrenal ARF, or based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

10. The use of Carcinoembryonic antigen-related cell adhesion molecule 5 for the diagnosis, risk stratification, prognosis, classifying and monitoring of renal status of a subject not receiving renal replacement therapy.

11. The use of one or more biomarkers comprising Carcinoembryonic antigen-related cell adhesion molecule 5, and optionally further comprising Tumor necrosis factor receptor superfamily member 10B, Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, and/or Cellular tumor antigen p53 for

(i) the diagnosis, risk stratification, prognosis, classifying and monitoring of renal status of a subject not in acute renal failure or

(ii) for assigning an increased likelihood of progressing to a worsening RIFLE stage to a subject, relative to the subject's current RIFLE stage.

Europäisches
Patentamt

European
Patent Office

Office européen
des brevets

# EUROPEAN SEARCH REPORT

Application Number

EP 16 18 9562

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2007/248989 A1 (DEVARAJAN PRASAD [US]) 25 October 2007 (2007-10-25) * the whole document * * abstract * * claim 1 * | 1-11 | INV. G01N33/53 G01N33/68 |
| A | S G COCA ET AL: "Biomarkers for the diagnosis and risk stratification of acute kidney injury: A systematic review", KIDNEY INTERNATIONAL, vol. 73, no. 9, 19 December 2007 (2007-12-19), pages 1008-1016, XP055027810, ISSN: 0085-2538, DOI: 10.1038/sj.ki.5002729 * the whole document * * abstract * | 1-11 | |
| A | US 2009/215046 A1 (SOREK ROTEM [IL] ET AL) 27 August 2009 (2009-08-27) * the whole document * * abstract * * sequence 863 * | 1-11 | |

TECHNICAL FIELDS
SEARCHED (IPC)

G01N

The present search report has been drawn up for all claims

| Place of search | Date of completion of the search | Examiner |
|---|---|---|
| The Hague | 17 January 2017 | Gall-Truchot, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 3 151 005 A1

## ANNEX TO THE EUROPEAN SEARCH REPORT
## ON EUROPEAN PATENT APPLICATION NO.

EP 16 18 9562

17-01-2017

| Patent document cited in search report | Publication date | Patent family member(s) | Publication date |
|---|---|---|---|
| US 2007248989 A1 | 25-10-2007 | US 2007248989 A1<br>WO 2007124419 A1 | 25-10-2007<br>01-11-2007 |
| US 2009215046 A1 | 27-08-2009 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61238115 A
- US 61238118 B
- US 61238120 B
- US 61238121 B
- US 61238123 B
- US 61238125 B
- US 61238127 B
- US 61238129 B
- US 61238134 B
- US 61243991 B
- US 61243997 B
- US 61244002 B
- US 61243993 B
- US 61238128 B
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

**Non-patent literature cited in the description**

- Harrison's Principles of Internal Medicine. McGraw Hill, 1741-1830
- Current Medical Diagnosis & Treatment. McGraw Hill, 2008, 785-815
- Merck Manual
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), 204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 ((4)), 177-197
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant.,* 2008, vol. 23, 1203-1210
- Merck Manual of Diagnosis and Therapy. Merck Research Laboratories, 1999
- **WIJEYSUNDERA et al.** *JAMA,* 2007, vol. 297, 1801-9
- **HANLEY, J. A. ; MCNEIL, B.J.** The meaning and use of the area under a receiver operating characteristic (ROC) curve. *Radiology,* 1982, vol. 143, 29-36